(19) Europäisches Patentamt
European Patent Office
Office européen des brevets

(11) EP 4 279 991 A1

(12) EUROPEAN PATENT APPLICATION

(43) Date of publication:
**22.11.2023 Bulletin 2023/47**

(21) Application number: **23171817.2**

(22) Date of filing: **05.05.2023**

(51) International Patent Classification (IPC):
***G03F 7/004** (2006.01)* ***G03F 7/039** (2006.01)*

(52) Cooperative Patent Classification (CPC):
**G03F 7/0045; G03F 7/0392; G03F 7/0397**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.05.2022 JP 2022081146**

(71) Applicant: **SHIN-ETSU CHEMICAL CO., LTD.**
**Tokyo 1000005 (JP)**

(72) Inventors:
- **Fukushima, Masahiro**
  **Niigata (JP)**
- **Watanabe, Satoshi**
  **Niigata (JP)**
- **Hatakeyama, Jun**
  **Niigata (JP)**
- **Masunaga, Keiichi**
  **Niigata (JP)**
- **Kotake, Masaaki**
  **Niigata (JP)**
- **Matsuzawa, Yuta**
  **Niigata (JP)**

(74) Representative: **Sonnenhauser, Thomas Martin**
**Wuesthoff & Wuesthoff**
**Patentanwälte PartG mbB**
**Schweigerstraße 2**
**81541 München (DE)**

(54) **NOVEL SULFONIUM SALT, RESIST COMPOSITION, AND PATTERNING PROCESS**

(57) The present invention is a sulfonium salt represented by the following formula (1),

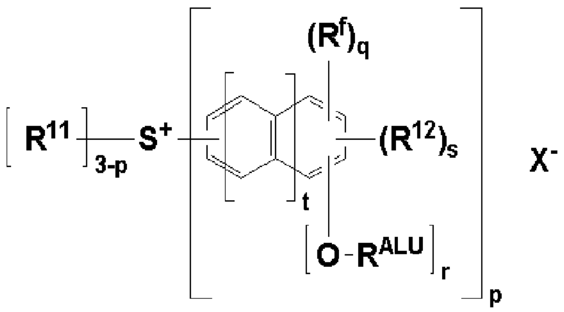

(1)

wherein "p" represents an integer of 1 to 3; $R^{11}$ represents a hydrocarbyl group having 1 to 20 carbon atoms; $R^f$ represents a fluorine atom or a fluorine-atom-containing C1 to C6 group selected from alkyl, alkoxy, and sulfide; "q" represents an integer of 1 to 4; $R^{ALU}$ represents an acid-labile group; "r" represents an integer of 1 to 4; $R^{12}$ represents a hydrocarbyl group having 1 to 20 carbon atoms; "s" represents an integer of 0 to 4; "t" represents an integer of 0 to 2; $R^f$ and - O-$R^{ALU}$ are bonded to adjacent carbon atoms; and X-represents a non-nucleophilic counterion having no polymerizable group. This provides a sulfonium salt used for a resist composition having excellent solvent solubility, high sensitivity and high contrast, and excellent lithographic performance such as exposure latitude and LWR; a resist composition containing the sulfonium salt as a photoacid generator; and a patterning process using the resist composition.

EP 4 279 991 A1

## Description

TECHNICAL FILED

The present invention relates to a novel sulfonium salt compound, a resist composition containing the sulfonium salt compound, and a patterning process.

BACKGROUND ART

As higher integration and higher speed of LSI have been achieved in recent years, the pattern rule has been required to be miniaturized, and far ultraviolet ray lithography and extreme ultraviolet ray (EUV) lithography are promising as the next-generation fine processing technology. Among these, photolithography using ArF excimer laser light is essential technology for ultrafine processing of 0.13 μm or smaller.

The ArF lithography began to be partially used from production of 130-nm node devices, and has become main lithography technology from 90-nm node devices. Although 157-nm lithography using $F_2$ laser was initially promising as the next 45-nm node lithography technology, development delay due to problems was pointed out, and thereby ArF immersion lithography has rapidly emerged (Non Patent Document 1) to be in a practical use stage. The ArF immersion lithography can design a numerical aperture (NA) of a projection lens to be 1.0 or more and can achieve high resolution by interposing a liquid having higher refractive index than air, such as water, ethylene glycol, and glycerin, between the projection lens and a wafer. This immersion lithography requires a resist composition hardly eluted into water.

In the ArF lithography, a highly sensitive resist composition that can exhibit sufficient resolution with a small exposure dose is required to prevent deterioration of a precise and expensive optical material. The most common method for achieving this requirement is selecting a component having high transparency at a wavelength of 193 nm. For example, proposed as a base polymer are polyacrylic acid and a derivative thereof, a norbornene-maleic anhydride alternating polymer, polynorbornene, a ring-opening metathesis polymer, a hydrogenated ring-opening metathesis polymer, etc., and an outcome is obtained in a certain degree in terms of increase in the transparency of the resin itself.

In recent years, in addition to a positive-tone resist with alkali aqueous solution development, a negative-tone resist with organic solvent development has attracted attention. To resolve an extremely fine hole pattern, which cannot be achieved by positive-tone exposure, by the negative-tone exposure, the negative-tone resist forms a negative-type pattern by using a positive-type resist composition having high resolution and developing the exposed pattern with an organic solvent. Furthermore, combining twice development of alkali aqueous solution development and organic solvent development has been investigated to obtain twice resolution. As an ArF resist composition for the negative-tone developments with an organic solvent, conventional positive-type ArF resist compositions can be used, and Patent Documents 1 to 3 describe patterning processes using these compositions.

To adapt rapid miniaturization in recent years, development of resist compositions is progressing in addition to the process technology. Various investigations are made also on a photoacid generator, and a sulfonium salt composed of a triphenylsulfonium cation and a perfluoroalkanesulfonate anion is commonly used. However, a perfluoroalkanesulfonic acid, which is an acid to be generated, particularly perfluorooctanesulfonic acid (PFOS), has hard degradability, bioconcentration property, and toxicity concern. Thus, it is difficult to be applied for the resist composition, and a photoacid generator to generate perfluorobutanesulfonic acid is used at present. However, when such a photoacid generator is used for the resist composition, the acid to be generated largely diffuses to be difficult to achieve the high resolution. For this problem, various partially fluorine-substituted alkanesulfonic acids and salts thereof have been developed. For example, Patent Document 1 describes, as conventional art, a photoacid generator to generate an α,α-difluoroalkanesulfonic acid by exposure, specifically di(4-tert-butylphenyl)iodonium 1,1-difluoro-2-(1-naphthyl)ethanesulfonate, and a photoacid generator to generate an α,α,β,β-tetrafluoroalkanesulfonic acid. Although having a reduced fluorine substitution rate, these acids have no decomposable substituent, such as an ester structure, and thereby are not satisfactory from the viewpoint of environmental safety with easy degradability. In addition, these photoacid generators have problems such as: a limit of molecular design for changing balkiness of the alkanesulfonic acid; and an expensive starting material having fluorine atoms.

As a line width of a circuit has been miniaturized, an effect of deterioration in contrast due to acid diffusion have become further considerable in the resist composition. This effect, which is caused by the pattern size being close to a length of the acid diffusion, deteriorates mask fidelity and deteriorates pattern rectangularity due to increase in a size error on a wafer relative to a value of size error of a mask (mask error factor (MEF)). Thus, to sufficiently obtain contribution of the shortened wavelength of the light source and the higher NA, increase in dissolution contrast or inhibition of the acid diffusion is further required compared with conventional materials. One of the solutions, which lowers the baking temperature, reduces the acid diffusion and consequently enables to improve the MEF, but the sensitivity is necessarily lowered.

Introducing a balky substituent or polar group into the photoacid generator is effective for inhibiting the acid

diffusion. Patent Document 4 describes a photoacid generator having 2-acyloxy-1,1,3,3,3-pentafluoropropane-1-sulfonic acid, which has excellent solubility and stability in a resist solvent, and capable of a wide molecular design. In particular, a photoacid generator having 2-(1-adamantyloxy)-1,1,3,3,3-pentafluoropropane-1-sulfonic acid, which has the introduced balky substituent, has a small acid diffusion. Patent Documents 5 to 7 describe photoacid generators having an introduced condensed lactone, sultone, or thiolactone, as the polar group. Although improvement of performance by the effect of inhibiting the acid diffusion derived from introducing the polar group is confirmed in a certain degree, these photoacid generators are still insufficient for highly controlling the acid diffusion, and the lithographic performance collectively including the MEF, a pattern shape, and the sensitivity, are not satisfactory.

Introducing the polar group into an anion of the photoacid generator is effective for inhibiting the acid diffusion, but is disadvantageous from the viewpoint of solvent solubility. Patent Documents 8 and 9 attempt to introduce an alicyclic group into a cation portion of the photoacid generator to achieve the solvent solubility, and specifically, a cyclohexane ring or an adamantane ring is introduced. Although introducing such an alicyclic group improves the solubility, a certain amount of carbon numbers is required to achieve the solubility. As a result, the molecular structure of the photoacid generator becomes balky to deteriorate lithographic performance such as line width roughness (LWR) and critical dimension uniformity (CDU) in fine pattern formation.

For improving the dissolution contrast, Patent Documents 10 and 11 introduce an acid-labile group into an anion or cation of the photoacid generator. Many of these photoacid generators have a structure in which a carboxylic acid is protected with the acid-labile group. A deprotection reaction of the protective group with an acid proceeds before and after the exposure. However, the polar group to be generated is the carboxyl group, and thereby swelling due to a developer occurs during alkali development to cause a problem of pattern collapse in fine pattern formation. Development of a novel photoacid generator is important for meeting the requirement of further miniaturization, and desired is development of a photoacid generator having sufficiently controlled acid diffusion and excellent solvent solubility, and effectively inhibiting the pattern collapse.

## CITATION LIST

### PATENT LITERATURE

Patent Document 1: JP 2008-281974 A
Patent Document 2: JP 2008-281975 A
Patent Document 3: JP 4554665 B
Patent Document 4: JP 2007-145797 A
Patent Document 5: JP 5061484 B
Patent Document 6: JP 2016-147879 A
Patent Document 7: JP 2015-63472 A
Patent Document 8: JP 5573098 B
Patent Document 9: JP 6461919 B
Patent Document 10: JP 5544078 B
Patent Document 11: JP 5609569 B

### NON PATENT LITERATURE

Non Patent Document 1: Journal of Photopolymer Science and Technology Vol. 17, No. 4, pp. 587-601 (2004).

## SUMMARY OF INVENTION

### TECHNICAL PROBLEM

For the recent requirement of high resolution of the resist pattern, a resist composition using a conventional sulfonium salt-type photoacid generator cannot sufficiently inhibit the acid diffusion, and consequently deteriorates lithographic performance such as the contrast, MEF, and the line width roughness (LWR). In addition, such a resist composition has a problem of the pattern collapse due to the swelling in fine pattern formation.

The present invention is made in view of the above circumstances. An object of the present invention is to provide: a sulfonium salt used for a resist composition having excellent solvent solubility, high sensitivity and high contrast, and having excellent lithographic performance such as exposure latitude (EL) and LWR; a resist composition compositing this sulfonium salt as a photoacid generator; and a patterning process using this resist composition.

SOLUTION TO PROBLEM

To solve the above problem, the present invention provides a sulfonium salt represented by the following formula (1),

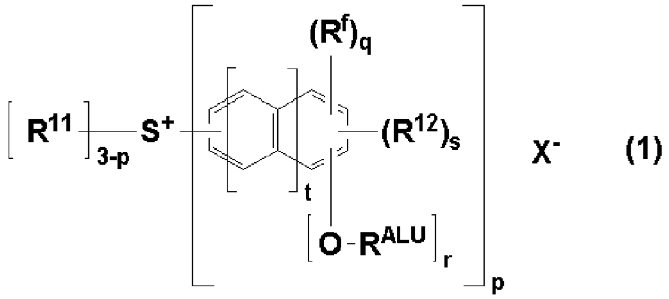

wherein "p" represents an integer of 1 to 3; $R^{11}$ represents a hydrocarbyl group having 1 to 20 carbon atoms and optionally having a heteroatom; two of the three substituents bonded to the sulfonium cation are optionally bonded each other to form a ring together with the sulfur atom to which the two substituents are bonded; $R^f$ represents a fluorine atom, a fluorine-atom-containing alkyl group, a fluorine-atom-containing alkoxy group, or a fluorine-atom-containing sulfide group, each group having 1 to 6 carbon atoms; "q" represents an integer of 1 to 4, and when $q \geq 2$, $R^f$ may be same as or different from each other; $R^{ALU}$ represents an acid-labile group formed together with the adjacent oxygen atom; "r" represents an integer of 1 to 4; $R^{12}$ represents a hydrocarbyl group having 1 to 20 carbon atoms and optionally having a heteroatom; "s" represents an integer of 0 to 4; "t" represents an integer of 0 to 2; $q + r + s \leq 5$ when t = 0, $q + r + s \leq 7$ when t = 1, and $q + r + s \leq 9$ when t = 2; $R^f$ and $-O-R^{ALU}$ are bonded to adjacent carbon atoms; and $X^-$ represents a non-nucleophilic counterion having no polymerizable group.

Such a sulfonium salt can provide a resist composition having excellent solvent solubility, high sensitivity and high contrast, and having excellent lithographic performance such as exposure latitude (EL) and LWR, by blending with the resist composition.

In the present invention, $R^{ALU}$ in the formula (1) is preferably represented by the following formula (ALU-1) or (ALU-2),

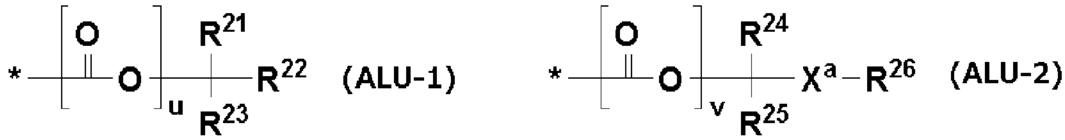

wherein in the formula (ALU-1), $R^{21}$, $R^{22}$, and $R^{23}$ each independently represent a hydrocarbyl group having 1 to 10 carbon atoms and optionally having a substituent; any two of $R^{21}$, $R^{22}$, and $R^{23}$ are optionally bonded each other to form a ring; "u" represents an integer of 0 or 1; in the formula (ALU-2), $R^{24}$ and $R^{25}$ each independently represent a hydrogen atom or a hydrocarbyl group having 1 to 10 carbon atoms and optionally having a substituent; $R^{26}$ represents a hydrocarbyl group having 1 to 20 carbon atoms, or $R^{26}$ is optionally bonded to $R^{24}$ or $R^{25}$ each other to form a heterocyclic group having 3 to 20 carbon atoms together with $X^a$ and the carbon atom to which $R^{24}$ and $R^{25}$ are bonded; $-CH_2-$ contained in the hydrocarbyl group and the heterocyclic group is optionally substituted with -O- or -S-; $X^a$ represents an oxygen atom or a sulfur atom; "v" represents an integer of 0 or 1; and "*" represents a bond to the adjacent oxygen atom.

Such a sulfonium salt can provide a resist composition having further excellent lithographic performance by blending with the resist composition.

In the sulfonium salt, X- being the non-nucleophilic counterion having no polymerizable group in the formula (1) preferably represents a sulfonate anion, an imide anion, or a methide anion.

Such a non-nucleophilic counterion can be suitably used for the sulfonium salt.

The present invention also provides a photoacid generator comprising the above sulfonium salt.

The inventive sulfonium salt is suitable for a photoacid generator.

The present invention also provides a resist composition comprising the above photoacid generator

The inventive resist composition has excellent solvent solubility, high sensitivity and high contrast, and excellent lithographic performance such as exposure latitude (EL) and LWR.

In this case, the resist composition preferably further comprises a base resin having a repeating unit represented by the following formula (a1) or (a2),

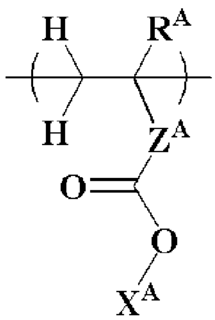

(a1)

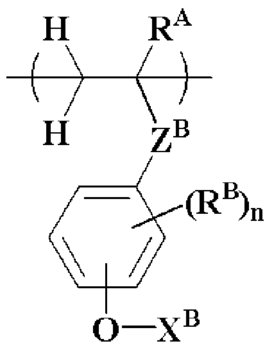

(a2)

wherein $R^A$ each independently represents a hydrogen atom, a fluorine atom, a methyl group, or a trifluoromethyl group; $Z^A$ represents a single bond, a phenylene group, a naphthylene group, or (main chain)-C(=O)-O-$Z^{A1}$-; $Z^{A1}$ represents a linear, branched, or cyclic alkanediyl group having 1 to 10 carbon atoms, a phenylene group or a naphthylene group, the alkanediyl group optionally having a hydroxy group, an ether bond, an ester bond, or a lactone ring; $Z^B$ represents a single bond or (main chain)-C(=O)-O-; $X^A$ and $X^B$ each independently represent an acid-labile group; $R^B$ represents a linear, branched, or cyclic monovalent hydrocarbon group having 1 to 20 carbon atoms and optionally having a heteroatom; and "n" represents an integer of 0 to 4.

Such a base resin can be suitably used for the inventive resist composition.

In the inventive resist composition, the above base resin preferably further has a repeating unit represented by the following formula (b1) or (b2),

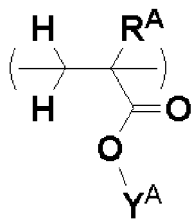

(b1)

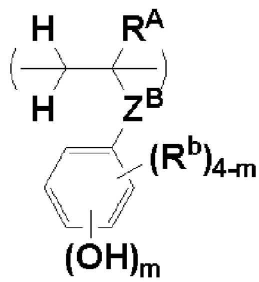

(b2)

wherein $R^A$ and $Z^B$ represent the same as above; $Y^A$ represents a hydrogen atom or a polar group having one or more structures selected from a hydroxy group other than a phenolic hydroxy group, a cyano group, a carbonyl group, a carboxy group, an ether bond, an ester bond, a sulfonate ester bond, a sulfonamide bond, a carbonate bond, a lactone ring, a sultone ring, a sulfur atom, and a carboxylic anhydride; $R^b$ represents a linear, branched, or cyclic monovalent hydrocarbon group having 1 to 20 carbon atoms and optionally having a heteroatom; and "m" represents an integer of 1 to 4.

A base polymer having such a repeating unit can be suitably used as the base resin blended with the inventive resist composition.

In the inventive resist composition, the base resin preferably further has at least one repeating unit selected from repeating units represented by the following formulae (C1) to (C4),

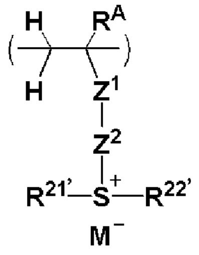

(C1)

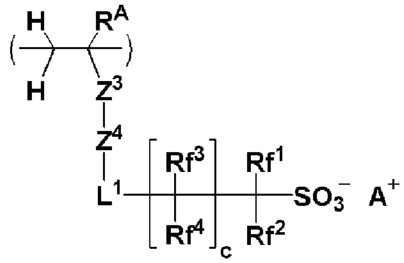

(C2)

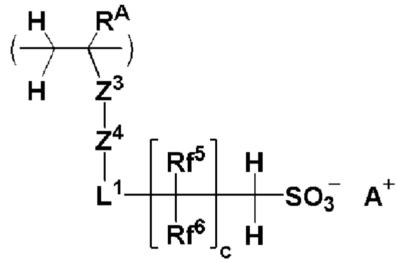

(C3)

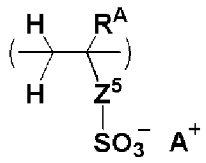

(C4)

wherein $R^A$ represents the same as above; $Z^1$ represents a single bond or a phenylene group; $Z^2$ represents a single bond, *-C(=O)-O-$Z^{21}$-, *-C(=O)-NH-$Z^{21}$-, or *-O-$Z^{21}$-; $Z^{21}$ represents an aliphatic hydrocarbylene group having 1 to 6 carbon atoms, a phenylene group, or a divalent group obtained by combining these groups, $Z^{21}$ optionally having a carbonyl group, an ester bond, an ether bond, or a hydroxy group; $Z^3$ represents a single bond, a phenylene group, a naphthylene group, or *-C(=O)-O-$Z^{31}$-; $Z^{31}$ represents an aliphatic hydrocarbylene group having 1 to 10 carbon atoms, a phenylene group or a naphthylene group, the aliphatic hydrocarbylene group optionally having a hydroxy group, an ether bond, an ester bond, or a lactone ring; $Z^4$ represents a single bond, a methylene group, or *-$Z^{41}$-C(=O)-O-; $Z^{41}$ represents a hydrocarbylene group having 1 to 20 carbon atoms and optionally having a heteroatom, an ether bond, or an ester bond; $Z^5$ represents a single bond, a methylene group, an ethylene group, a phenylene group, a fluorinated phenylene group, a phenylene group substituted with a trifluoromethyl group, *-C(=O)-O-$Z^{51}$-, *-C(=O)-N(H)-$Z^{51}$-, or *-O-$Z^{51}$-; $Z^{51}$ represents an aliphatic hydrocarbylene group having 1 to 6 carbon atoms, a phenylene group, a fluorinated phenylene group, or a phenylene group substituted with a trifluoromethyl group, and $Z^{51}$ optionally has a carbonyl group, an ester bond, an ether bond, or a hydroxy group; "*" represents an attachment point to a carbon atom in the main chain or to a group bonding to the main chain; $R^{21'}$ and $R^{22'}$ each independently represent a hydrocarbyl group having 1 to 20 carbon atoms and optionally having a heteroatom; $R^{21'}$ and $R^{22'}$ are optionally bonded each other to form a ring together with the sulfur atom to which $R^{21'}$ and $R^{22'}$ are bonded; $L^1$ represents a single bond, an ether bond, an ester bond, a carbonyl group, a sulfonate ester bond, a carbonate bond, or a carbamate bond; $Rf^1$ and $Rf^2$ each independently represent a fluorine atom or a fluorinated alkyl group having 1 to 6 carbon atoms; $Rf^3$ and $Rf^4$ each independently represent a hydrogen atom, a fluorine atom, or a fluorinated alkyl group having 1 to 6 carbon atoms; $Rf^5$ and $Rf^6$ each independently represent a hydrogen atom, a fluorine atom, or a fluorinated alkyl group having 1 to 6 carbon atoms; not all $Rf^5$ and $Rf^6$ simultaneously represent hydrogen atoms; $M^-$ represents a non-nucleophilic counterion; $A^+$ represents an onium cation; and "c" represents an integer of 0 to 3.

A base resin having such a repeating unit can be further suitably used as the base resin blended with the inventive resist composition.

The inventive resist composition preferably further comprises an organic solvent.

Such a resist composition has excellent operability.

The inventive resist composition preferably further comprises a compound represented by the following formula (5) or (6),

$$R^{q1}\text{-}SO_3^-\ Mq^+ \qquad (5)$$

$$R^{q2}\text{-}CO_2^-\ Mq^+ \qquad (6)$$

wherein $R^{q1}$ represents a hydrogen atom or a monovalent hydrocarbon group having 1 to 40 carbon atoms and optionally having a heteroatom, $R^{q1}$ excluding a group in which a hydrogen atom bonded to a carbon atom at an $\alpha$-position of the sulfo group is substituted with a fluorine atom or a fluoroalkyl group; $R^{q2}$ represents a hydrogen atom or a monovalent

hydrocarbon group having 1 to 40 carbon atoms and optionally having a heteroatom; and $Mq^+$ represents an onium cation.

The inventive resist composition can control the acid diffusion with containing the above compound as a quencher.

The inventive resist composition preferably further comprises a photoacid generator other than the above photoacid generator.

Such a resist composition has good resolution.

The inventive resist composition preferably further comprises an amine compound.

The amine compound functions as a quencher, and the acid diffusion can be further suitably controlled.

The inventive resist composition preferably further comprises: a surfactant insoluble or hardly soluble in water and soluble in an alkaline developer; and/or a surfactant insoluble or hardly soluble in water and an alkaline developer.

In the ArF immersion exposure without a resist protective film, such a surfactant has a function of reducing penetration of water or leaching by orientation on a surface of the resist film. Thus, such a surfactant is useful for inhibiting elution of a watersoluble component from the resist film to reduce damage of an exposure apparatus. Such a surfactant is also useful because such a surfactant becomes soluble during development with an alkaline aqueous solution after the exposure and post exposure bake (PEB), and hardly forms a foreign matter causing a defect.

The present invention also provides a patterning process comprising steps of: forming a resist film on a substrate using the above resist composition; exposing the resist film to high energy ray; and developing the exposed resist film using a developer.

Such a patterning process uses the inventive resist composition, and can form a pattern with high sensitivity and high contrast, and excellent lithographic performance such as exposure latitude (EL) and LWR.

In this case, the high energy ray is preferably KrF excimer laser light, ArF excimer laser light, electron beam, or extreme ultraviolet ray having a wavelength of 3 to 15 nm.

Such high energy ray can be suitably used for the inventive patterning process.

## ADVANTAGEOUS EFFECTS OF INVENTION

As noted above, pattern forming using the resist composition containing the inventive sulfonium salt as the photoacid generator can form a resist pattern having high contrast and good sensitivity, excellent lithographic performance such as MEF and LWR, and inhibited pattern collapse. In particular, in photolithography using high energy ray such as KrF excimer laser light, ArF excimer laser light, electron beam (EB), and EUV, the chemically amplified resist composition containing the inventive sulfonium salt as the photoacid generator has excellent solvent solubility, high sensitivity and high contrast, and excellent lithographic performance such as exposure latitude (EL) and LWR.

## BRIEF DESCRIPTION OF DRAWINGS

FIG. 1 is a $^1H$-NMR spectrum of a compound obtained in Example I-1.
FIG. 2 is a $^1H$-NMR spectrum of a compound obtained in Example I-2.
FIG. 3 is a $^1H$-NMR spectrum of a compound obtained in Example I-3.
FIG. 4 is a $^1H$-NMR spectrum of a compound obtained in Example I-4.
FIG. 5 is a $^1H$-NMR spectrum of a compound obtained in Example 1-5.

## DESCRIPTION OF EMBODIMENTS

As noted above, there have been demands for the developments of a photoacid generator having sufficiently inhibited acid diffusion, excellent solvent solubility, excellent lithographic performance such as LER, and effectively inhibiting the pattern collapse.

The present inventors have earnestly made study to achieve the above object, and consequently found that a sulfonium salt having a specific structure has excellent solvent solubility and that a resist composition, such as a chemically amplified resist composition, using this sulfonium salt as a photoacid generator has high sensitivity and high contrast, excellent lithographic performance such as EL and LWR, and extremely effectively inhibiting pattern collapse in fine pattern formation. These findings have led to the completion of the present invention.

Specifically, the present invention is a sulfonium salt represented by the following formula (1),

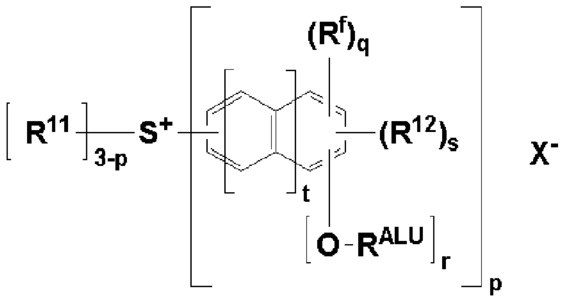

(1)

wherein "p" represents an integer of 1 to 3; $R^{11}$ represents a hydrocarbyl group having 1 to 20 carbon atoms and optionally having a heteroatom; two of the three substituents bonded to the sulfonium cation are optionally bonded each other to form a ring together with the sulfur atom to which the two substituents are bonded; $R^f$ represents a fluorine atom, a fluorine-atom-containing alkyl group, a fluorine-atom-containing alkoxy group, or a fluorine-atom-containing sulfide group, each group having 1 to 6 carbon atoms; "q" represents an integer of 1 to 4, and when $q \geq 2$, $R^f$ may be same as or different from each other; $R^{ALU}$ represents an acid-labile group formed together with the adjacent oxygen atom; "r" represents an integer of 1 to 4; $R^{12}$ represents a hydrocarbyl group having 1 to 20 carbon atoms and optionally having a heteroatom; "s" represents an integer of 0 to 4; "t" represents an integer of 0 to 2; $q + r + s \leq 5$ when t = 0, $q + r + s \leq 7$ when t = 1, and $q + r + s \leq 9$ when t = 2; $R^f$ and $-O-R^{ALU}$ are bonded to adjacent carbon atoms; and $X^-$ represents a non-nucleophilic counterion having no polymerizable group.

Hereinafter, the present invention will be described in detail, but the present invention is not limited thereto.

Sulfonium Salt

The inventive sulfonium salt is represented by the following formula (1).

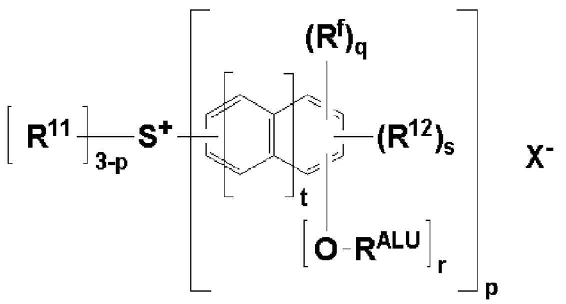

(1)

Hereinafter, the structure in the parenthesis of the formula (1) is also referred to as $Ar^f$. That is, $Ar^f$ represents a structure represented by the following formula (1-1), and the formula (1) can also be represented as the following formula (1-2).

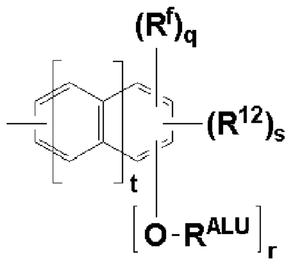

(1-1)

$[R^{11}]_{3-p}S^+Ar^f_p$ X- (1-2)

In the formula (1), "p" represents an integer of 1 to 3.

In the formula (1), $R^{11}$ represents a hydrocarbyl group having 1 to 20 carbon atoms and optionally having a heteroatom. The hydrocarbyl group may be a saturated or unsaturated group, and may be any of linear, branched, and cyclic groups. Specific examples thereof include: alkyl groups having 1 to 20 carbon atoms, such as a methyl group, an

ethyl group, an n-propyl group, an isopropyl group, an n-butyl group, and a tert-butyl group; cyclic saturated hydrocarbyl groups having 3 to 20 carbon atoms, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups having 2 to 20 carbon atoms, such as a vinyl group, an allyl group, a propenyl group, a butenyl group, and a hexenyl group; cyclic unsaturated hydrocarbyl groups having 3 to 20 carbon atoms, such as a cyclohexenyl group; aryl groups having 2 to 20 carbon atoms, such as a phenyl group and a naphthyl group; aralkyl groups having 7 to 20 carbon atoms, such as a benzyl group, a 1-phenylethyl group, and a 2-phenylethyl group; and groups obtained by combining these groups. Among these, aryl groups are preferable. A part or all of hydrogen atoms in the hydrocarbyl group are optionally substituted with a group having a heteroatom, such as an oxygen atom, a sulfur atom, a nitrogen atom, and a halogen atom. A part of $-CH_2-$ constituting the hydrocarbyl group is optionally substituted with a group having a heteroatom, such as an oxygen atom, a sulfur atom, and a nitrogen atom. As a result, optionally contained are a hydroxy group, a cyano group, a fluorine atom, a chlorine atom, a bromine atom, an iodine atom, a carbonyl group, an ether bond, an ester bond, a sulfonate ester bond, a carbonate bond, a lactone ring, a sultone ring, a carboxylic anhydride, a haloalkyl group, etc.

When p = 1, any two of the aromatic ring $Ar^f$ and the two $R^{11}$ are optionally bonded each other to form a ring together with the sulfur atom to which these groups are bonded. When p = 2, any two of the two aromatic rings $Ar^f$ and the one $R^{11}$ are optionally bonded each other to form a ring together with the sulfur atom to which these groups are bonded. When p = 3, any two of the three aromatic rings $Ar^f$ are optionally bonded each other to form a ring together with the sulfur atom to which these groups are bonded. Examples of a sulfonium cation in these cases include those represented by the following formulae.

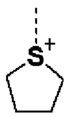
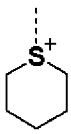
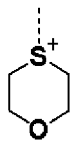
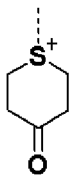
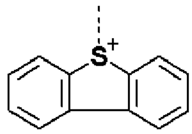
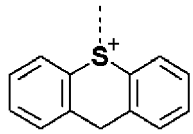
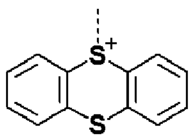
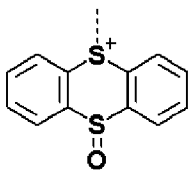
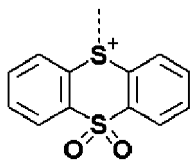
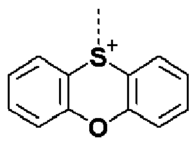
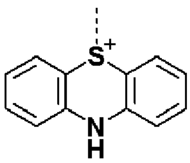
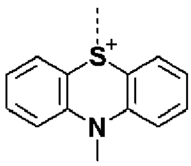
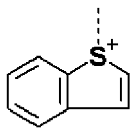
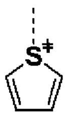

In the formula, a broken line represents a residual attachment point of the sulfonium cation.

In the formula (1), "t" represents an integer of 0 to 2. "t = 0" represents a benzene ring, "t = 1" represents a naphthalene ring, and "t = 2" represents an anthracene ring. "t" preferably represents 0, which represents a benzene ring, from the viewpoint of the solvent solubility.

In the formula (1), $R^f$ represents a fluorine atom or a fluorine-atom-containing alkyl group, alkoxy group, or sulfide group having 1 to 6 carbon atoms. Examples of the fluorine-atom-containing alkyl group having 1 to 6 carbon atoms include a fluoromethyl group, a difluoromethyl group, a trifluoromethyl group, a 2,2,2-trifluoroethyl group, a pentafluoroethyl group, a pentafluoropropyl group, a 1,1,1,3,3,3-hexafluoro-2-propyl group, and a nonafluorobutyl group. Examples of the fluorine-atom-containing alkoxy group having 1 to 6 carbon atoms include a fluoromethoxy group, a difluoromethoxy group, a trifluoromethoxy group, a 2,2,2-trifluoroethoxy group, a pentafluoroethoxy group, a pentafluoropropoxy group, a 1,1,1,3,3,3-hexafluoro-2-propoxy group, and a nonafluorobutoxy group. Examples of the fluorine-atom-containing sulfide group having 1 to 6 carbon atoms include a fluorothiomethoxy group, a difluorothiomethoxy group, a trifluorothiomethoxy group, a 2,2,2-trifluorothioethoxy group, a pentafluorothioethoxy group, a pentafluorothiopropoxy group, a 1,1,1,3,3,3-hexafluoro-2-thiopropoxy group, and a nonafluorothiobutoxy group. Among these, $R^f$ preferably represents a fluorine atom or a fluorine-atom-containing alkoxy group having 1 to 6 carbon atoms, and further preferably a fluorine atom or a trifluoromethoxy group.

In the formula (1), "q" represents an integer of 1 to 4, and when $q \geq 2$, $R^f$ may be same as or different from each other. From the viewpoint of easiness of raw material availability, "q" preferably represents 1 or 2.

In the formula (1), $R^{12}$ represents a hydrocarbyl group having 1 to 20 carbon atoms and optionally having a heteroatom. The hydrocarbyl group may be a saturated or unsaturated group, and may be any of linear, branched, and cyclic groups. Specific examples thereof include: alkyl groups having 1 to 20 carbon atoms, such as a methyl group, an ethyl group, an n-propyl group, an isopropyl group, an n-butyl group, and a tert-butyl group; cyclic saturated hydrocarbyl groups having 3 to 20 carbon atoms, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups having 2 to 20 carbon atoms, such as a vinyl group, an allyl group, a propenyl group, a butenyl group, and a hexenyl group; cyclic unsaturated hydrocarbyl groups having 3 to 20 carbon atoms, such as a cyclohexenyl group; aryl groups having 2 to 20 carbon atoms, such as a phenyl group and a naphthyl group; aralkyl groups having 7 to 20 carbon atoms, such as a benzyl group, a 1-phenylethyl group, and a 2-phenylethyl group; and groups obtained by combining these groups. Among these, aryl groups are preferable. A part or all of hydrogen atoms in the hydrocarbyl group are optionally substituted with a group having a heteroatom, such as an oxygen atom, a sulfur atom, a nitrogen atom, and a halogen atom. A part of $-CH_2-$ constituting the hydrocarbyl group is optionally substituted with a group having a heteroatom, such as an oxygen atom, a sulfur atom, and a nitrogen atom. As a result, optionally contained are a hydroxy group, a cyano group, a fluorine atom, a chlorine atom, a bromine atom, an iodine atom, a carbonyl group, an ether bond, an ester bond, a sulfonate ester bond, a carbonate bond, a lactone ring, a sultone ring, a carboxylic anhydride, a haloalkyl group, etc.

In the formula (1), "s" represents an integer of 0 to 4, and preferably represents 0 or 1.

In the formula (1), $R^{ALU}$ represents an acid-labile group formed together with the adjacent oxygen atom. Here, the acid-labile group means an acidic functional group, such as a phenolic hydroxy group and a carboxyl group, is substituted with one or more functional groups decomposable in the presence of an acid. The acid-labile group is not particularly limited as long as the acid-labile group is decomposed in the presence of an acid to release the functional group having alkali solubility.

In the formula (1), "r" represents an integer of 1 to 4, and when $r \geq 2$, $R^{ALU}$ may be same as or different from each other.

When t = 0, the aromatic ring is a benzene ring, and $q + r + s \leq 5$. When t = 1, the aromatic ring is a naphthalene ring, and $q + r + s \leq 7$. When t = 2, the aromatic ring is an anthracene ring, and $q + r + s \leq 9$. $R^f$ and $-O-R^{ALU}$ are bonded to adjacent carbon atoms. When "q" or "r" represents 2 or more, at least one pair of $R^f$ and $-O-R^{ALU}$ are bonded to the adjacent carbon atoms, and preferably all of $R^f$ and $-O-R^{ALU}$ are bonded to the adjacent carbon atoms. As described later, a sulfonium salt having the structure in which $R^f$ and $-O-R^{ALU}$ are bonded to the adjacent carbon atoms can provide a resist composition that can form a pattern having high dissolution contrast, excellent LWR of a line pattern or CDU of a hole pattern, and hardly causing collapse by a synergistic effect of these groups.

Specifically, the acid-labile group $R^{ALU}$ is preferably a structure represented by the following formula (ALU-1) or (ALU-2).

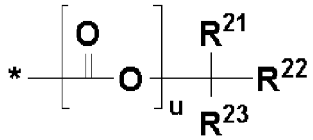 (ALU-1) 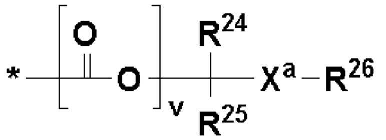 (ALU-2)

In the formula (ALU-1), $R^{21}$, $R^{22}$, and $R^{23}$ each independently represent a hydrocarbyl group having 1 to 10 carbon atoms and optionally having a substituent. Any two of $R^{21}$, $R^{22}$, and $R^{23}$ are optionally bonded each other to form a ring. "u" represents an integer of 0 or 1. In the formula (ALU-2), $R^{24}$ and $R^{25}$ each independently represent a hydrogen atom or a hydrocarbyl group having 1 to 10 carbon atoms and optionally having a substituent. $R^{26}$ represents a hydrocarbyl group having 1 to 20 carbon atoms, or $R^{26}$ is optionally bonded to $R^{24}$ or $R^{25}$ each other to form a heterocyclic group having 3 to 20 carbon atoms together with $X^a$ and the carbon atom to which $R^{24}$ and $R^{25}$ are bonded. $-CH_2-$ contained in the hydrocarbyl group and the heterocyclic group is optionally substituted with -O- or -S-; $X^a$ represents an oxygen atom or a sulfur atom. "v" represents an integer of 0 or 1. "*" represents a bond to the adjacent oxygen atom.

Hydrogen atoms in $R^{21}$ to $R^{26}$ are optionally substituted. When $R^{21}$ to $R^{26}$ has an aromatic ring, such as a benzene ring, a naphthalene ring, and an indene ring, a part or all of hydrogen atoms in the aromatic ring are optionally substituted. Examples of such a substituent include a fluorine atom, a chlorine atom, a bromine atom, an iodine atom, a methyl group, a methoxy group, a trifluoromethyl group, a trifluoromethoxy group, a nitro group, and a cyano group.

Examples of the structure of the acid-labile group represented by the formula (ALU-1) include the following structures, but the structure is not limited thereto. "*" represents a bond to the adjacent oxygen atom.

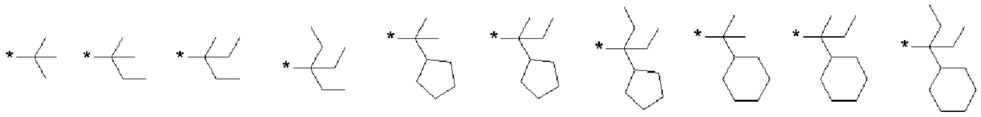

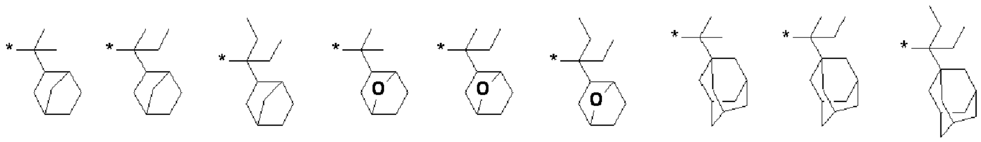

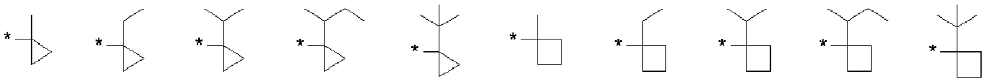

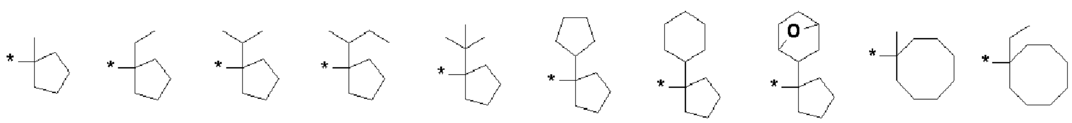

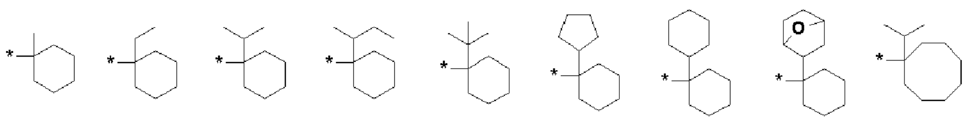

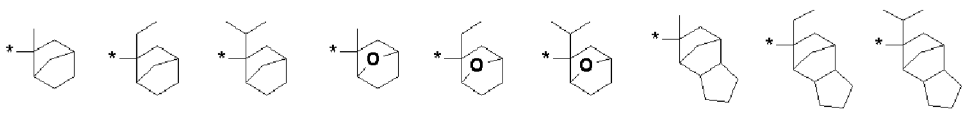

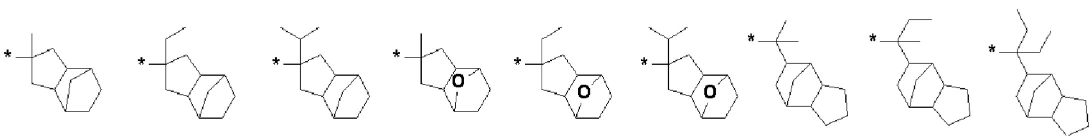

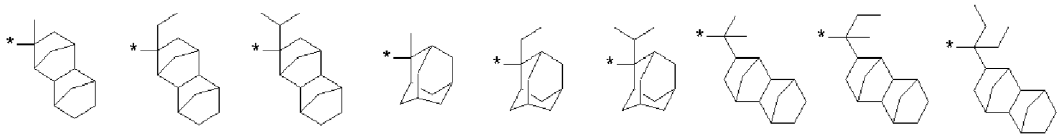

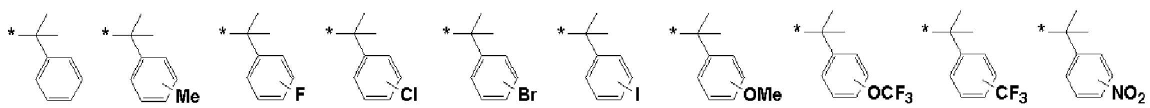

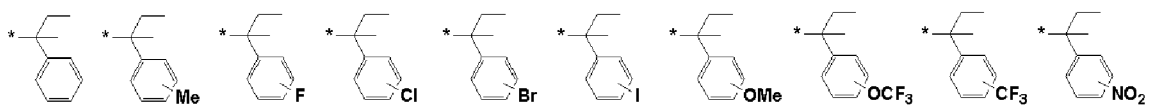

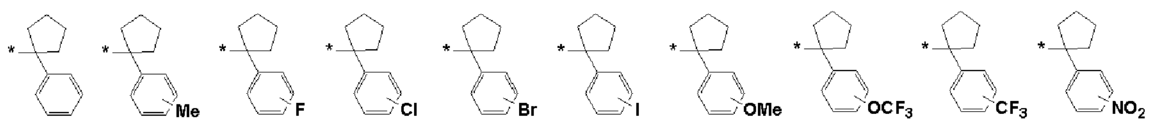

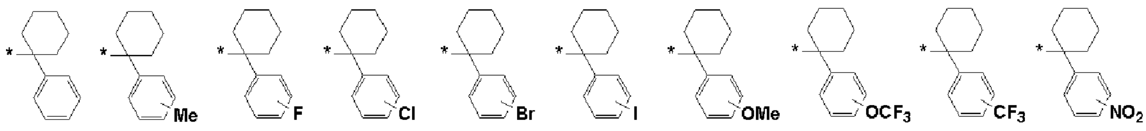

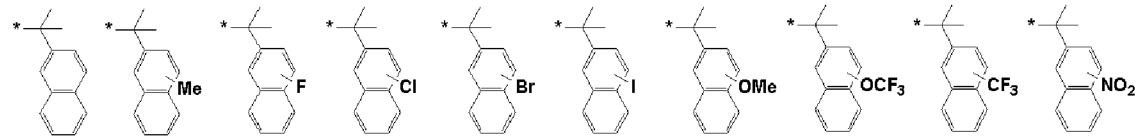

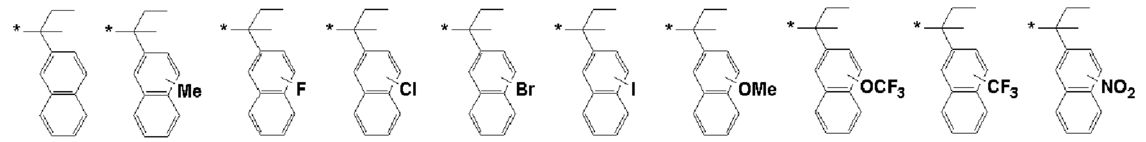

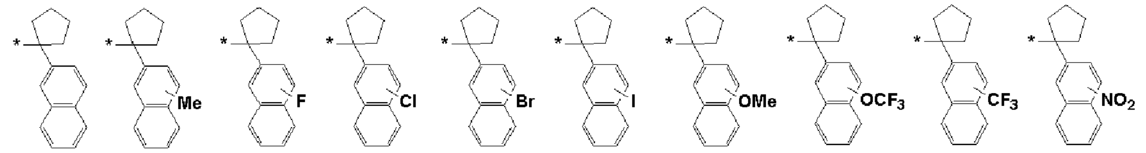

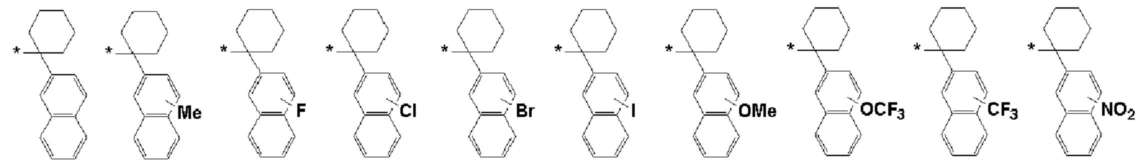

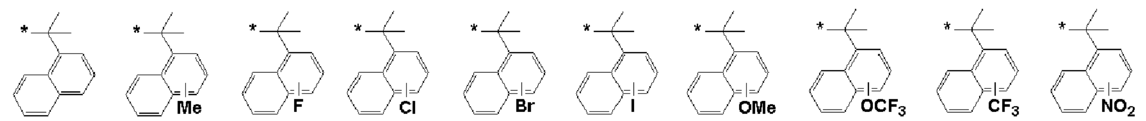

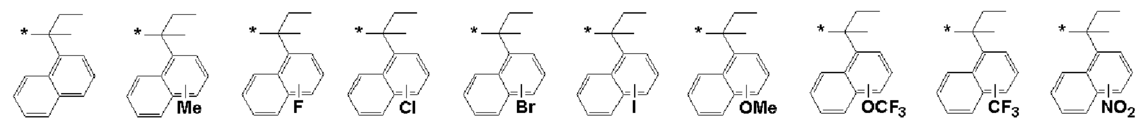

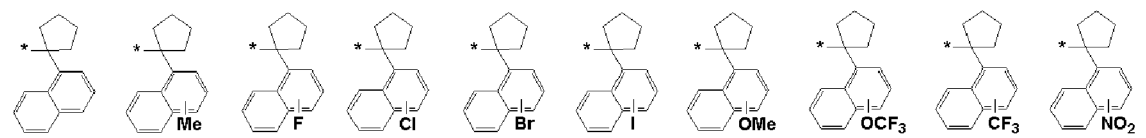

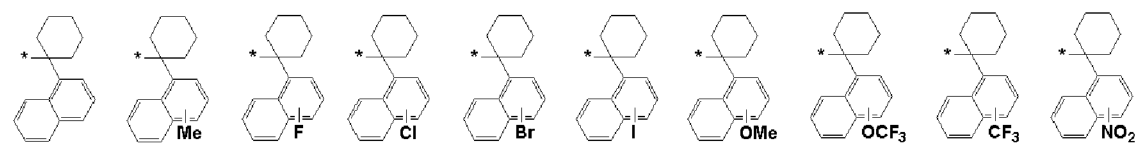

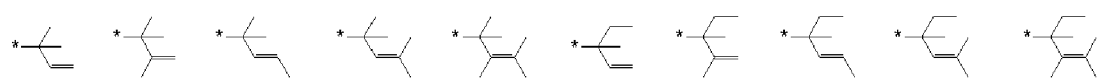

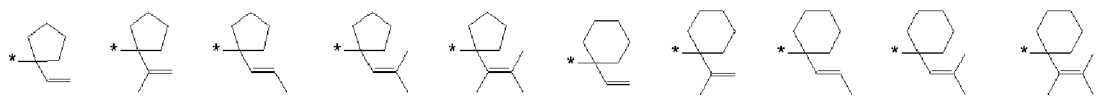

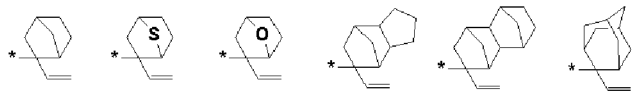

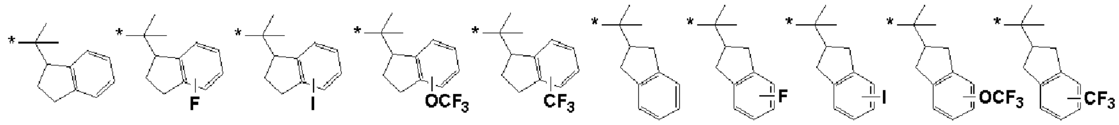

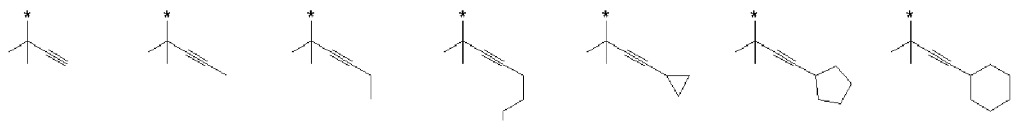

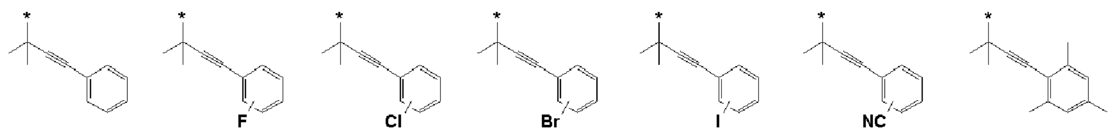

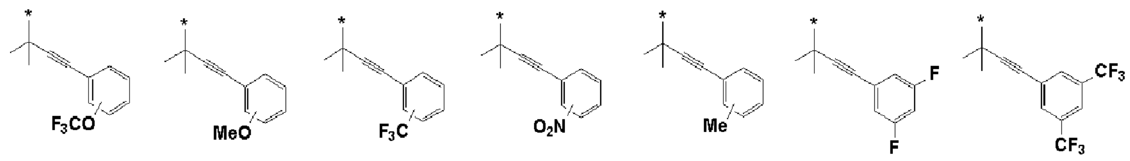

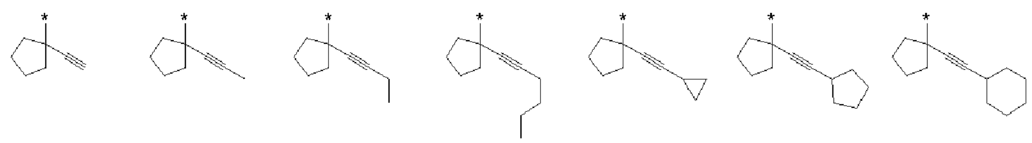

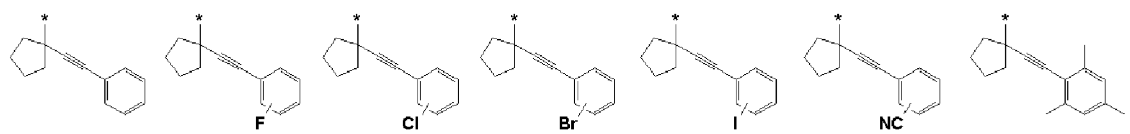

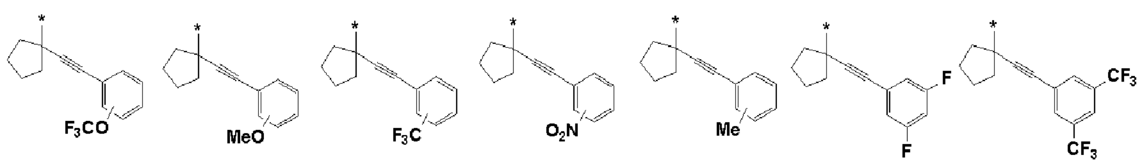

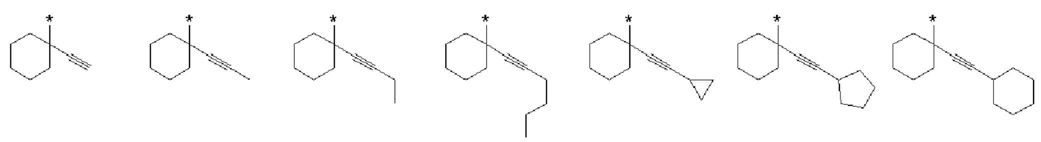

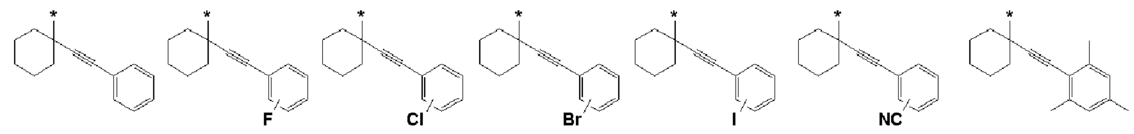

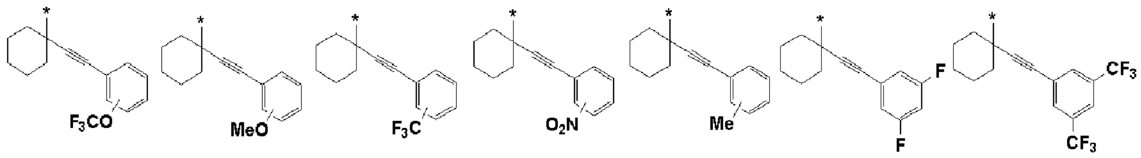

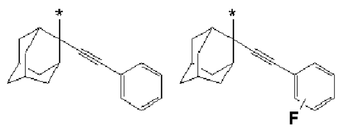

Examples of the structure of the acid-labile group represented by the formula (ALU-2) include the following structures, but the structure is not limited thereto. "*" represents a bond to the adjacent oxygen atom. The oxygen atom in the following structures may be substituted with a sulfur atom.

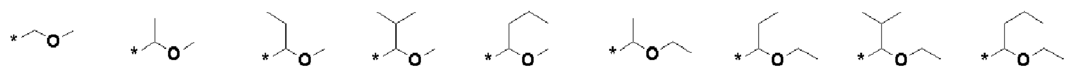

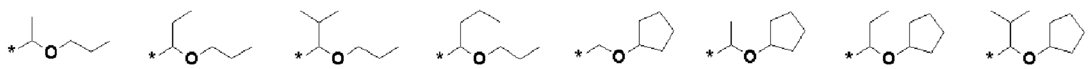

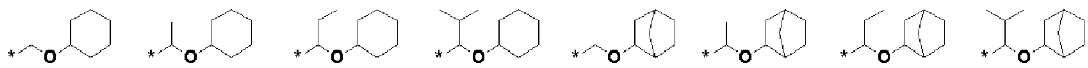

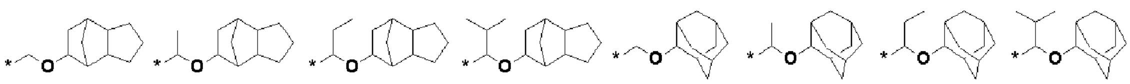

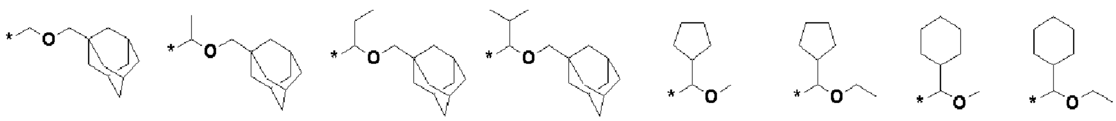

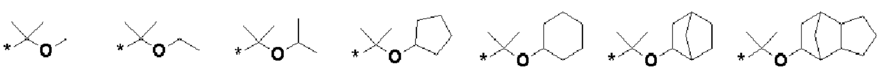

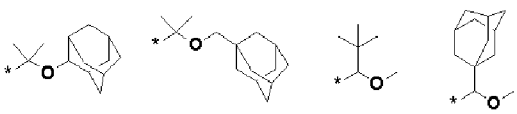

Examples of the cation of the sulfonium salt represented by the formula (1) include the following cations, but the cation is not limited thereto.

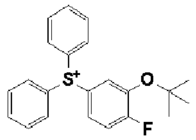
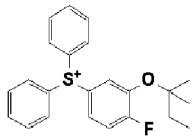
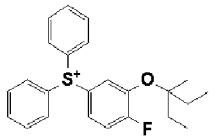
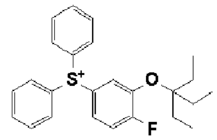

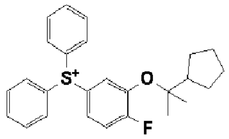
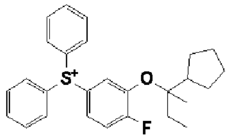
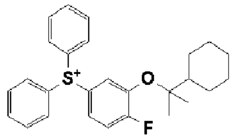
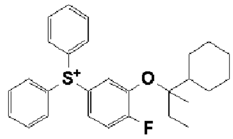

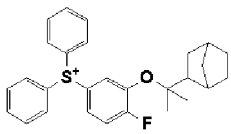
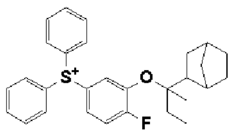
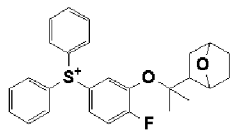
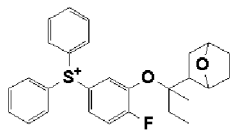

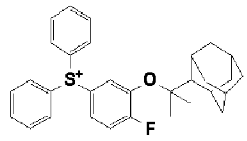
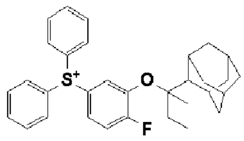
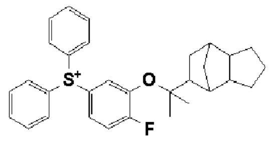
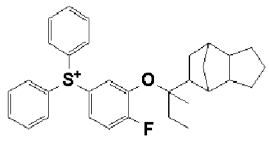

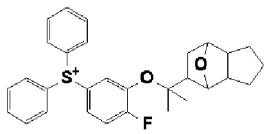
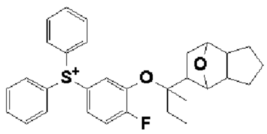
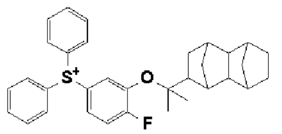
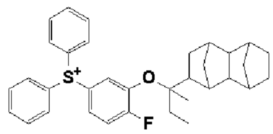

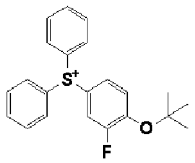
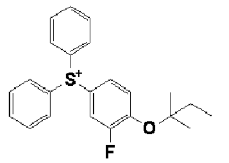
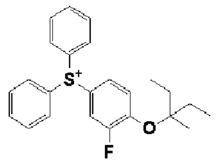
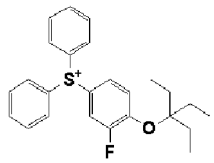

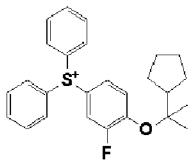
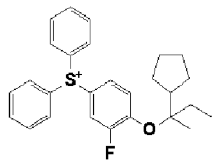
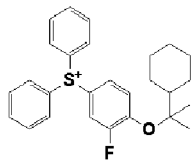
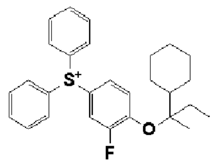

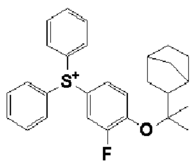
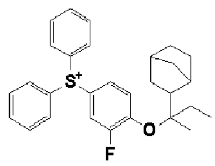
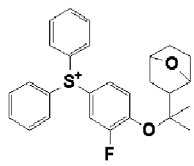
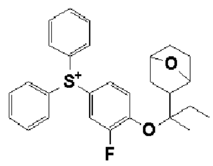

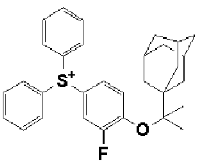
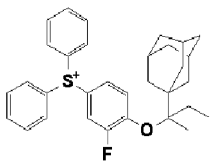
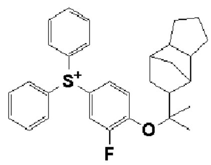
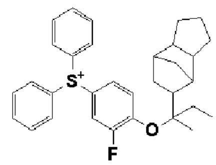

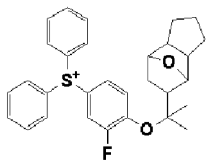 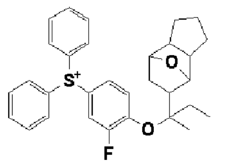 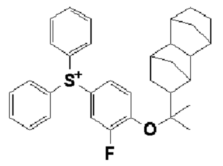 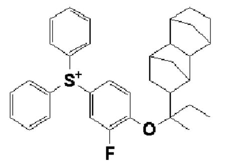

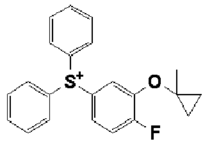 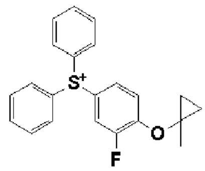 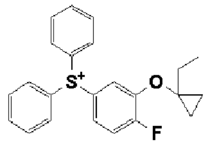 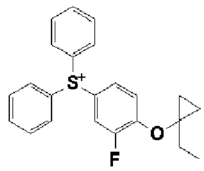

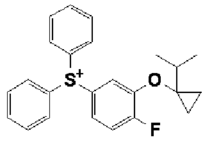 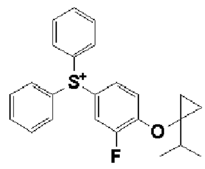 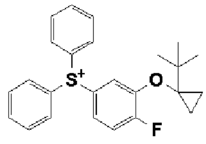 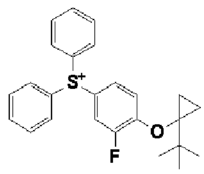

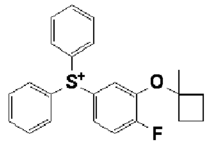 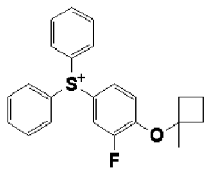 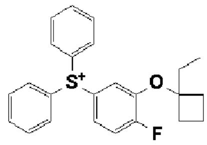 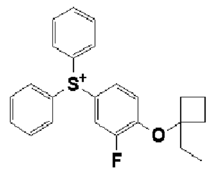

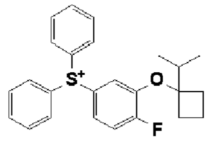 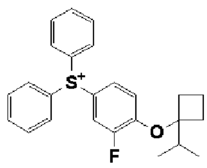 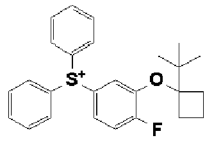 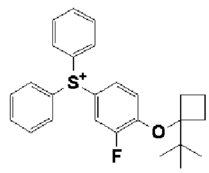

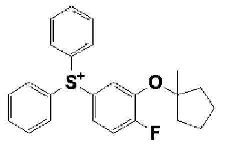 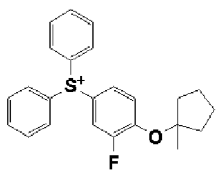 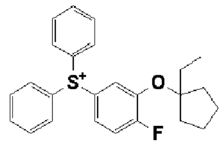 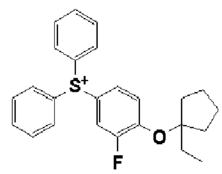

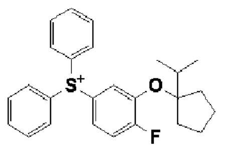 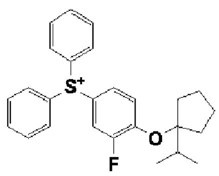 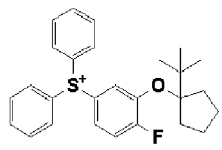 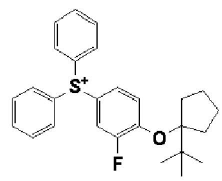

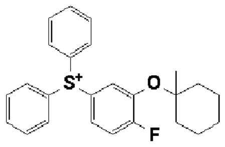 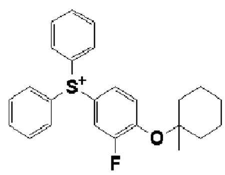 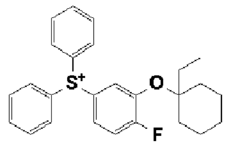 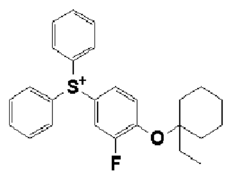

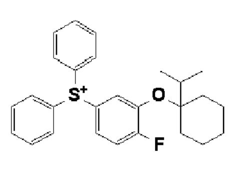
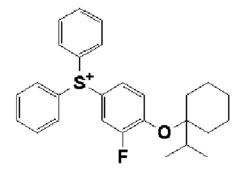
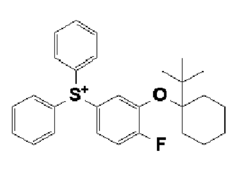
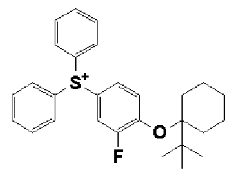
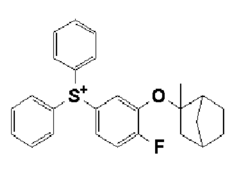
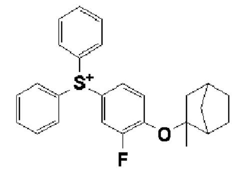
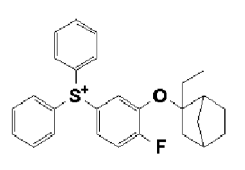
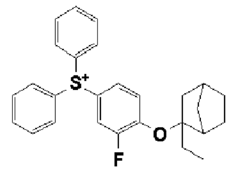
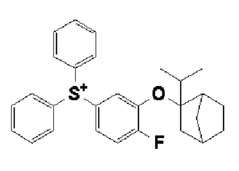
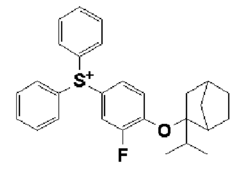
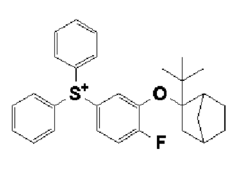
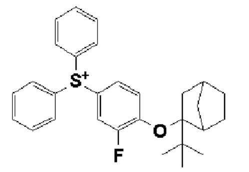
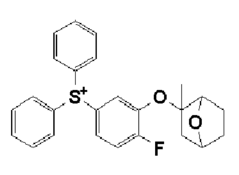
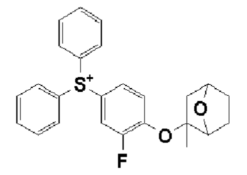
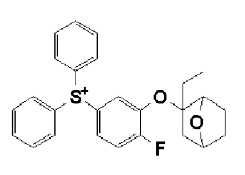
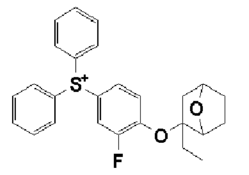
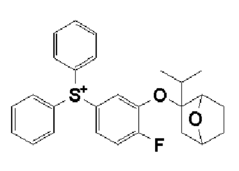
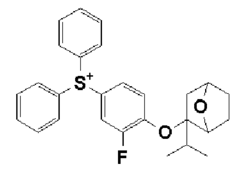
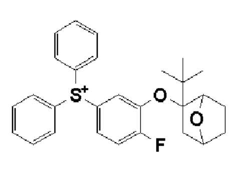
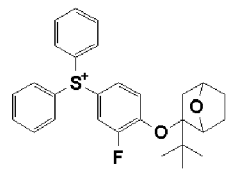
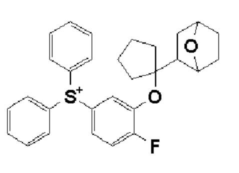
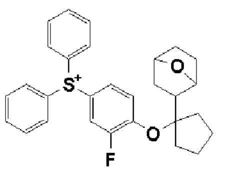
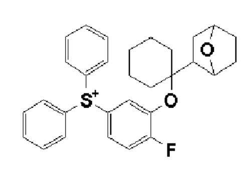
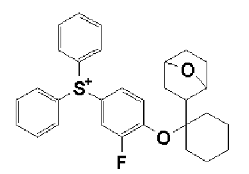
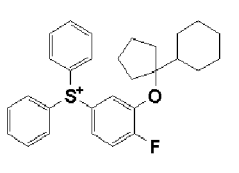
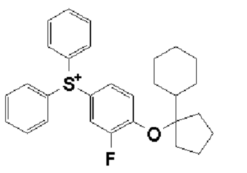
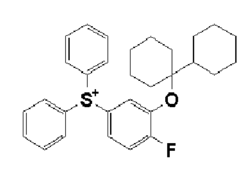
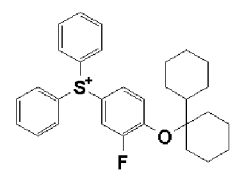
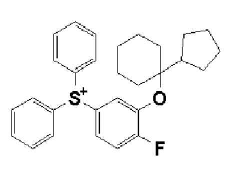
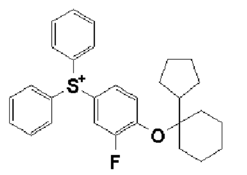
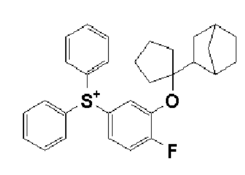
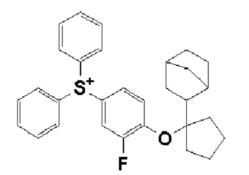

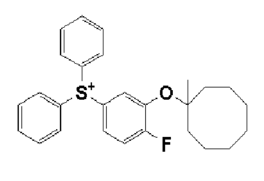 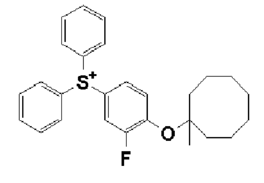 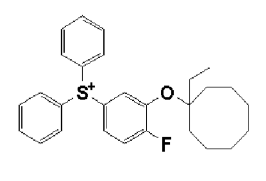 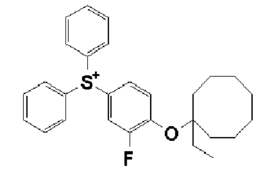

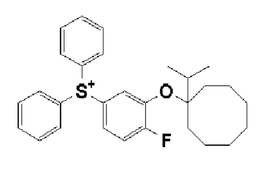 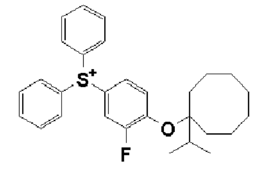 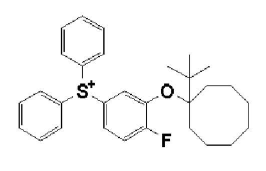 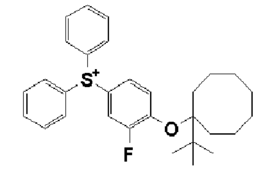

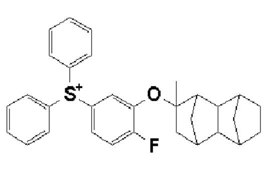 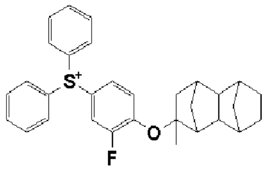 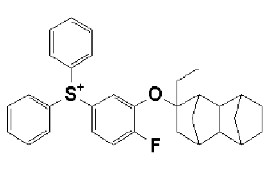 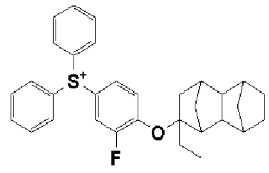

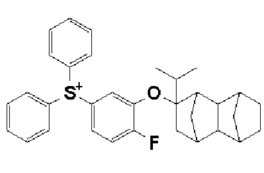 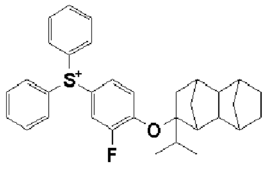 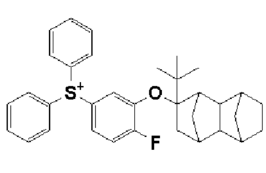 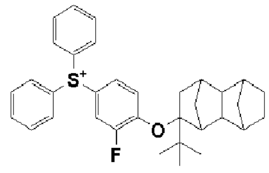

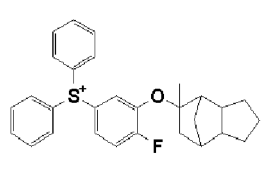 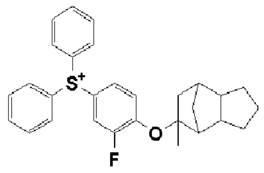 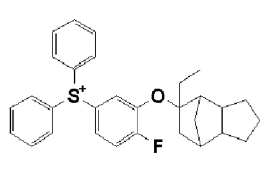 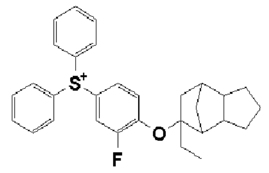

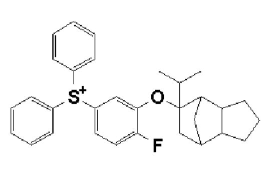 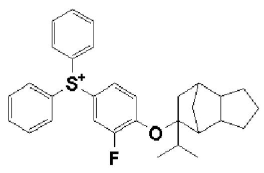 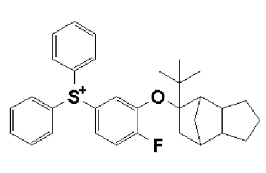 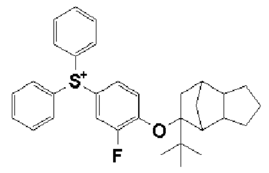

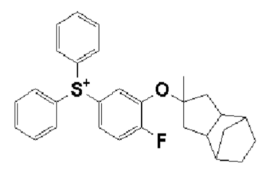 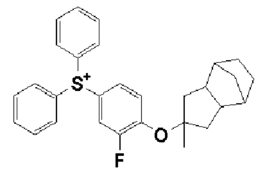 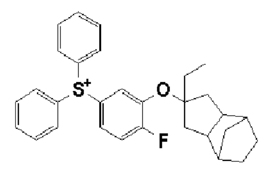 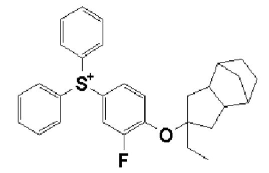

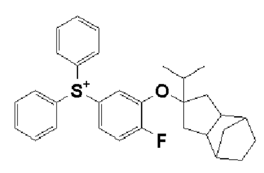 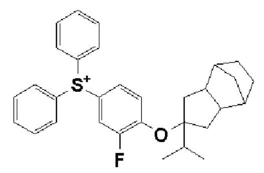 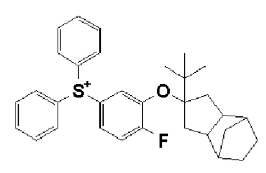 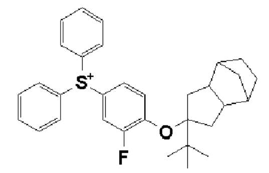

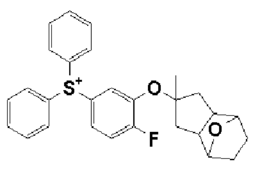 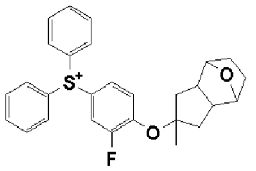 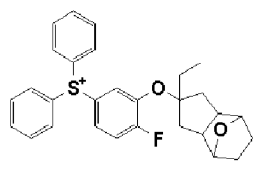 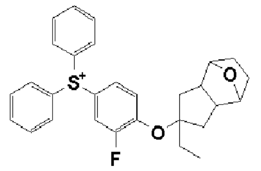

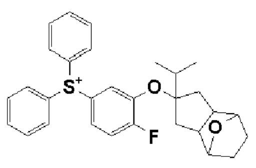 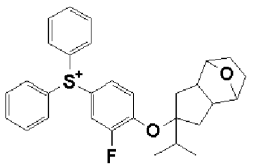 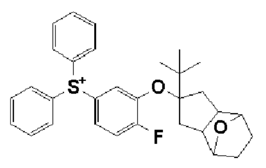 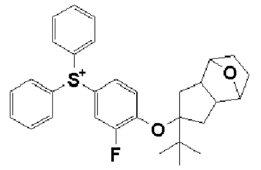

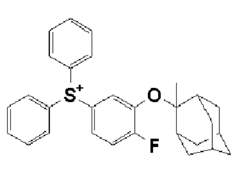 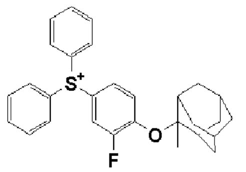 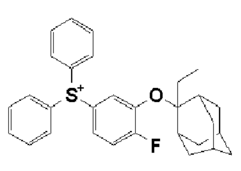 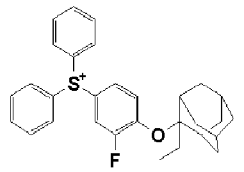

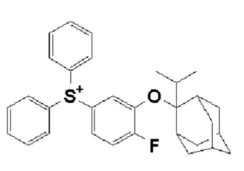 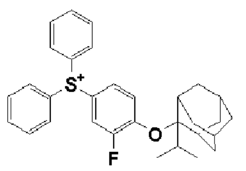 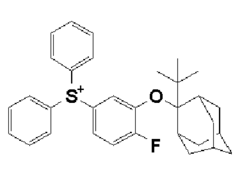 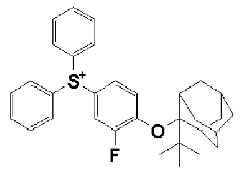

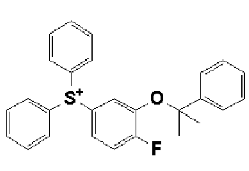 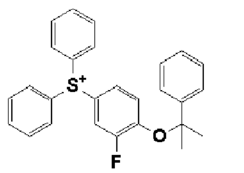 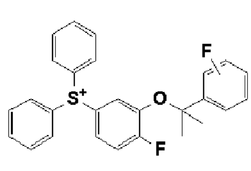 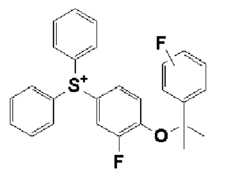

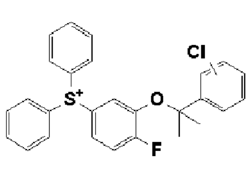 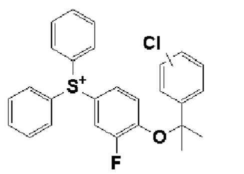 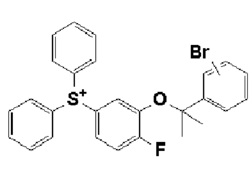 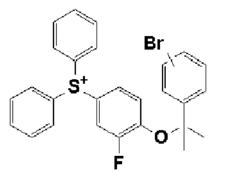

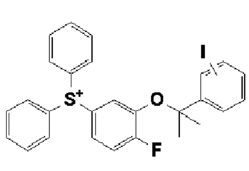 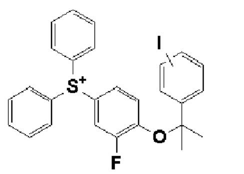 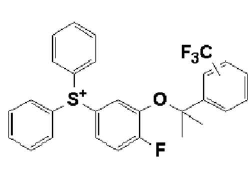 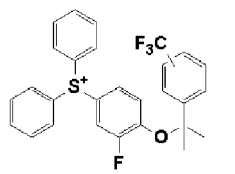

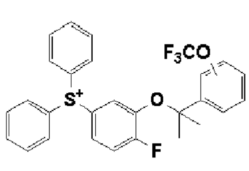 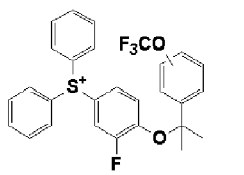 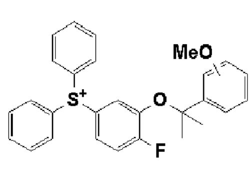 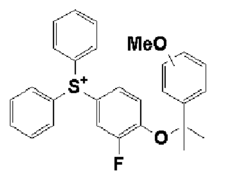

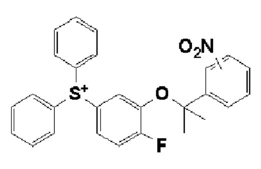 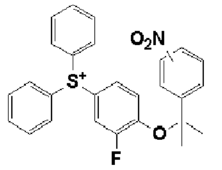 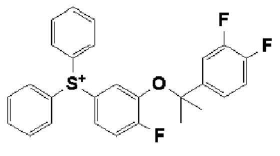 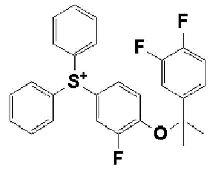

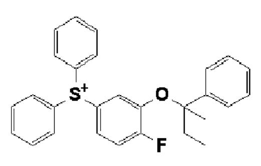 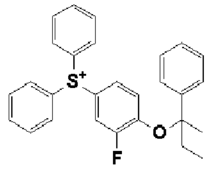 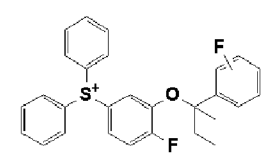 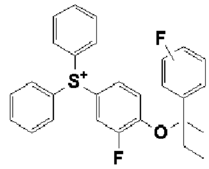

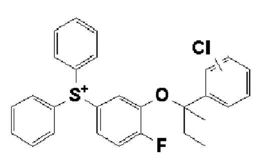 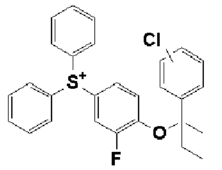 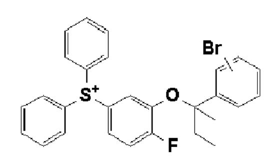 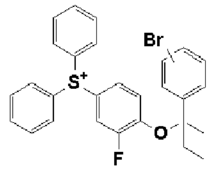

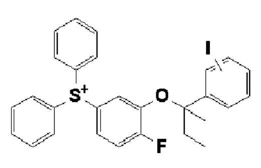 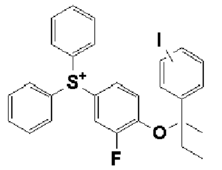 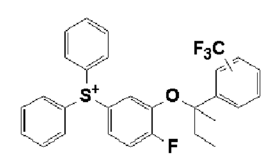 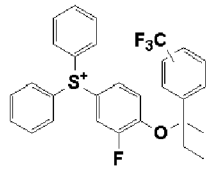

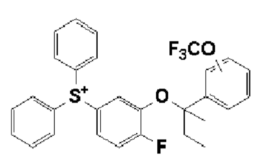 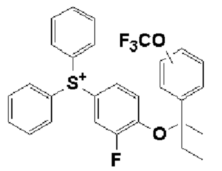 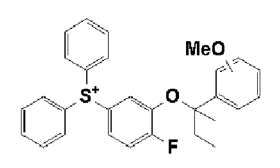 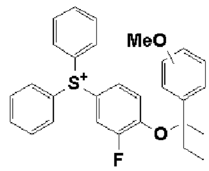

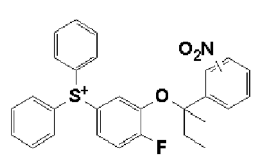 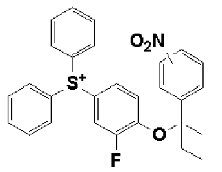 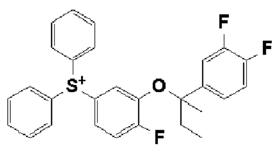 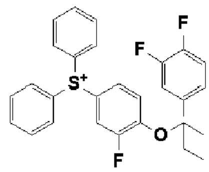

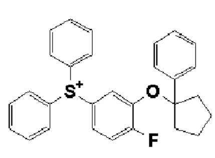 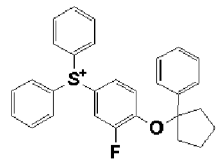 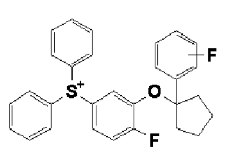 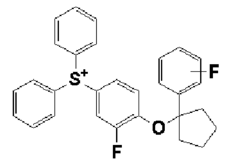

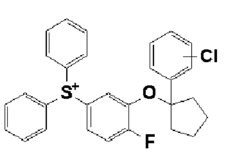 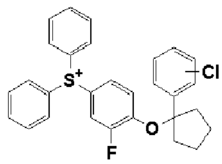 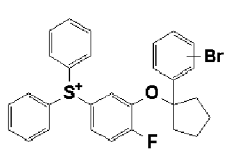 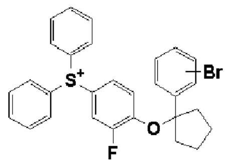

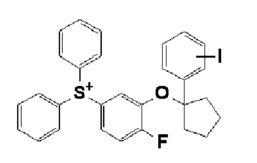 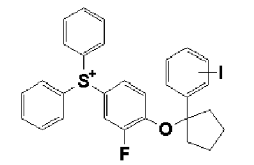 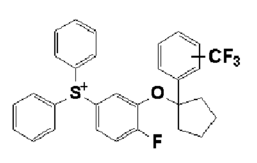 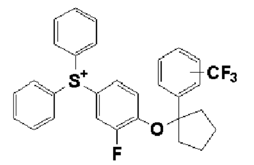

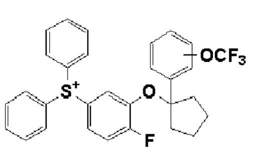 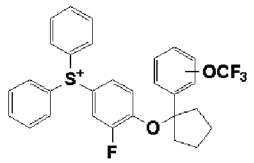 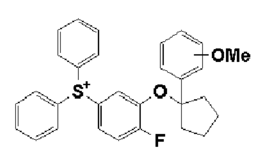 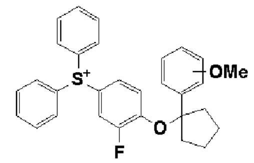

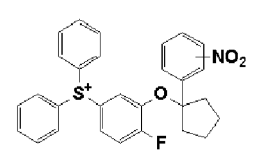 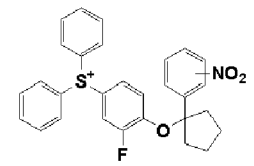 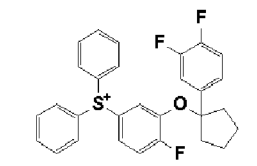 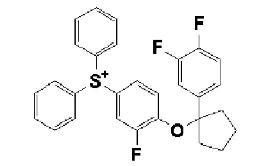

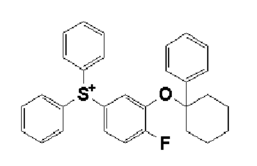 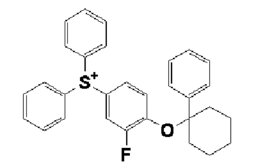 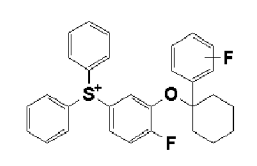 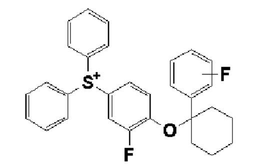

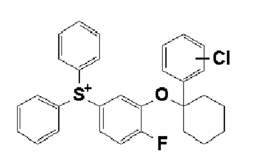 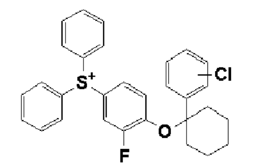 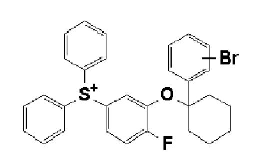 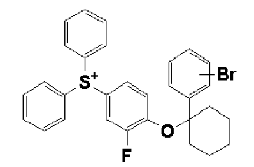

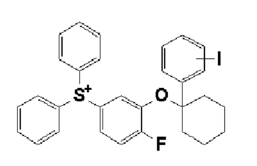 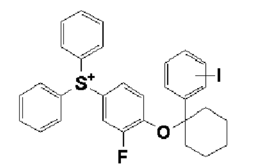 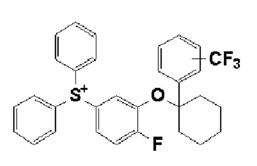 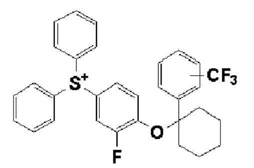

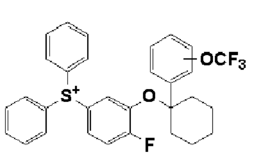 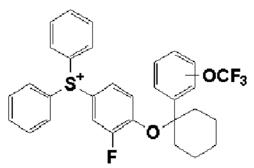 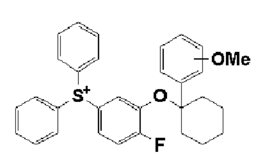 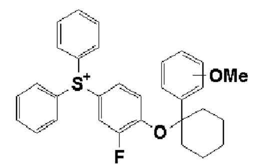

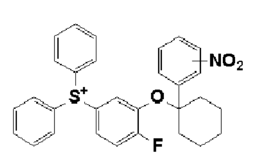 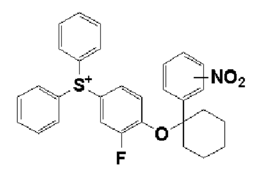 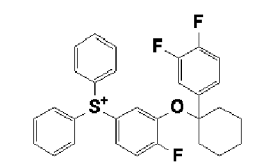 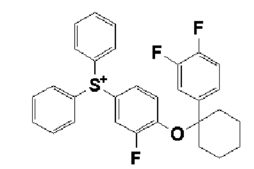

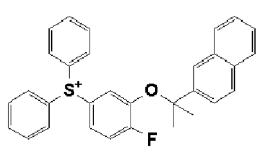
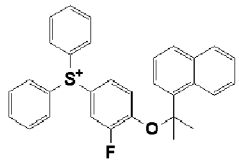
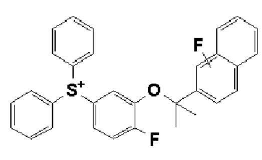
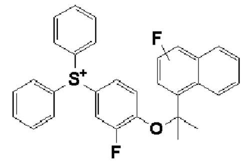

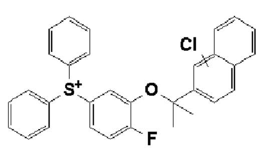
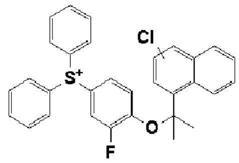
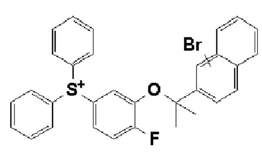
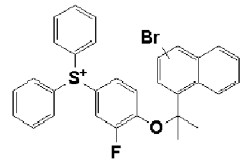

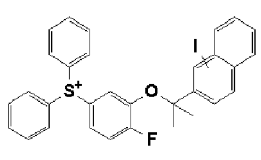
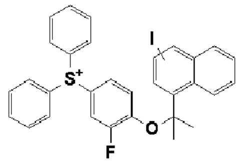
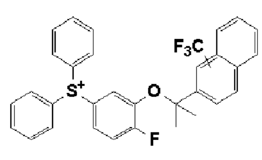
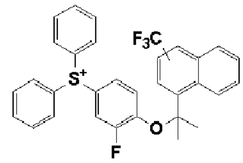

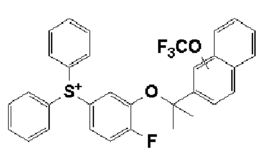
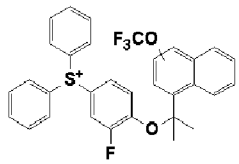
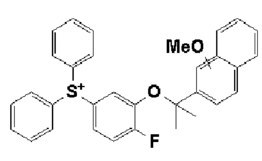
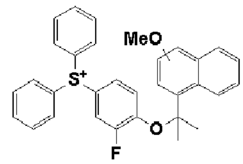

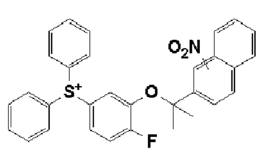
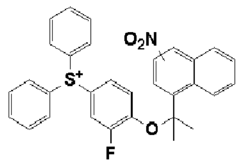
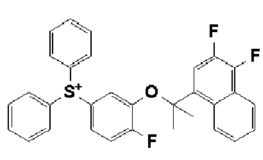
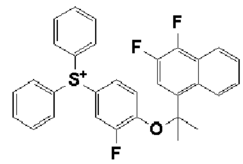

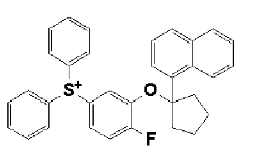
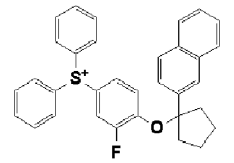
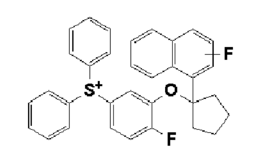
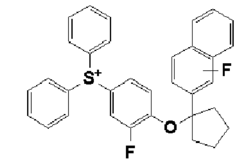

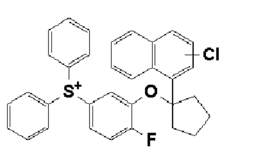
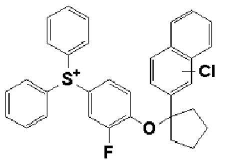
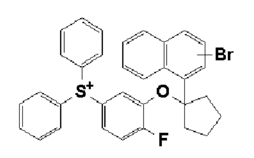
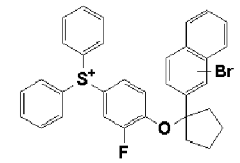

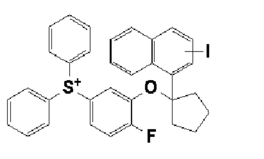
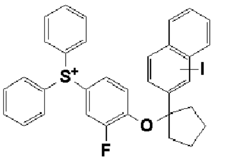
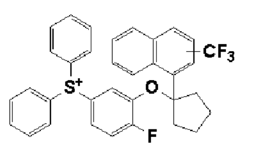
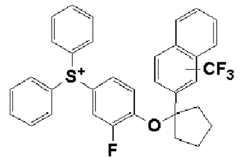

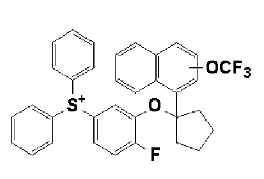
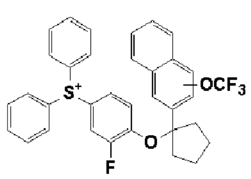
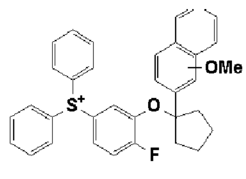
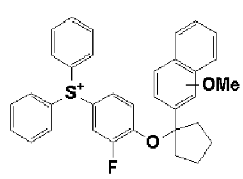

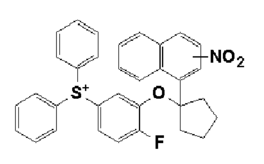
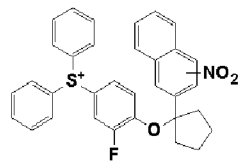
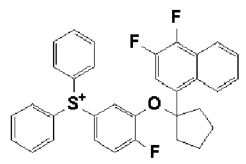
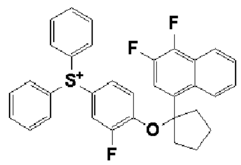

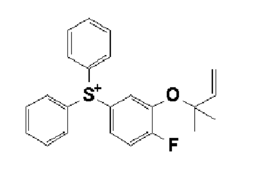
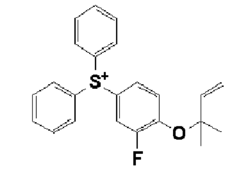
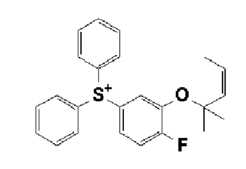
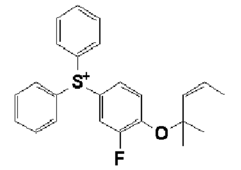

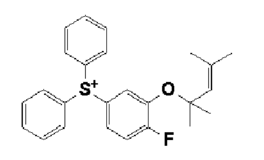
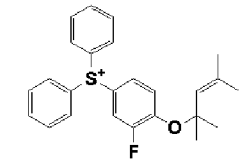
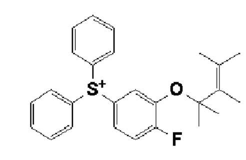
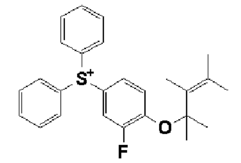

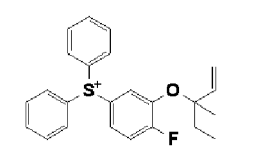
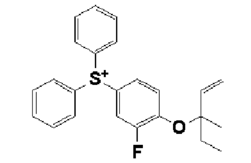
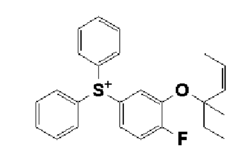
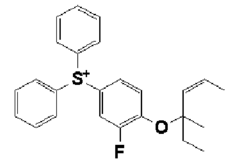

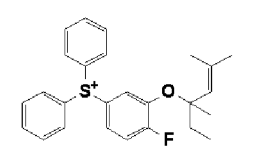
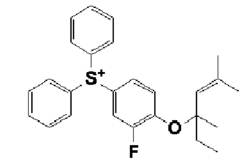
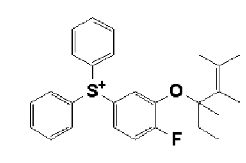
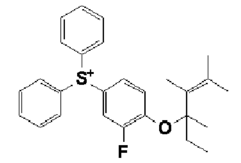

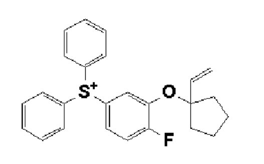
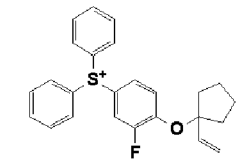
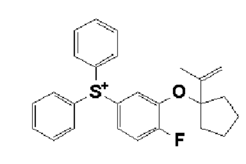
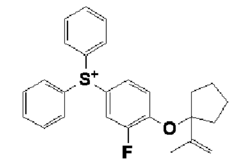

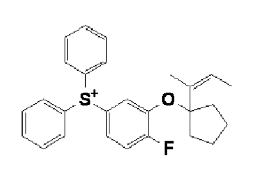
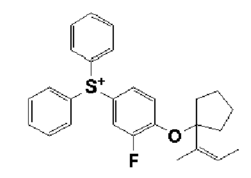
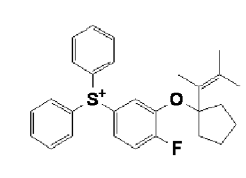
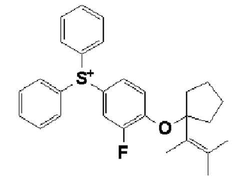

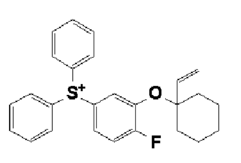
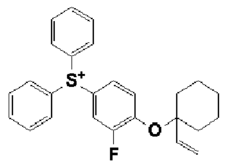
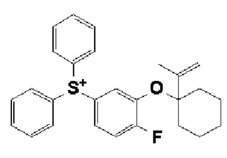
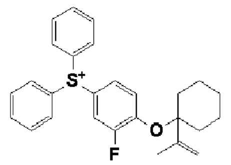

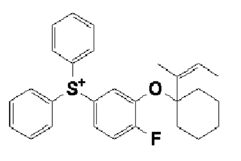
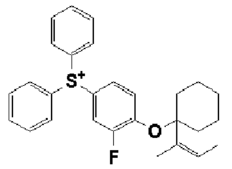
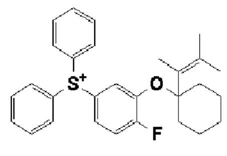
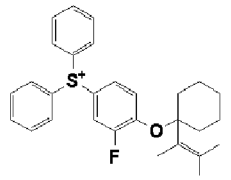

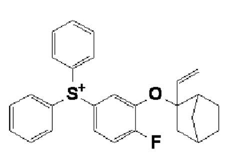
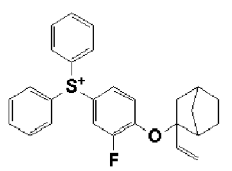
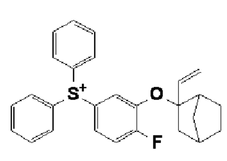
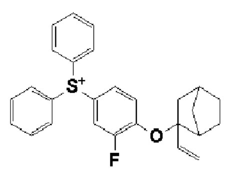

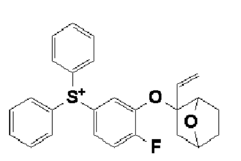
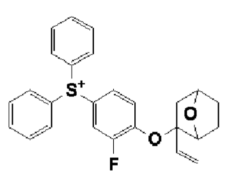
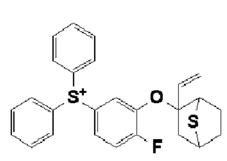
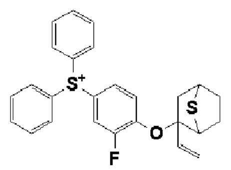

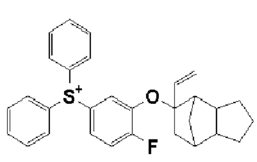
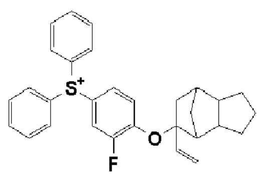
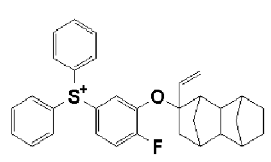
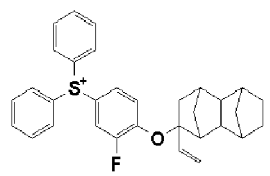

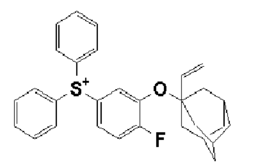
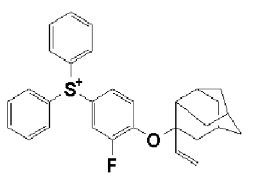
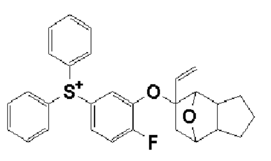
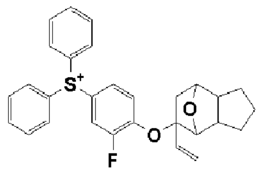

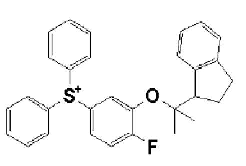
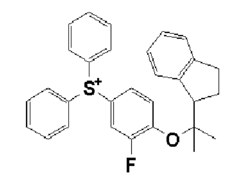
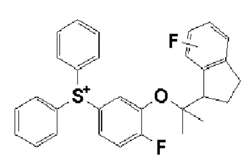
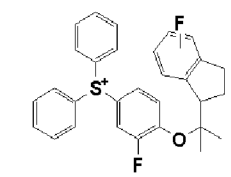

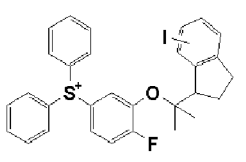
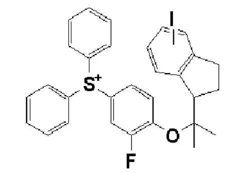
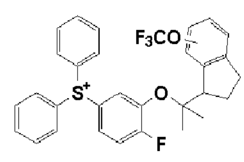
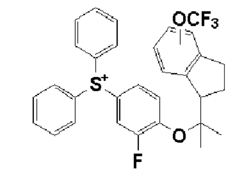

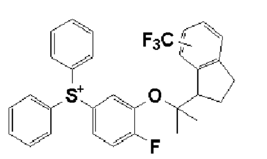 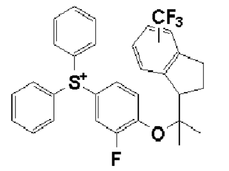 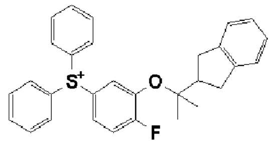 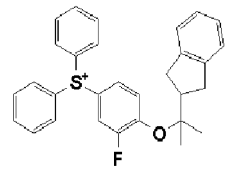

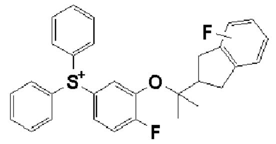 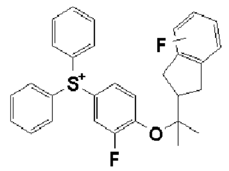 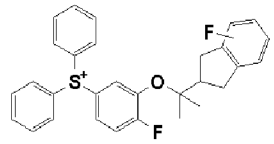 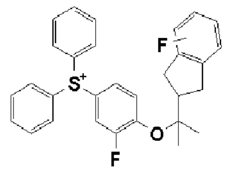

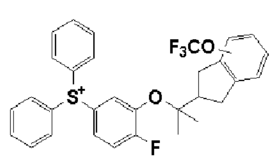 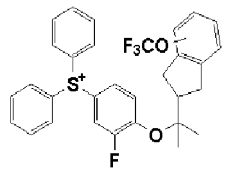 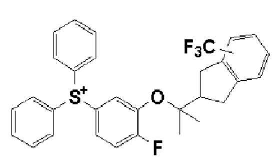 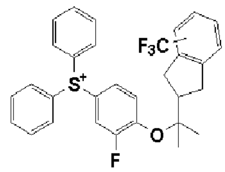

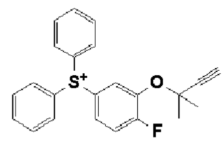 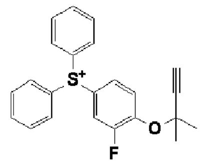 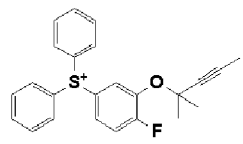 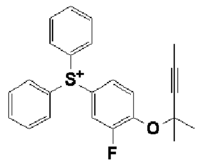

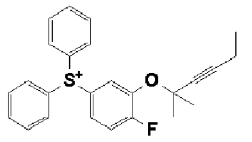 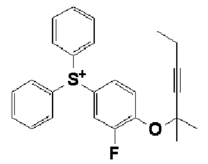 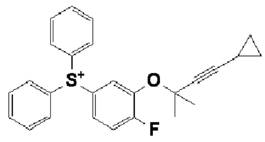 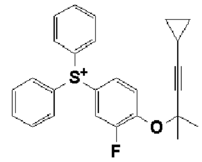

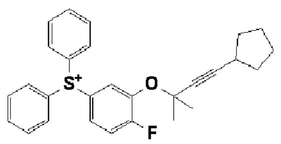 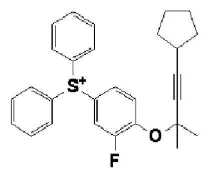 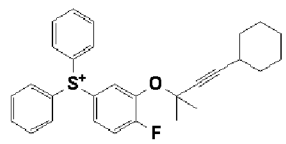 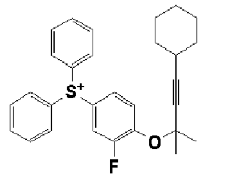

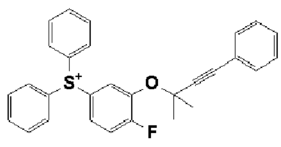 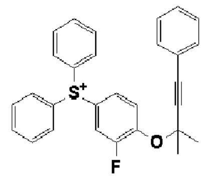 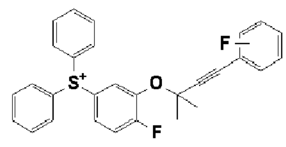 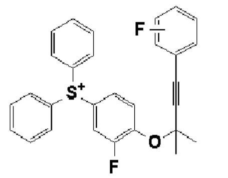

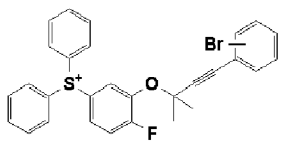 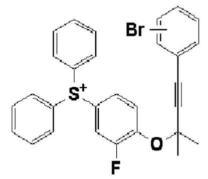 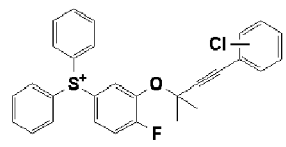 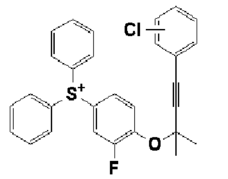

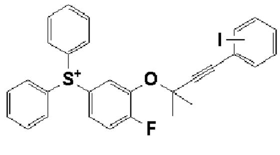
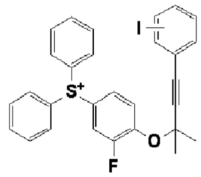
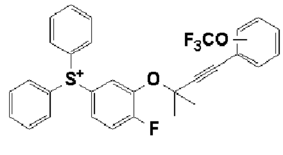
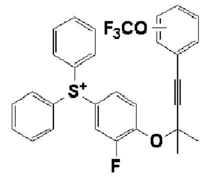

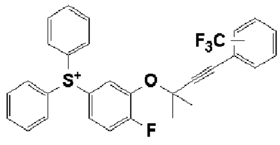
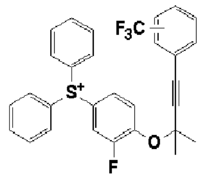
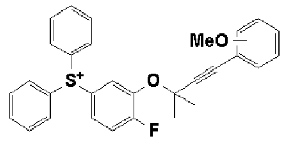
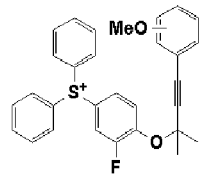

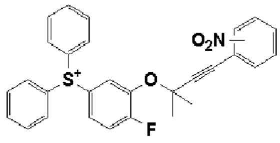
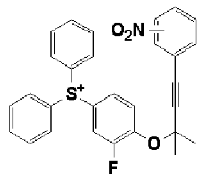
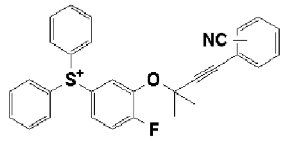
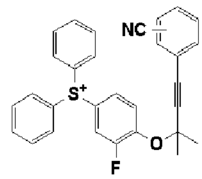

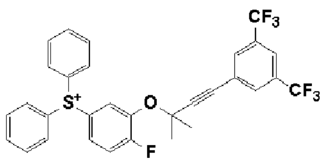
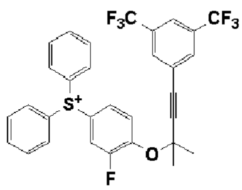
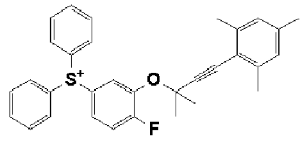
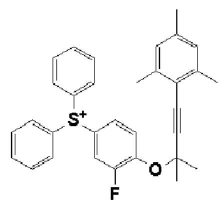

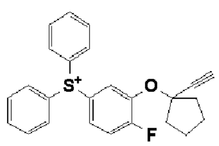
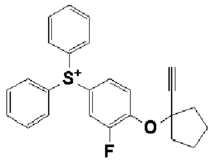
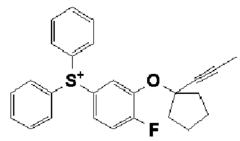
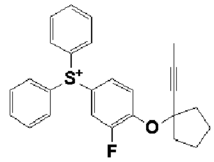

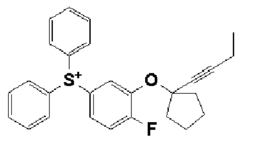
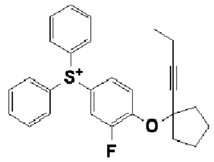
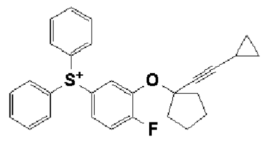
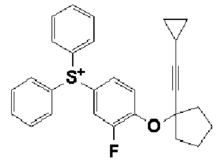

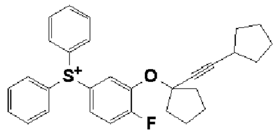
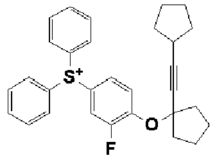
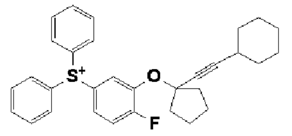
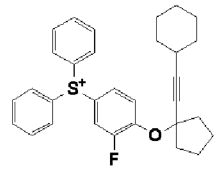

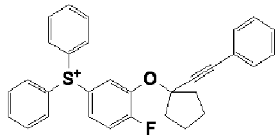
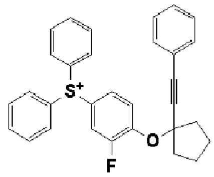
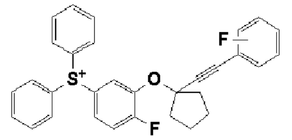
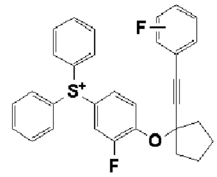

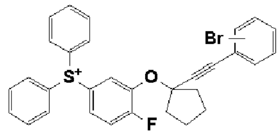
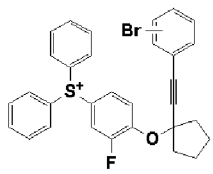
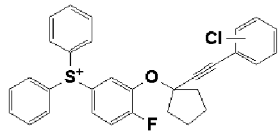
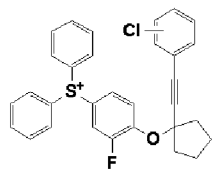

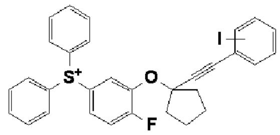
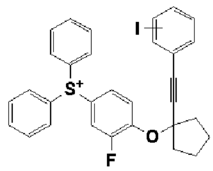
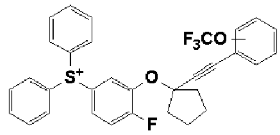
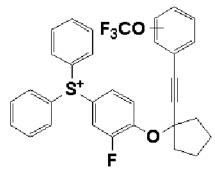

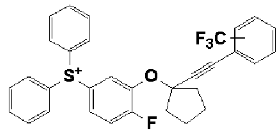
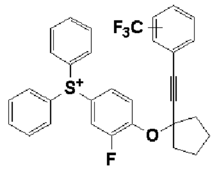
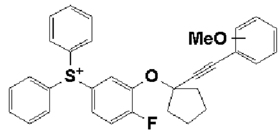
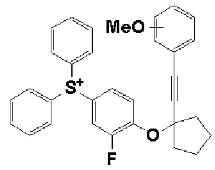

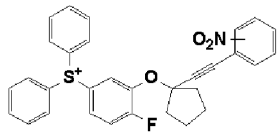
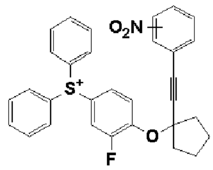
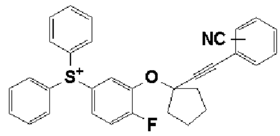
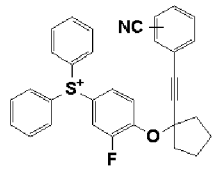

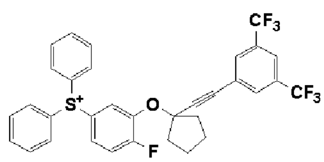
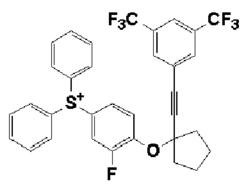
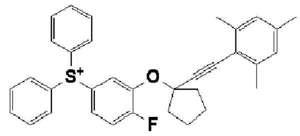
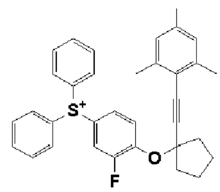

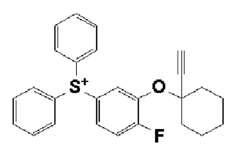
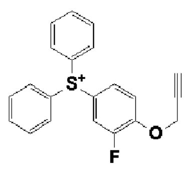
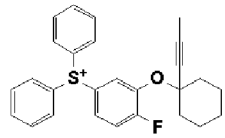
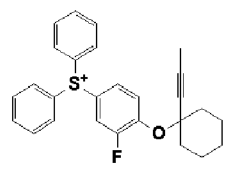

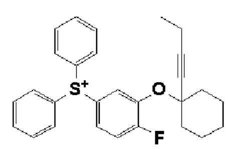
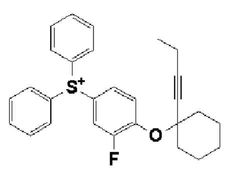
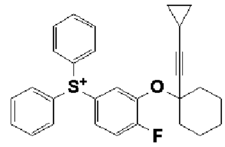
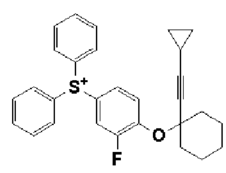

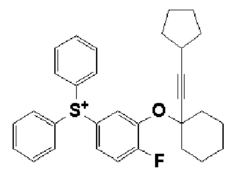
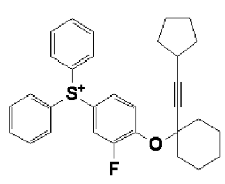
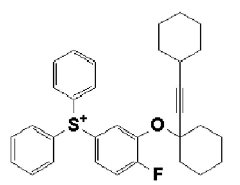
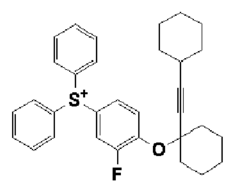

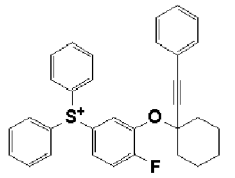 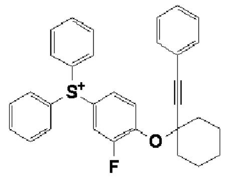 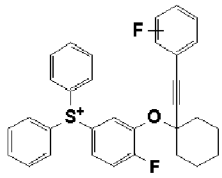 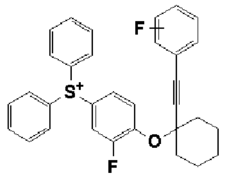

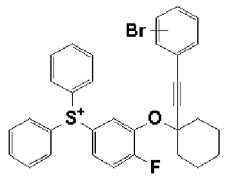 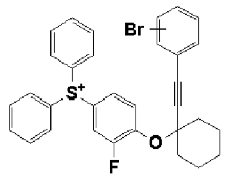 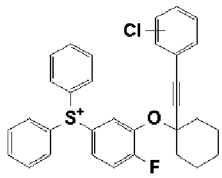 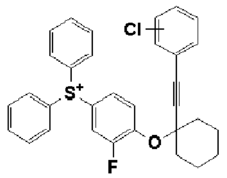

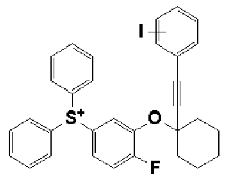 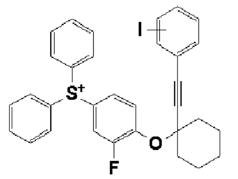 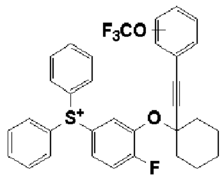 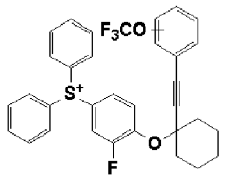

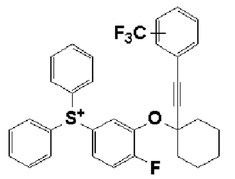 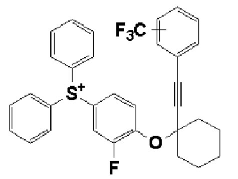 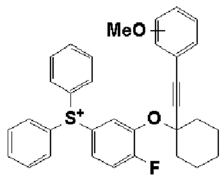 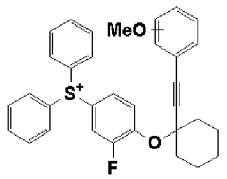

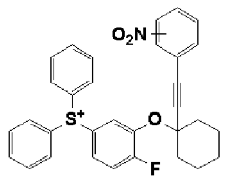 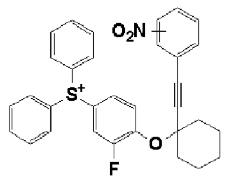 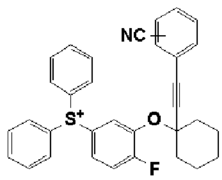 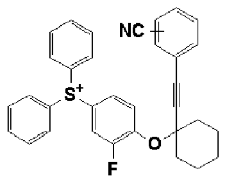

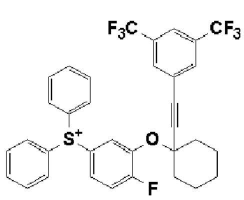 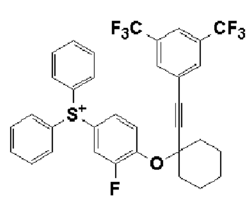 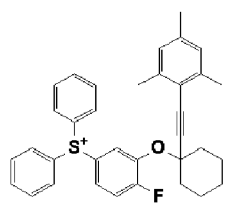 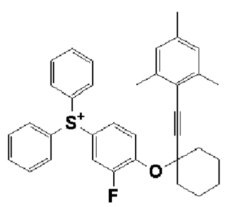

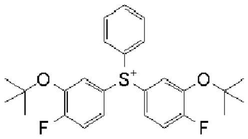 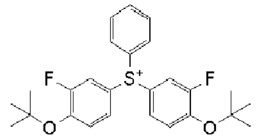 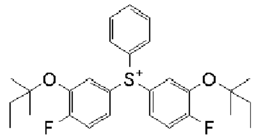 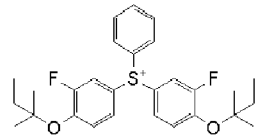

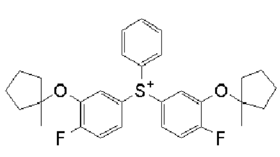 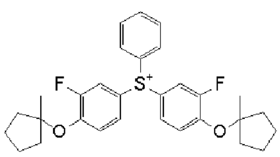 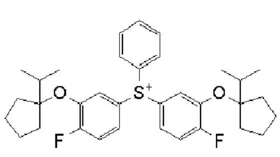 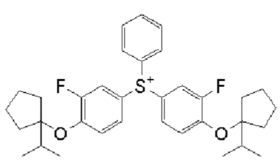

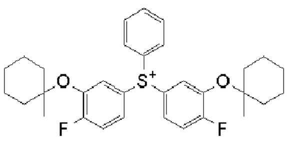 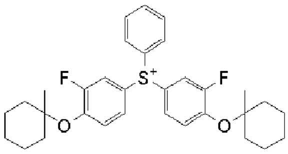 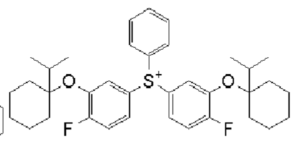 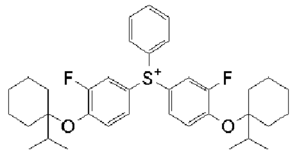

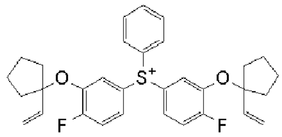 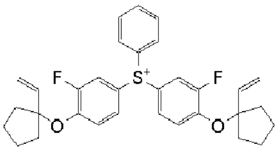 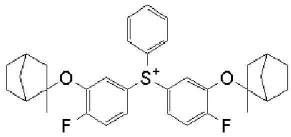 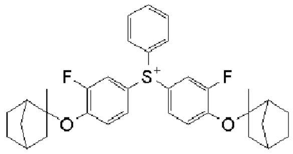

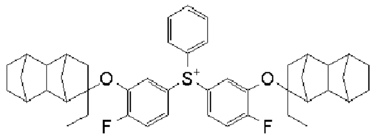 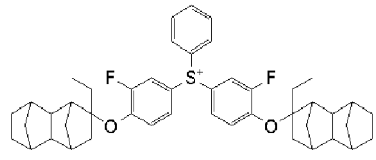

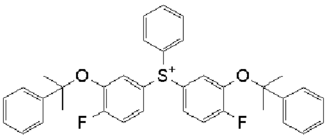 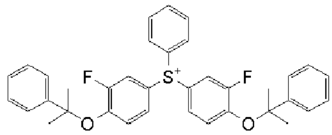 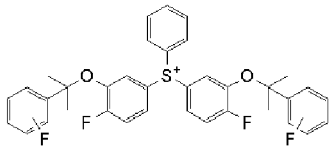

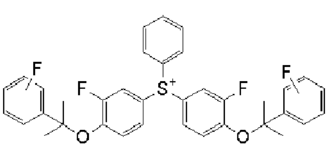 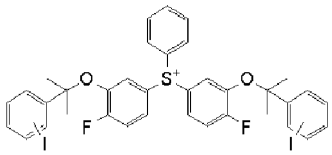 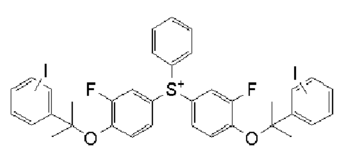

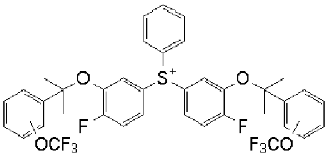 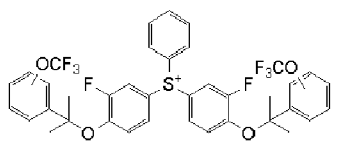 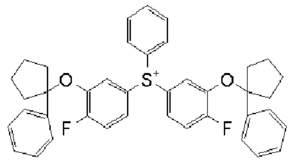

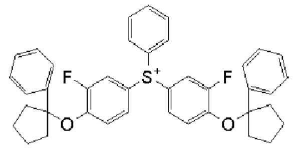 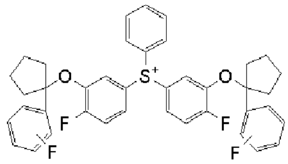 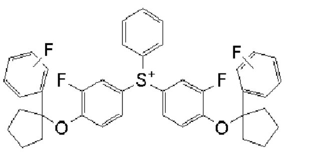

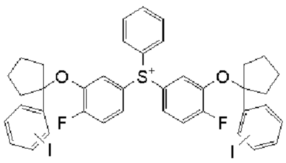 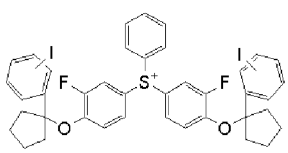 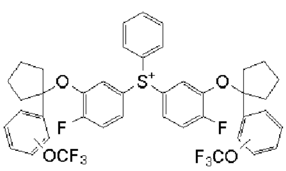 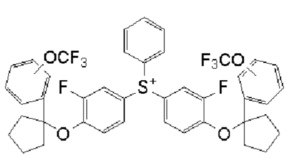

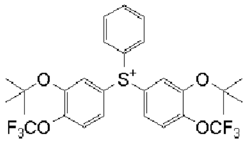 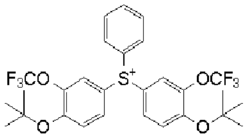 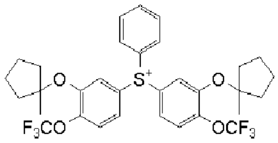 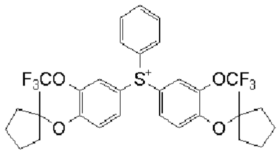

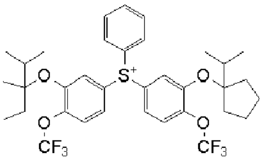 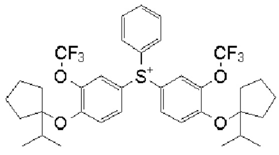 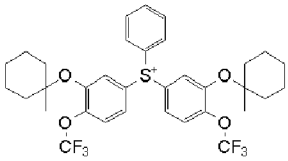 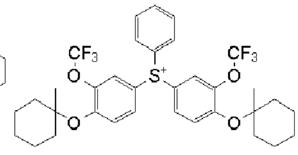

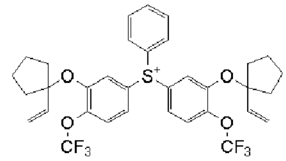 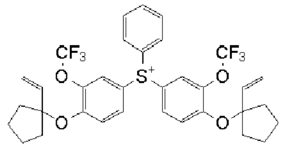 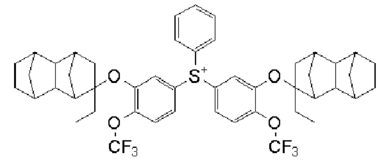

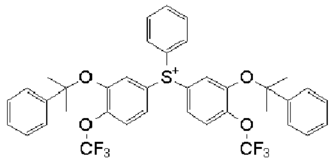 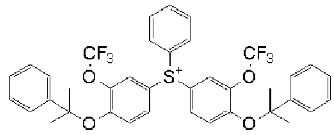 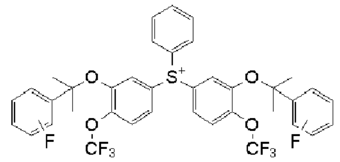

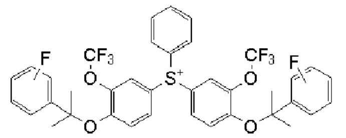 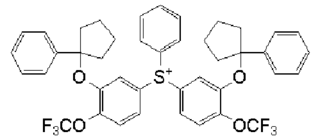 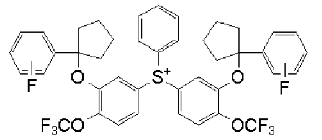

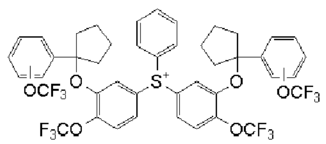 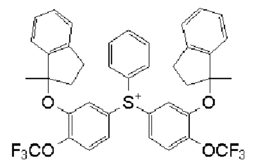 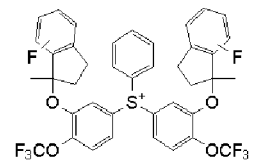

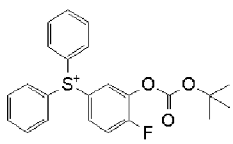 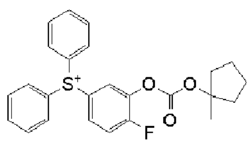 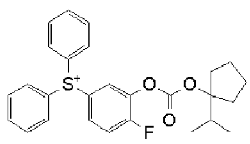 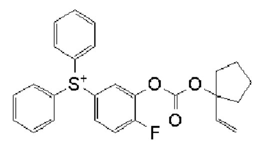

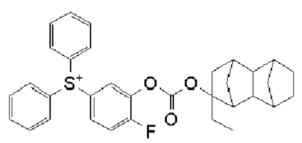 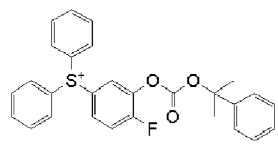 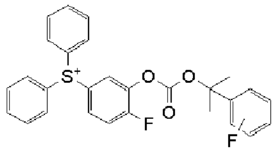 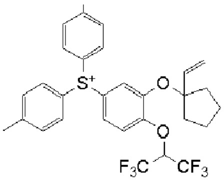

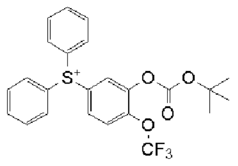 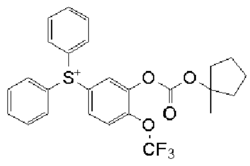 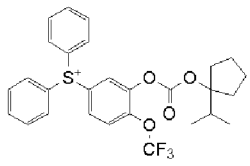 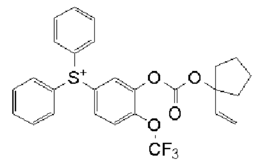

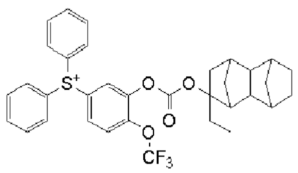 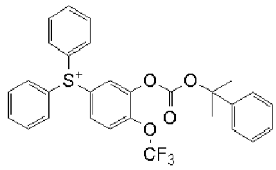 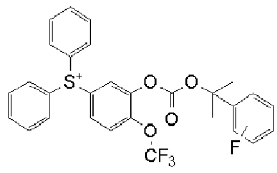 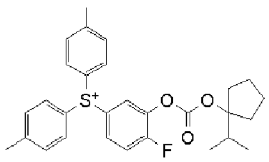

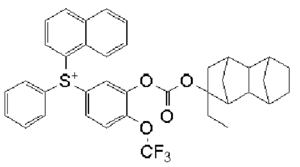 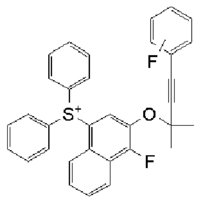 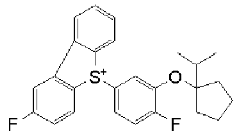 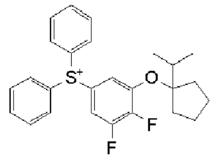

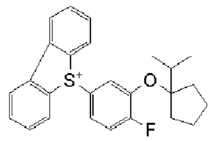 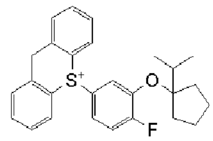 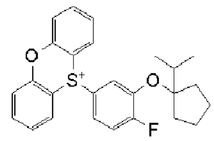 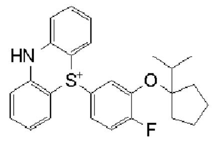

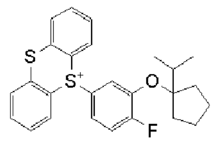 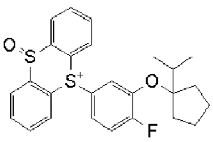 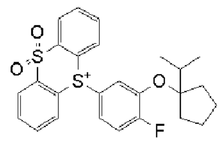 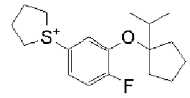

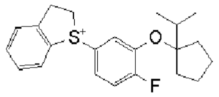 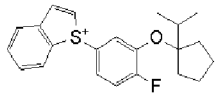 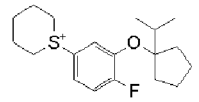 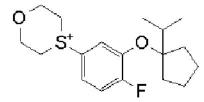

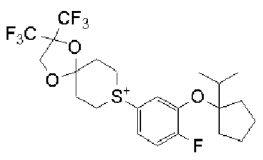 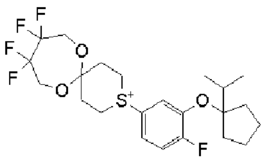 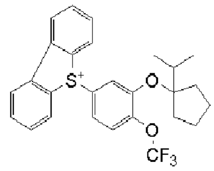 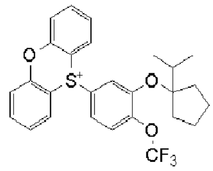

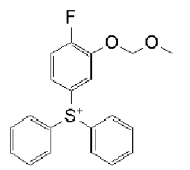 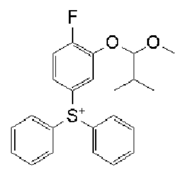 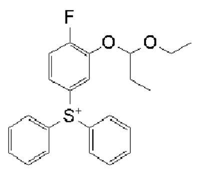 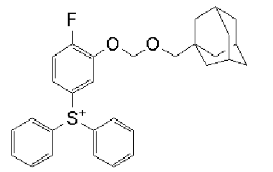

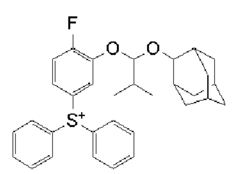 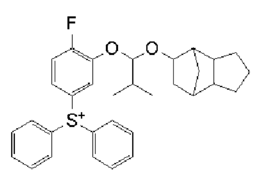 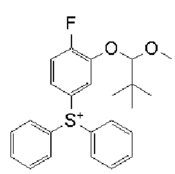 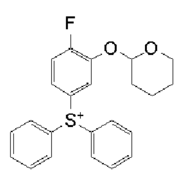

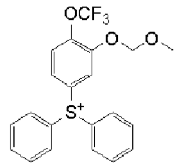
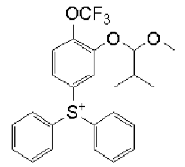
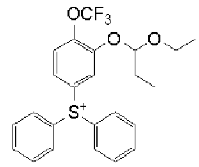
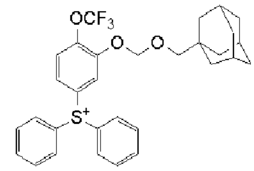

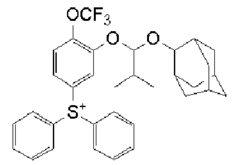
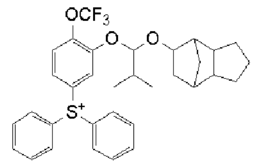
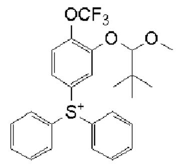
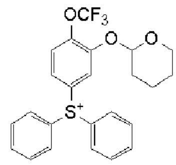

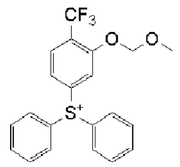
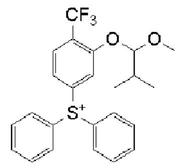
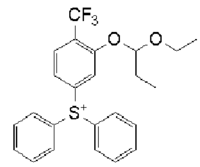
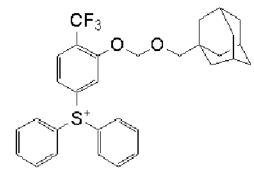

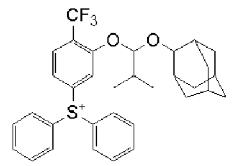
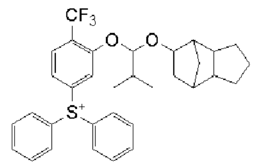
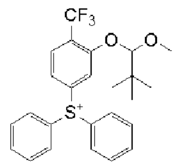
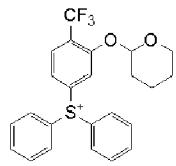

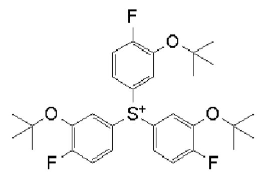
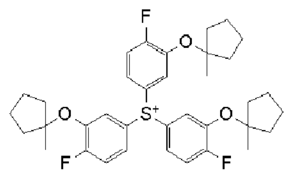
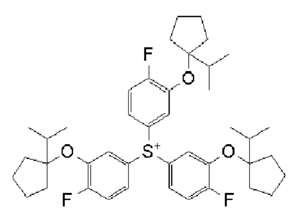
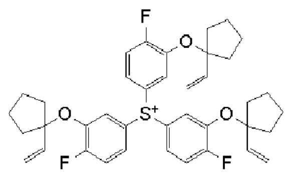

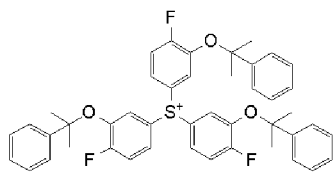
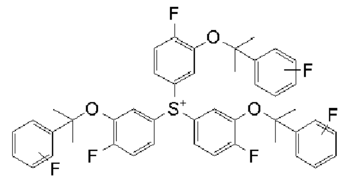
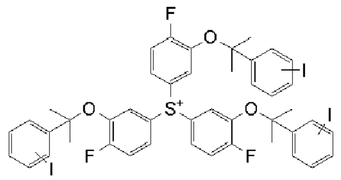

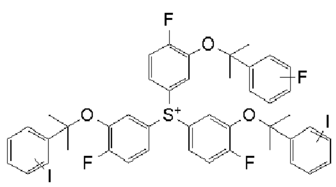
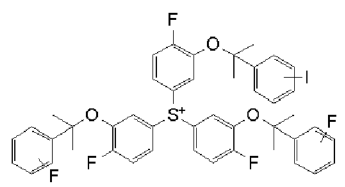
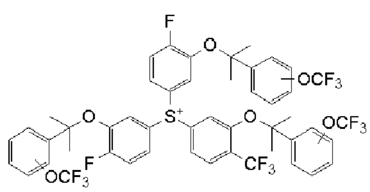

In the formula (1), $X^-$ represents a non-nucleophilic counterion having no polymerizable group. The non-nucleophilic counterion is preferably a sulfonate anion, an imide anion, or a methide anion. Specific examples of the non-nucleophilic counterion, such as the sulfonate anion (sulfonate ion), the imide anion (imide ion), and the methide anion (methide ion), include: halide ions, such as a chloride ion and a bromide ion; fluoroalkylsulfonate ions, such as a triflate ion, a 1,1,1-trifluoroethanesulfonate ion, and a nonafluorobutanesulfonate ion; arylsulfonate ions, such as a tosylate ion, a benzenesulfonate ion, a 4-fluorobenzenesulfonate ion, and a 1,2,3,4,5-pentafluorobenzenesulfonate ion; alkylsulfonate ions, such as a mesylate ion and a butanesulfonate ion; imide ions, such as a bis(trifluoromethylsulfonyl)imide ion, a bis(perfluoroethylsulfonyl)imide ion, and a bis(perfluorobutylsulfonyl)imide ion; and methide ions, such as a tris(trifluoromethylsulfonyl)methide ion and a tris(perfluoroethylsulfonyl)methide ion.

Other examples of the non-nucleophilic counterion include anions selected from the following formulae (1A) to (1D).

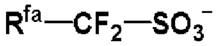 (1A)

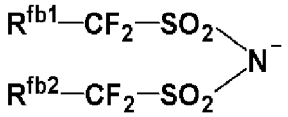 (1B)

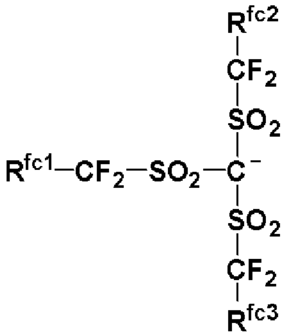 (1C)

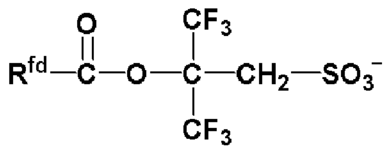 (1D)

In the formula (1A), $R^{fa}$ represents a fluorine atom or a hydrocarbyl group having 1 to 40 carbon atoms and optionally having a heteroatom. The hydrocarbyl group may be a saturated or unsaturated group, and may be any of linear, branched, and cyclic groups. Specific examples thereof include groups same as those exemplified as a hydrocarbyl group represented by $R^{fa1}$ in the formula (1A'), described later.

The anion represented by the formula (1A) is preferably represented by the following formula (1A').

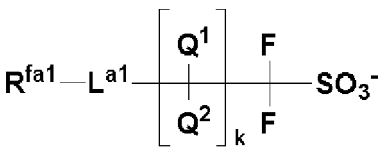 (1A')

In the formula (1A'), $Q^1$ and $Q^2$ each independently represent a hydrogen atom, a fluorine atom, or a fluorinated alkyl group having 1 to 6 carbon atoms. To improve the solvent solubility, at least one of $Q^1$ and $Q^2$ preferably represents a trifluoromethyl group. "k" represents an integer of 0 to 4, and particularly preferably 1. $R^{fa1}$ represents a hydrocarbyl group having 1 to 50 carbon atoms and optionally having a heteroatom. The heteroatom is preferably an oxygen atom, a nitrogen atom, a sulfur atom, a halogen atom, etc., and more preferably an oxygen atom. The hydrocarbyl group particularly preferably has 6 to 30 carbon atoms in terms of obtaining high resolution in fine pattern formation.

The hydrocarbyl group represented by $R^{fa1}$ in the formula (1A') may be a saturated or unsaturated group, and may be any of linear, branched, and cyclic groups. Specific examples thereof include: alkyl groups having 1 to 38 carbon atoms, such as a methyl group, an ethyl group, an n-propyl group, an isopropyl group, a butyl group, an isobutyl group, a sec-butyl group, a tert-butyl group, a pentyl group, a neopentyl group, a hexyl group, a heptyl group, a 2-ethylhexyl group, a nonyl group, an undecyl group, a tridecyl group, a pentadecyl group, a heptadecyl group, and an icosanyl group; cyclic saturated hydrocarbyl groups having 3 to 38 carbon atoms, such as a cyclopentyl group, a cyclohexyl group, a 1-adamantyl group, a 2-adamantyl group, a 1-adamantylmethyl group, a norbornyl group, a norbornylmethyl group, a tricyclodecanyl group, a tetracyclododecanyl group, a tetracyclododecanylmethyl group, and a dicyclohexylmethyl group; unsaturated aliphatic hydrocarbyl groups having 2 to 38 carbon atoms, such as an allyl group and a 3-cyclohexenyl group; aryl groups having 6 to 38 carbon atoms, such as a phenyl group, a 1-naphthyl group, a 2-naphthyl group, and a 9-fluorenyl group; aralkyl groups having 7 to 38 carbon atoms, such as a benzyl group and a diphenylmethyl group; and groups obtained by combining these groups.

A part or all of hydrogen atoms in the hydrocarbyl group are optionally substituted with a group having a heteroatom, such as an oxygen atom, a sulfur atom, a nitrogen atom, and a halogen atom. A part of $-CH_2-$ in the hydrocarbyl group is optionally substituted with a group having a heteroatom, such as an oxygen atom, a sulfur atom, and a nitrogen atom. As a result, optionally contained are a hydroxy group, a fluorine atom, a chlorine atom, a bromine atom, an iodine atom, a cyano group, a nitro group, a carbonyl group, an ether bond, an ester bond, a sulfonate ester bond, a carbonate bond, a lactone ring, a sultone ring, a carboxylic anhydride (-C(=O)-O-C(=O)-), a haloalkyl group, etc. Examples of the hydrocarbyl group having a heteroatom include a tetrahydrofuryl group, a methoxymethyl group,

an ethoxymethyl group, a methylthiomethyl group, an acetamidomethyl group, a trifluoroethyl group, a (2-methoxyethoxy)methyl group, an acetoxymethyl group, a 2-carboxy-1-cyclohexyl group, a 2-oxopropyl group, a 4-oxo-1-adamantyl group, and a 3-oxocyclohexyl group.

In the formula (1A'), $L^{a1}$ represents a single bond, an ether bond, an ester bond, a sulfonate ester bond, a carbonate bond, or a carbamate bond. From the viewpoint of the synthesis, $L^{a1}$ preferably represents an ether bond or an ester bond, and further preferably an ester bond.

Examples of the anion represented by the formula (1A) include the following anions, but the anion is not limited thereto. In the following formulae, $Q^1$ represents the same as above, and Ac represents an acetyl group.

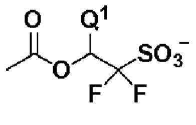
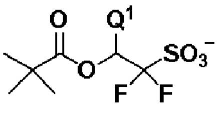
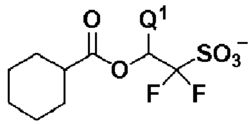
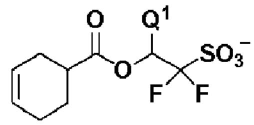

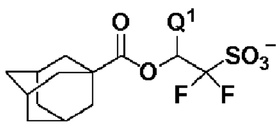
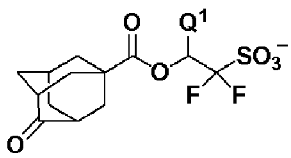
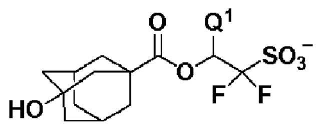

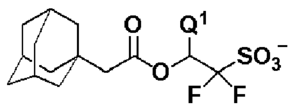
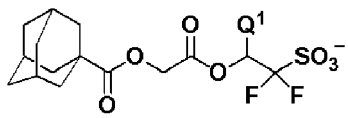
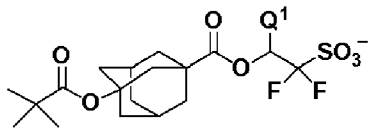

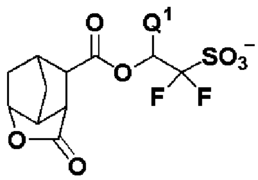
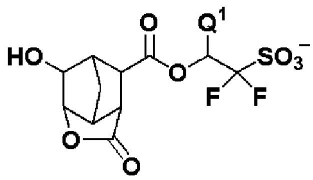
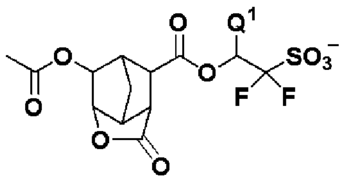

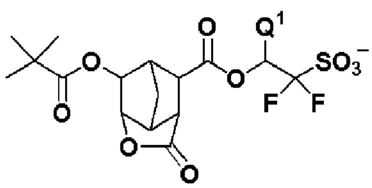
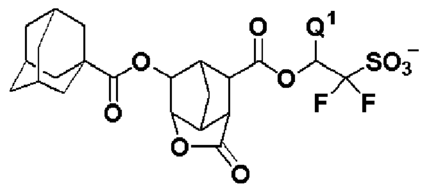

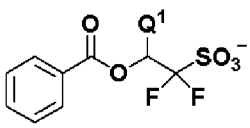
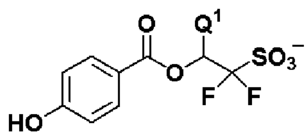
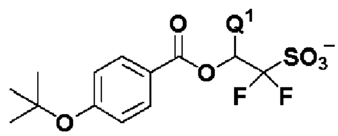

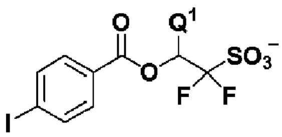
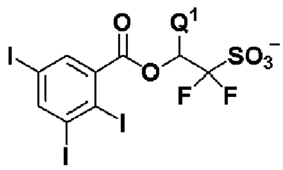
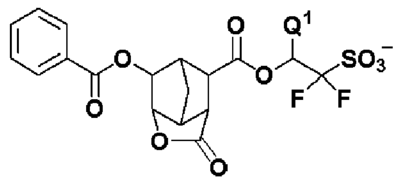

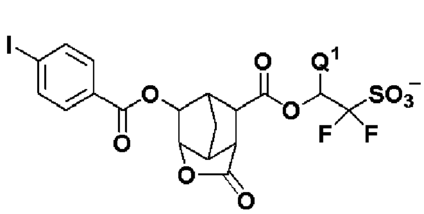
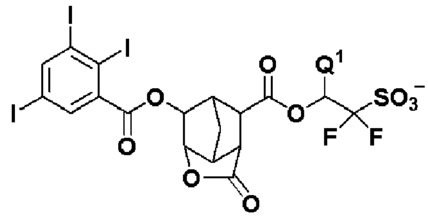

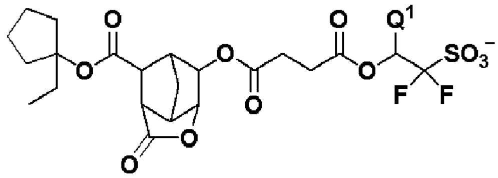

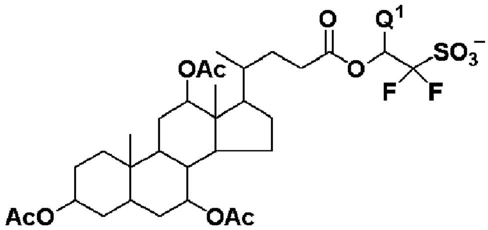
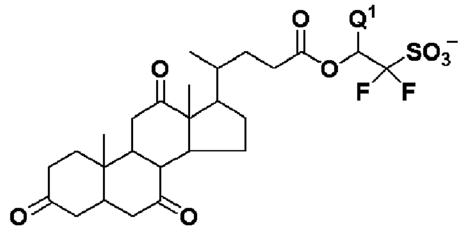

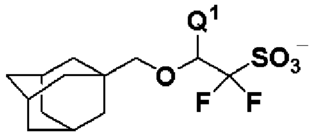
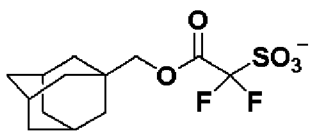
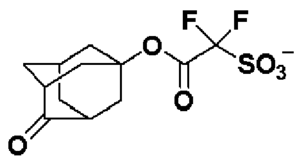

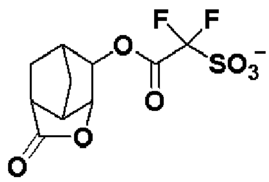
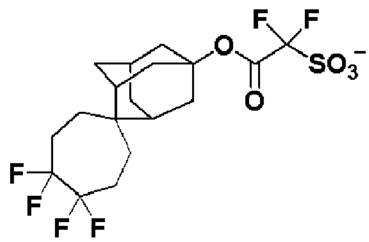

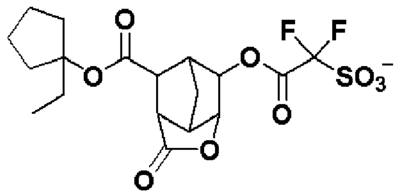
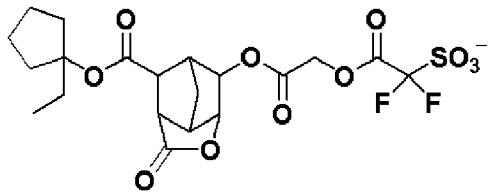

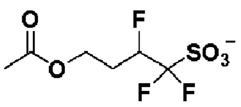
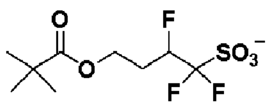
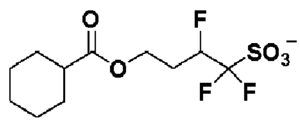

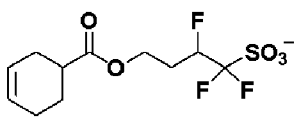
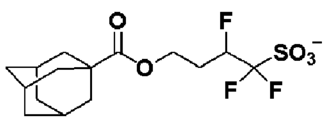
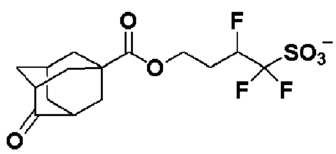

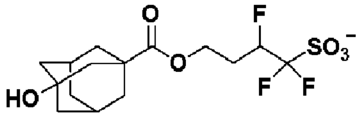
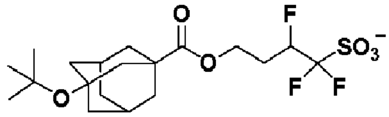

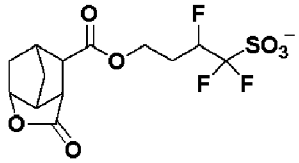
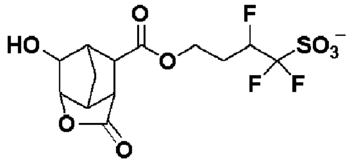
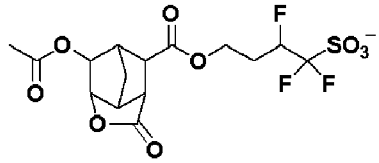

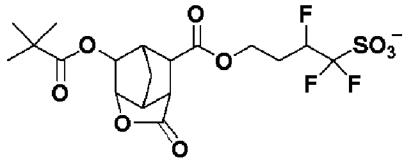
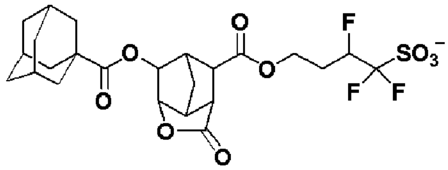

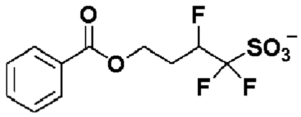
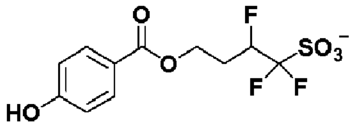
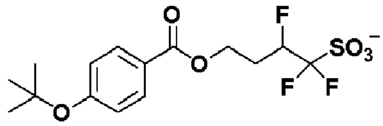

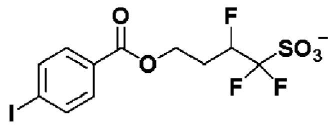
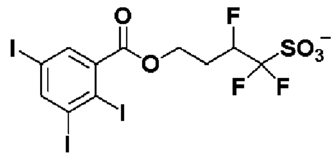

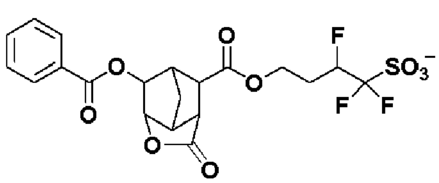
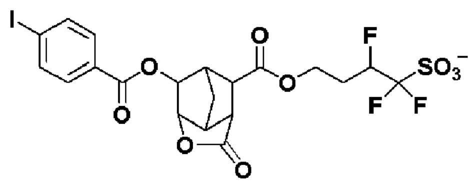

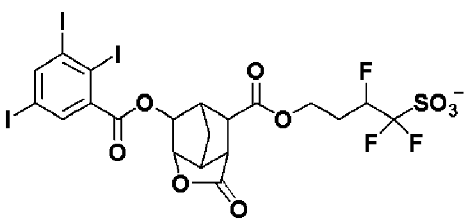
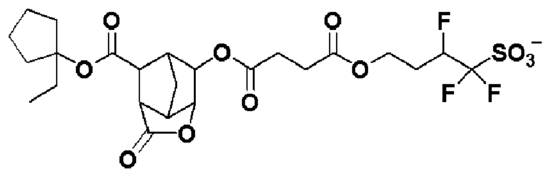

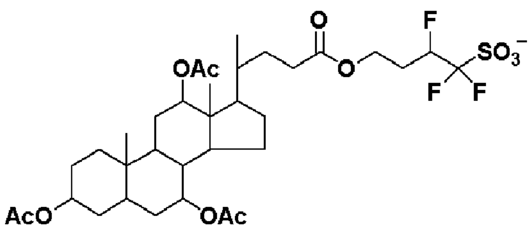
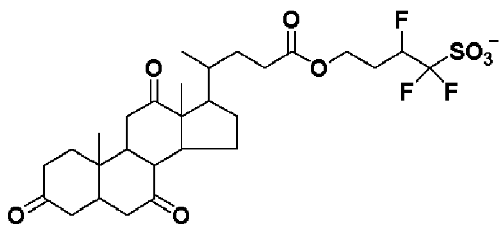

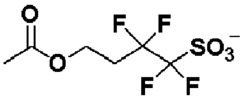 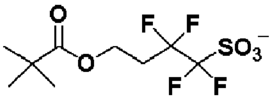 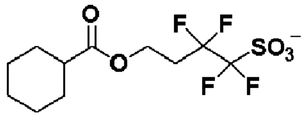

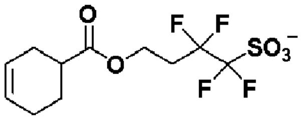 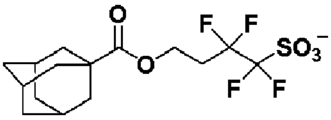 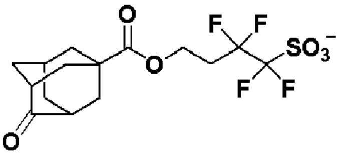

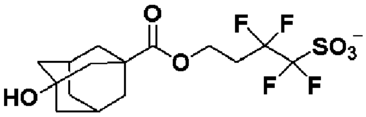 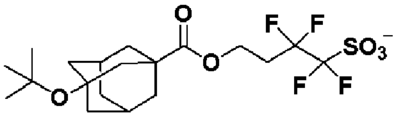

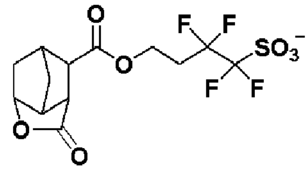 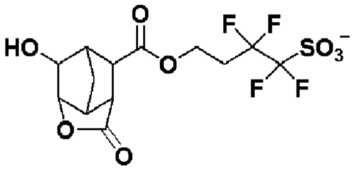 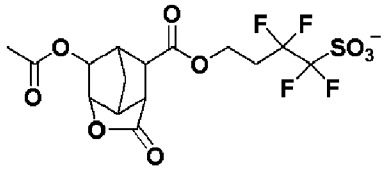

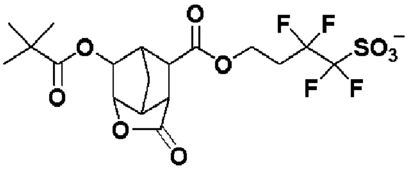 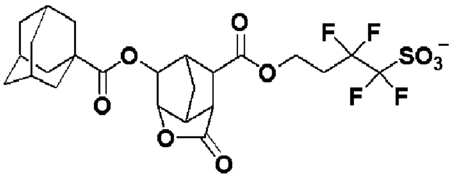

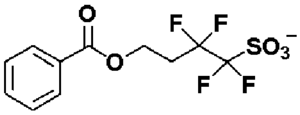 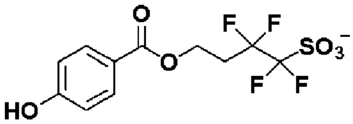 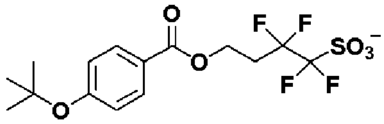

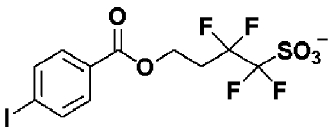 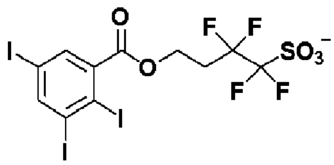

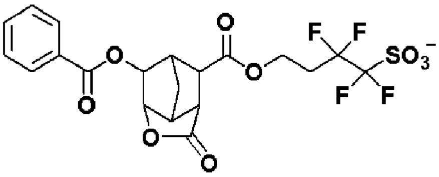 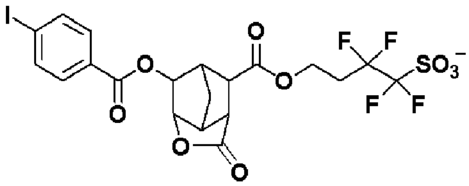

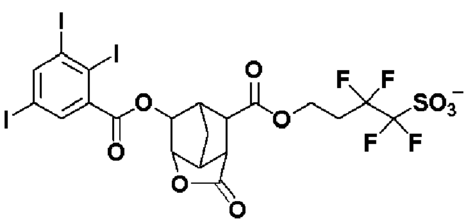 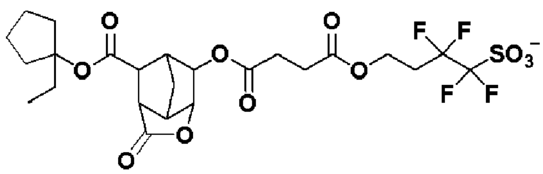

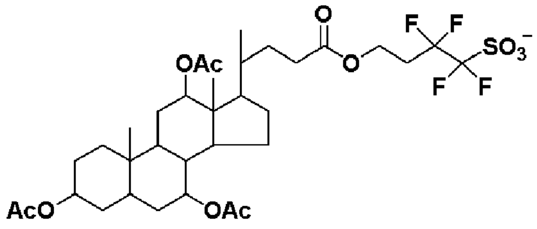 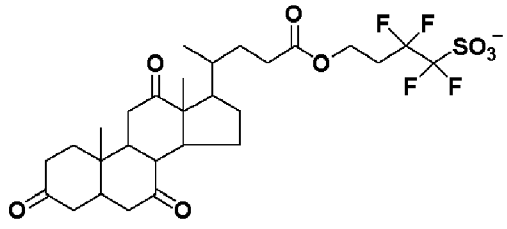

In the formula (1B), $R^{fb1}$ and $R^{fb2}$ each independently represent a fluorine atom, or a hydrocarbyl group having 1 to 40 carbon atoms and optionally having a heteroatom. The hydrocarbyl group may be a saturated or unsaturated group, and may be any of linear, branched, and cyclic groups. Specific examples thereof include groups same as those exemplified as the hydrocarbyl group represented by $R^{fa1}$ in the formula (1A'). $R^{fb1}$ and $R^{fb2}$ preferably represent a fluorine atom or a linear fluorinated alkyl group having 1 to 4 carbon atoms. $R^{fb1}$ and $R^{fb2}$ are optionally bonded each other to form a ring together with a bond ($-CF_2-SO_2-N^--SO_2-CF_2-$) to which $R^{fb1}$ and $R^{fb2}$ are bonded. In this case, the group obtained by bonding $R^{fb1}$ and $R^{fb2}$ each other is preferably a fluorinated ethylene group or a fluorinated propylene group.

In the formula (1C), $R^{fc1}$, $R^{fc2}$, and $R^{fc3}$ each independently represent a fluorine atom, or a hydrocarbyl group having 1 to 40 carbon atoms and optionally having a heteroatom. The hydrocarbyl group may be a saturated or unsaturated group, and may be any of linear, branched, and cyclic groups. Specific examples thereof include groups same as those exemplified as the hydrocarbyl group represented by $R^{fa1}$ in the formula (1A'). $R^{fc1}$, $R^{fc2}$, and $R^{fc3}$ preferably represent a fluorine atom or a linear fluorinated alkyl group having 1 to 4 carbon atoms. $R^{fc1}$ and $R^{fc2}$ are optionally bonded each other to form a ring together with a bond ($-CF_2-SO_2-C^--SO_2-CF_2-$) to which $R^{fc1}$ and $R^{fc2}$ are bonded. In this case, the group obtained by bonding $R^{fc1}$ and $R^{fc2}$ each other is preferably a fluorinated ethylene group or a fluorinated propylene group.

In the formula (1D), $R^{fd}$ represents a hydrocarbyl group having 1 to 40 carbon atoms and optionally having a heteroatom. The hydrocarbyl group may be a saturated or unsaturated group, and may be any of linear, branched, and cyclic groups. Specific examples thereof include groups same as those exemplified as the hydrocarbyl group represented by $R^{fa1}$ in the formula (1A').

Examples of the anion represented by the formula (1D) include the following anions, but the anion is not limited thereto.

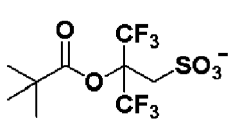 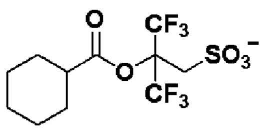 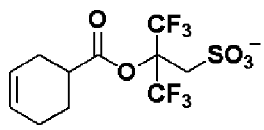

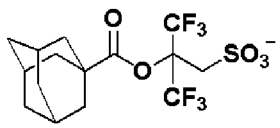 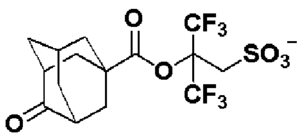 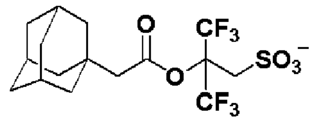

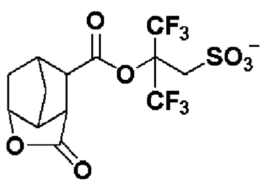 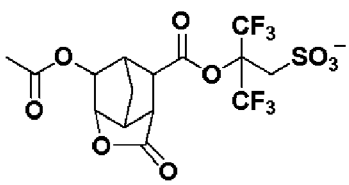 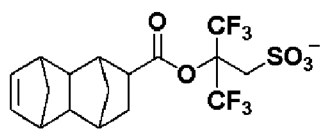

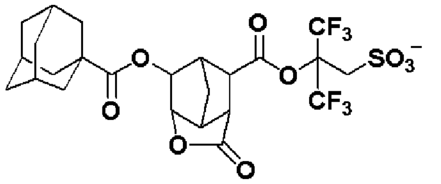

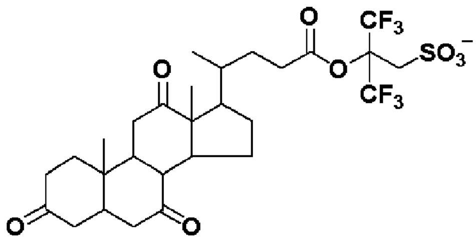

Examples of the non-nucleophilic counterion further include an anion having an aromatic ring substituted with an iodine atom or a bromine atom. Examples of such an anion include anions represented by the following formula (1E).

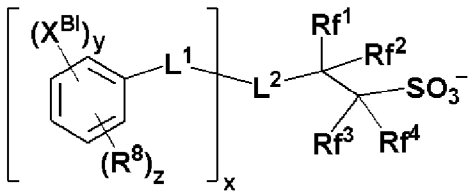

(1E)

In the formula (1E), "x" represents an integer satisfying $1 \leq x \leq 3$. "y" and "z" represent an integer satisfying $1 \leq y \leq 5$, $0 \leq z \leq 3$, and $1 \leq y + z \leq 5$. "y" preferably represents an integer satisfying $1 \leq y \leq 3$, and more preferably 2 or 3. "z" preferably represents an integer satisfying $0 \leq z \leq 2$.

In the formula (1E), $X^{BI}$ represents an iodine atom or a bromine atom. When "x" and/or "y" represent 2 or more, $X^{BI}$ may be same as or different from each other.

In the formula (1E), $L^1$ represents a single bond, an ether bond, an ester bond, or a saturated hydrocarbylene group having 1 to 6 carbon atoms and optionally having an ether bond or an ester bond. The saturated hydrocarbylene group may be any of linear, branched, and cyclic groups.

In the formula (1E), $L^2$ represents a single bond or a divalent linking group having 1 to 20 carbon atoms when "x" represents 1, and $L^2$ represents a (x + 1)-valent linking group having 1 to 20 carbon atoms when "x" represents 2 or 3. The linking group optionally has an oxygen atom, a sulfur atom, or a nitrogen atom.

In the formulae (1E), $R^8$ represents a hydroxy group, a carboxy group, a fluorine atom, a chlorine atom, a bromine atom, an amino group, a hydrocarbyl group having 1 to 20 carbon atoms, a hydrocarbyloxy group having 1 to 20 carbon atoms, a hydrocarbylcarbonyl group having 2 to 20 carbon atoms, a hydrocarbyloxycarbonyl group having 2 to 10 carbon atoms, a hydrocarbylcarbonyloxy group having 2 to 20 carbon atoms, a hydrocarbylsulfonyloxy group having 1 to 20 carbon atoms, $-N(R^{8A})(R^{8B})$, $-N(R^{8C})-C(=O)-R^{8D}$, or $-N(R^{8C})-C(=O)-OR^{8D}$. The hydrocarbyl group, the hydrocarbyloxy group, the hydrocarbylcarbonyl group, the hydrocarbyloxycarbonyl group, the hydrocarbylcarbonyloxy group, and the hydrocarbylsulfonyloxy group optionally have a fluorine atom, a chlorine atom, a bromine atom, a hydroxy group, an amino group, or an ether bond. $R^{8A}$ and $R^{8B}$ each independently represent a hydrogen atom or a saturated hydrocarbyl group having 1 to 6 carbon atoms. $R^{8C}$ represents a hydrogen atom or a saturated hydrocarbyl group having 1 to 6 carbon atoms and optionally having a halogen atom, a hydroxy group, a saturated hydrocarbyloxy group having 1 to 6 carbon atoms, a saturated hydrocarbylcarbonyl group having 2 to 6 carbon atoms, or a saturated hydrocarbylcarbonyloxy group having 2 to 6 carbon atoms. $R^{8D}$ represents an aliphatic hydrocarbyl group having 1 to 16 carbon atoms, an aryl group having 6 to 12 carbon atoms, or an aralkyl group having 7 to 15 carbon atoms, and optionally having a halogen atom, a hydroxy group, a saturated hydrocarbyloxy group having 1 to 6 carbon atoms, a saturated hydrocarbylcarbonyl group having 2 to 6 carbon atoms, or a saturated hydrocarbylcarbonyloxy group having 2 to 6 carbon atoms. The aliphatic hydrocarbyl group may be a saturated or unsaturated group, and may be any of linear, branched, and cyclic groups. The hydrocarbyl group, the hydrocarbyloxy group, the hydrocarbylcarbonyl group, the hydrcarbyloxycarbonyl group, the hydrocarbylcarbonyloxy group, and the hydrocarbylsulfonyloxy group may be any of linear, branched, and cyclic groups. When "x" and/or "z" represent 2 or more, each $R^8$ may be same as or different from each other.

Among these, $R^8$ preferably represents a hydroxy group, $-N(R^{8C})-C(=O)-R^{8D}$, $-N(R^{8C})-C(=O)-O-R^{8D}$, a fluorine atom, a chlorine atom, a bromine atom, a methyl group, and a methoxy group, etc.

In the formula (1E), $Rf^1$ to $Rf^4$ each independently represent a hydrogen atom, a fluorine atom, or a trifluoromethyl group, and at least one of them represents a fluorine atom or a trifluoromethyl group. $Rf^1$ and $Rf^2$ are optionally integrated to form a carbonyl group. In particular, both of $Rf^3$ and $Rf^4$ preferably represent fluorine atoms. $Rf^1$ to $Rf^4$ herein are applied only in the formula (1E).

Examples of the anion of the onium salt represented by the formula (1E) include the following anions, but the anion is not limited thereto. In the following formulae, $X^{BI}$ represents the same as above.

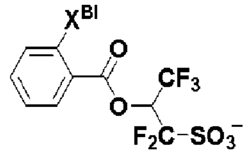 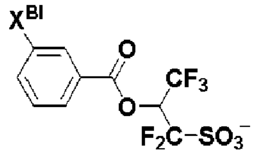 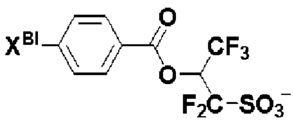 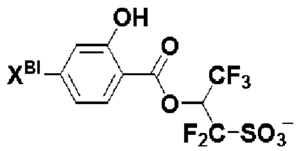

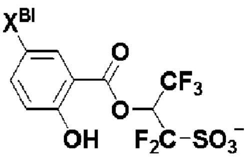 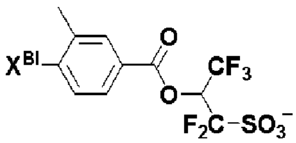 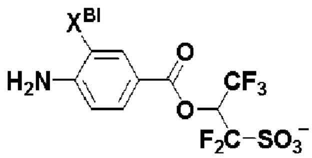 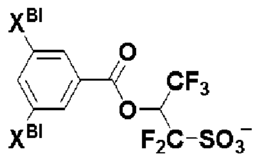

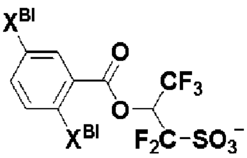 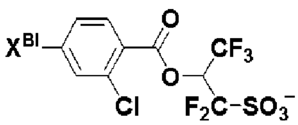 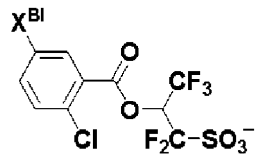 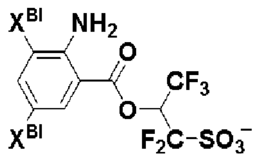

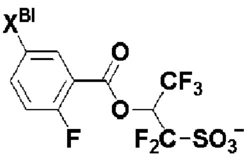 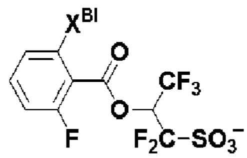 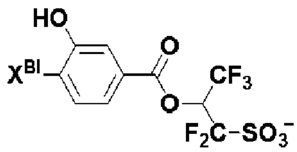 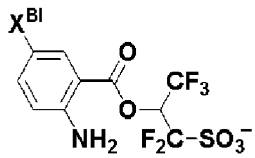

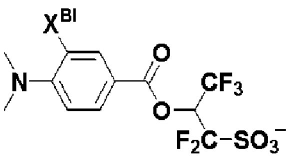 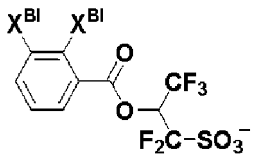 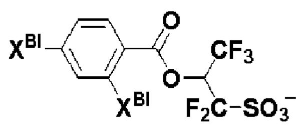 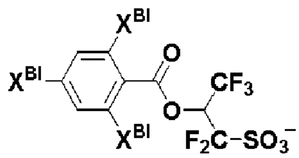

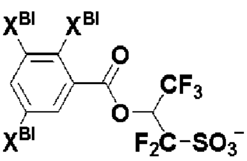 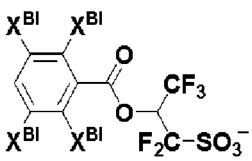 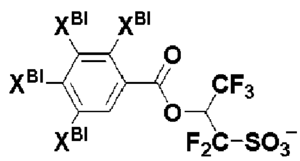 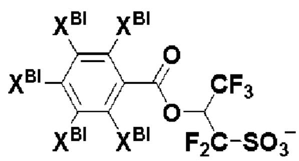

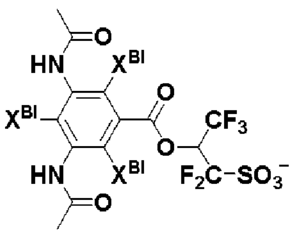 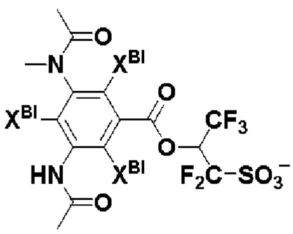 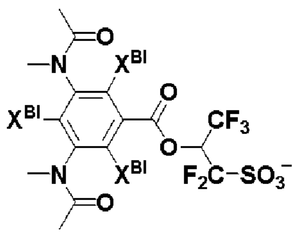

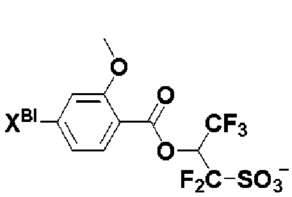 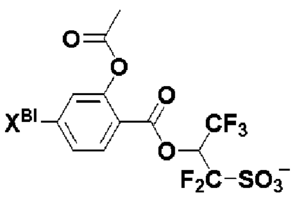 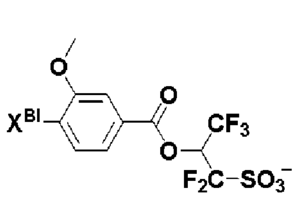

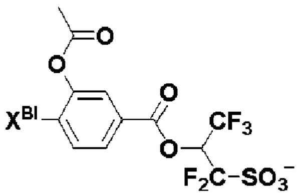
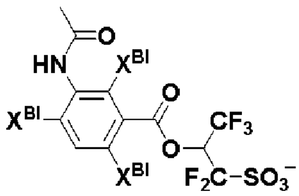

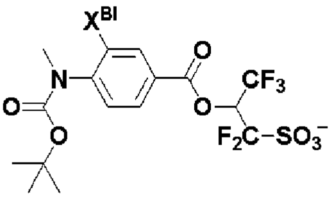
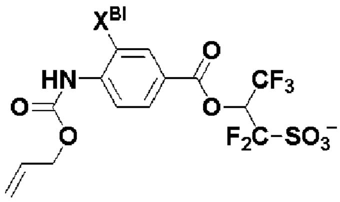
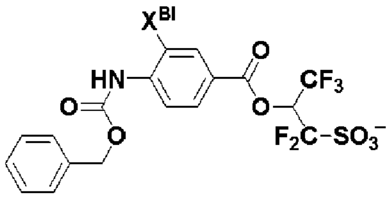

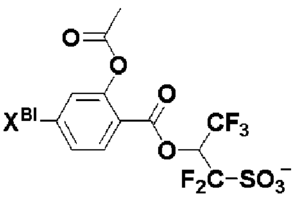
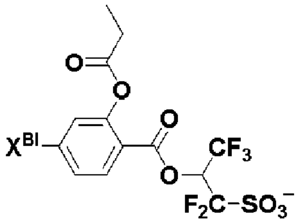
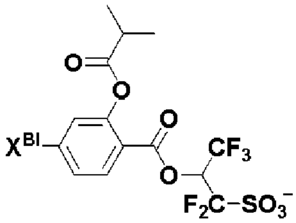

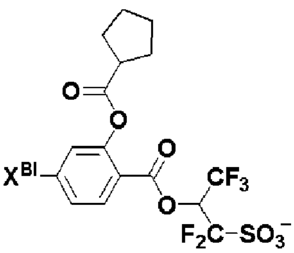
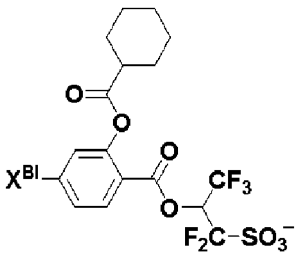
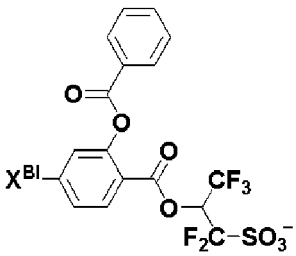

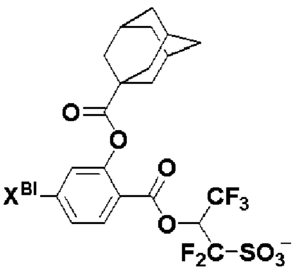
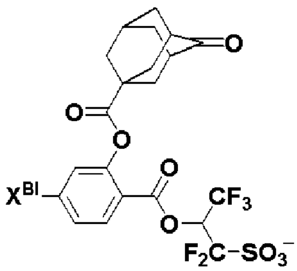
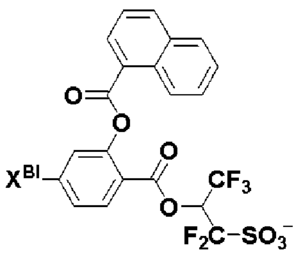

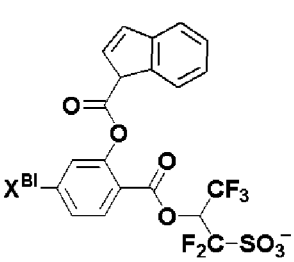
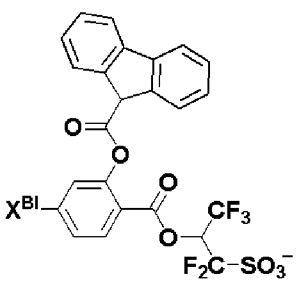
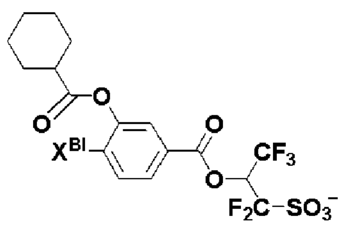

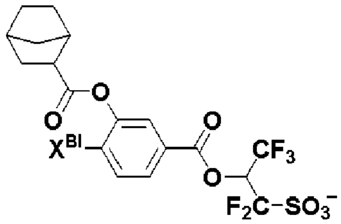 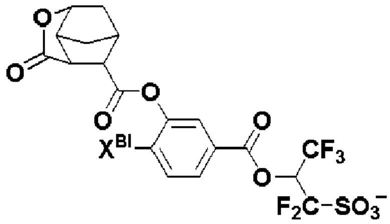

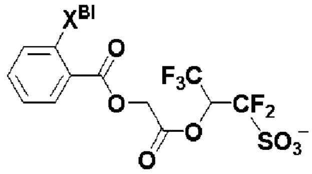 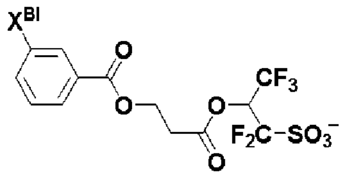 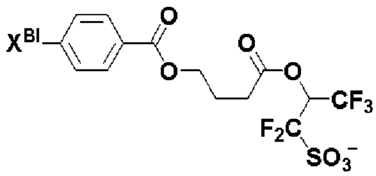

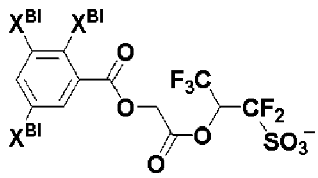 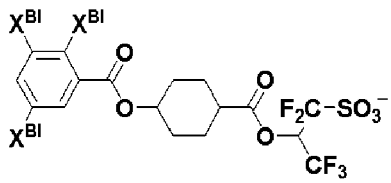

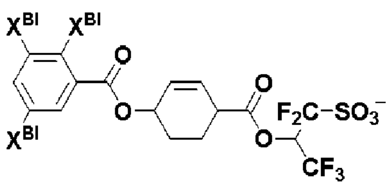 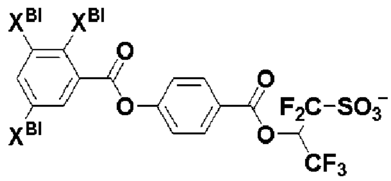

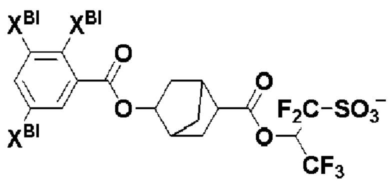 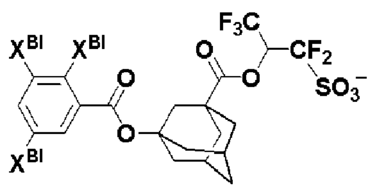

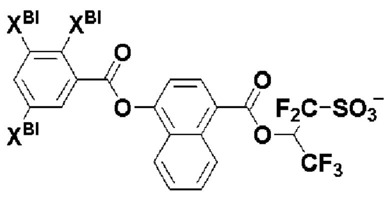 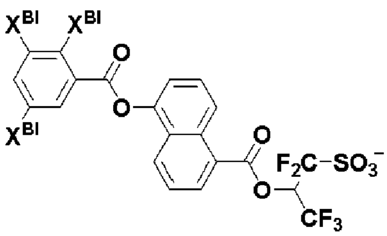 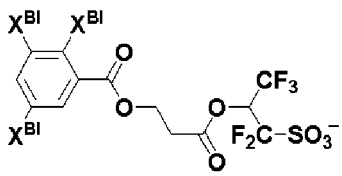

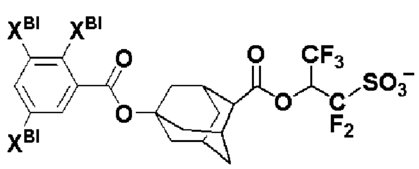 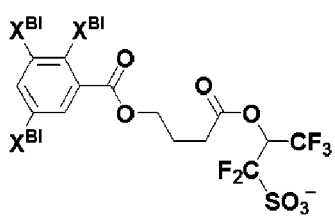

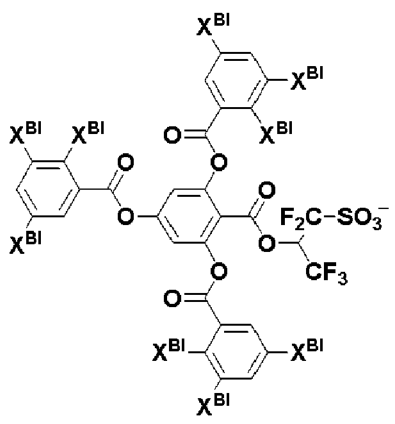

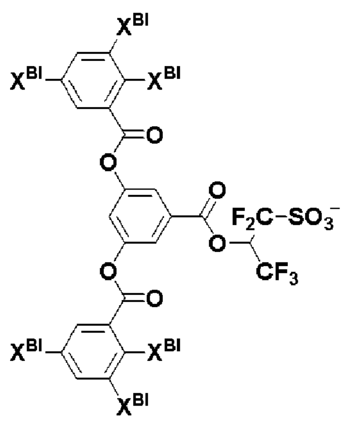

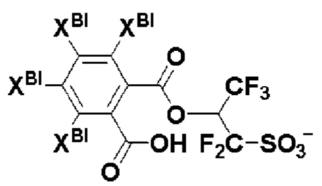

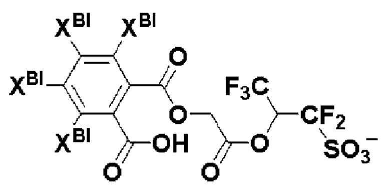

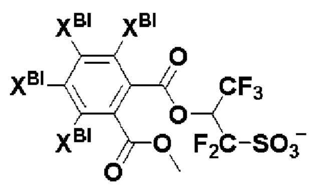

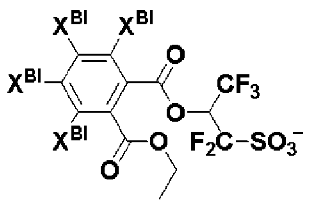

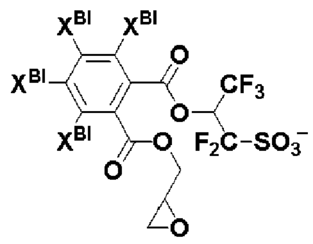

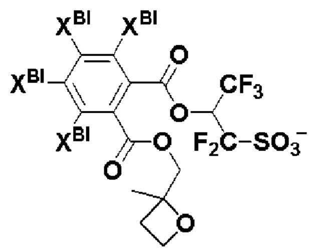

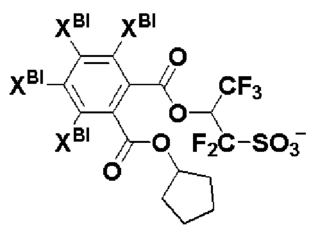

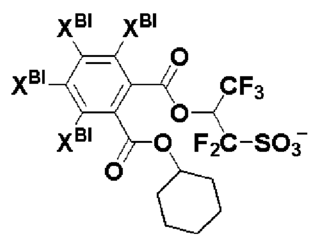

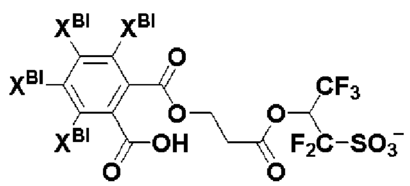

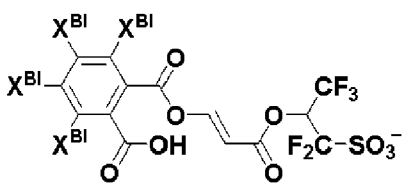

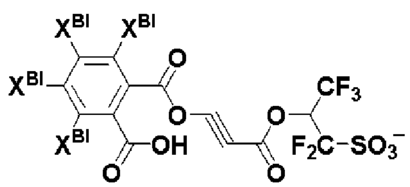

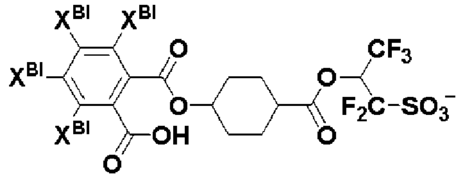

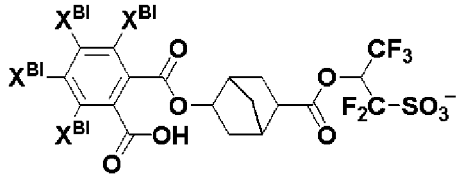

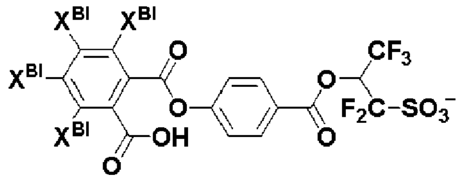
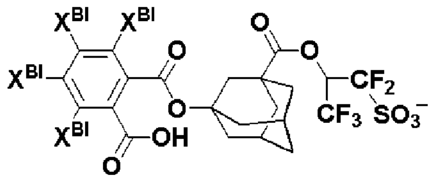

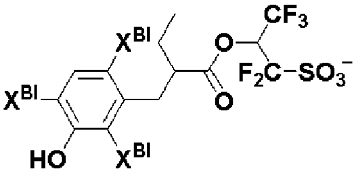
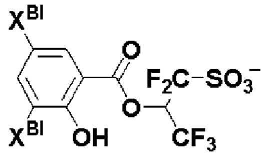
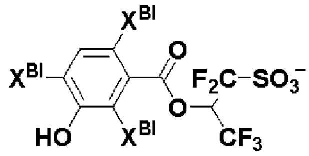

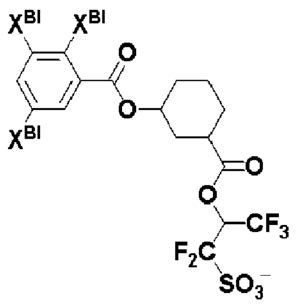
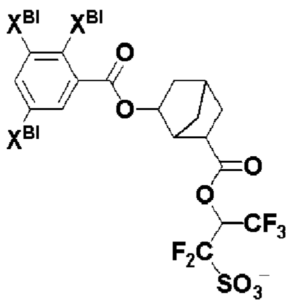
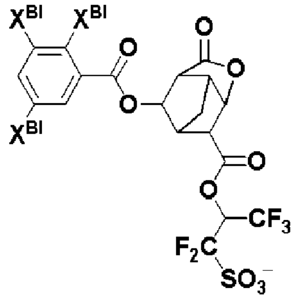

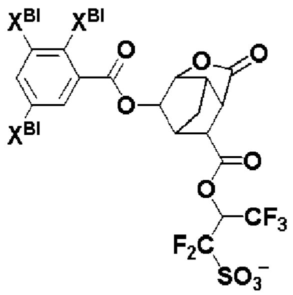
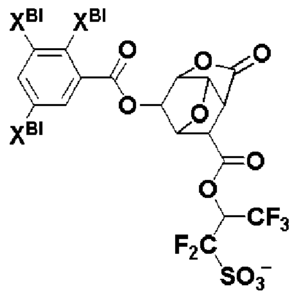

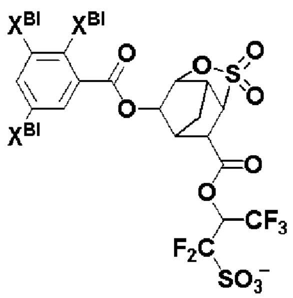
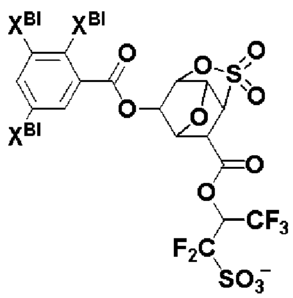
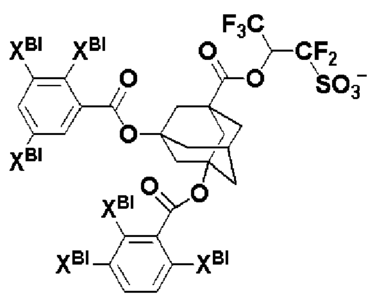

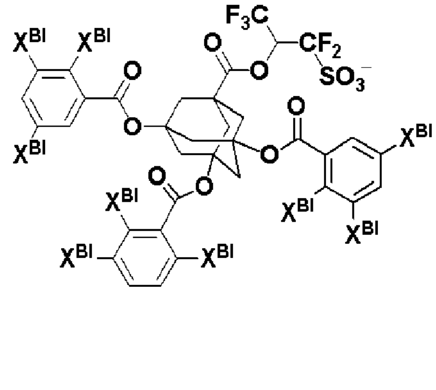

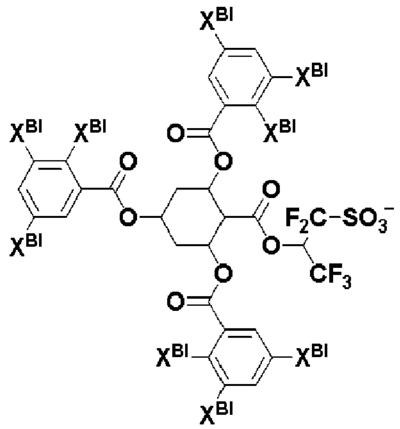

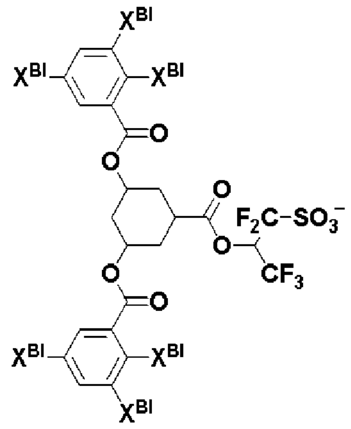

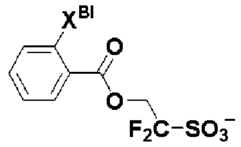

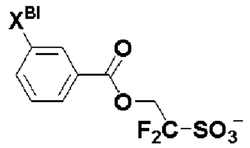

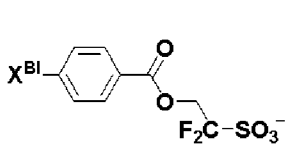

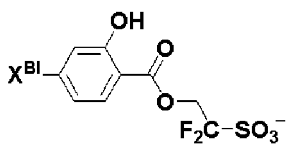

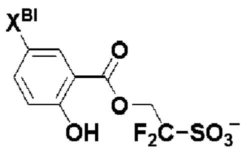

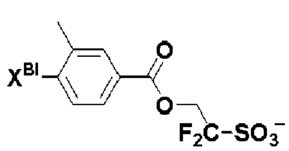

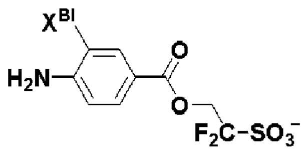

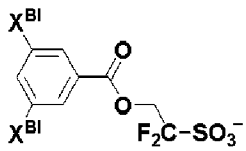

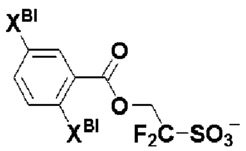

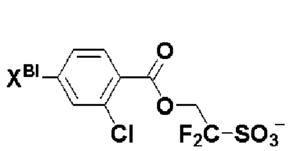

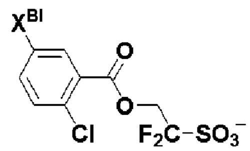

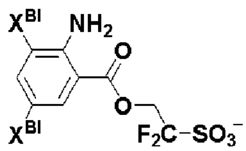

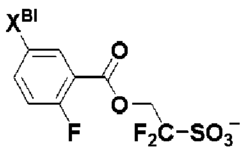

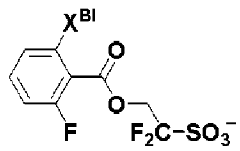

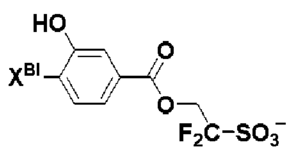

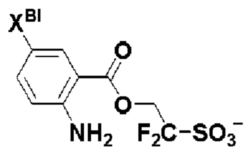

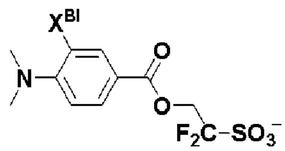

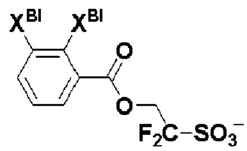

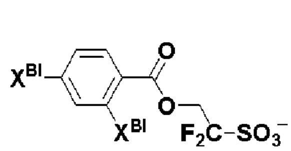

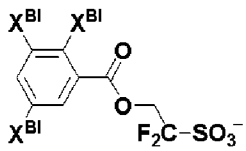

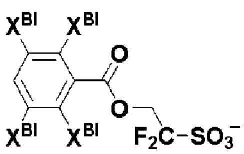

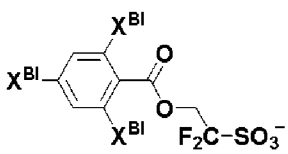

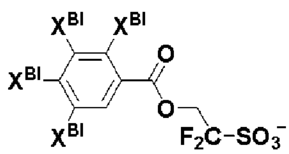

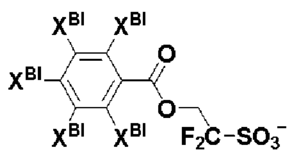

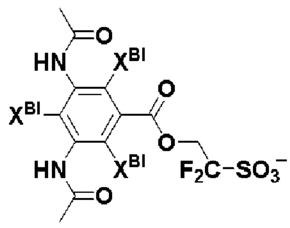
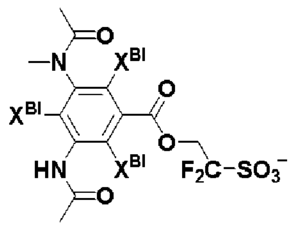
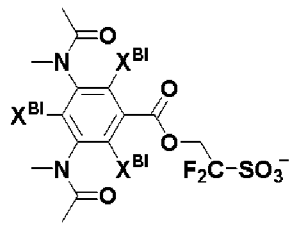

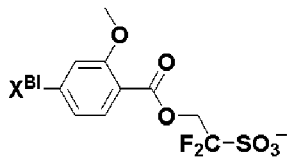
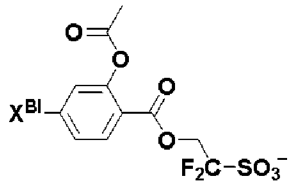
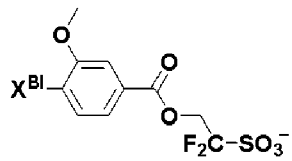

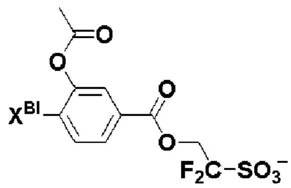
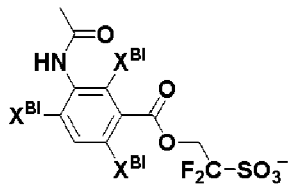

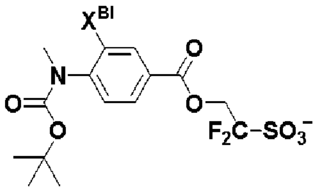
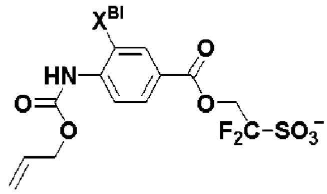
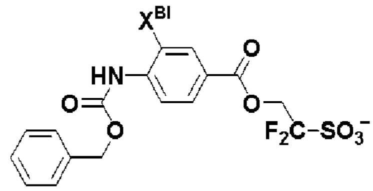

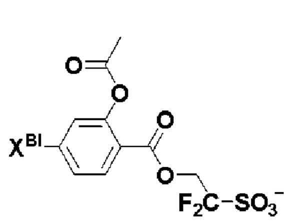
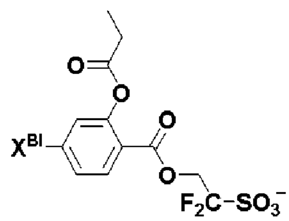
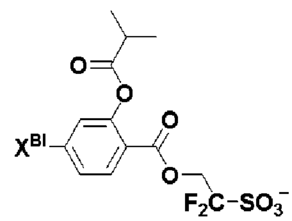

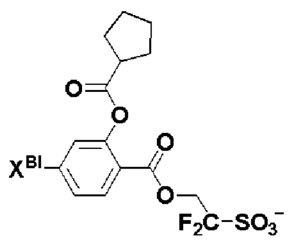
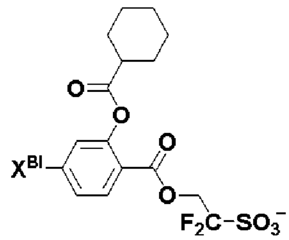
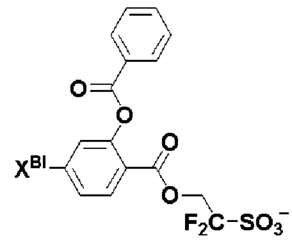

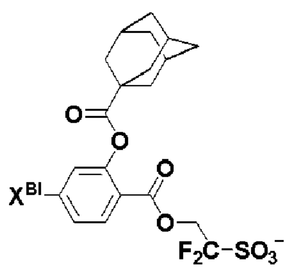
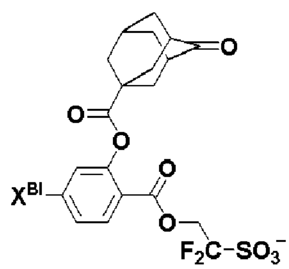
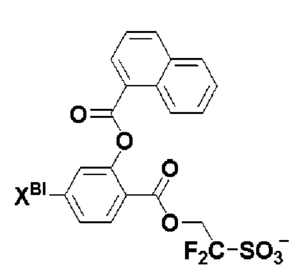

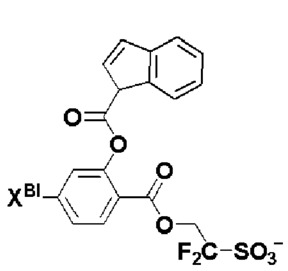
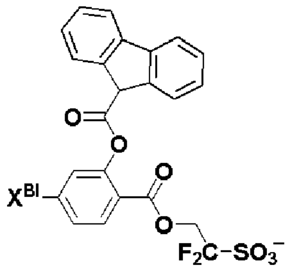
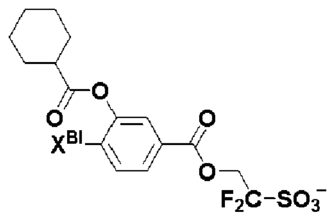

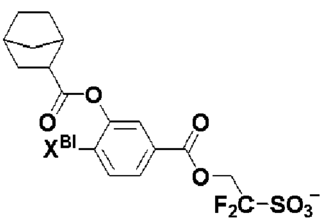
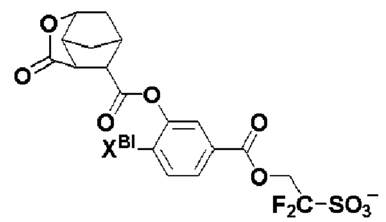

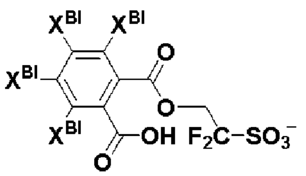
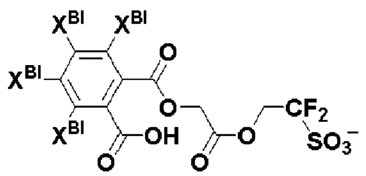
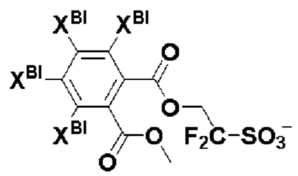

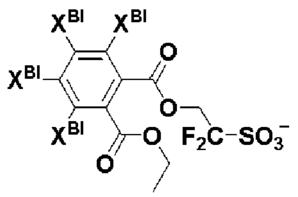
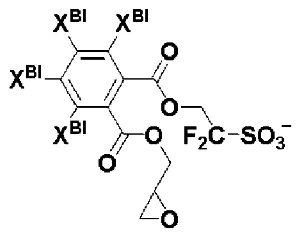
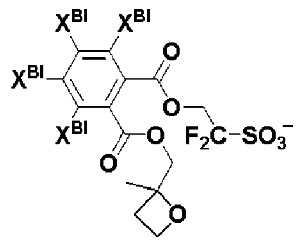

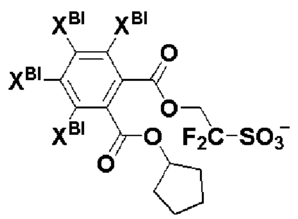
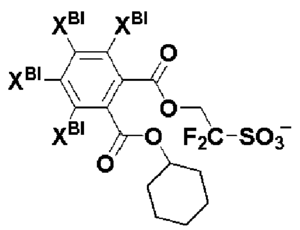
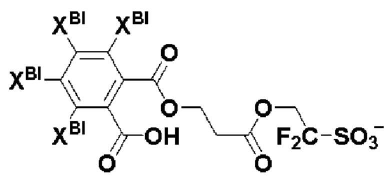

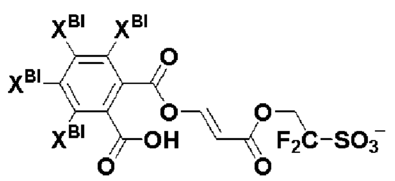
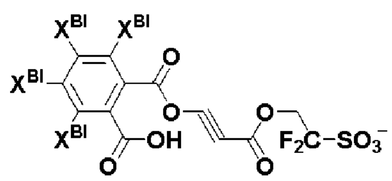

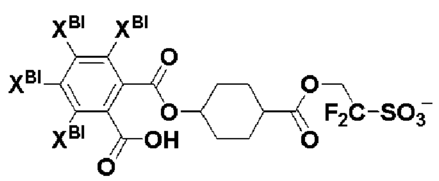
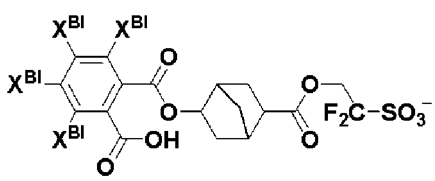

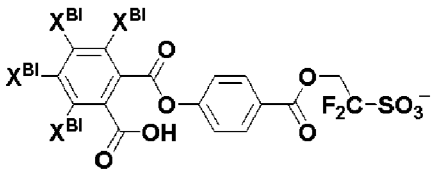
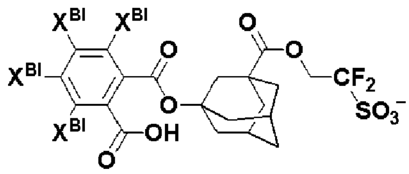

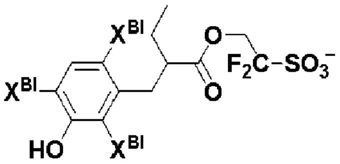
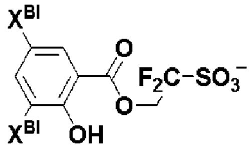
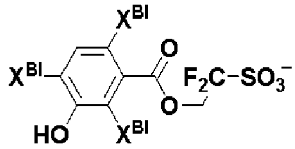

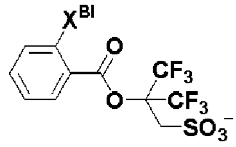
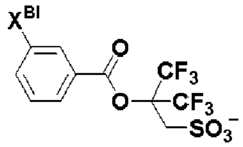
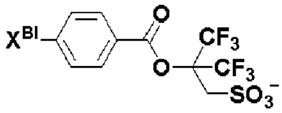
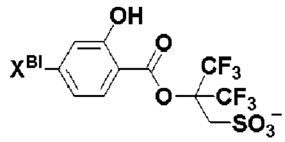

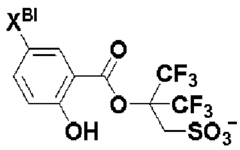
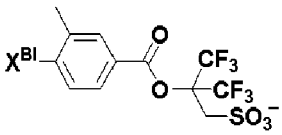
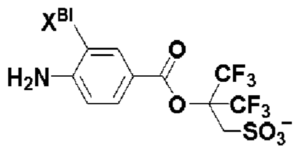
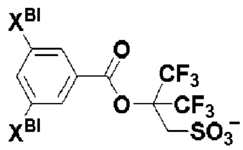

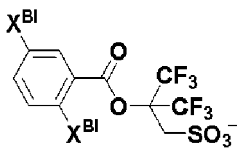
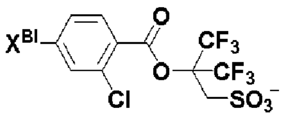
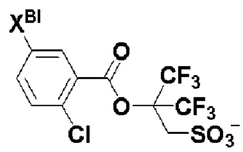
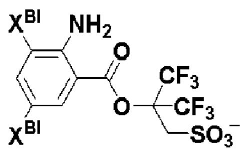

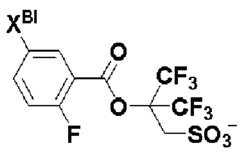
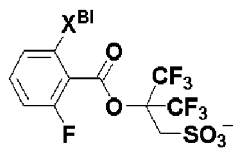
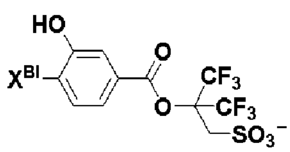
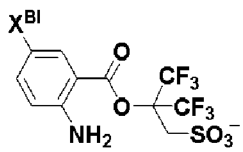

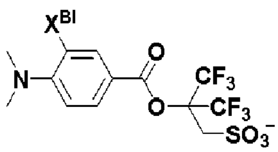
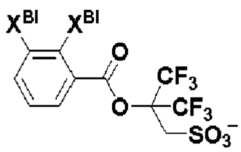
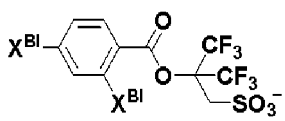
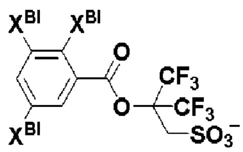

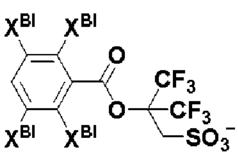
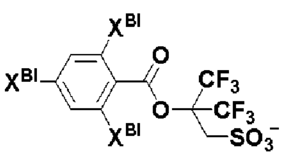
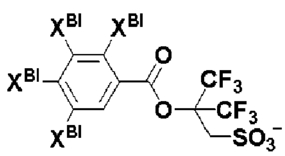
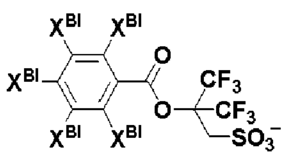

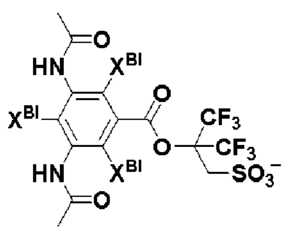
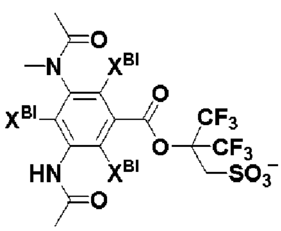
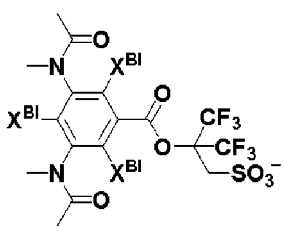

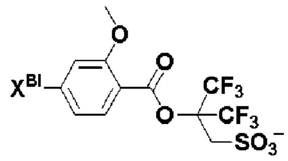
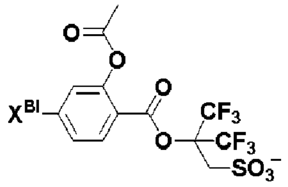
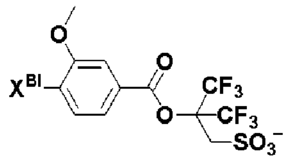

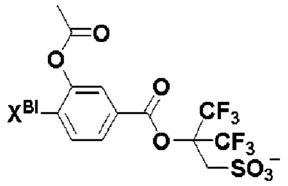
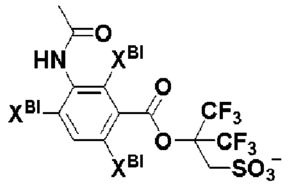

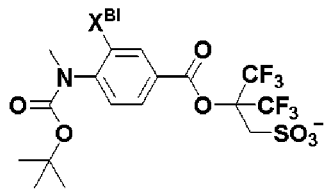
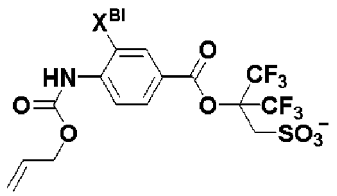
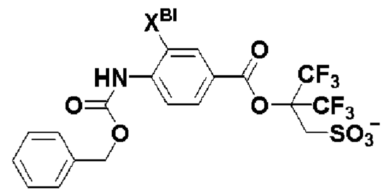

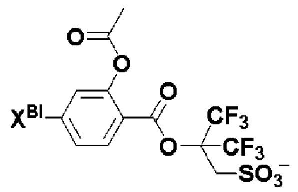
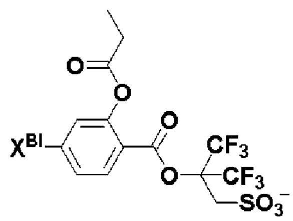
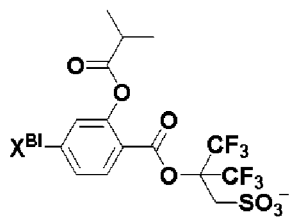

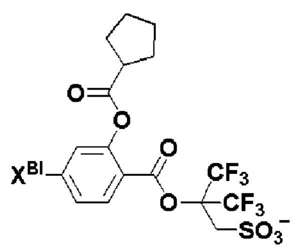
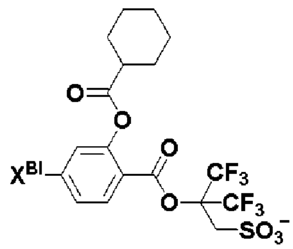
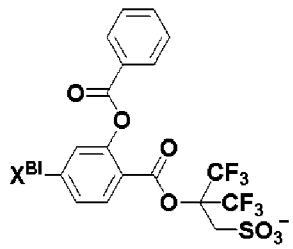

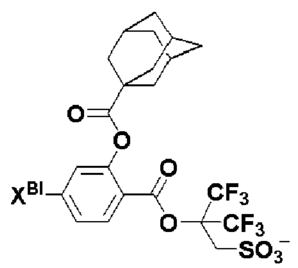
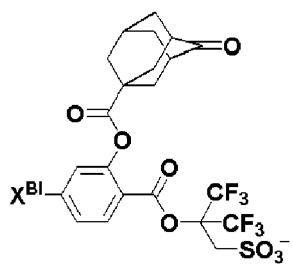
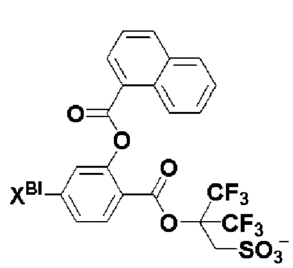

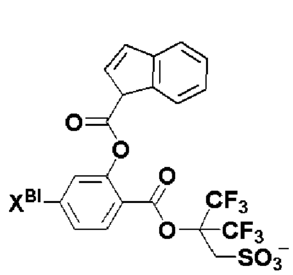
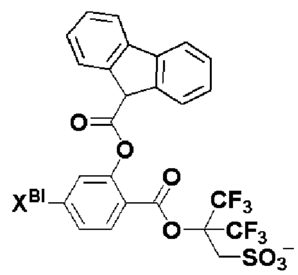
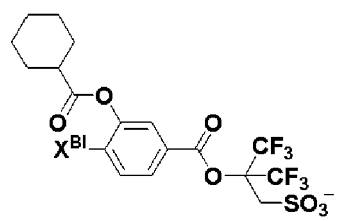

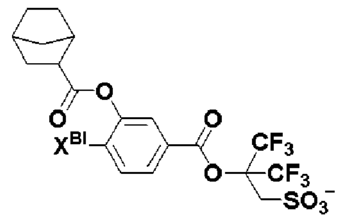
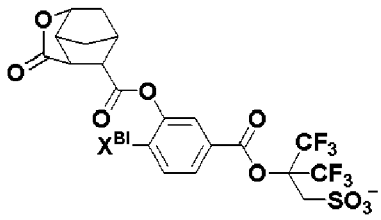

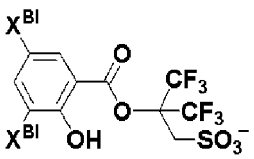
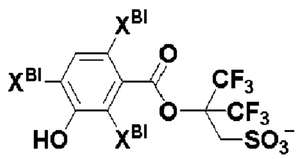

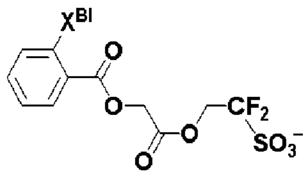
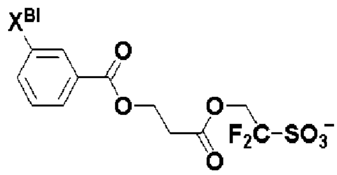
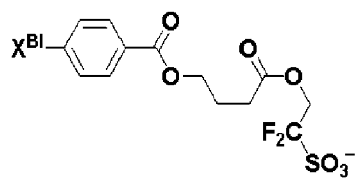

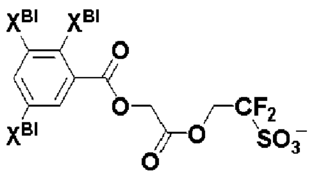
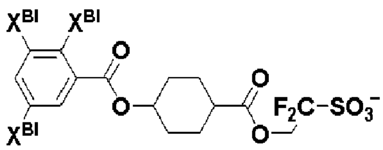

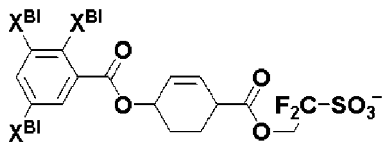
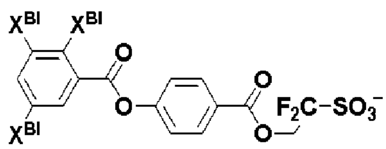

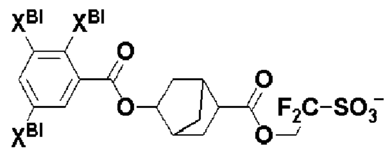
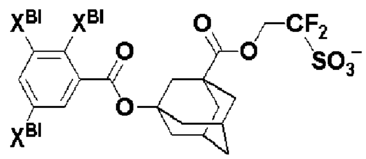

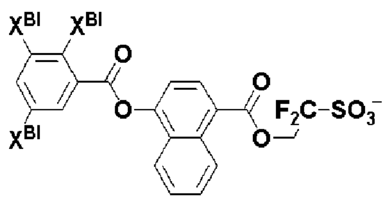
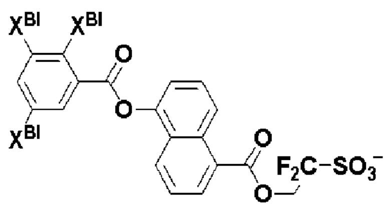

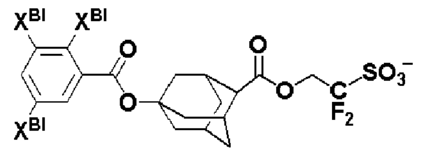

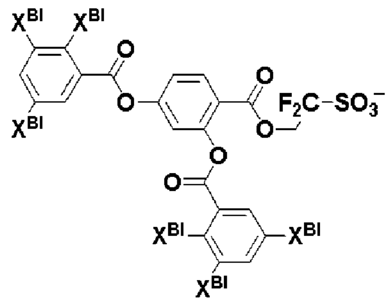

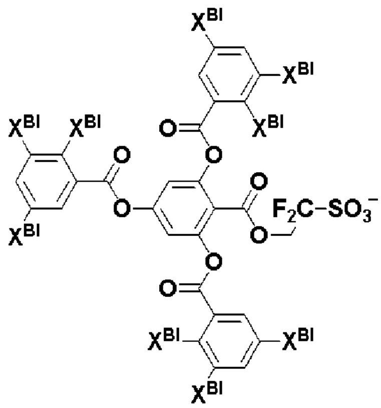

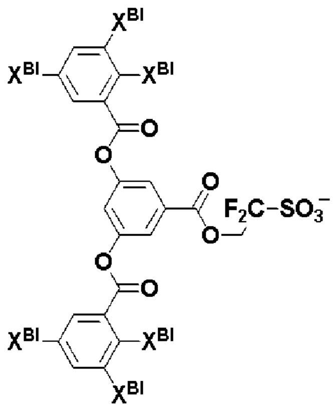

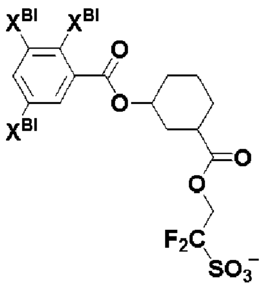

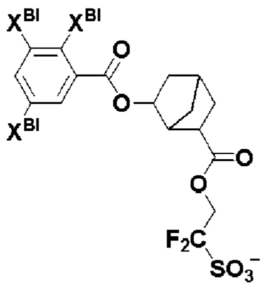

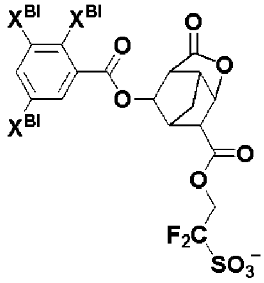

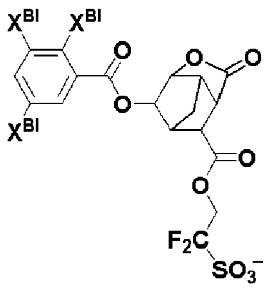

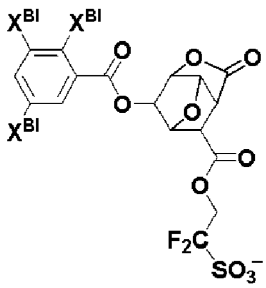

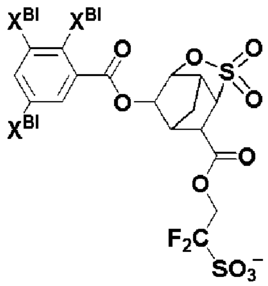

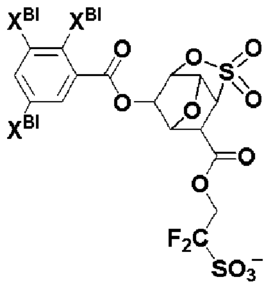

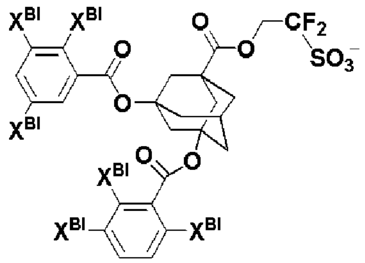

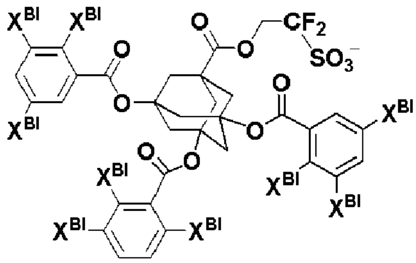

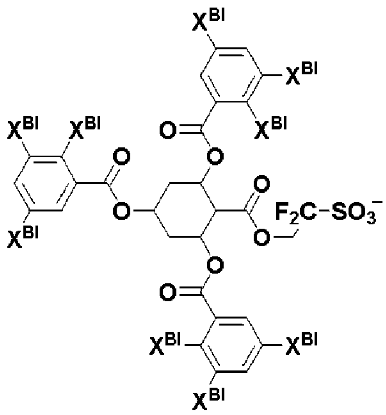

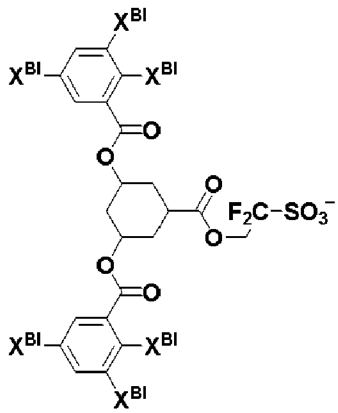

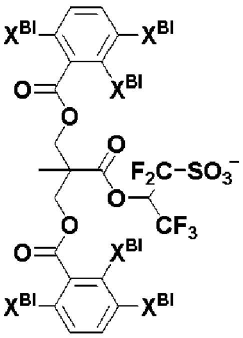

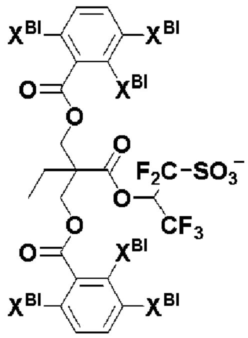

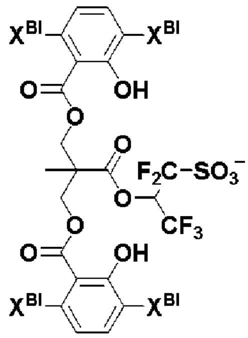

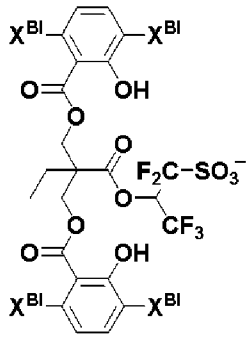

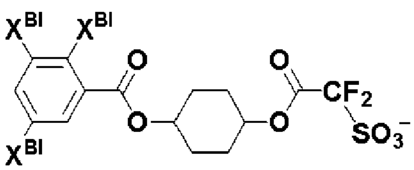

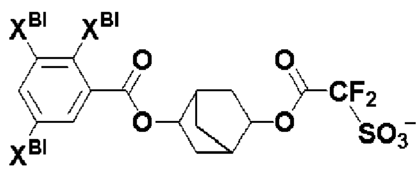

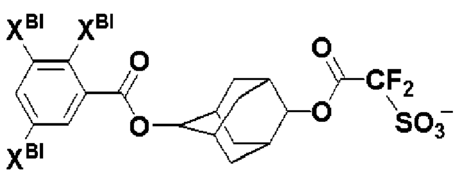

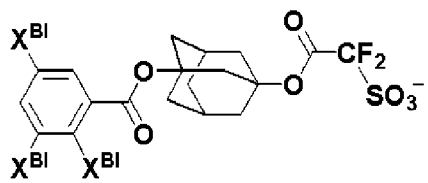

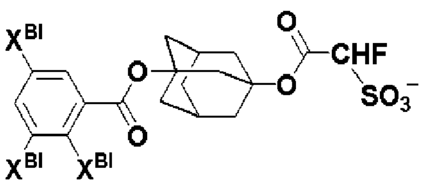

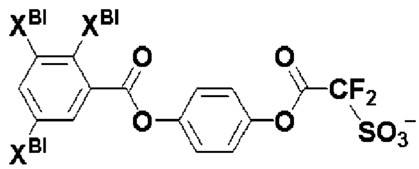

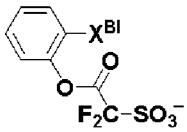
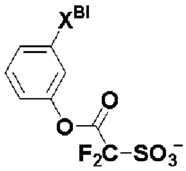
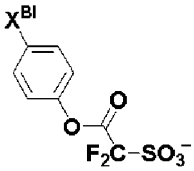
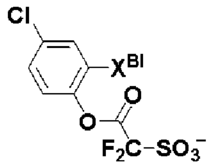
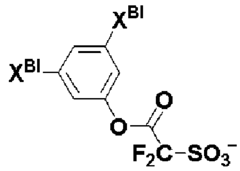

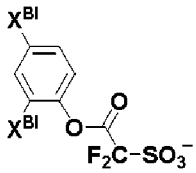
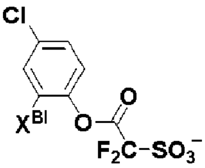
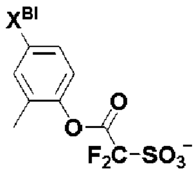
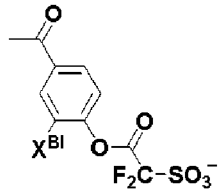
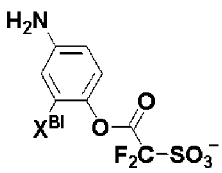

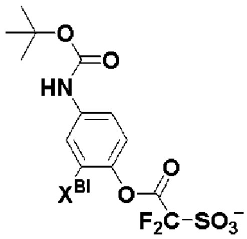
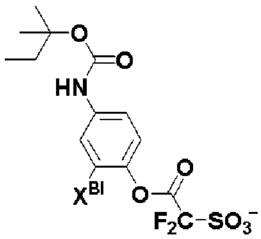
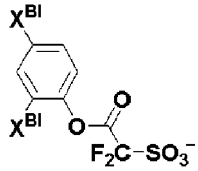
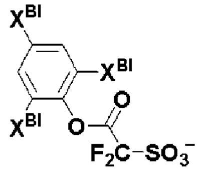

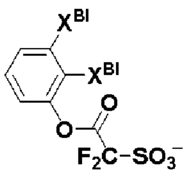
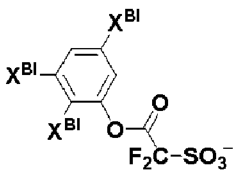
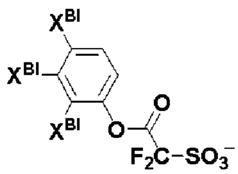
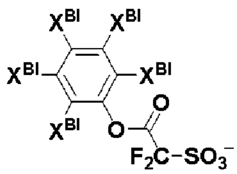

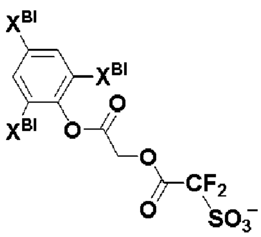
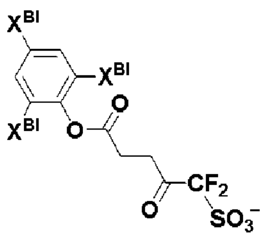
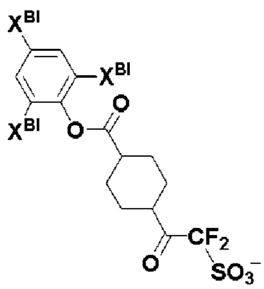

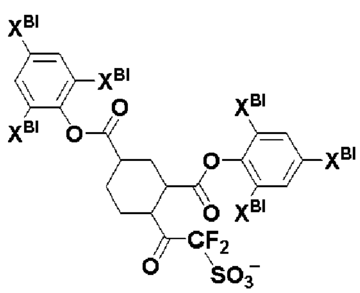
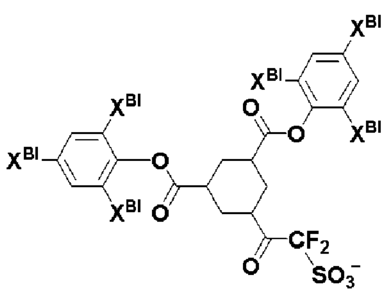

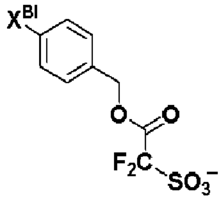
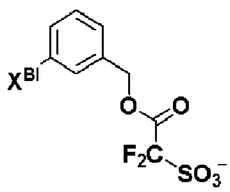
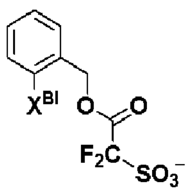

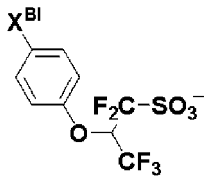
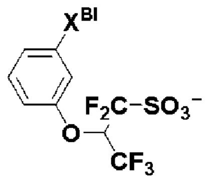
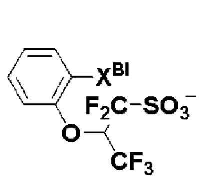
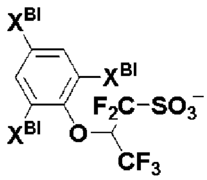

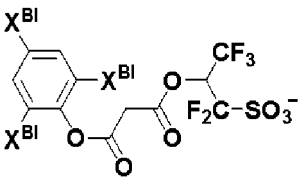
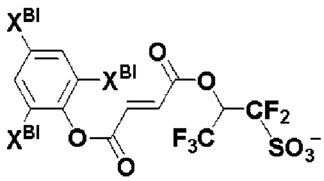
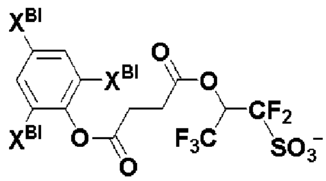

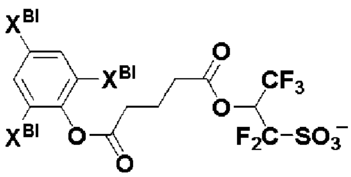
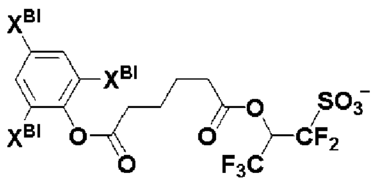

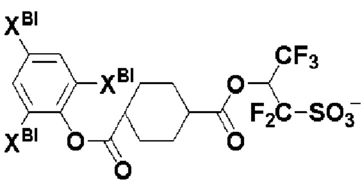
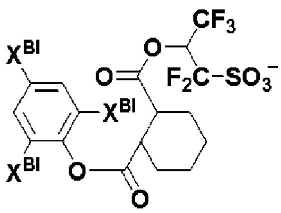
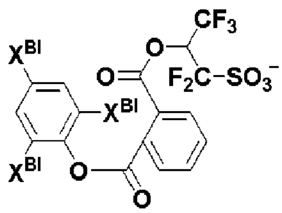

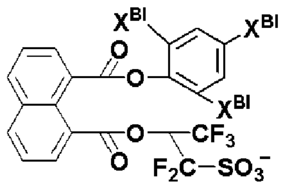
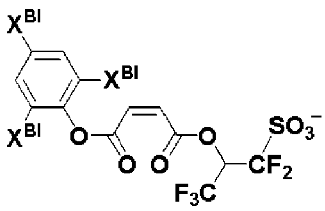

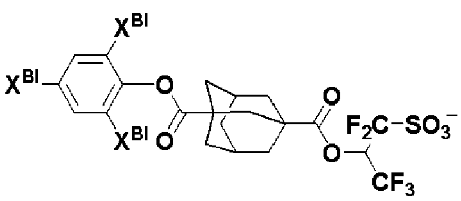
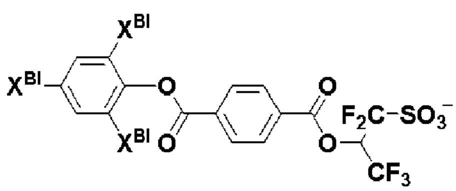

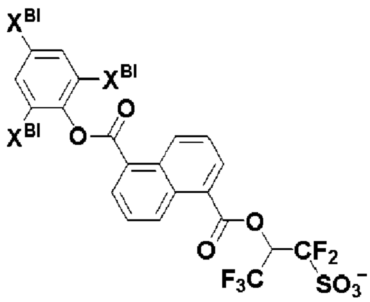
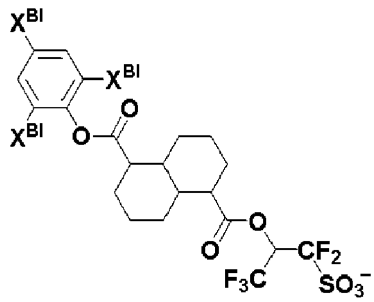

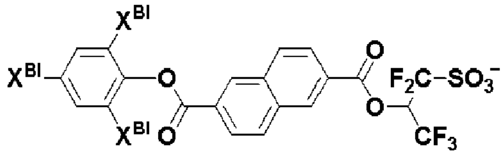
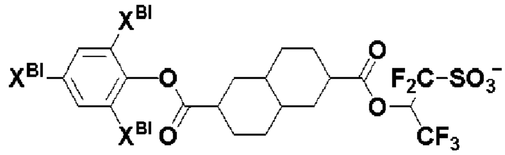

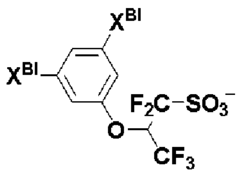
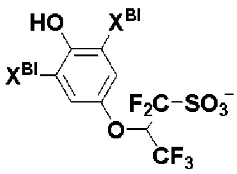
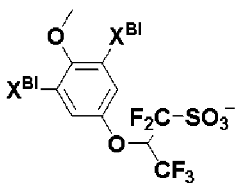
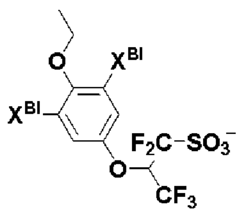

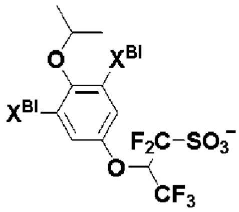
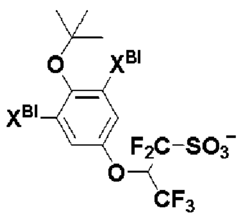
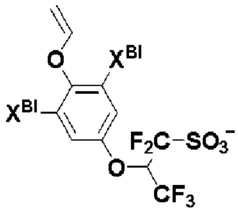
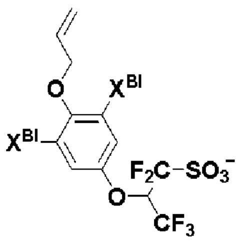

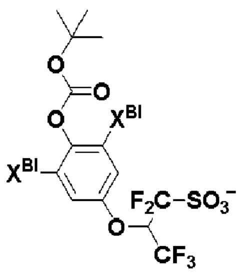
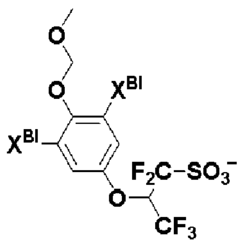

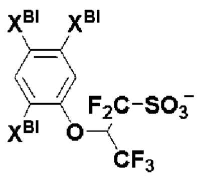
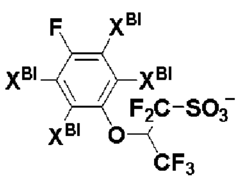
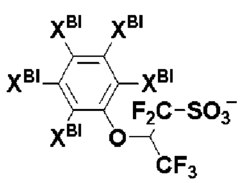

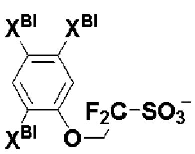
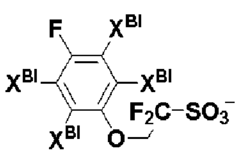
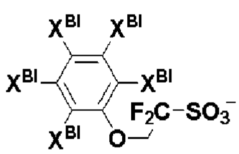
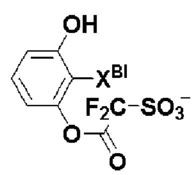

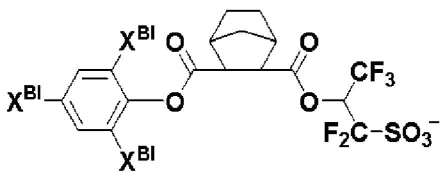
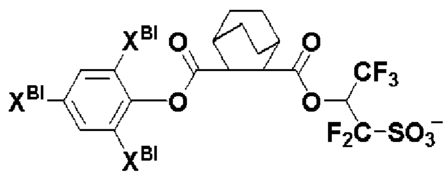
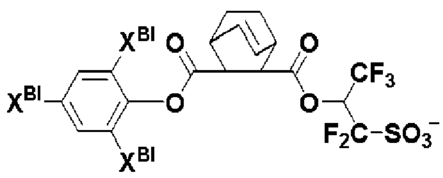
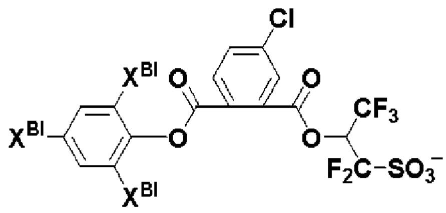
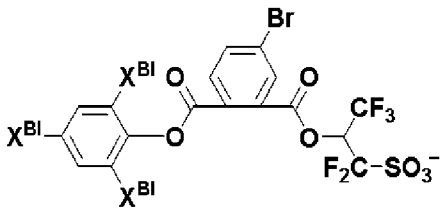
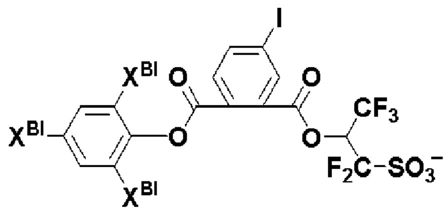
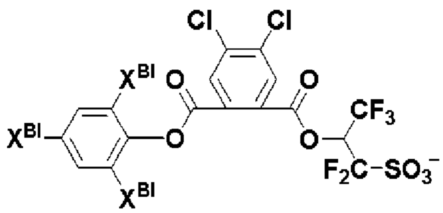
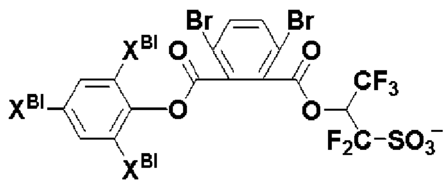
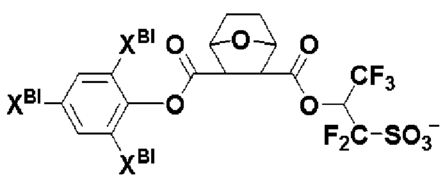
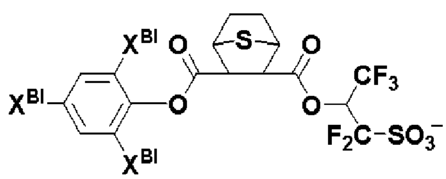
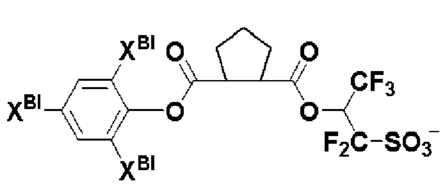
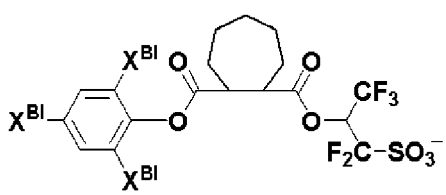
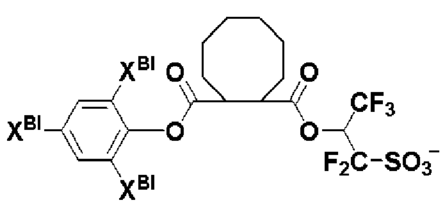
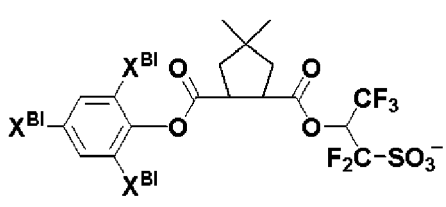

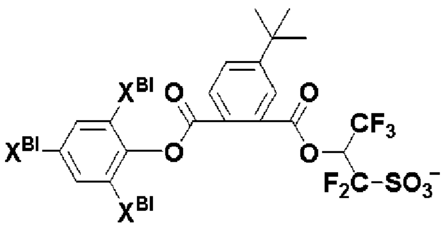

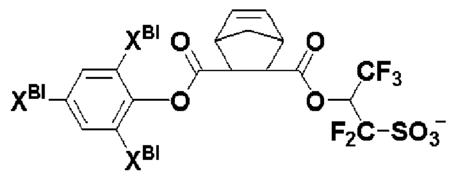

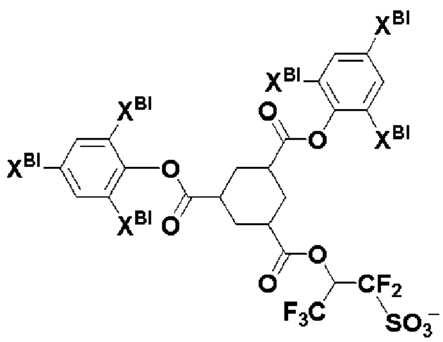

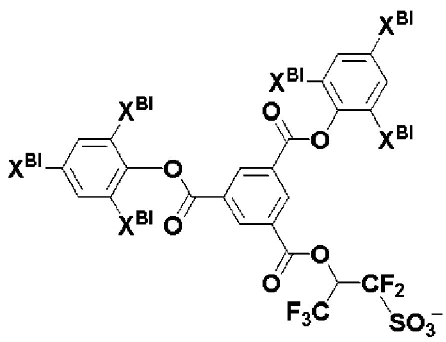

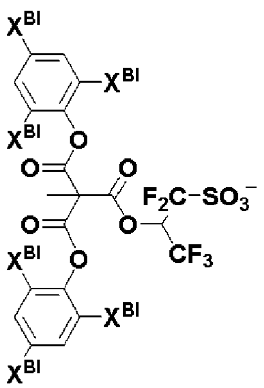

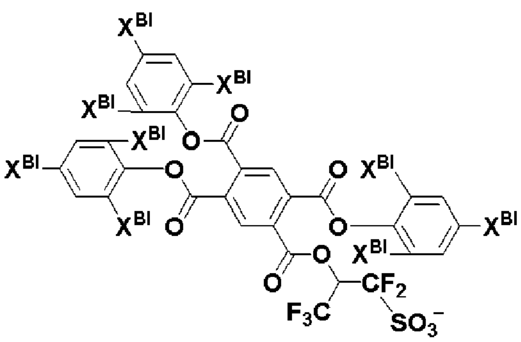

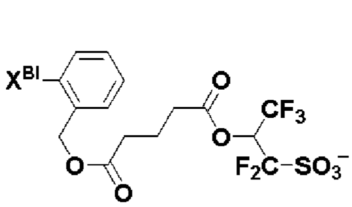

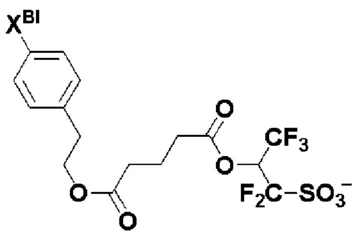

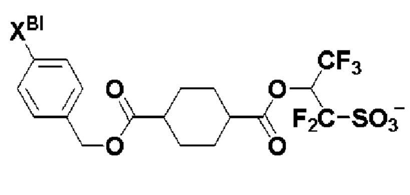

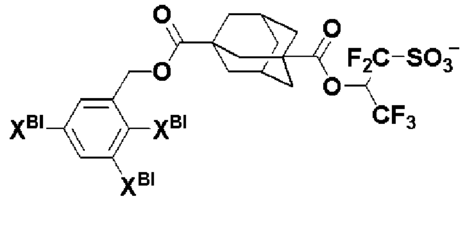

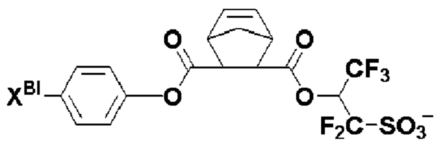

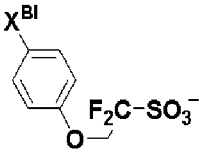
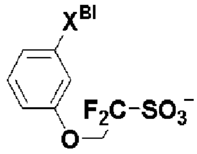
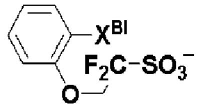
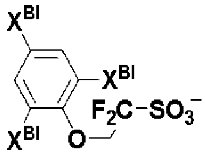

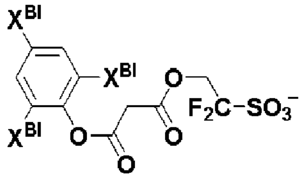
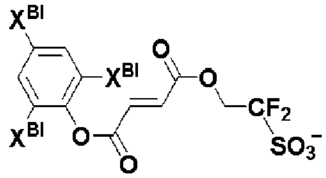
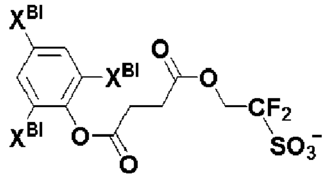

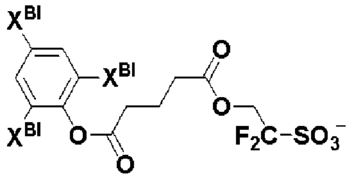
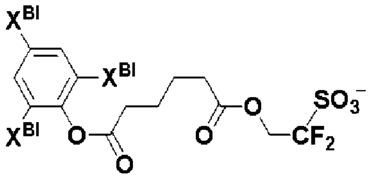

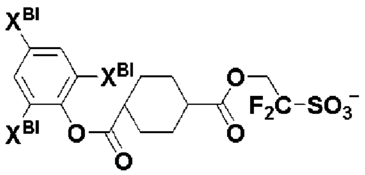
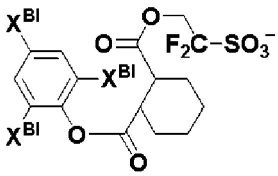
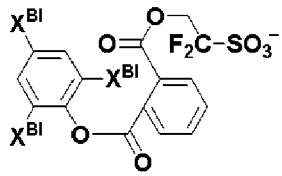

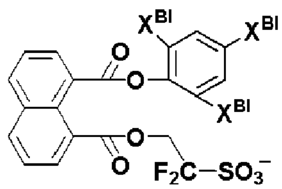
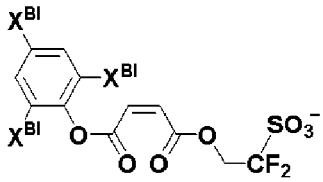

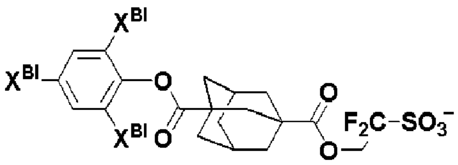
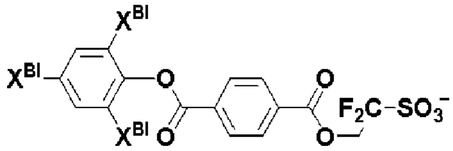

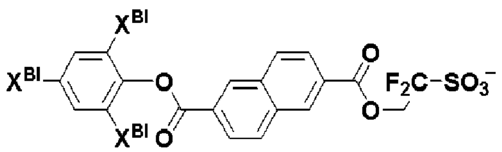
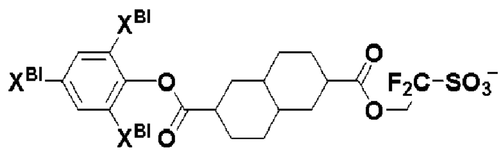

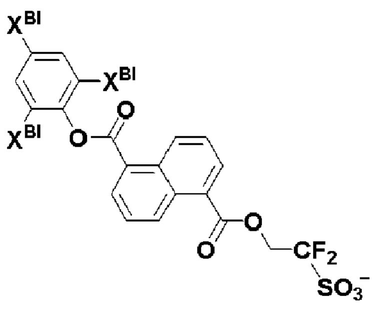
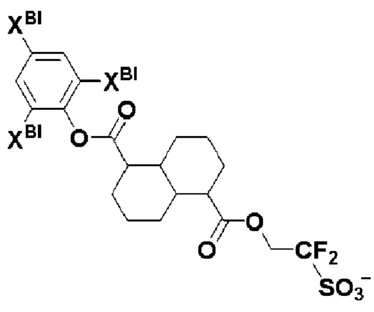

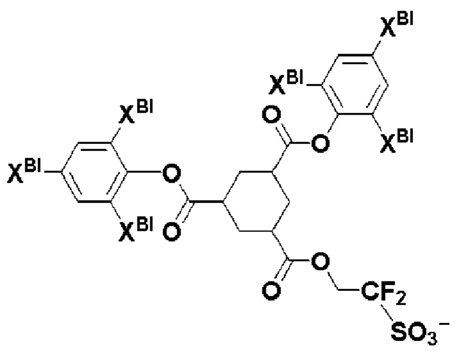

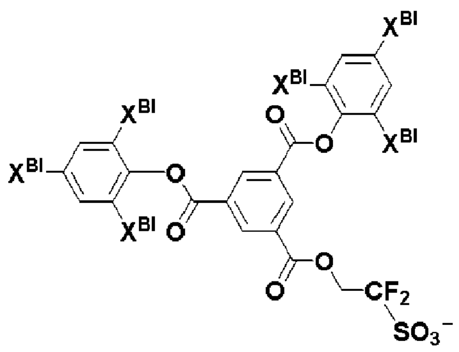

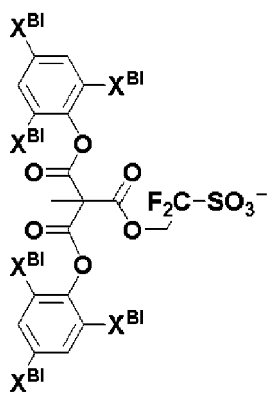

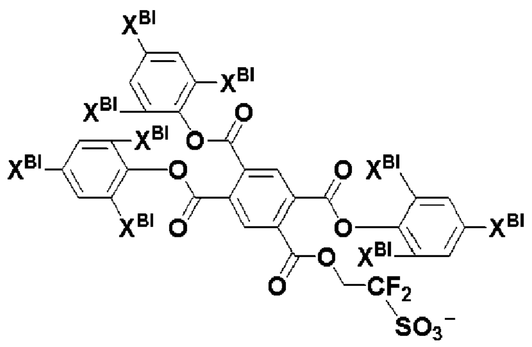

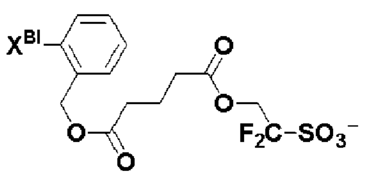

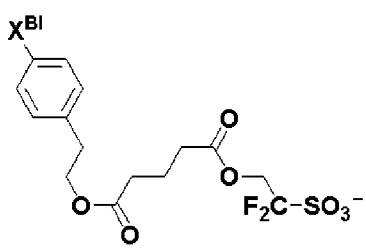

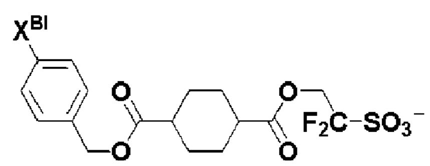

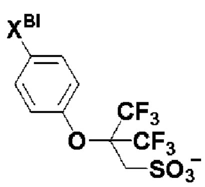

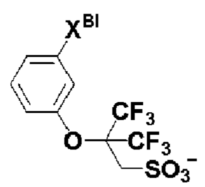

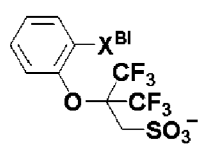

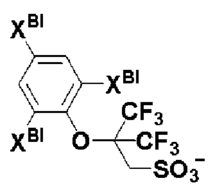

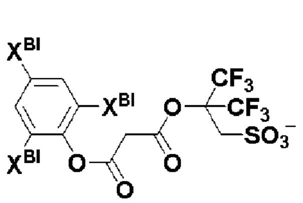

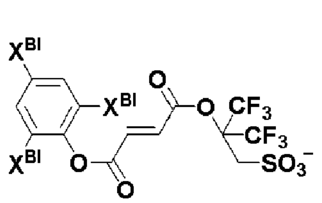

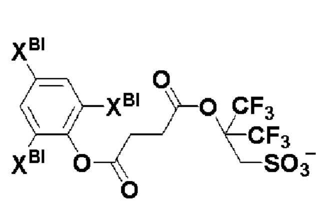

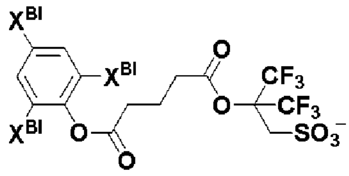
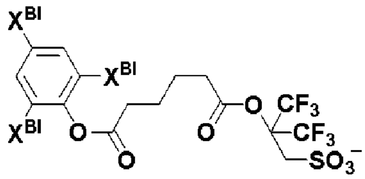

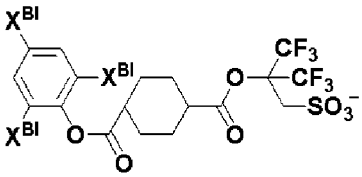
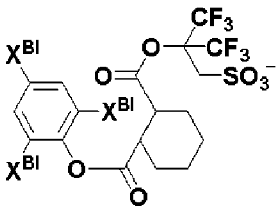
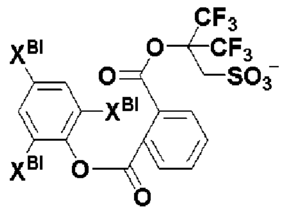

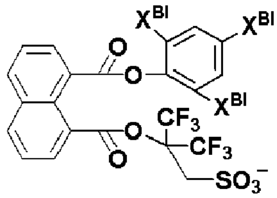
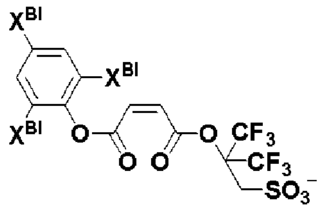

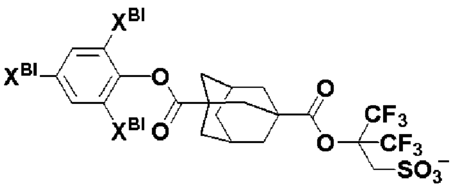
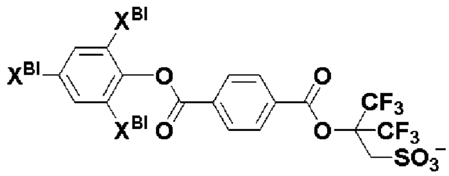

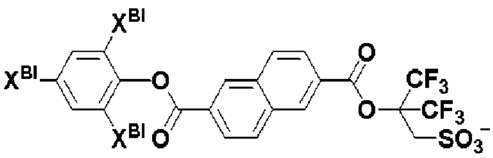
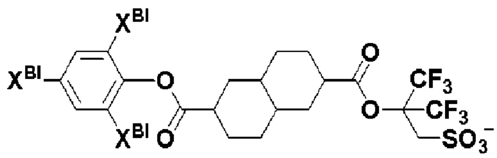

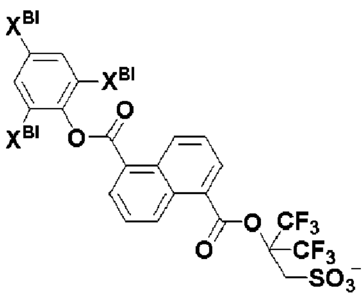
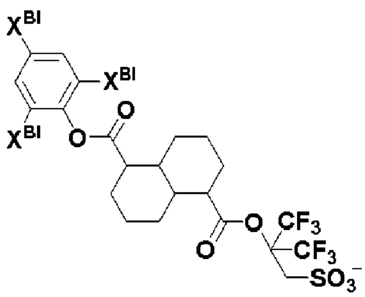

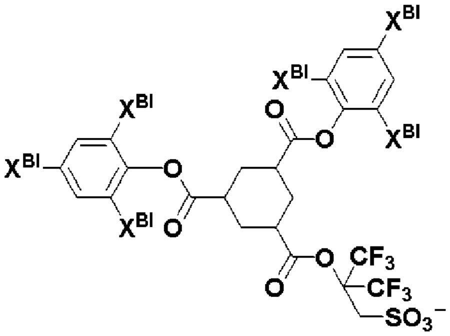
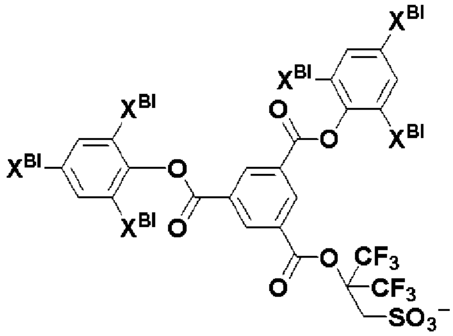

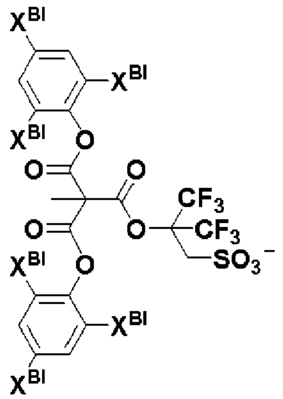

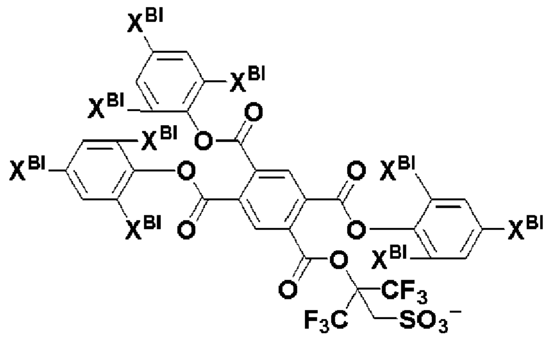

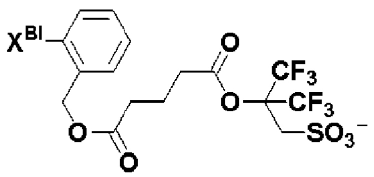

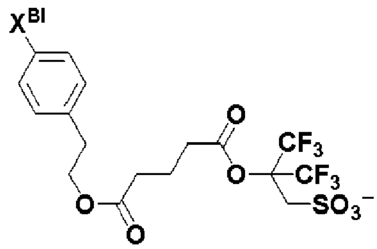

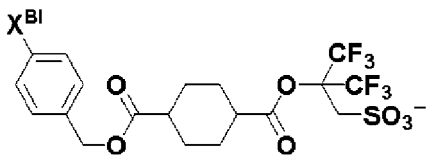

Usable as the non-nucleophilic counterion are: a fluorobenzenesulfonate anion bonded to an aromatic group having an iodine atom, described in JP 6648726 B; an anion having a mechanism in which the anion is decomposed by an acid, described in WO 2021/200056 and JP 2021-070692 A; an anion having a cyclic ether group, described in JP 2018-180525 A and JP 2021-35935 A; and an anion described in JP 2018-092159 A.

In addition, usable as the non-nucleophilic counterion are: an anion of a balky benzenesulfonic acid derivative having no fluorine atom, described in JP 2006-276759 A, JP 2015-117200 A, JP 2016-65016 A, and JP 2019-202974 A; and a benzenesulfonate anion or alkylsulfonate anion having no fluorine atom and bonded to an aromatic group having an iodine atom, described in JP 6645464 B.

In addition, usable as the non-nucleophilic counterion are: an anion of a bissulfonic acid, described in JP 2015-206932 A; an anion having a sulfonate on one side and a sulfonamide or sulfonimide differing therefrom on the other side, described in WO 2020/158366; and an anion having a sulfonate on one side and a carboxylate on the other side, described in JP 2015-024989 A.

Specific examples of the inventive sulfonium salt include any combination of the aforementioned anions and cations.

The inventive sulfonium salt (1) can be synthesized by a known method. For example, a corresponding sulfoxide is firstly reacted with a Grignard reagent in the presence of a halosilicon reagent to synthesize a sulfonium salt having the sulfonium cation. Then, the synthesized sulfonium salt and a corresponding anion can be subjected to a salt-exchange reaction to be converted into the target sulfonium salt. The salt exchange with the corresponding anion can be easily performed by a known method, and JP 2007-145797 A can be referred, for example.

The above manufacturing method is just an example, and a method for manufacturing the inventive sulfonium salt is not limited thereto.

The inventive sulfonium salt has structural features of: the acid-labile group bonded instead of the hydrogen atom of the hydroxy group on the aromatic ring of the sulfonium cation; the fluorine-atom-containing substituent; and these groups being bonded to adjacent carbon atoms. The acid-labile group in an exposed portion causes a deprotection reaction by a generated acid to generate an aromatic hydroxy group. This generation enhances the contrast between the exposed portion and the unexposed portion. The adjacent fluorine-atom-containing substituent increases the solubility of the sulfonium salt itself in a resist solvent, and increases the acidity of the aromatic hydroxy group generated in the exposed portion with its electron withdrawing property. When the resist film is developed with an alkaline developer after the exposure, compatibility between the generated aromatic hydroxy group and the alkaline developer increases to

effectively remove the exposed portion with the developer. The aromatic hydroxy group adjacent to the fluorine-atom-containing substituent, which does not attract the alkaline developer to the unexposed portion with the water repellent effect of the fluorine atom compared with a carboxyl group, is considered to have an effect of reducing swelling due to the alkaline developer. This effect inhibits collapse of the resist pattern produced in the unexposed portion. With a synergistic effect of these, using the inventive sulfonium salt can form a pattern having high dissolution contrast, excellent LWR of a line pattern or CDU of a hole pattern, and hardly causing collapse. Thus, the inventive sulfonium salt is suitable for the positive-type resist material.

Photoacid Generator

As noted above, the above sulfonium salt can be suitably used as a photoacid generator.

Resist Composition

The present invention provides: a photoacid generator comprising the above sulfonium salt; and a resist composition comprising this photoacid generator. The inventive resist composition is preferably a chemically amplified resist composition. Hereinafter, the inventive resist composition will be described with a chemically amplified resist composition as an example.

The inventive chemically amplified resist composition is not particularly limited as long as the resist composition contains the photoacid generator comprising the above sulfonium salt, and the inventive chemically amplified resist composition can comprise:

(A) a photoacid generator comprising of the sulfonium salt represented by the formula (1);
(B) a base polymer; and
(C) an organic solvent.

The inventive chemically amplified resist composition optionally further comprises, as necessary,

(D) a quencher; and
(E) another photoacid generator.

The inventive chemically amplified resist composition optionally further comprises, as necessary,
(F) a surfactant insoluble or hardly soluble in water and soluble in an alkaline developer; and/or a surfactant insoluble or hardly soluble in water and an alkaline developer.

The inventive chemically amplified resist composition optionally further comprises, as necessary,
(G) another component.

(A) Photoacid Generator

The component (A) is a photoacid generator comprising the sulfonium salt represented by the formula (1). As noted above, the inventive resist composition can form a pattern having high dissolution contrast, excellent LER of a line pattern or CDU of a hole pattern, and hardly causing pattern collapse because of the structural features of the sulfonium salt.

A content of the photoacid generator of the component (A) comprising the sulfonium salt represented by the formula (1) is preferably 0.1 to 40 parts by mass, and more preferably 0.5 to 30 parts by mass, relative to 80 parts by mass of the base polymer, described later. The content of the component (A) within the above range is preferable because of good sensitivity and resolution, and no risk of problem of a foreign matter after the development or during peeling of the resist film. The photoacid generator being the component (A) may be used singly, or may be used in combination of two or more kinds thereof.

(B) Base Polymer

The base polymer being the component (B) is not particularly limited. For example, the base polymer has a repeating unit represented by the following formula (a1) (hereinafter, also referred to as the repeating unit a1) or a repeating unit represented by the following formula (a2) (hereinafter, also referred to as the repeating unit a2).

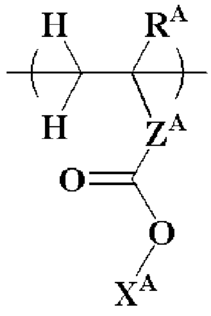

(a1)

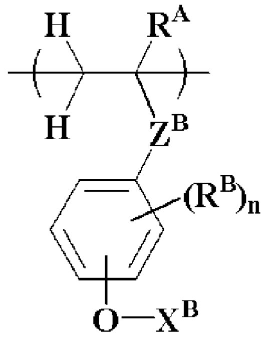

(a2)

In the formulae (a1) and (a2), $R^A$ each independently represents a hydrogen atom, a fluorine atom, a methyl group, or a trifluoromethyl group. $Z^A$ represents a single bond, (main chain)-C(=O)-O-$Z^{A1}$-, or a phenylene group or naphthylene group optionally having a C1 to C10 alkoxy group optionally having a fluorine atom, or a phenylene group or naphthylene group optionally having a halogen atom. $Z^{A1}$ represents a linear, branched, or cyclic alkanediyl group (aliphatic hydrocarbylene group) having 1 to 20 carbon atom, a phenylene group, or a naphthylene group, the alkanediyl group optionally having a heteroatom, an alkoxy group having 1 to 10 carbon atoms and optionally having a fluorine atom, a hydroxy group, an ether bond, an ester bond, or a lactone ring. $Z^B$ represents a single bond or (main chain) -C(=O)-O-. $X^A$ and $X^B$ each independently represent an acid-labile group. Here, "(main chain)" represents a bond between the above group and the polymer main chain.

In the formula (a2), $R^B$ represents a monovalent hydrocarbon group (hydrocarbyl group) having 1 to 20 carbon atoms and optionally having a heteroatom. The monovalent hydrocarbon group may be a saturated or unsaturated group, and may be any of linear, branched, and cyclic groups. Specific examples thereof include groups same as those exemplified in the description of $R^{12}$. "n" represents an integer of 0 to 4, and preferably 0 or 1.

Examples of the acid-labile group represented by $X^A$ and $X^B$ in the formulae (a1) and (a2) include groups described in JP 2013-80033 A and JP 2013-83821 A.

Typical examples of the acid-labile group include groups represented by the following formulae (AL-1) to (AL-3),

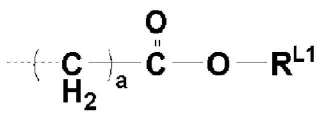

(AL-1)

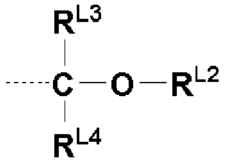

(AL-2)

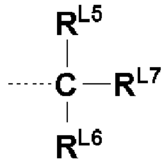

(AL-3)

wherein a broken line represents an attachment point.

In the formulae (AL-1) and (AL-2), $R^{L1}$ and $R^{L2}$ each independently represent a saturated hydrocarbyl group having 1 to 40 carbon atoms and optionally having a heteroatom such as an oxygen atom, a sulfur atom, a nitrogen atom, and a fluorine atom. The saturated hydrocarbyl group may be any of linear, branched, and cyclic groups. The saturated hydrocarbyl group preferably has 1 to 20 carbon atoms.

In the formula (AL-1), "a" represents an integer of 0 to 10, and preferably an integer of 1 to 5.

In the formula (AL-2), $R^{L3}$ and $R^{L4}$ each independently represent a hydrogen atom or a saturated hydrocarbyl group having 1 to 20 carbon atoms and optionally having a heteroatom such as an oxygen atom, a sulfur atom, a nitrogen atom, and a fluorine atom. The saturated hydrocarbyl group may be any of linear, branched, and cyclic groups. Any two of $R^{L2}$, $R^{L3}$, and $R^{L4}$ are optionally bonded each other to form a ring having 3 to 20 carbon atoms together with the carbon atom or with the carbon atom and the oxygen atom to which these groups are bonded. The ring is preferably a ring having 4 to 16 carbon atoms, and particularly preferably an aliphatic ring.

In the formula (AL-3), $R^{L5}$, $R^{L6}$, and $R^{L7}$ each independently represent a saturated hydrocarbyl group having 1 to 20 carbon atoms and optionally having a heteroatom such as an oxygen atom, a sulfur atom, a nitrogen atom, and a fluorine atom. The saturated hydrocarbyl group may be any of linear, branched, and cyclic groups. Any two of $R^{L5}$, $R^{L6}$, and $R^{L7}$ are optionally bonded each other to form a ring having 3 to 20 carbon atoms together with the carbon atom to which these groups are bonded. The ring is preferably a ring having 4 to 16 carbon atoms, and particularly preferably an aliphatic ring.

Examples of the repeating unit a1 include the following repeating units, but the repeating unit is not limited thereto. In the following formulae, $R^A$ and $X^A$ represent the same as above.

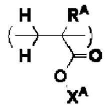
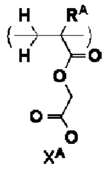
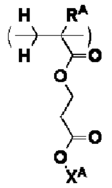
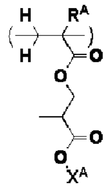
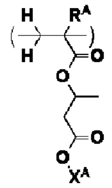
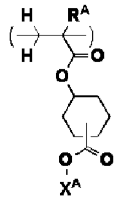

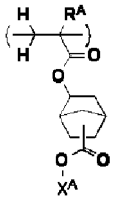
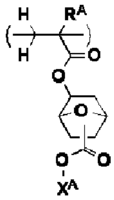
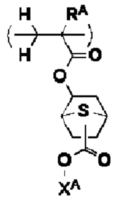
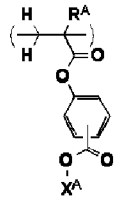
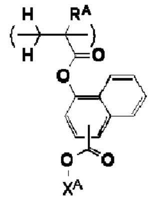
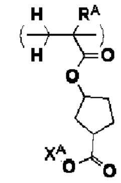

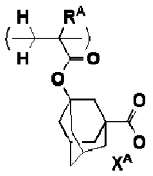
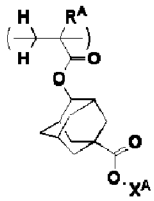
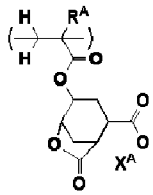
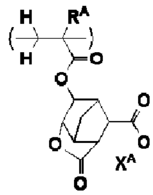
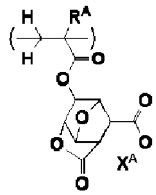
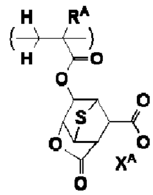

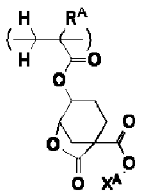
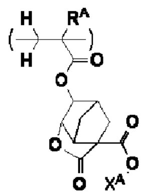
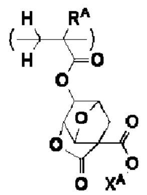
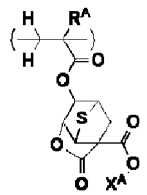
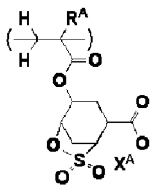
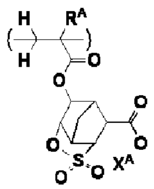

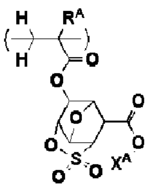
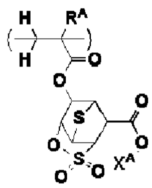
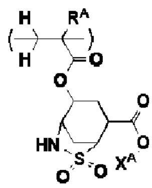
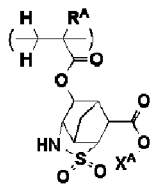
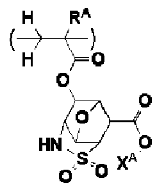
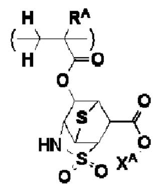

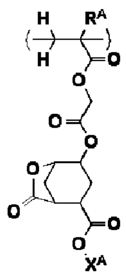
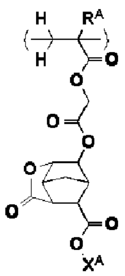
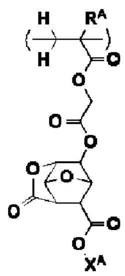
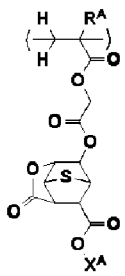
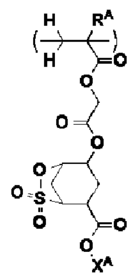
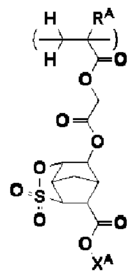

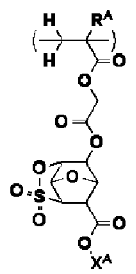 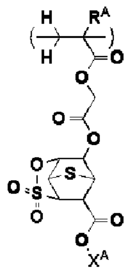 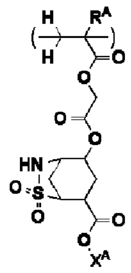 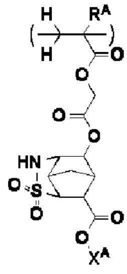 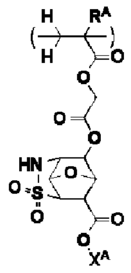 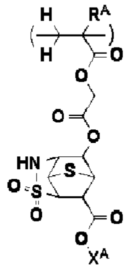

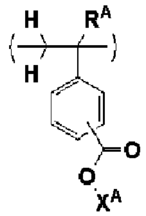 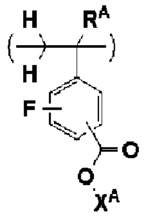 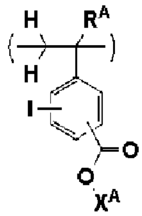 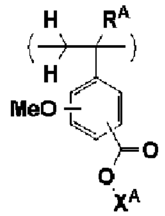 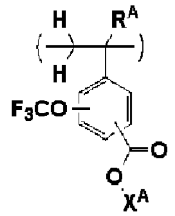

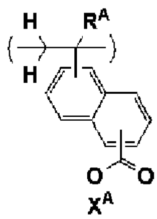 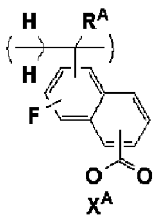 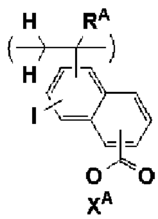 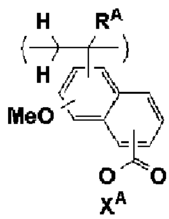 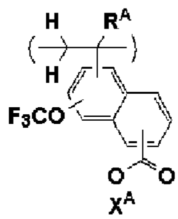

Examples of the repeating unit a2 include the following repeating units, but the repeating unit is not limited thereto. In the following formulae, $R^A$ and $X^B$ represent the same as above.

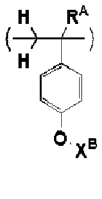 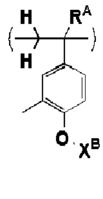 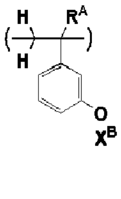 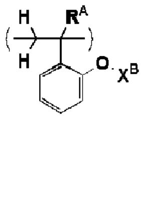 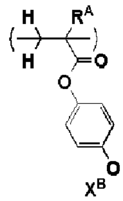 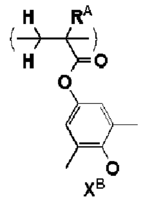

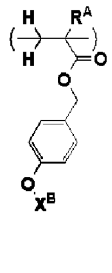 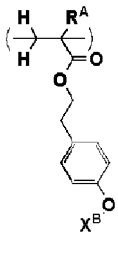 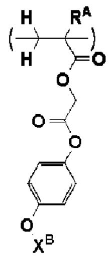 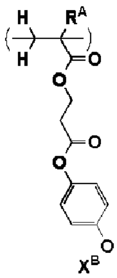 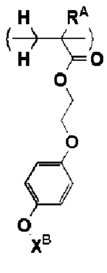

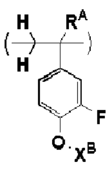 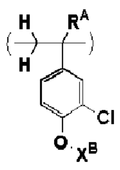 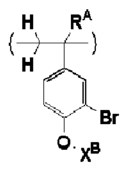 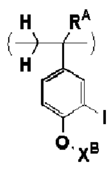 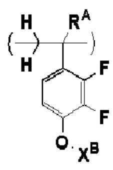 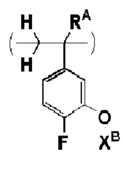

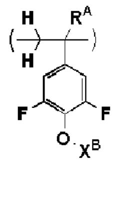
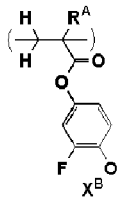
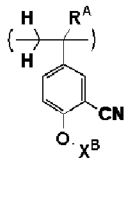
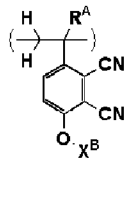
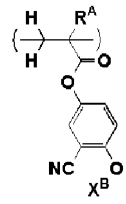
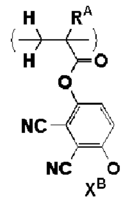

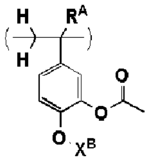
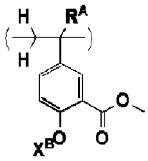
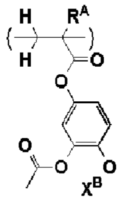
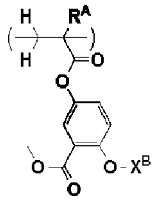
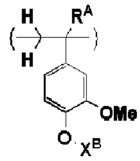
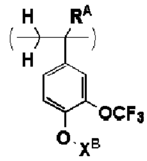

The base polymer preferably further has a repeating unit represented by the following formula (b1) (hereinafter, also referred to as the repeating unit b1) or a repeating unit represented by the following formula (b2) (hereinafter, also referred to as the repeating unit b2) .

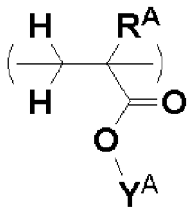
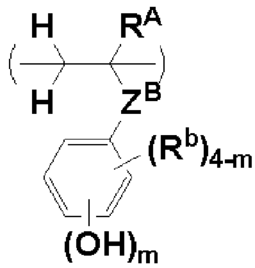

(b1) (b2)

In the formulae (b1) and (b2), $R^A$ and $Z^B$ represent the same as above. $Y^A$ represents a hydrogen atom or a polar group having one or more structures selected from a hydroxy group other than a phenolic hydroxy group, a cyano group, a carbonyl group, a carboxy group, an ether bond, an ester bond, a sulfonate ester bond, a sulfonamide bond, a carbonate bond, a lactone ring, a sultone ring, a sulfur atom, and a carboxylic anhydride. "m" represents an integer of 1 to 4.

$Y^A$ may be a hydrogen atom or a polar group having one or more structures selected from a hydroxy group other than a phenolic hydroxy group, a cyano group, a carbonyl group, a carboxy group, an ether bond, an ester bond, a sulfonate ester bond, a carbonate bond, a lactone ring, a sultone ring, and a carboxylic anhydride.

Examples of the repeating unit b1 include the following repeating units, but the repeating unit is not limited thereto. In the following formulae, $R^A$ represents the same as above.

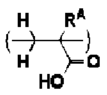
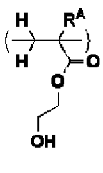
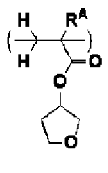
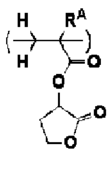
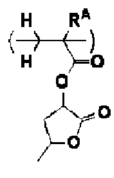
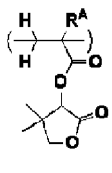

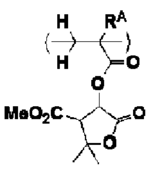
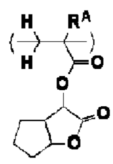
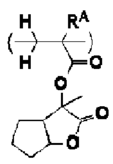
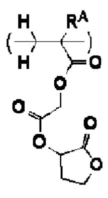
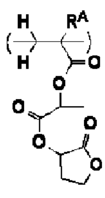
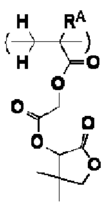

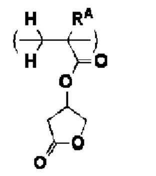 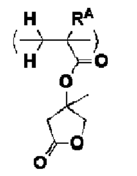 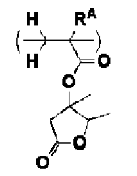 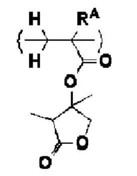 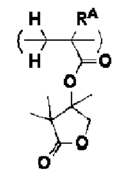 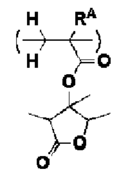

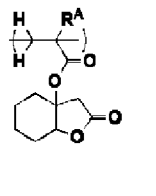 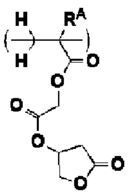 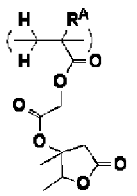 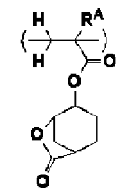 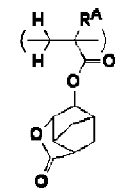 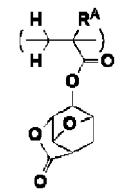

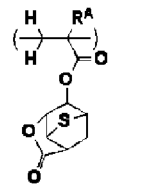 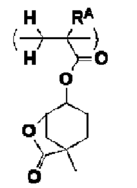 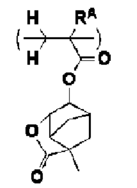 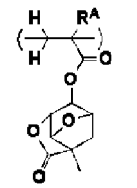 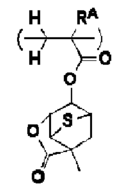 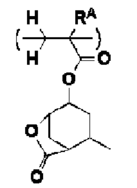

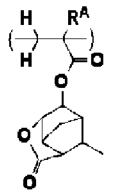 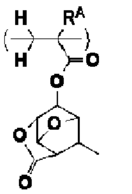 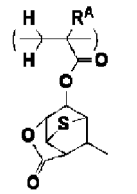 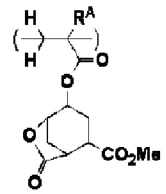 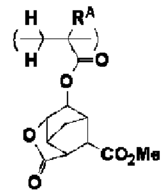 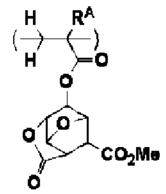

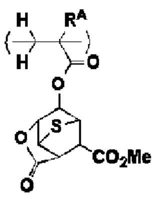 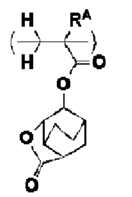 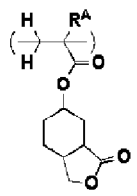 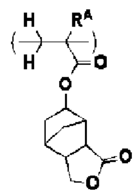 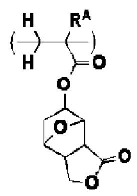 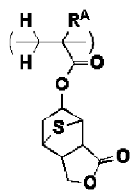

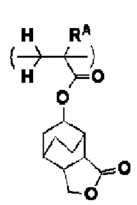 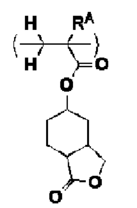 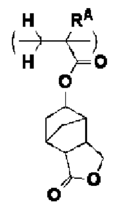 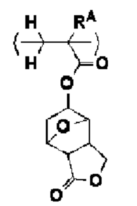 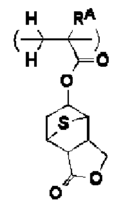 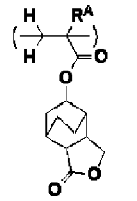

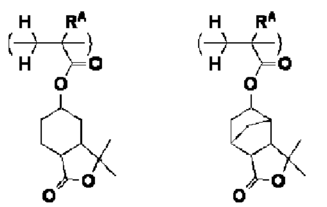 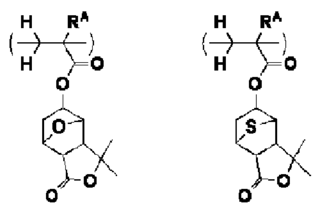 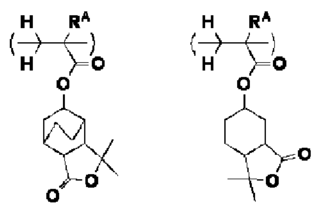

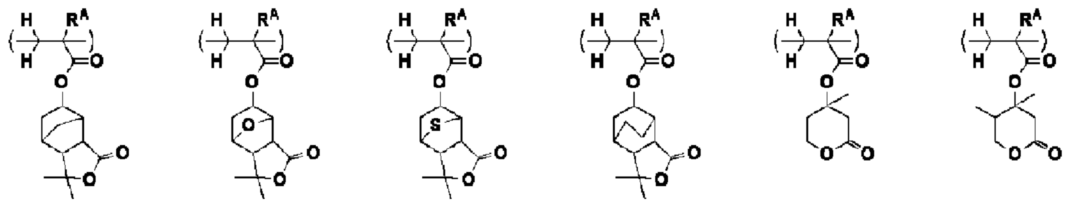

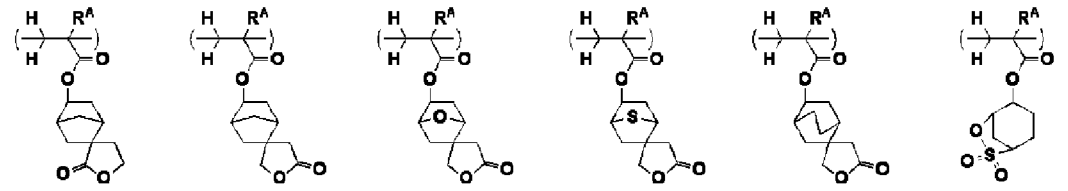

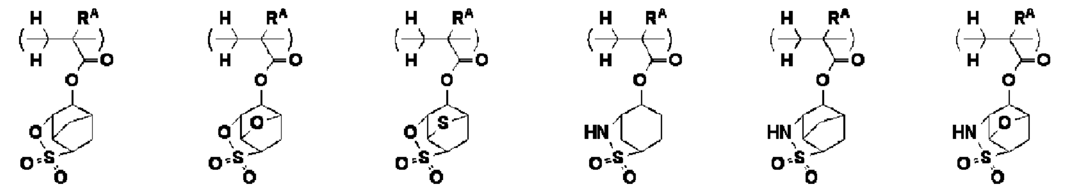

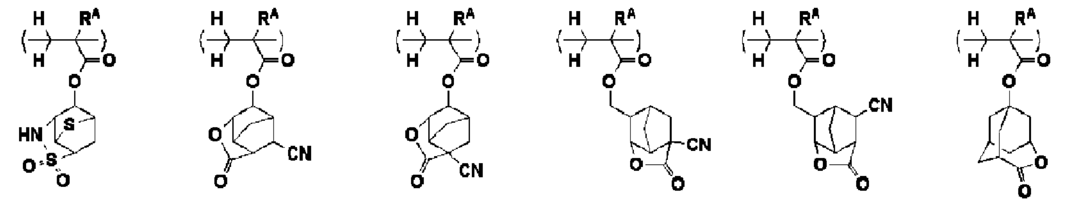

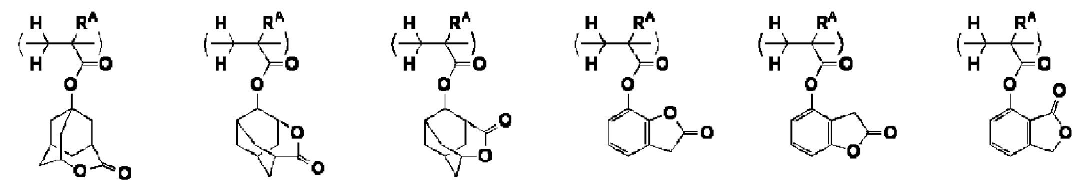

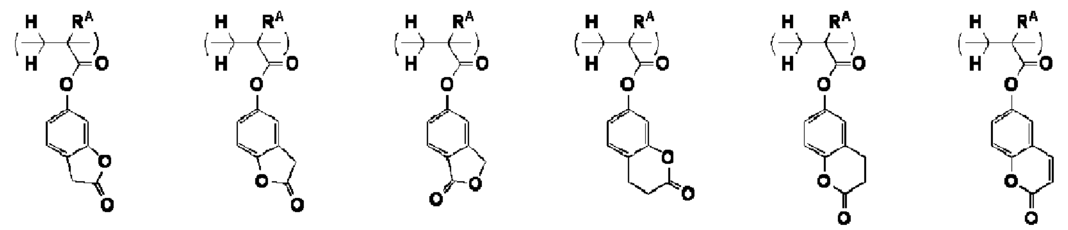

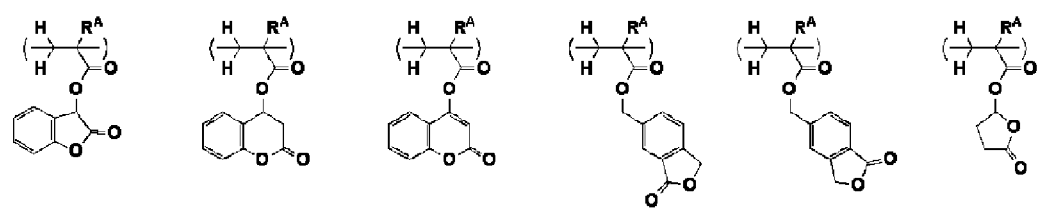

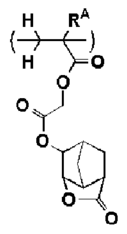 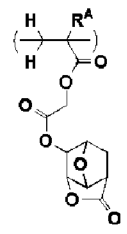 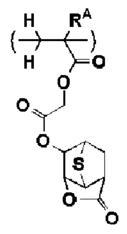 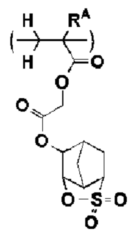 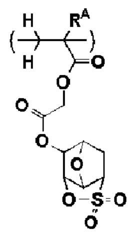 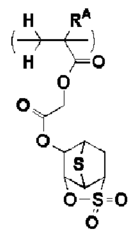

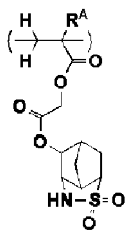 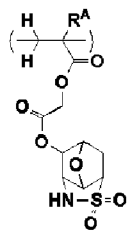 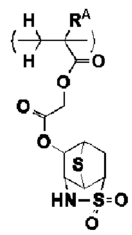 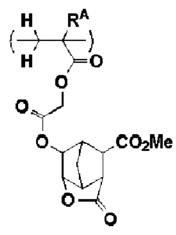 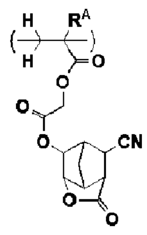 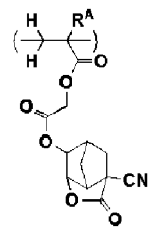

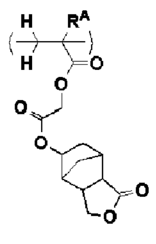 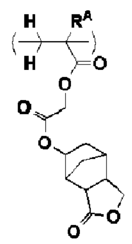 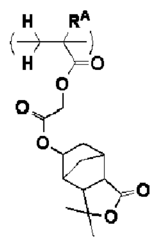 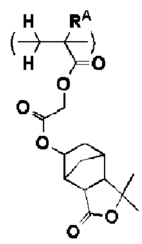 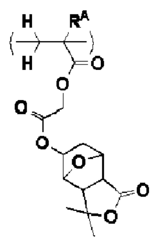 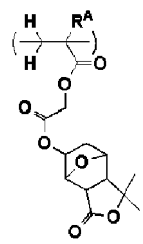

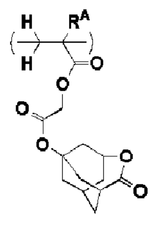 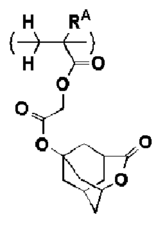 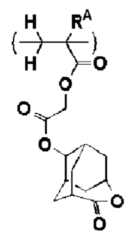 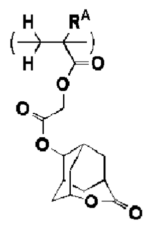 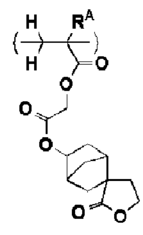 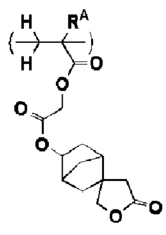

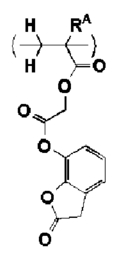 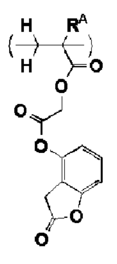 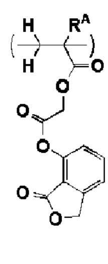 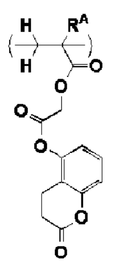 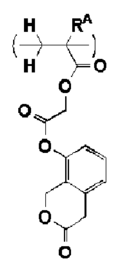 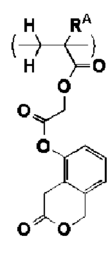

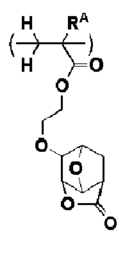 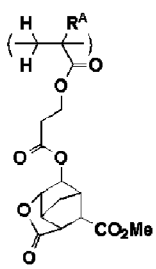 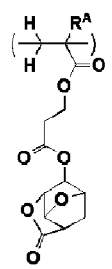 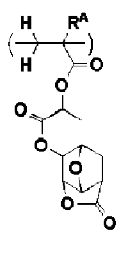 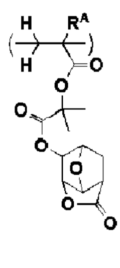 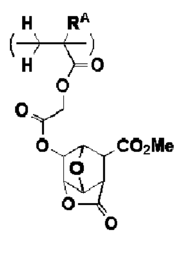

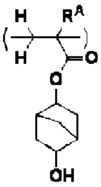
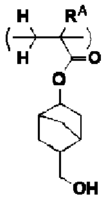
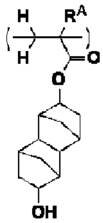
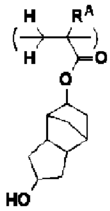
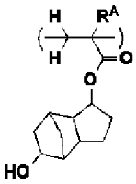
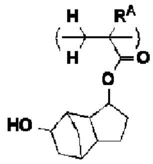

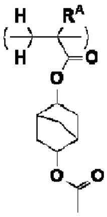
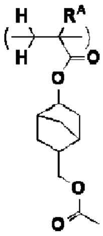
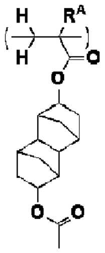
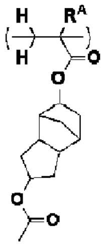
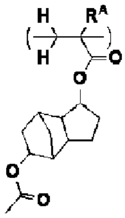
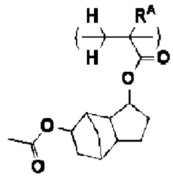

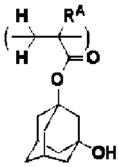
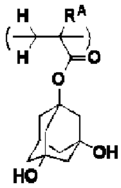
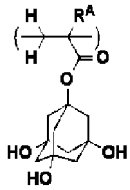
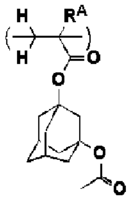
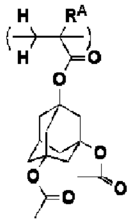
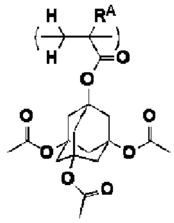

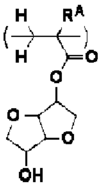
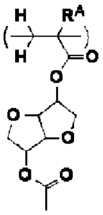
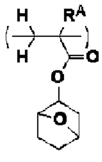
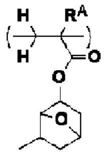
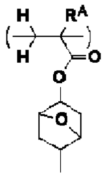
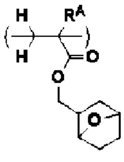

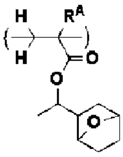
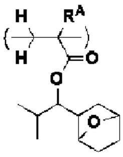
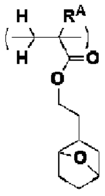
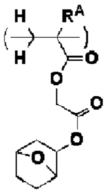
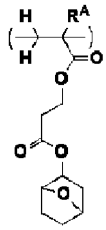
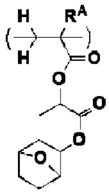

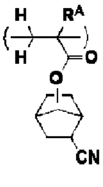
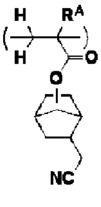
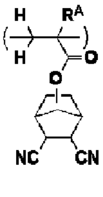
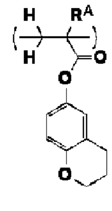
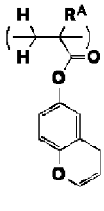
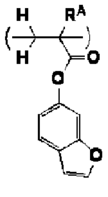

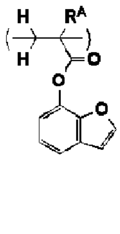 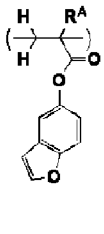 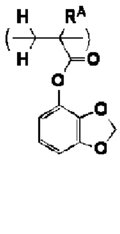 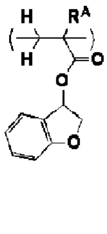 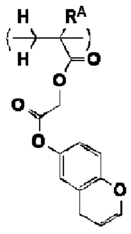 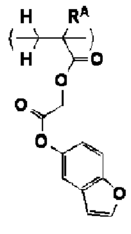

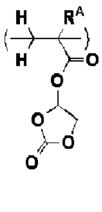 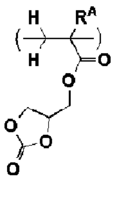 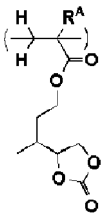 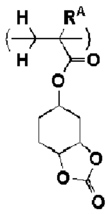 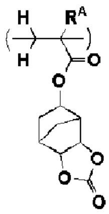 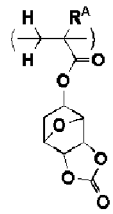

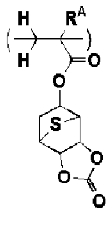 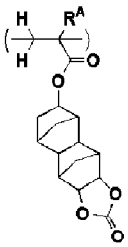 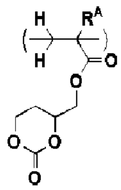 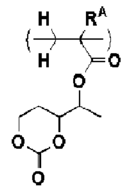 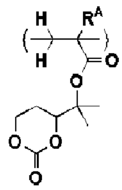 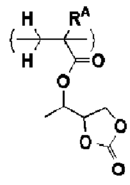

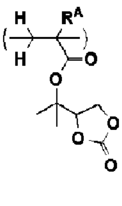 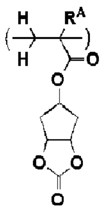 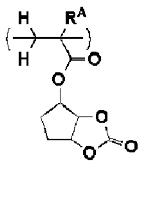 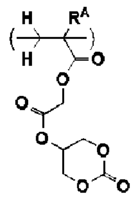 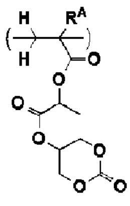 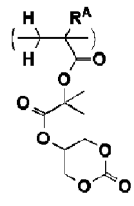

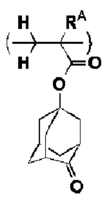 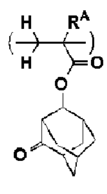 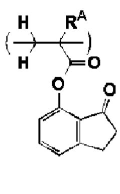 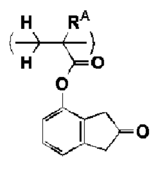 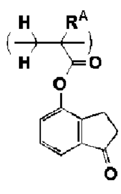 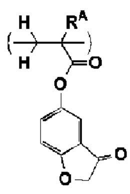

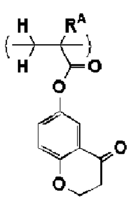 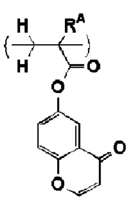 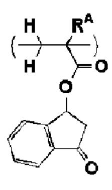

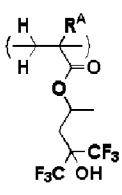 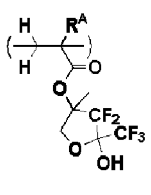 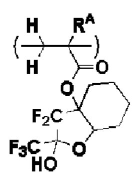 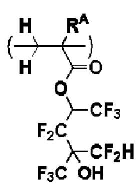 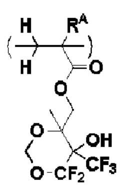 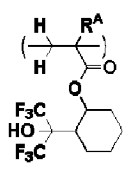

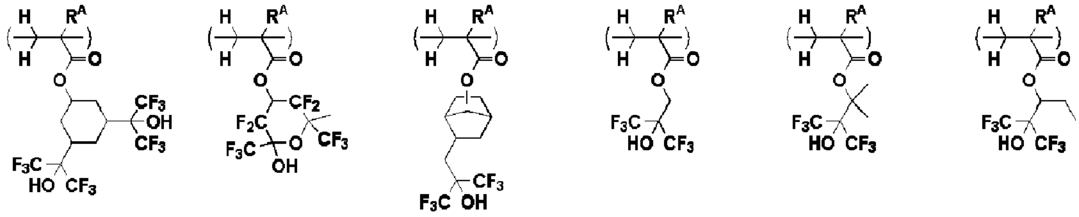

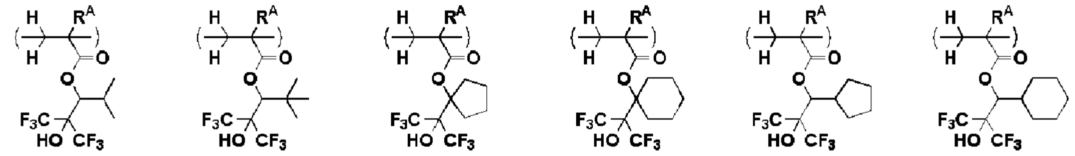

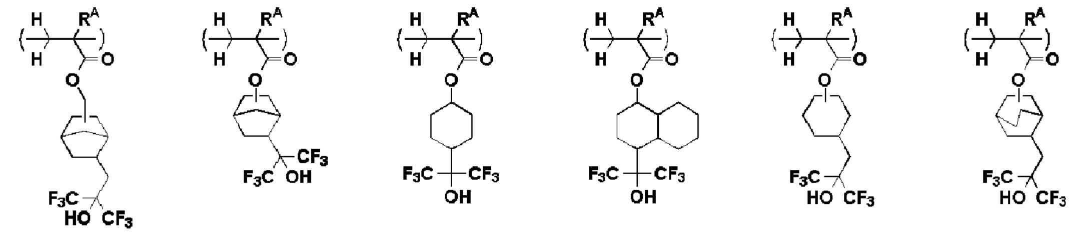

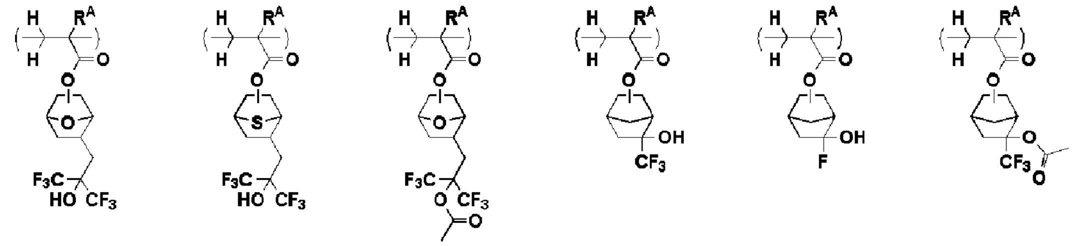

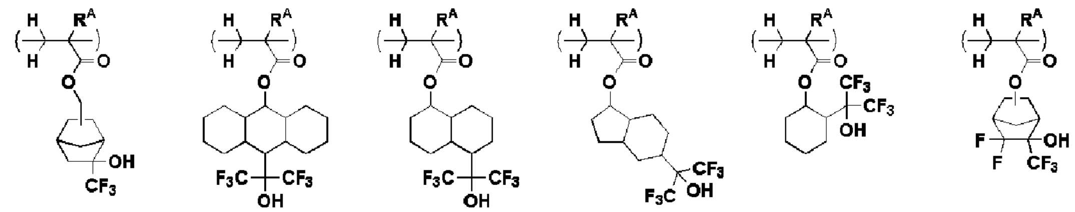

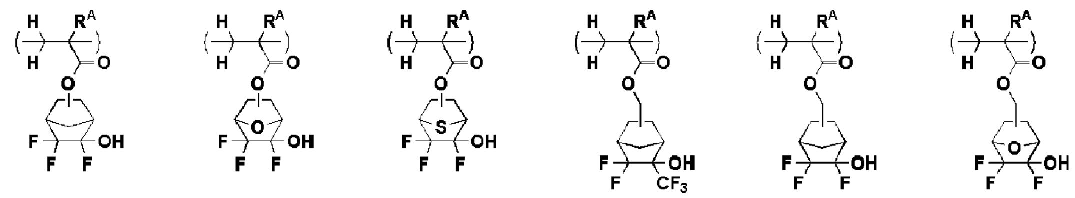

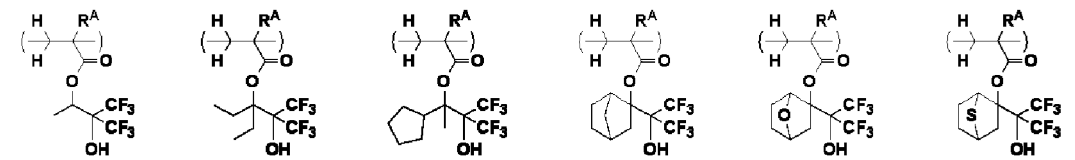

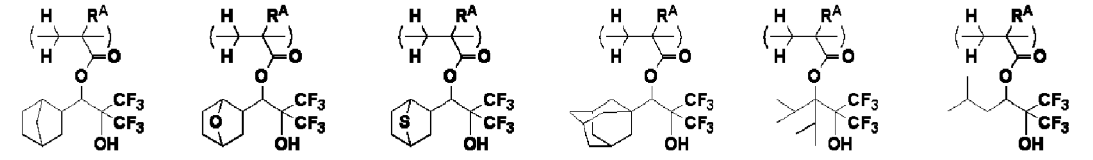

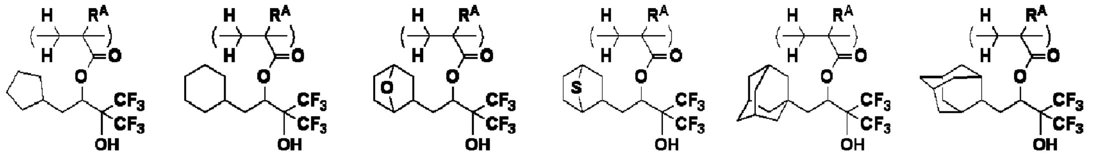

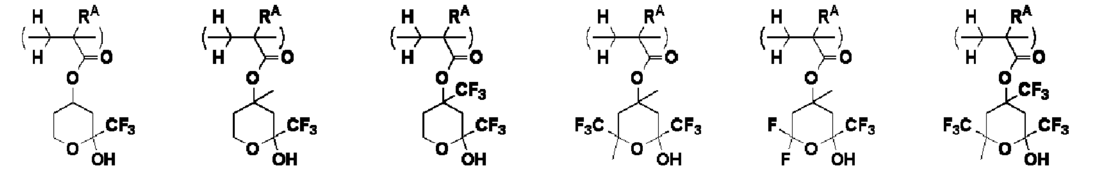

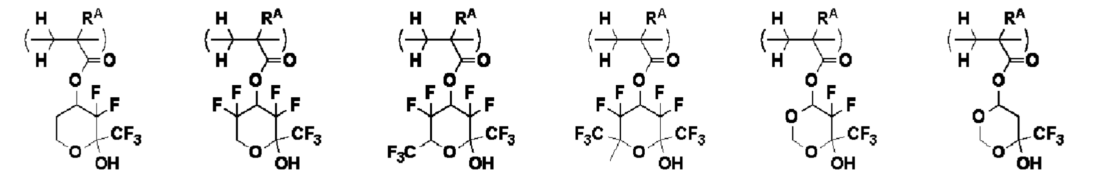

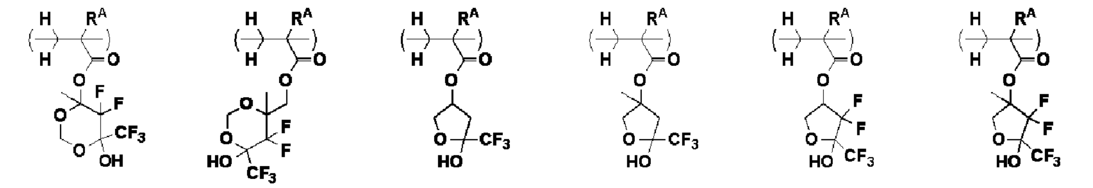

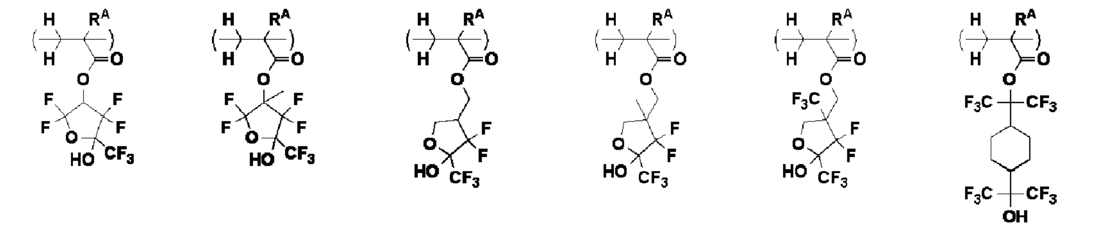

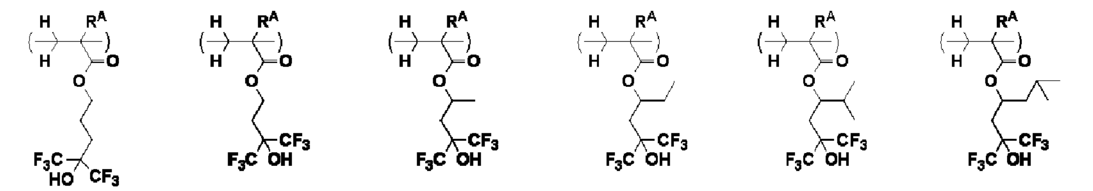

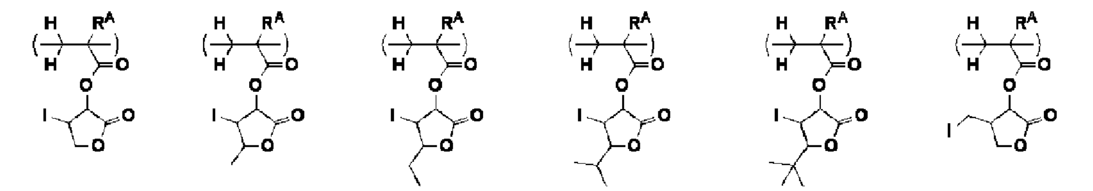

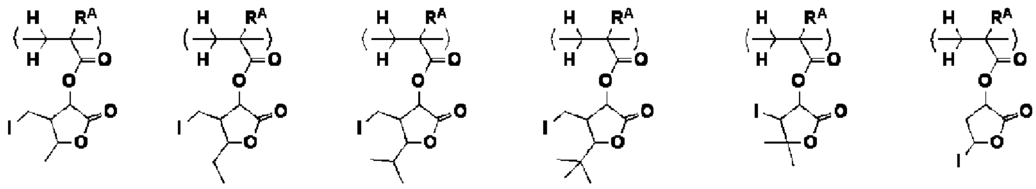

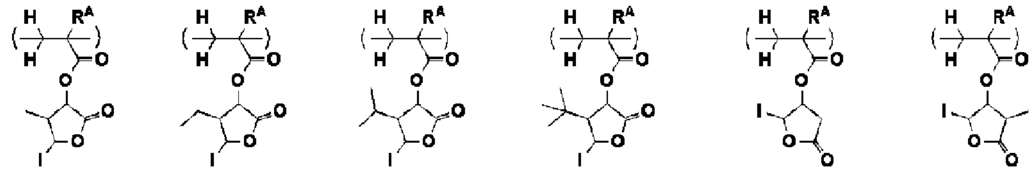

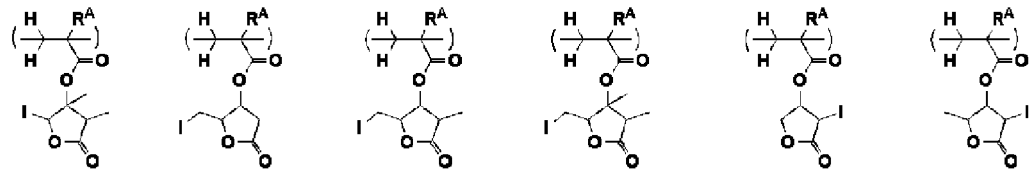

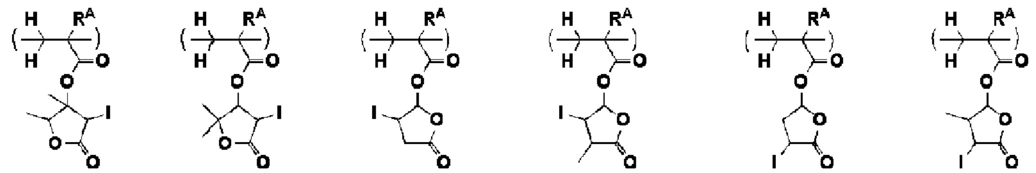

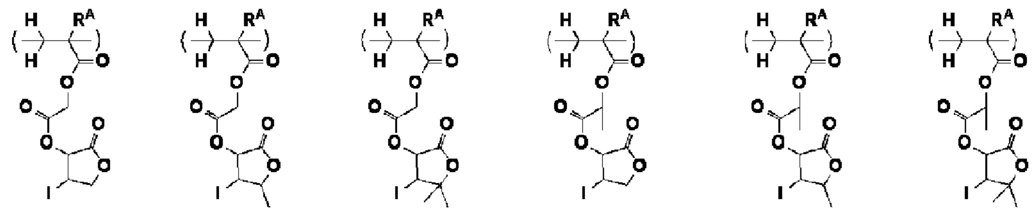

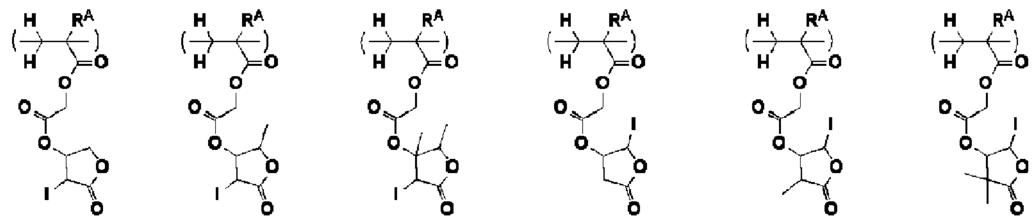

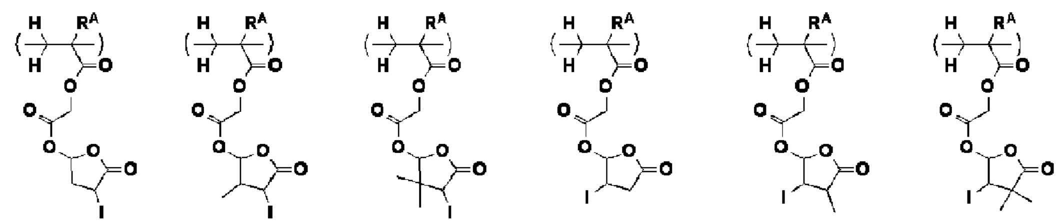

Examples of the repeating unit b2 include the following repeating units, but the repeating unit is not limited thereto. In the following formulae, $R^A$ represents the same as above.

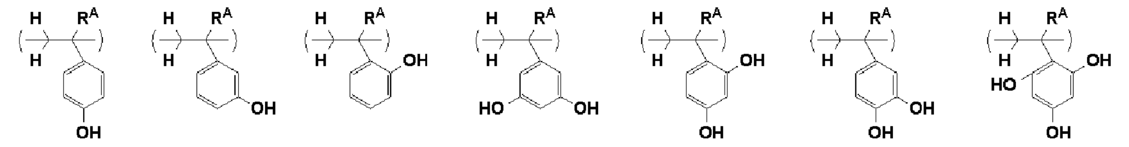

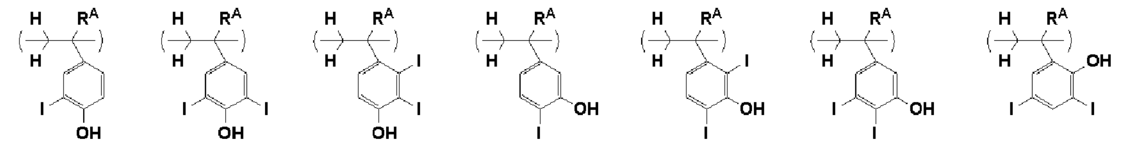

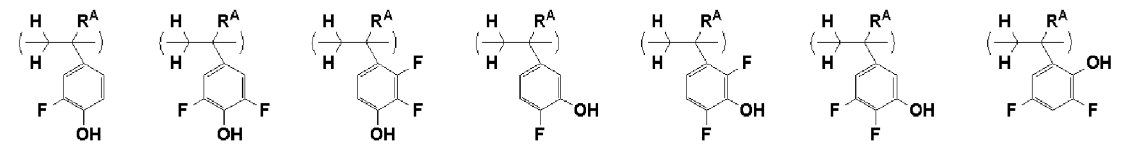

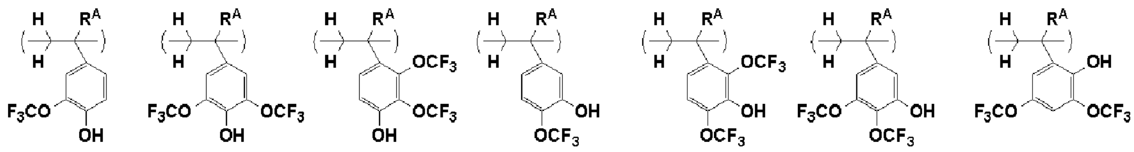

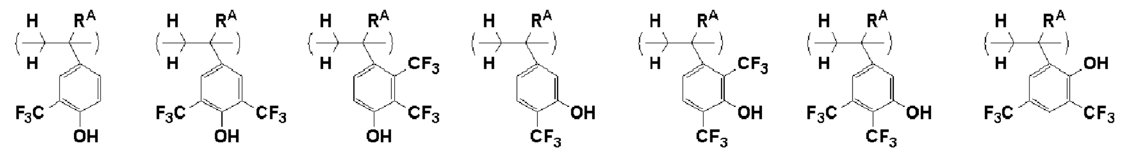

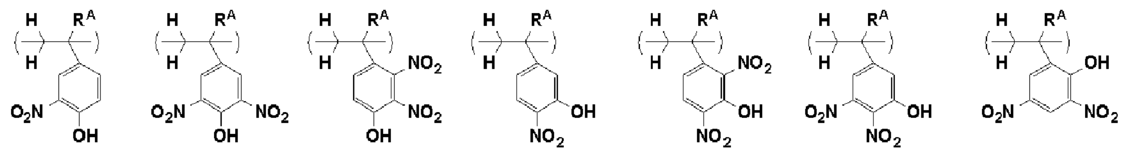

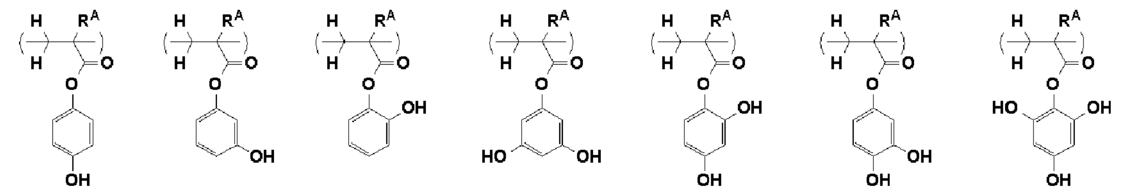

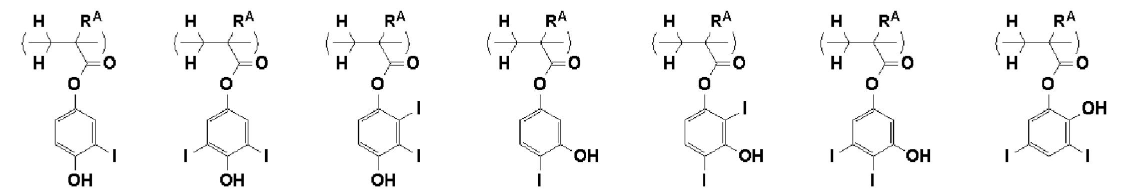

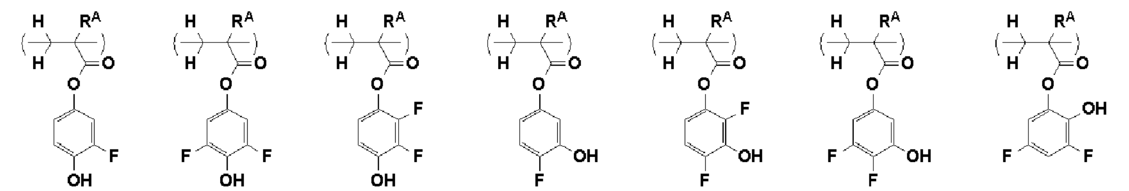

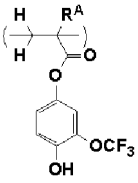 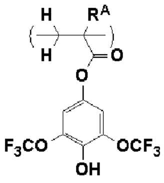 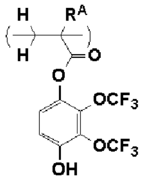 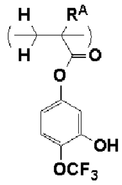 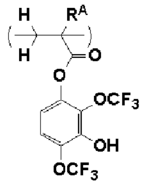 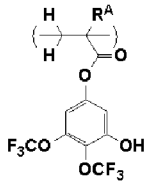 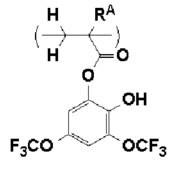

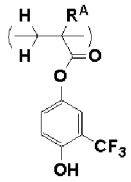 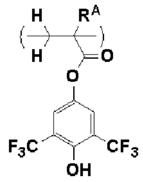 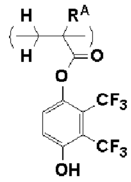 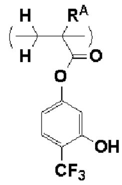 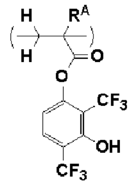 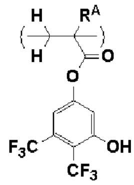 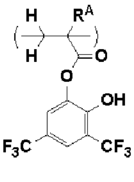

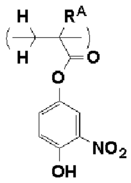 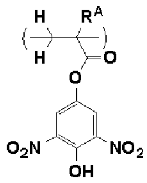 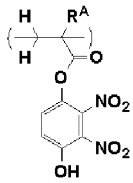 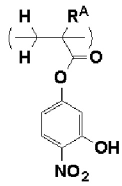 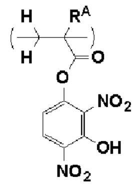 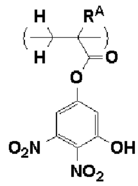 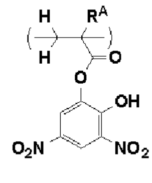

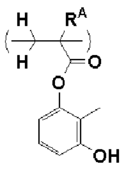 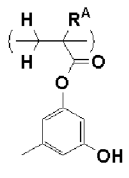 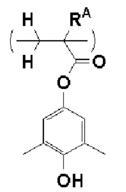 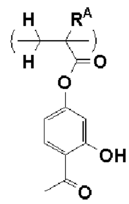 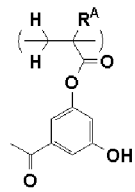 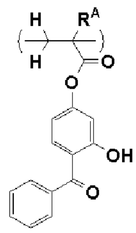 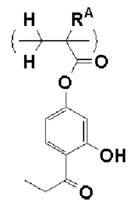

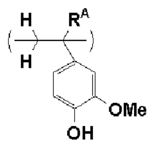 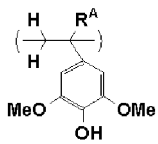 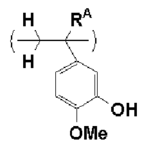 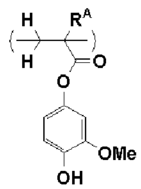 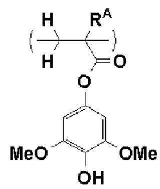 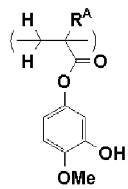

The repeating unit b1 or b2 particularly preferably has a lactone ring as the polar group in ArF lithography, and preferably has a phenol portion in KrF lithography, EB lithography, and EUV lithography.

The base polymer optionally further has a repeating unit represented by any one of the following formulae (C1) to (C4) (hereinafter, also referred to as the repeating units c1 to c4, respectively),

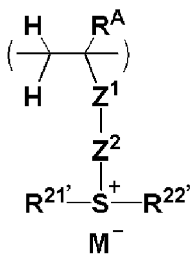
(C1)

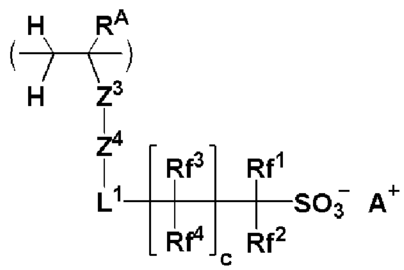
(C2)

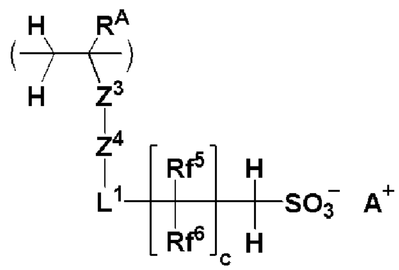
(C3)

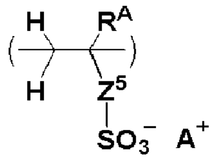

(C4)

wherein $R^A$ represents the same as above.

In the formulae (C1) to (C4), $R^A$ represent the same as above. $Z^1$ represents a single bond or a phenylene group. $Z^2$ represents a single bond, *-C(=O)-O-$Z^{21}$-, *-C(=O)-NH-$Z^{21}$-, or *-O-$Z^{21}$-. $Z^{21}$ represents an aliphatic hydrocarbylene group having 1 to 6 carbon atoms, a phenylene group, or a divalent group obtained by combining these groups, $Z^{21}$ optionally having a carbonyl group, an ester bond, an ether bond, or a hydroxy group. $Z^3$ represents a single bond, a phenylene group, a naphthylene group, or *-C(=O)-O-$Z^{31}$-. $Z^{31}$ represents an aliphatic hydrocarbylene group having 1 to 10 carbon atoms, a phenylene group, or a naphthylene group, the hydrocarbylene group optionally having a hydroxy group, an ether bond, an ester bond, or a lactone ring. $Z^4$ represents a single bond, a methylene group, or *-$Z^{41}$-C(=O)-O-. $Z^{41}$ represents a hydrocarbylene group having 1 to 20 carbon atoms and optionally having a heteroatom, an ether bond, or an ester bond. $Z^5$ represents a single bond, a methylene group, an ethylene group, a phenylene group, a fluorinated phenylene group, a phenylene group substituted with a trifluoromethyl group, *-C(=O)-O-$Z^{51}$-, *-C(=O)-N(H)-$Z^{51}$-, or *-O-$Z^{51}$-. $Z^{51}$ represents an aliphatic hydrocarbylene group having 1 to 6 carbon atoms, a phenylene group, a fluorinated phenylene group, or a phenylene group substituted with a trifluoromethyl group, and $Z^{51}$ optionally has a carbonyl group, an ester bond, an ether bond, or a hydroxy group. "*" represents an attachment point to a carbon atom in the main chain or to a group bonding to the main chain.

The aliphatic hydrocarbylene groups represented by $Z^{21}$, $Z^{31}$, and $Z^{51}$ may be any of linear, branched, and cyclic groups, and specific examples thereof include groups same as those exemplified in the description of $Z^A$ in the formula (a1) .

The hydrocarbylene group represented by $Z^{41}$ may be a saturated or unsaturated group, and may be any of linear, branched, and cyclic groups. Specific examples thereof include the following groups, but the aliphatic hydrocarbylene group is not limited thereto.

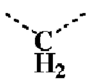 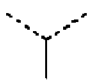 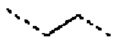 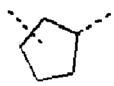 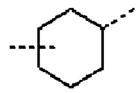 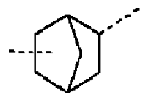

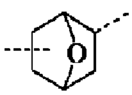 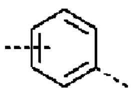 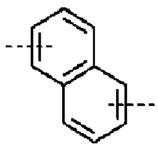 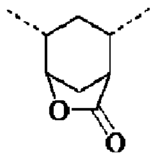 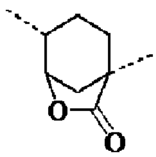 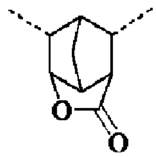

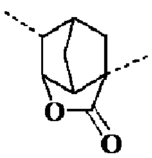 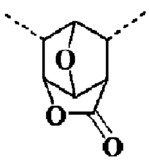 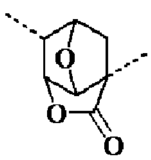 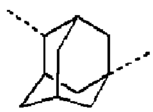 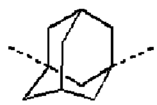

In the formulae, a broken line represents an attachment point.

In the formula (C1), $R^{21'}$ and $R^{22'}$ each independently represent a hydrocarbyl group having 1 to 20 carbon atoms and optionally having a heteroatom. The hydrocarbyl groups represented by $R^{21'}$ and $R^{22'}$ may be a saturated or unsaturated group, and may be any of linear, branched, and cyclic groups. Specific examples thereof include: alkyl groups, such as a methyl group, an ethyl group, an n-propyl group, an isopropyl group, an n-butyl group, and a tert-butyl group; cyclic saturated hydrocarbyl groups, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups, such as a vinyl group, an allyl group, a propenyl group, a butenyl group, and a hexenyl group; cyclic unsaturated hydrocarbyl groups, such as a cyclohexenyl group; aryl groups, such as a phenyl group, a naphthyl group, and a thienyl group; aralkyl groups, such as a benzyl group, a 1-phenylethyl group, and a 2-phenylethyl group;

and a group obtained by combining these groups. The hydrocarbyl group is preferably an aryl group. A part of hydrogen atoms in the hydrocarbyl group is optionally substituted with a group having a heteroatom, such as an oxygen atom, a sulfur atom, a nitrogen atom, and a halogen atom. Between carbon atoms in these groups, a group having a heteroatom such as an oxygen atom, a sulfur atom, and a nitrogen atom is optionally interposed. As a result, optionally contained are a hydroxy group, a cyano group, a carbonyl group, an ether bond, an ester bond, a sulfonate ester bond, a carbonate bond, a lactone ring, a sultone ring, a carboxylic anhydride, a haloalkyl group, etc.

$R^{21'}$ and $R^{22'}$ are optionally bonded each other to form a ring together with the sulfur atom to which $R^{21'}$ and $R^{22'}$ are bonded. Specific examples thereof include the following rings.

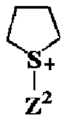
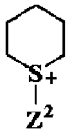
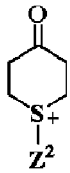
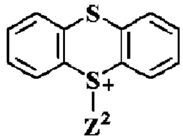
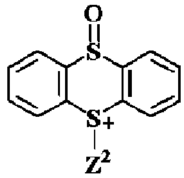
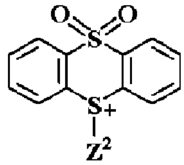

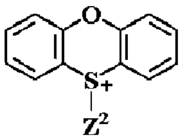
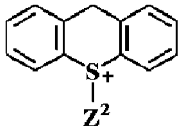
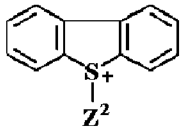
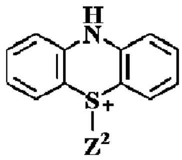
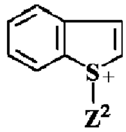

Examples of the cation of the repeating unit c1 include the following cations, but the cation is not limited thereto. In the following formulae, $R^A$ represents the same as above.

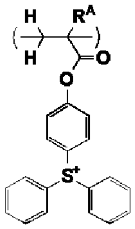
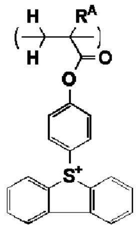
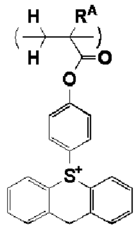
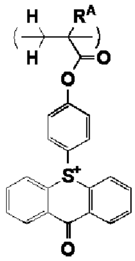
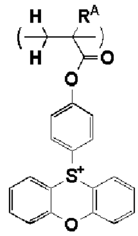
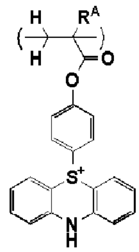

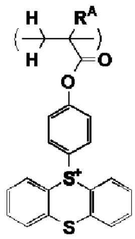
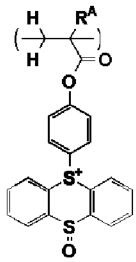
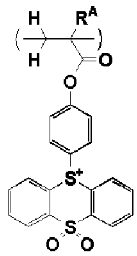
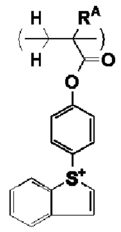
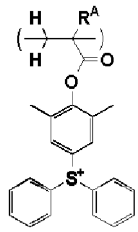
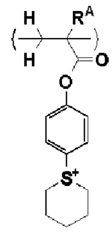

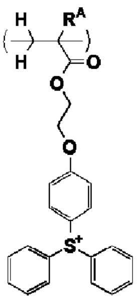
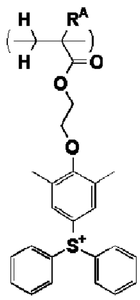
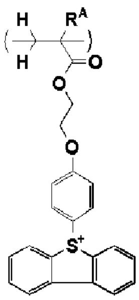
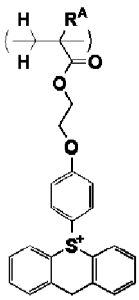
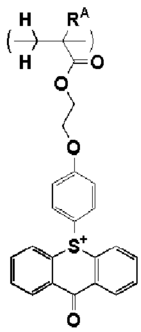
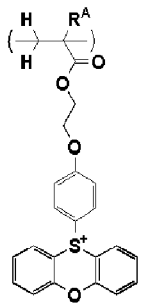

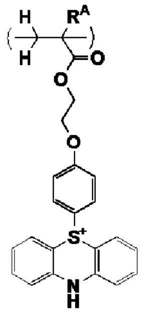
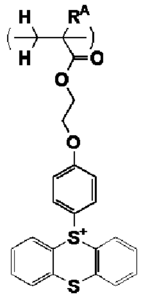
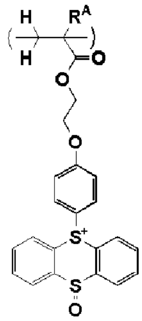
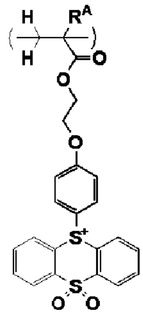
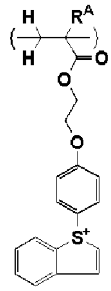
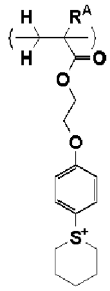

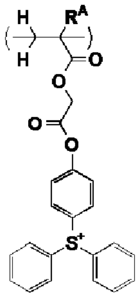
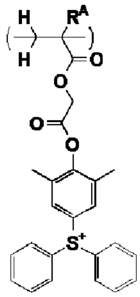
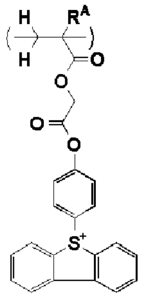
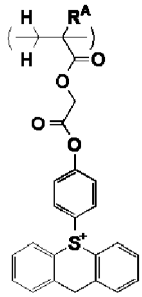
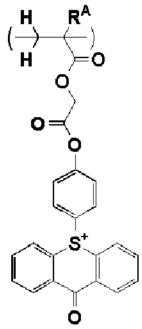
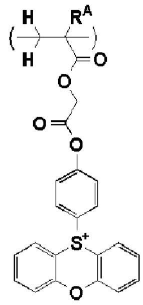

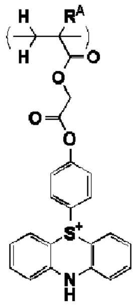
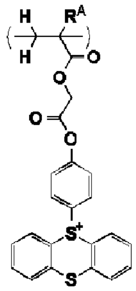
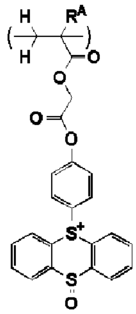
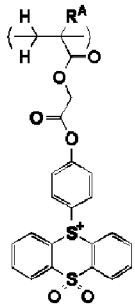
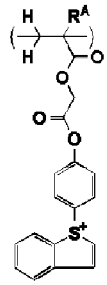
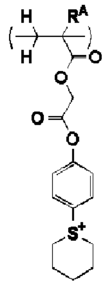

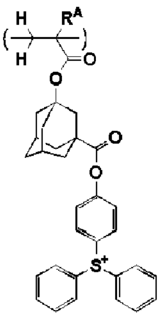
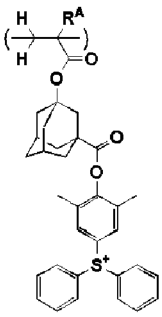
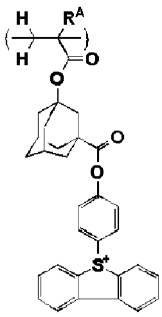
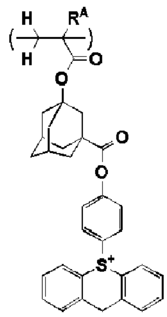
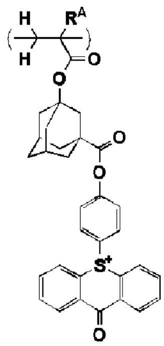
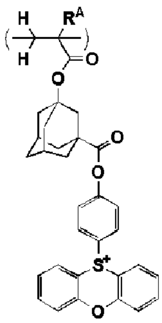

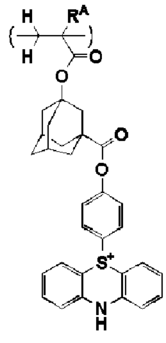 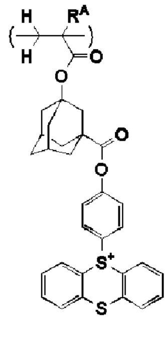 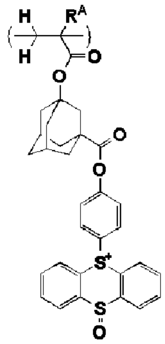 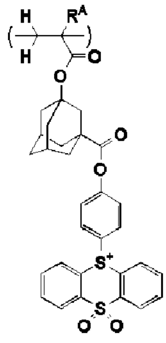 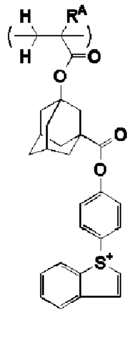 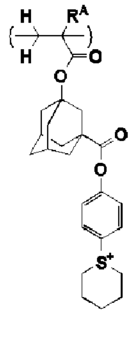

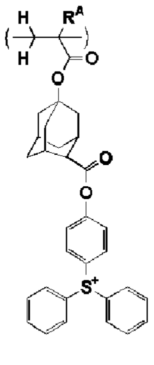 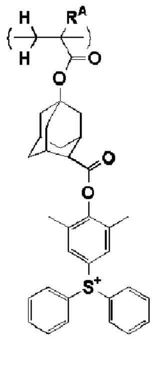 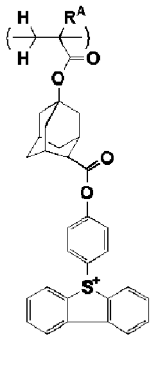 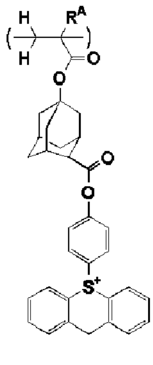 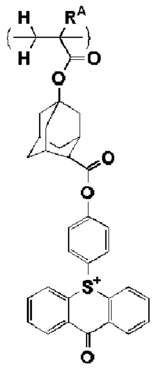 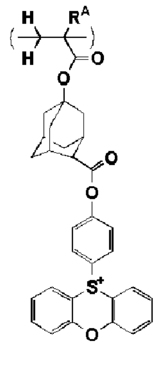

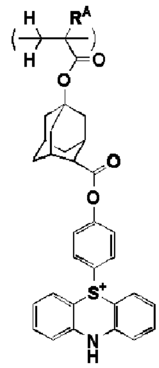 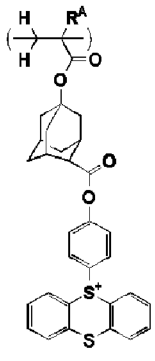 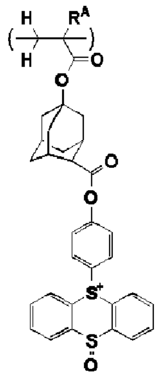 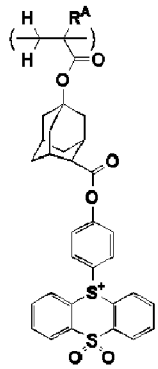 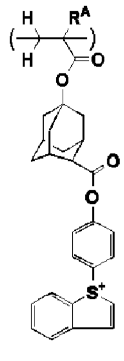 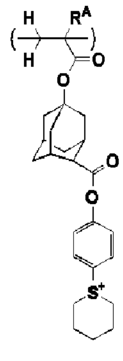

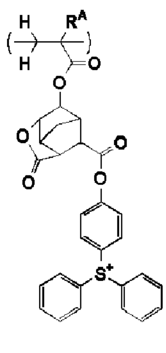 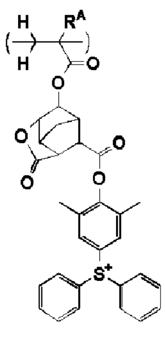 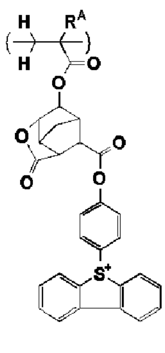 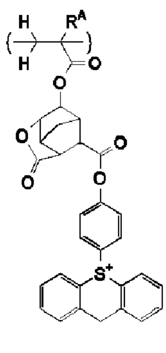 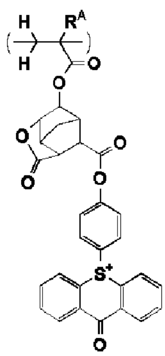 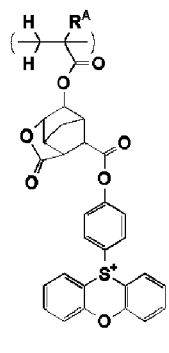

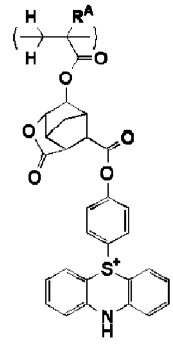 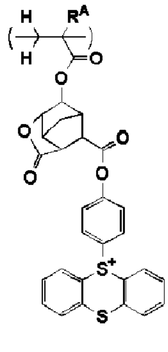 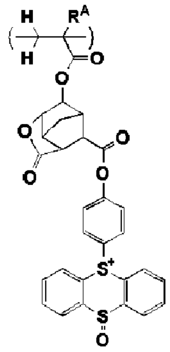 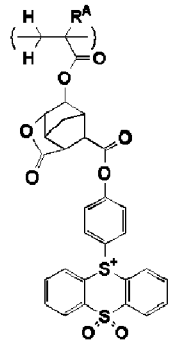 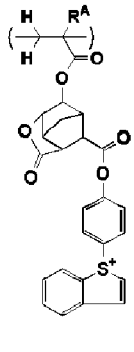 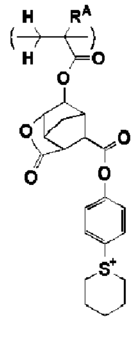

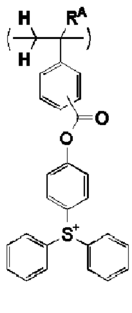 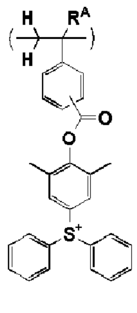 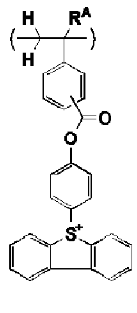 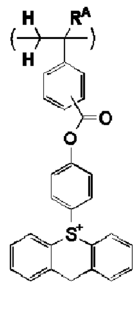 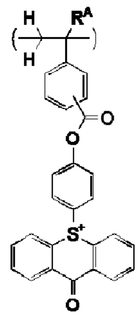 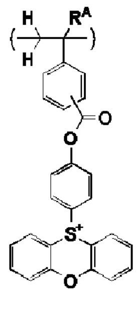

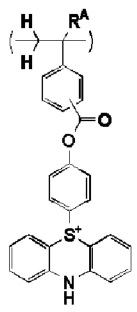 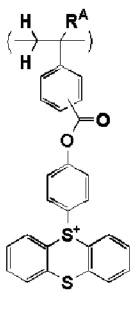 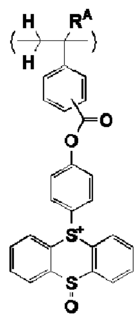 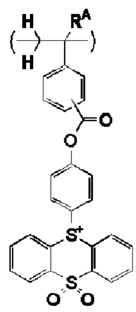 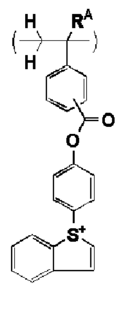 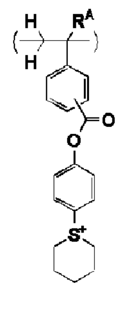

In the formula (C1), $M^-$ represents a non-nucleophilic counterion. Examples of the non-nucleophilic counterion include counterions same as those of X- in the formula (1).

In the formula (C2), $L^1$ represents a single bond, an ether bond, an ester bond, a carbonyl group, a sulfonate ester bond, a carbonate bond, or a carbamate bond. Among these, an ether bond, an ester bond, and a carbonyl group are preferable from the viewpoint of the synthesis, and an ester bond and a carbonyl bond are further preferable.

In the formula (C2), $Rf^1$ and $Rf^2$ each independently represent a fluorine atom or a fluorinated alkyl group having 1 to 6 carbon atoms. Among these, both $Rf^1$ and $Rf^2$ preferably represent fluorine atoms for increasing the acid strength of the generated acid. $Rf^3$ and $Rf^4$ each independently represent a hydrogen atom, a fluorine atom or a fluorinated alkyl group having 1 to 6 carbon atoms. Among these, at least one of $Rf^3$ and $Rf^4$ preferably represents a trifluoromethyl group for improving the solvent solubility.

In the formula (C2), "c" represents an integer of 0 to 3, and preferably 1.

Specific examples of the anion of the repeating unit represented by the formula (C2) include the following anions, but the anion is not limited thereto. In the following formulae, $R^A$ represents the same as above.

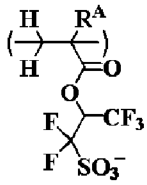 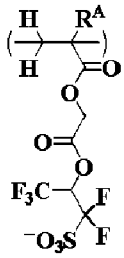 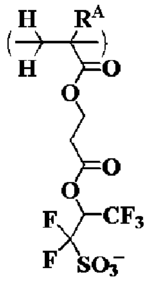 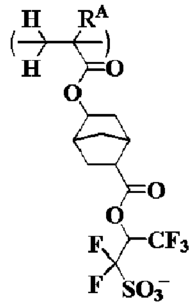 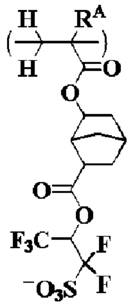 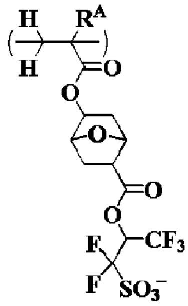

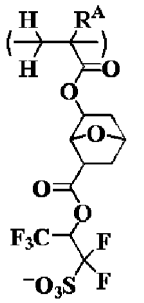 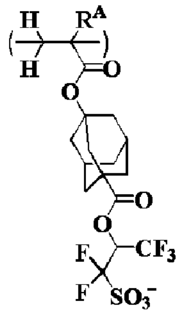 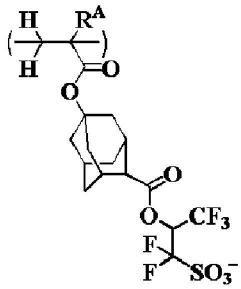 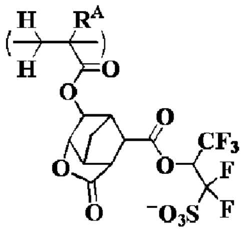 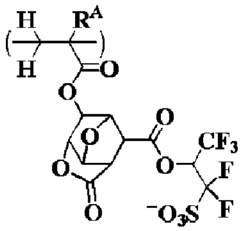

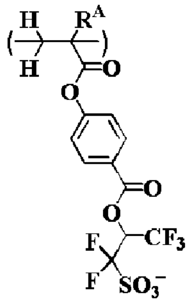 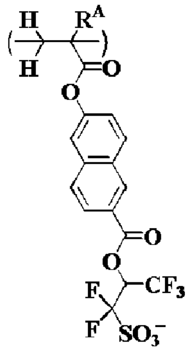 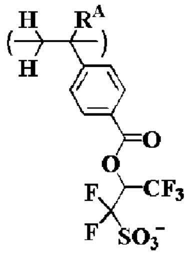 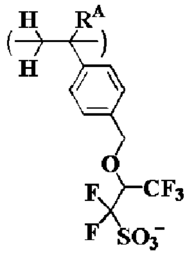

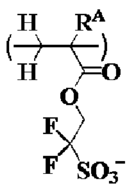 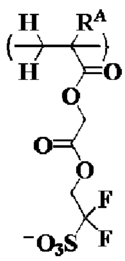 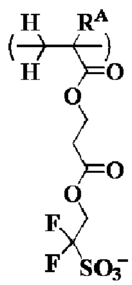 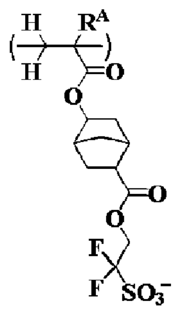 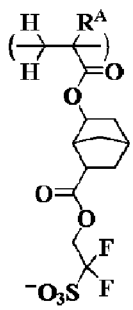 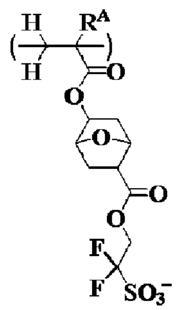

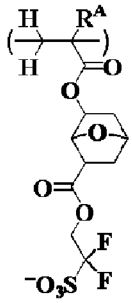 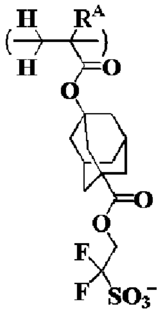 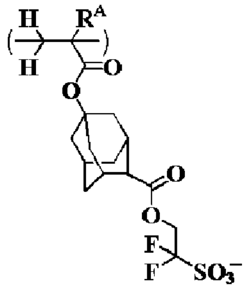 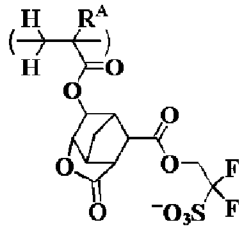 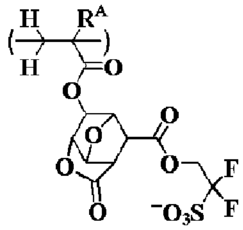

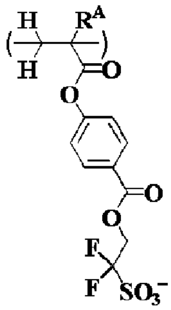
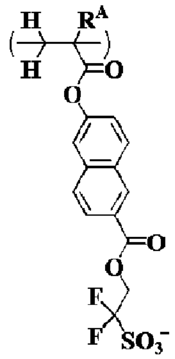
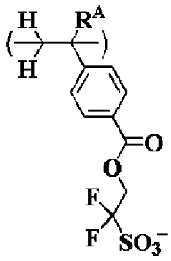
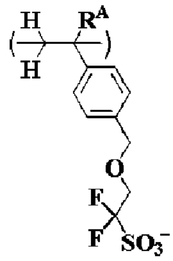

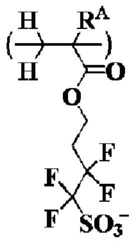
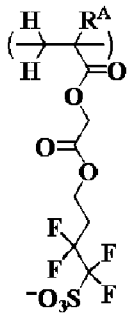
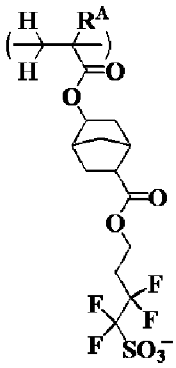
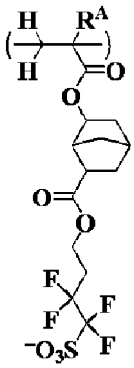
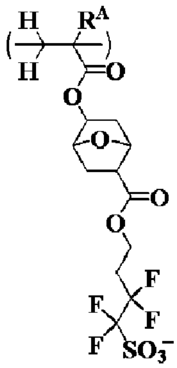
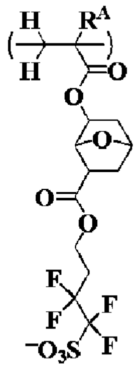

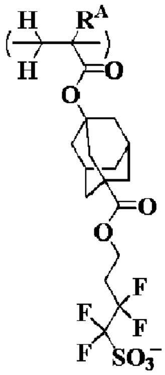
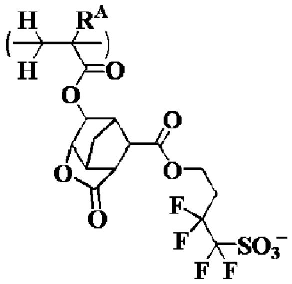
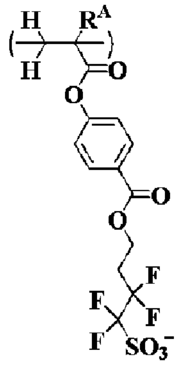
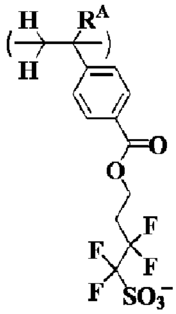

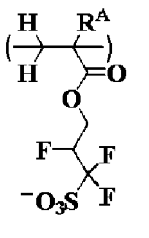
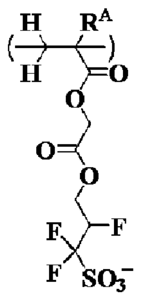
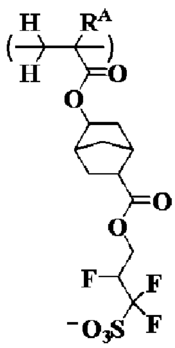
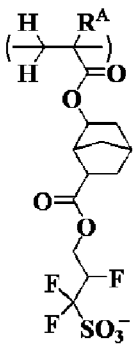
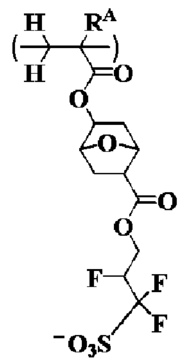
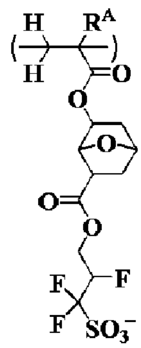

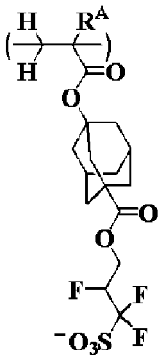
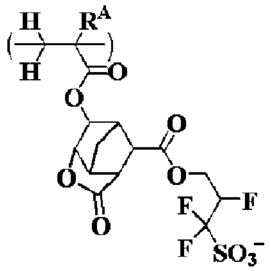
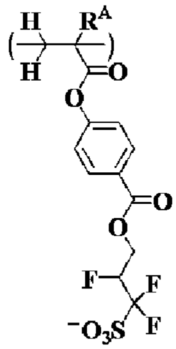
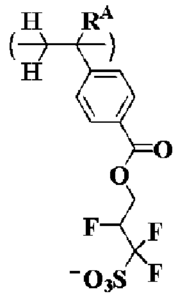

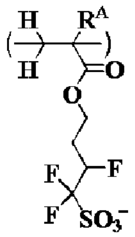
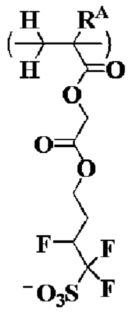
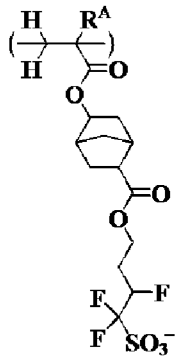
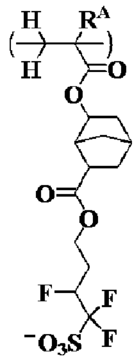
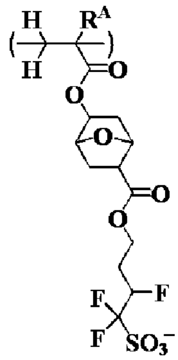
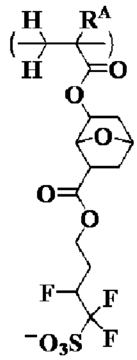

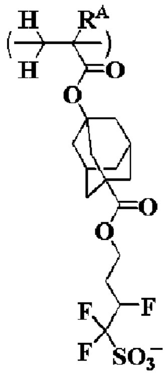
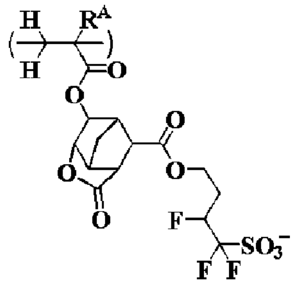
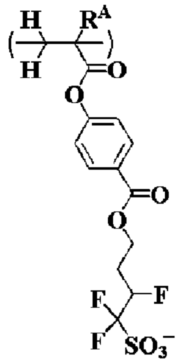
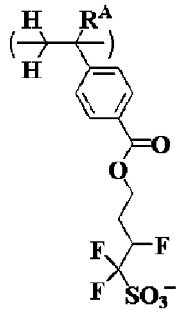

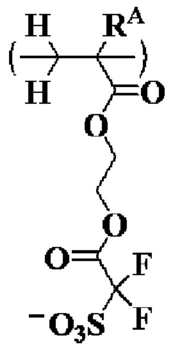
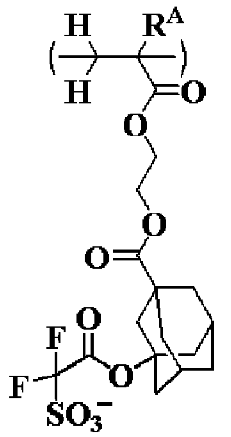
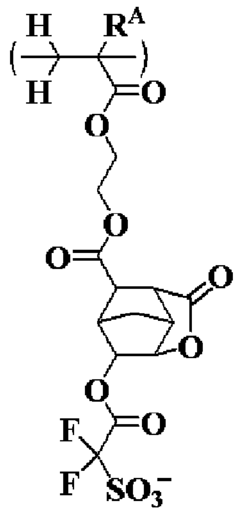
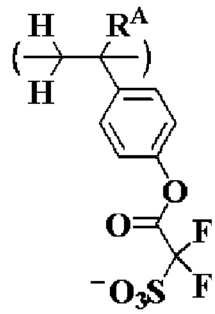

In the formula (C3), $L^1$ represents the same as above.

In the formula (C3), $Rf^5$ and $Rf^6$ each independently represent a hydrogen atom, a fluorine atom, or a fluorinated alkyl group having 1 to 6 carbon atoms. Among these, at least one of $Rf^5$ and $Rf^6$ preferably represents a trifluoromethyl group for improving the solvent solubility.

In the formula (C3), "c" represents an integer of 0 to 3, and preferably 1.

Specific examples of the anion of the repeating unit represented by the formula (C3) include the following anions, but the anion is not limited thereto. In the following formulae, $R^A$ represents the same as above.

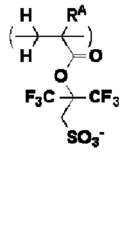
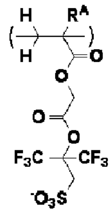
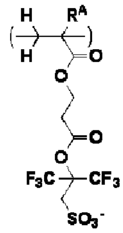
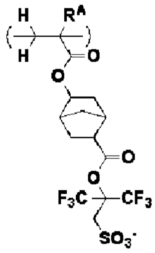
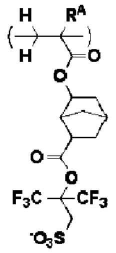
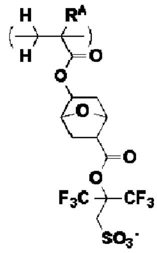
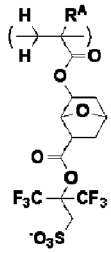

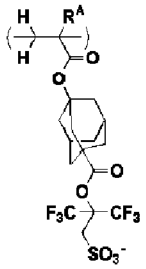
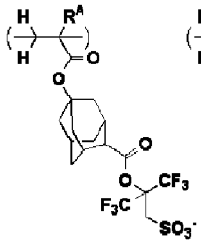
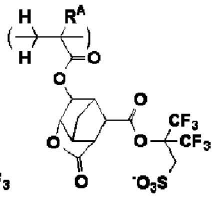
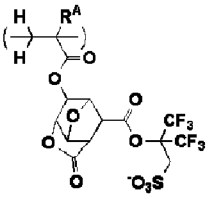
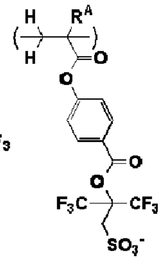
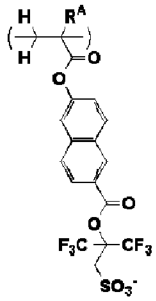

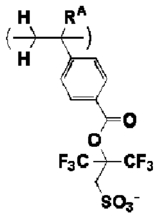
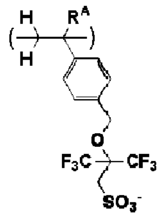

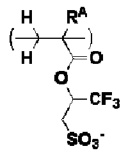
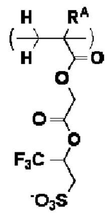
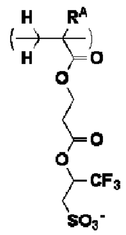
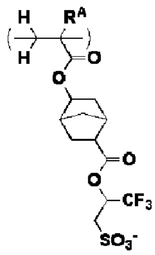
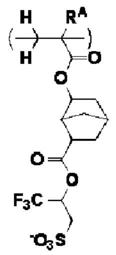
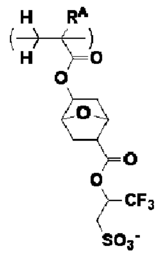
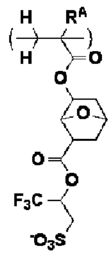

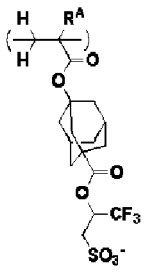
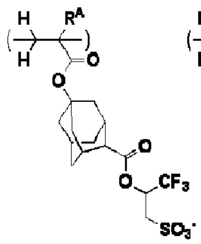
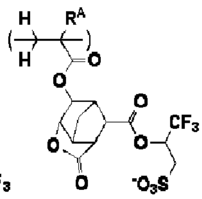
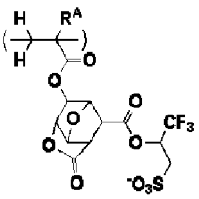
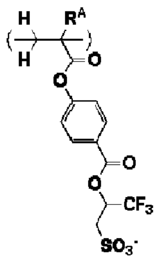
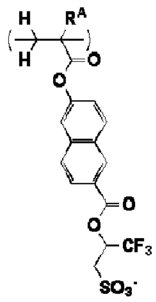

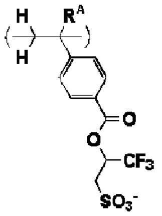
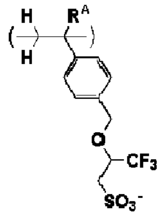

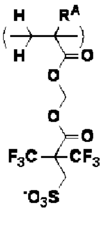
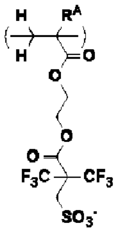
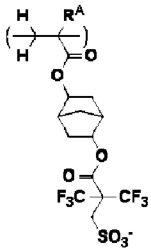
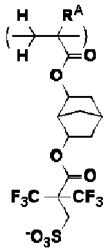
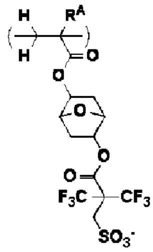
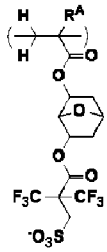
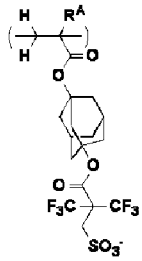

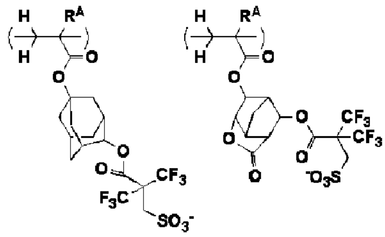
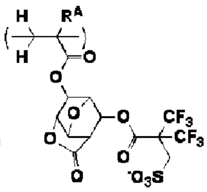
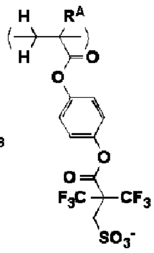
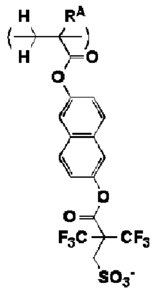
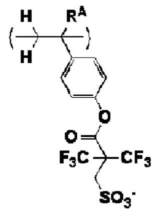

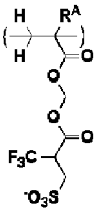
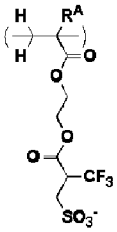
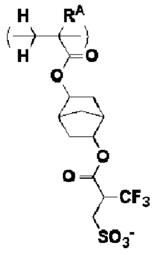
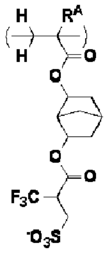
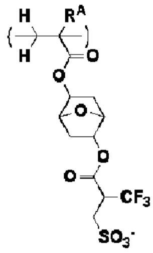
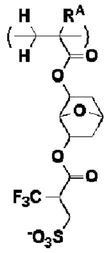
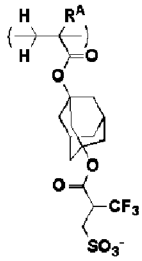

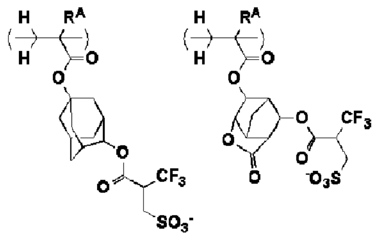
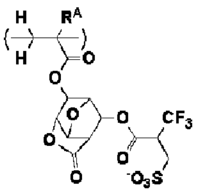
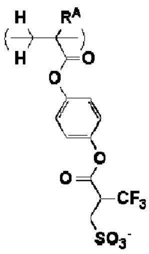
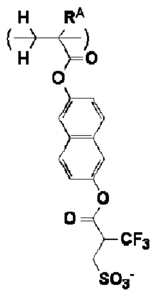
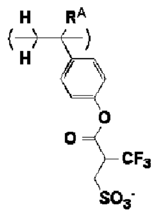

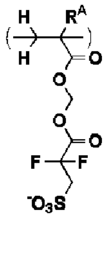 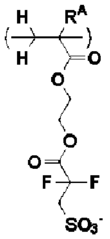 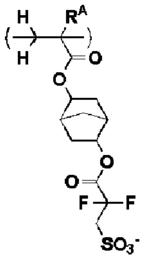 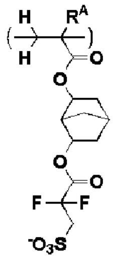 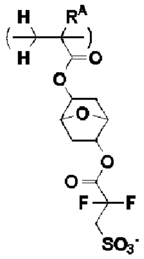 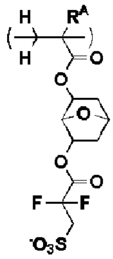 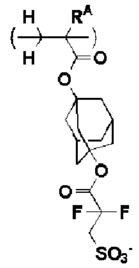

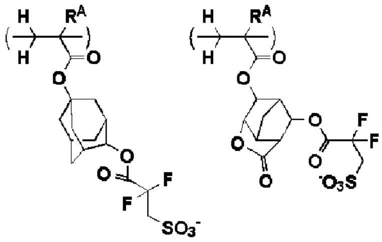 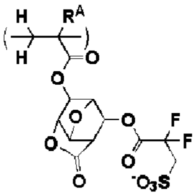 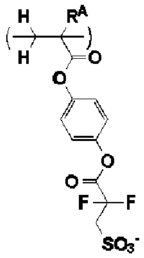 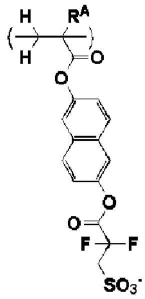 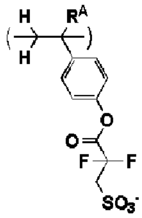

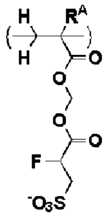 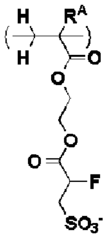 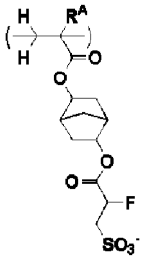 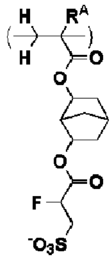 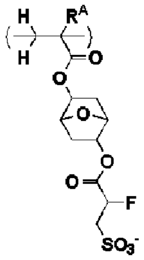 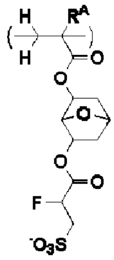 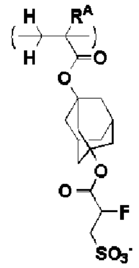

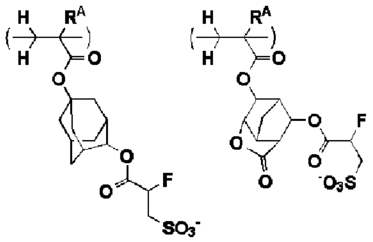 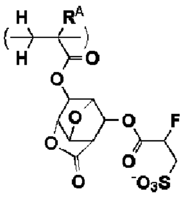 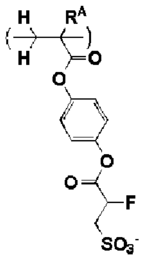 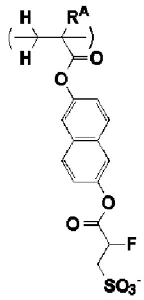 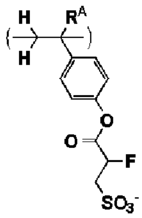

Specific examples of the anion of the repeating unit represented by the formula (C4) include the following anions, but the anion is not limited thereto. In the following formulae, $R^A$ represents the same as above.

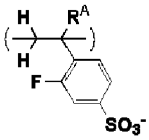 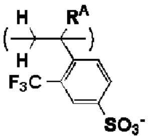 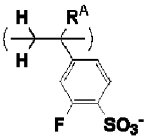 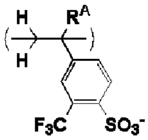 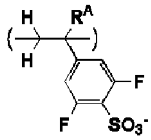 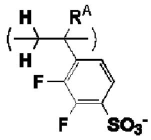 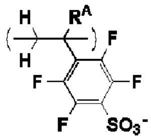

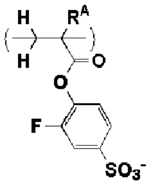 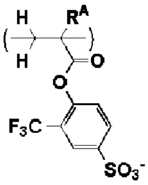 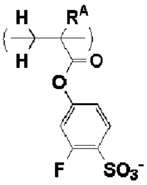 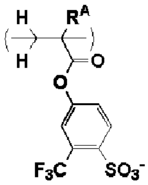 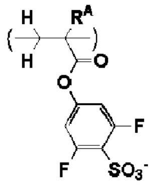 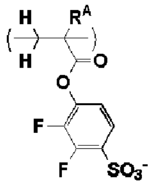 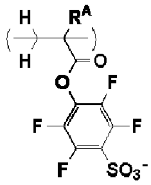

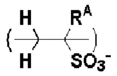 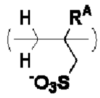 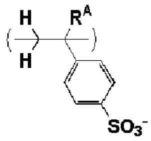 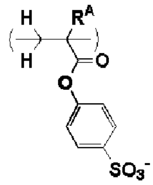 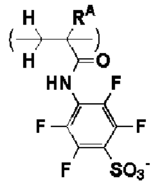

In the formulae (C2) to (C4), $A^+$ represents an onium cation. Examples of the onium cation include an ammonium cation, a sulfonium cation, and an iodonium cation. The onium cation is preferably a sulfonium cation or an iodonium cation, and more preferably a sulfonium cation represented by the following formula (cation-1) or an iodonium cation represented by the following formula (cation-2).

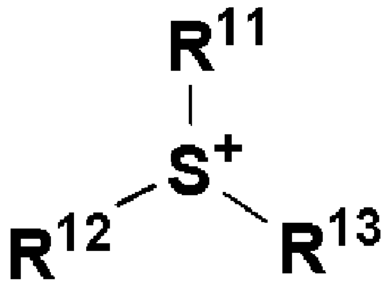

(cation-1)

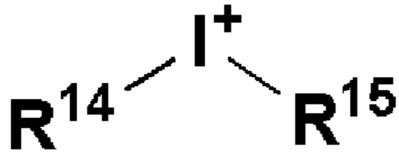

(cation-2)

In the formulae (cation-1) and (cation-2), $R^{11}$ to $R^{15}$ each independently represent a hydrocarbyl group having 1 to 30 carbon atoms and optionally having a heteroatom. The hydrocarbyl group may be a saturated or unsaturated group, and may be any of linear, branched, and cyclic groups. Specific examples thereof include: alkyl groups, such as a methyl group, an ethyl group, an n-propyl group, an isopropyl group, an n-butyl group, and a tert-butyl group; cyclic saturated hydrocarbyl groups, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups, such as a vinyl group, an allyl group, a propenyl group, a butenyl group, and a hexenyl group; cyclic unsaturated hydrocarbyl groups, such as a cyclohexenyl group; aryl groups, such as a phenyl group, a naphthyl group, and a thienyl group; aralkyl groups, such as a benzyl group, a 1-phenylethyl group, and a 2-phenylethyl group; and groups obtained by combining these groups. The hydrocarbyl group is preferably an aryl group. A part of hydrogen atoms in the hydrocarbyl group is optionally substituted with a group having a heteroatom, such as an oxygen atom, a sulfur atom, a nitrogen atom, and a halogen atom. Between carbon atoms in these groups, a group having a heteroatom such as an oxygen atom, a sulfur atom, and a nitrogen atom is optionally interposed. As a result, optionally contained are a hydroxy group, a cyano group, a carbonyl group, an ether bond, an ester bond, a sulfonate ester bond, a carbonate bond, a lactone ring, a sultone ring, a carboxylic anhydride, a haloalkyl group, etc. Note that $R^{11}$ to $R^{15}$ herein are applied only in the formulae (cation-1) and (cation-2).

$R^{11}$ and $R^{12}$ are optionally bonded each other to form a ring together with the sulfur atom to which $R^{11}$ and $R^{12}$ are bonded. In this case, examples of the sulfonium cation represented by the formula (cation-1) include cations represented by the following formulae.

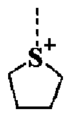 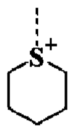 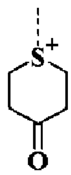 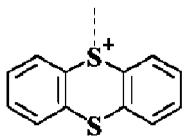 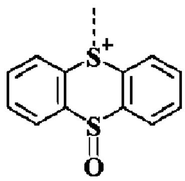 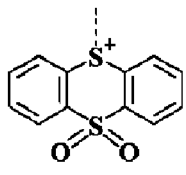

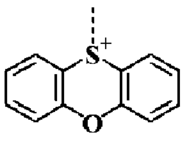 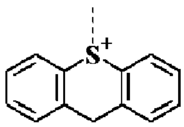 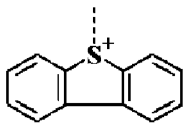 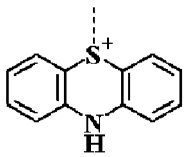 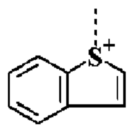

In the formulae, a broken line represents an attachment point to $R^{13}$.

Examples of the sulfonium cation represented by the formula (cation-1) include the following cations, but the sulfonium cation is not limited thereto.

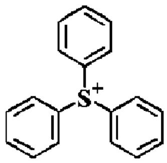 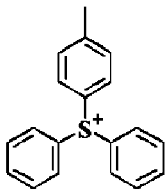 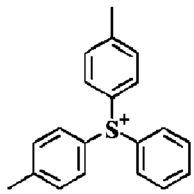 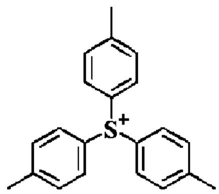

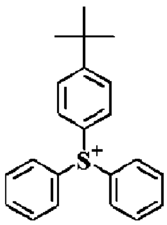 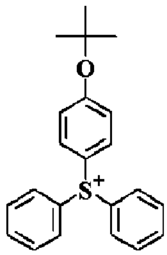 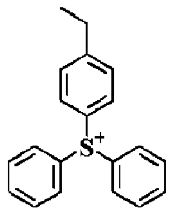 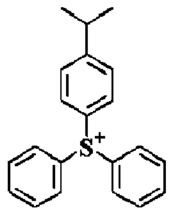 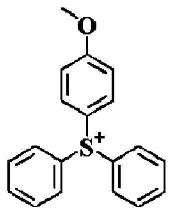

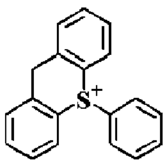 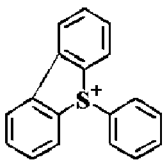 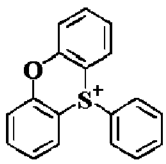 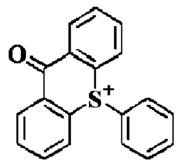 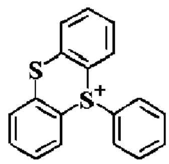

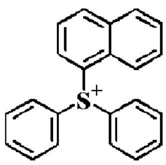 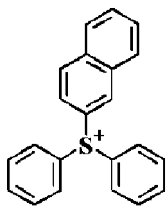 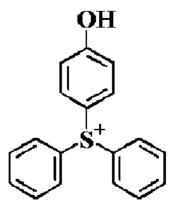 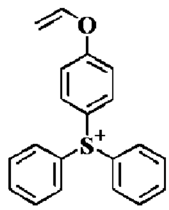 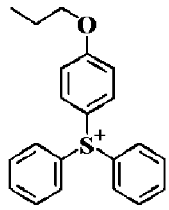

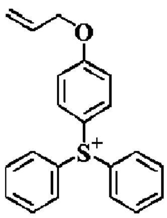 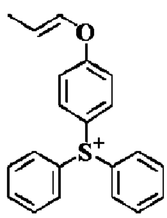 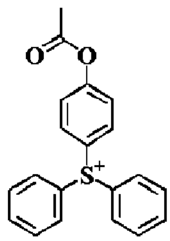 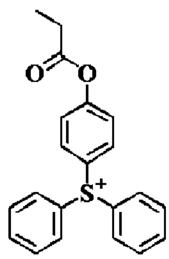 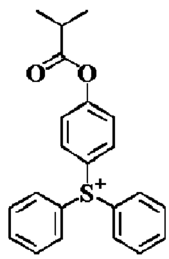

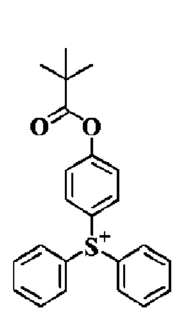
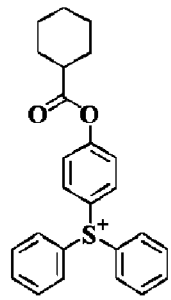
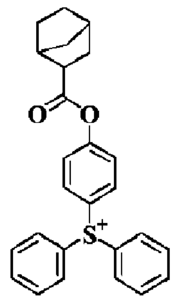
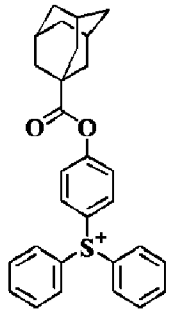
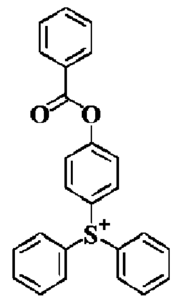

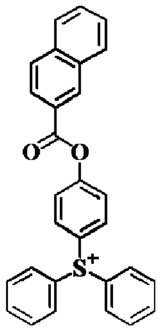
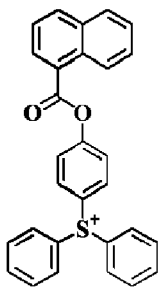
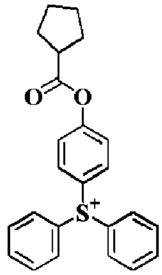
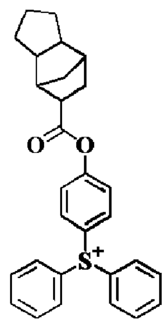
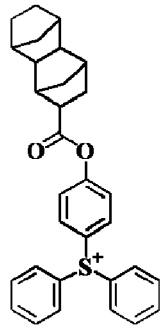

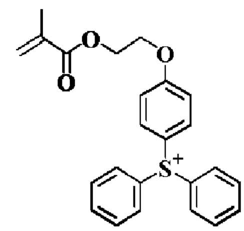
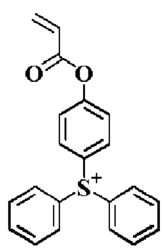
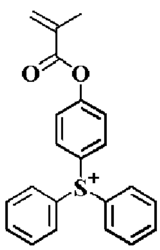
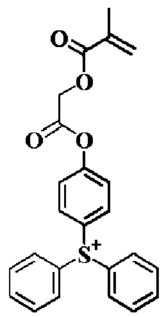
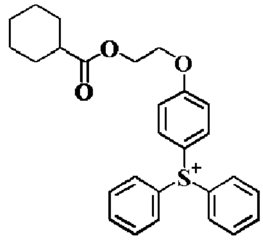

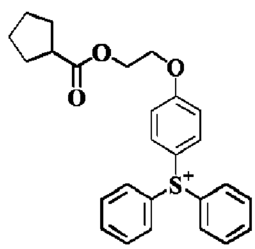
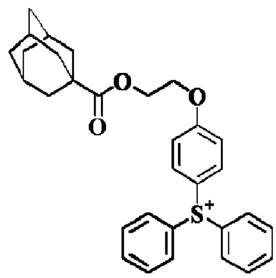
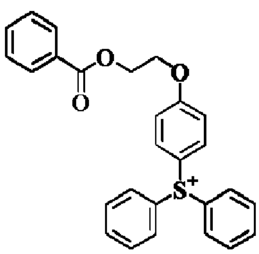
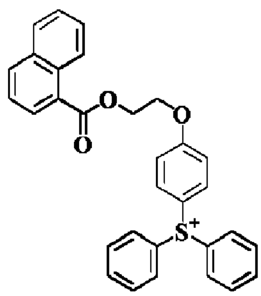

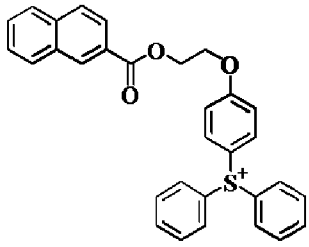
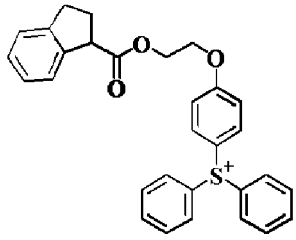
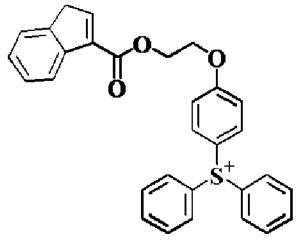

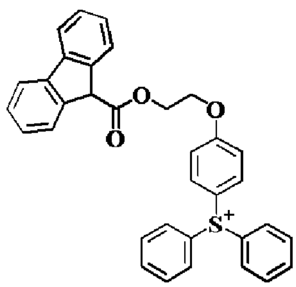
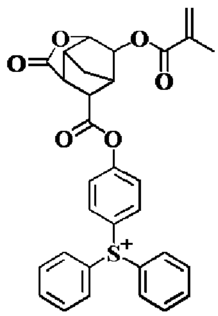
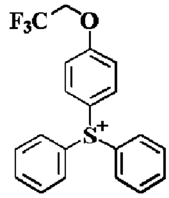
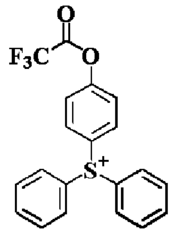
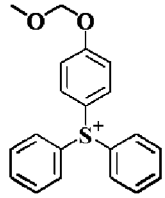

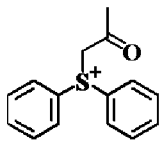
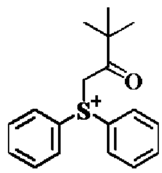
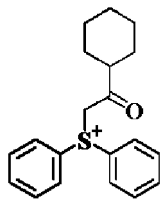
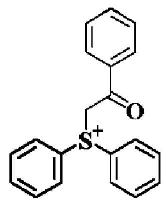

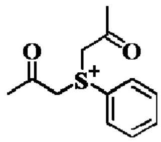
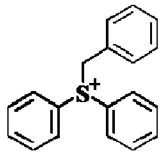
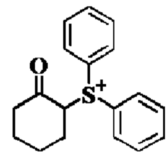
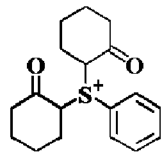

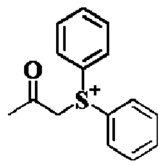
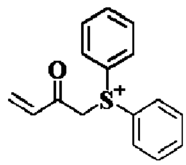
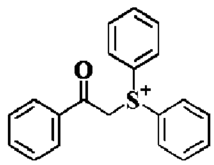
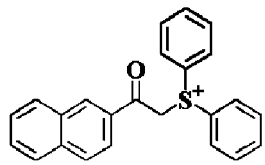

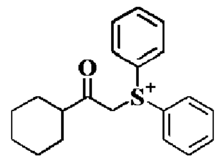
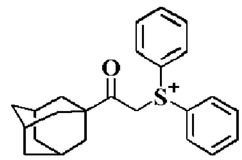
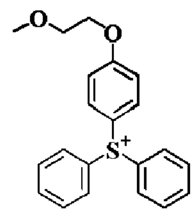
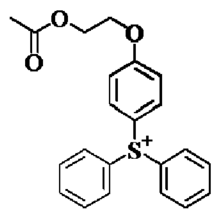

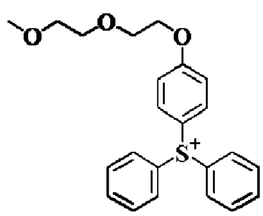
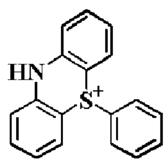
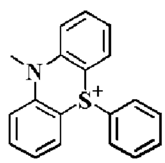

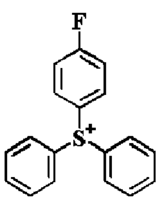
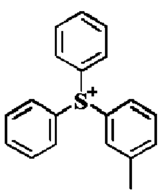
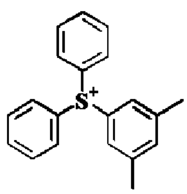
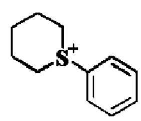
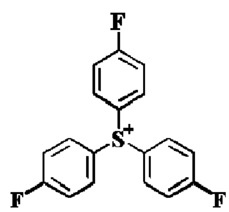

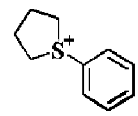 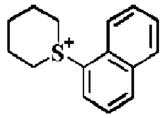 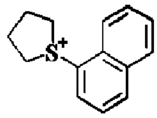 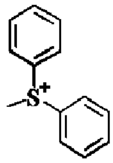 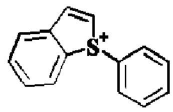

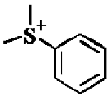 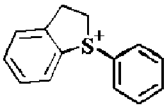 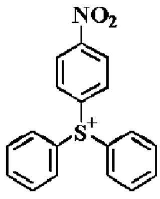 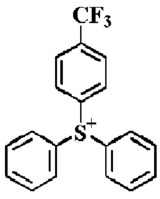 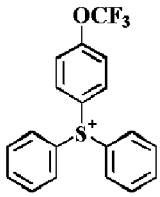

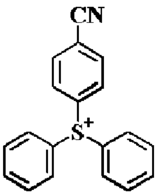 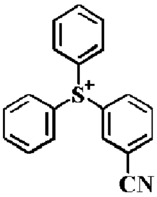 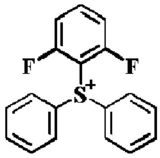 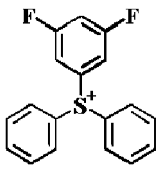 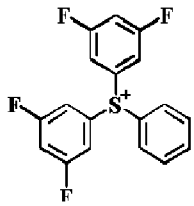

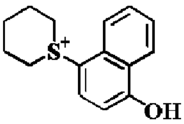 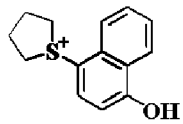 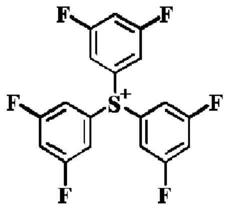 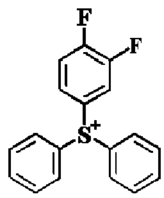

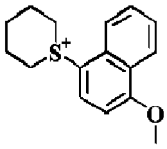 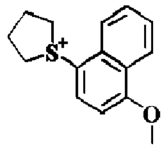 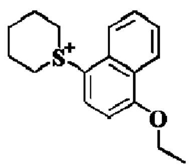 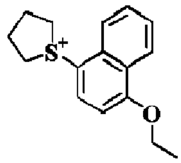

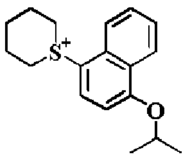 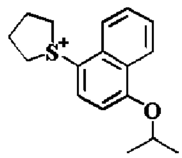 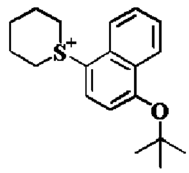 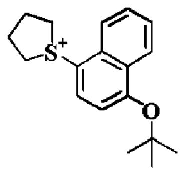

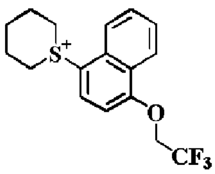 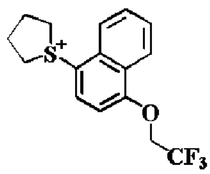 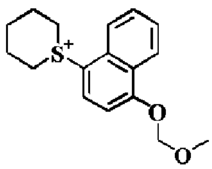 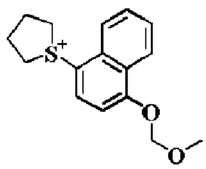

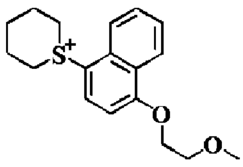 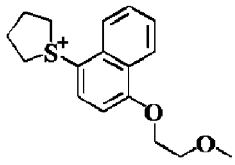 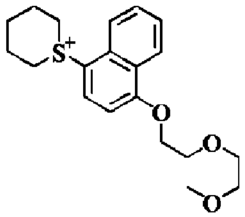 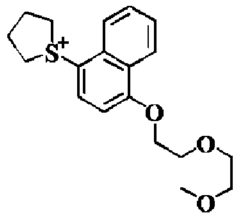

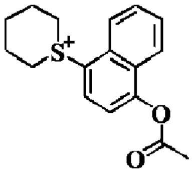 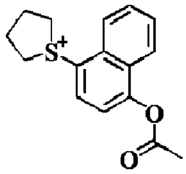 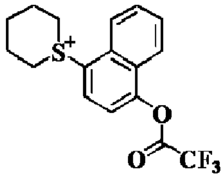 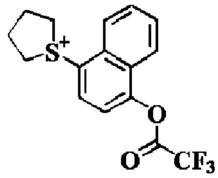

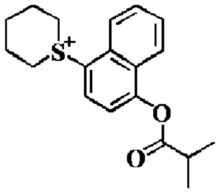 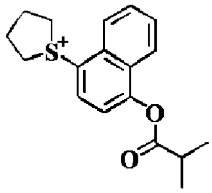 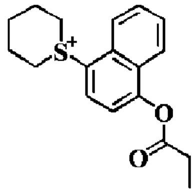 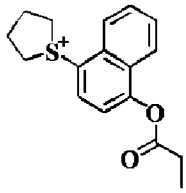

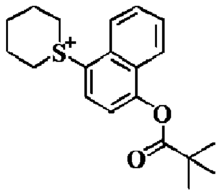 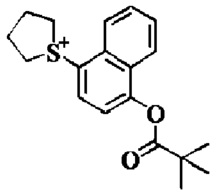 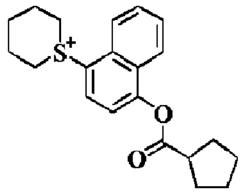 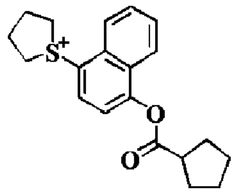

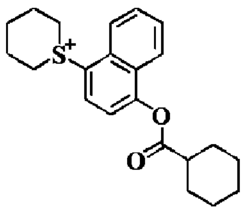 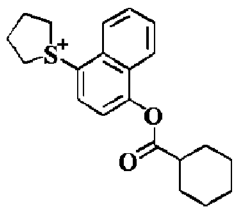 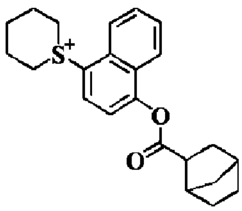 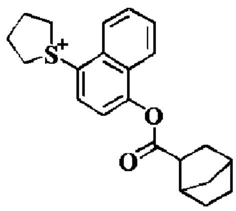

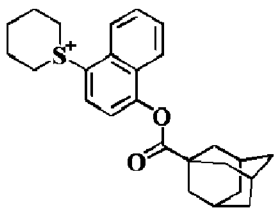 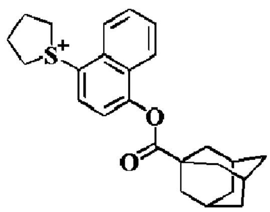 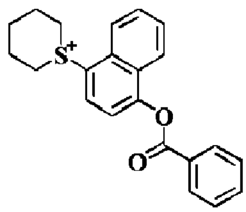 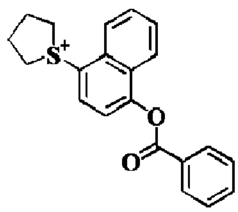

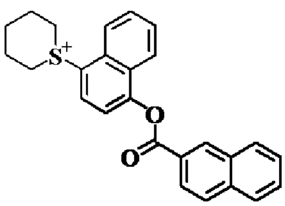 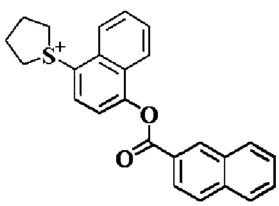 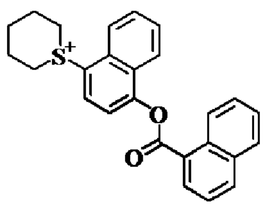 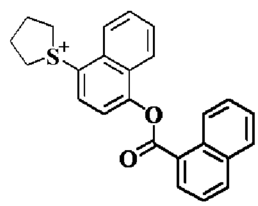

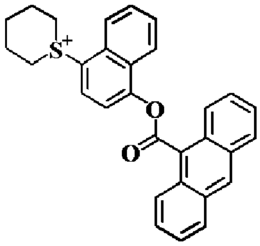
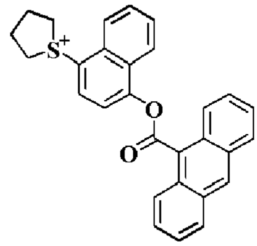
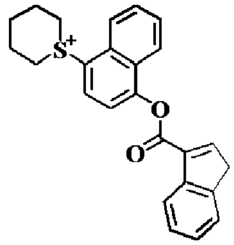
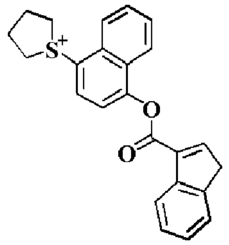

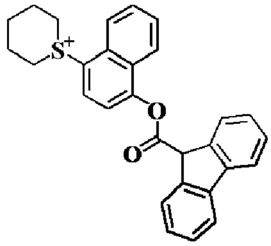
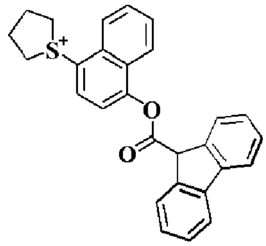
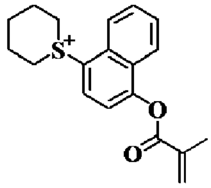
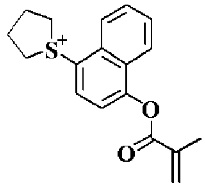

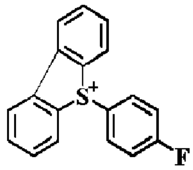
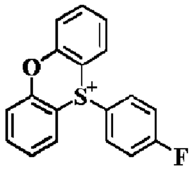
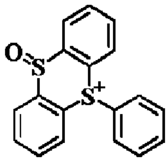
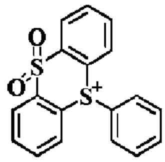

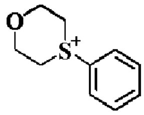
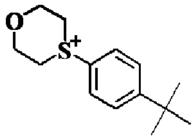
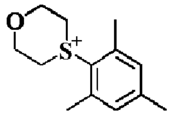

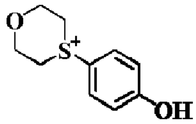
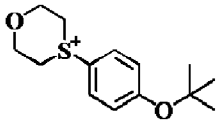
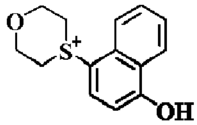
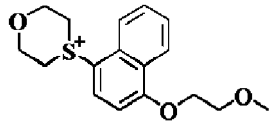

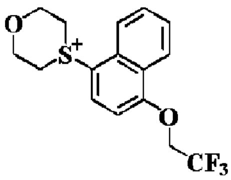
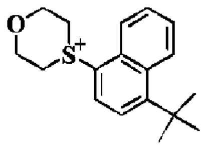
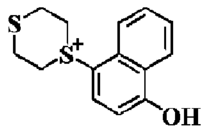
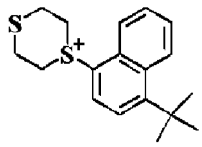

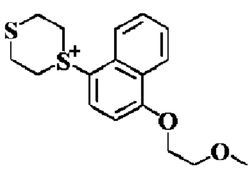
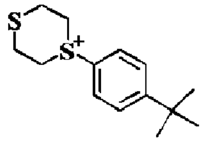
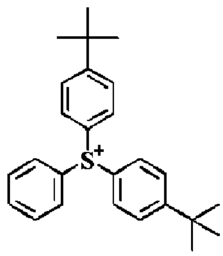
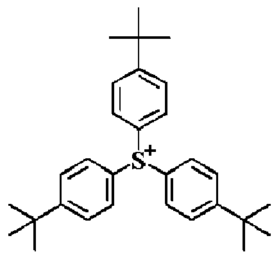

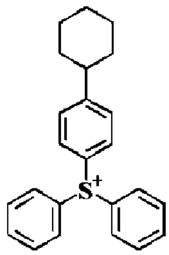
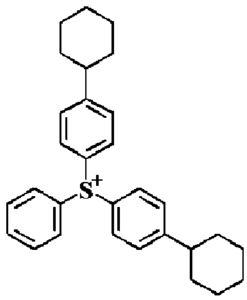
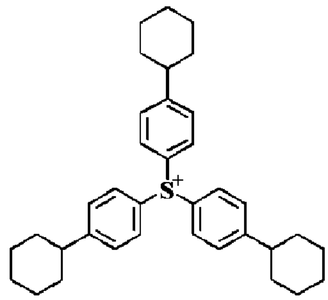

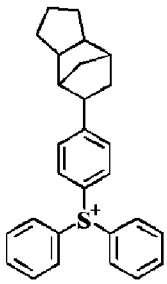
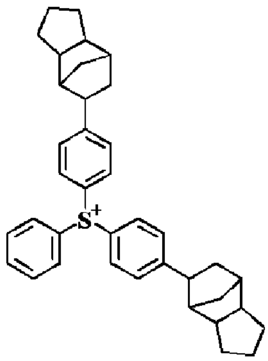
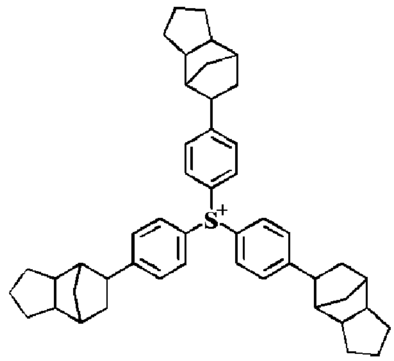

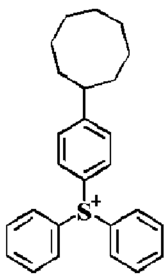
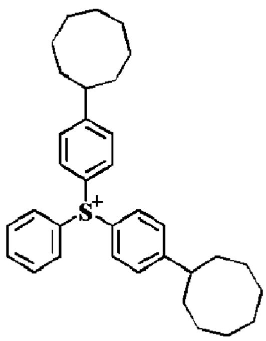
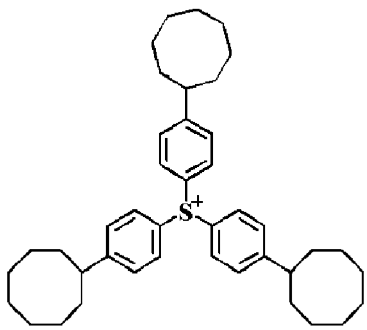

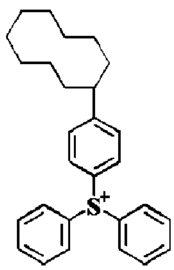
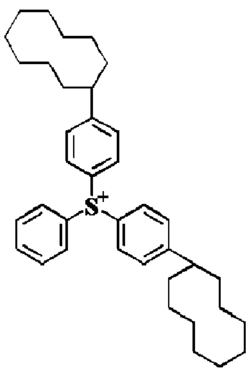
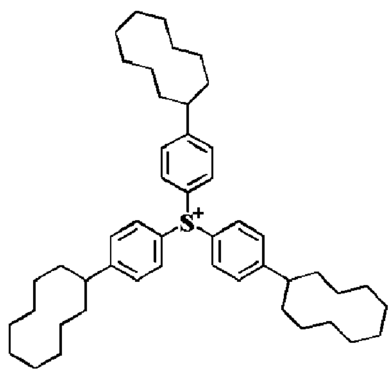

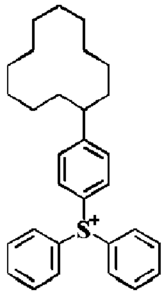
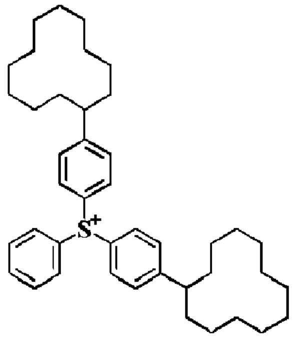
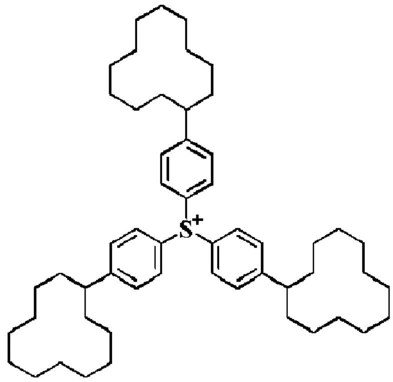

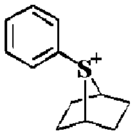
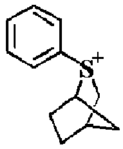
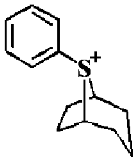
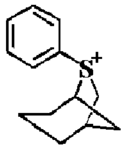
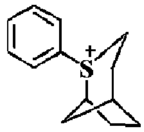

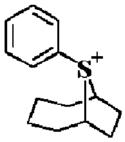
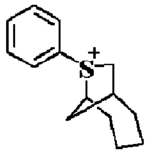
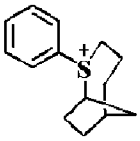
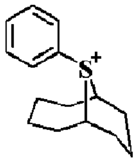
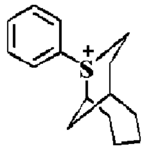

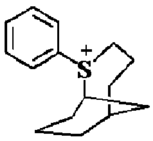
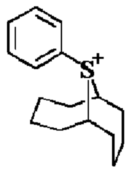
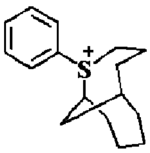
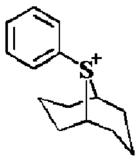
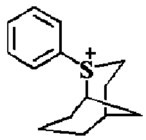

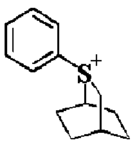
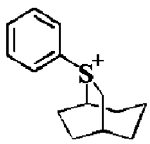
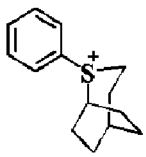
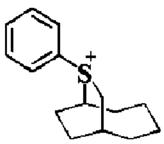
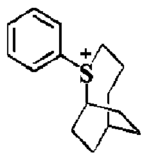

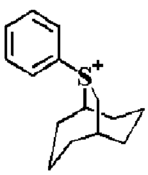
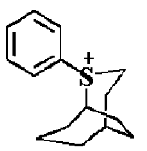
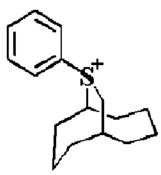
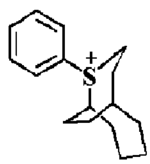
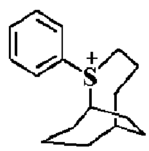

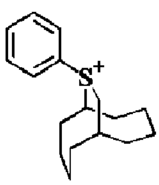
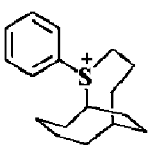
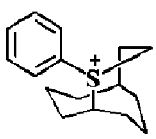
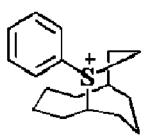
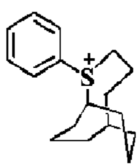

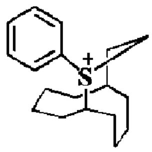
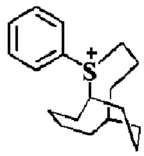
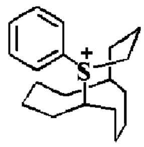

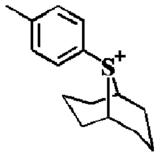
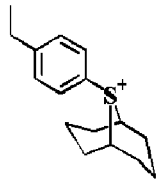
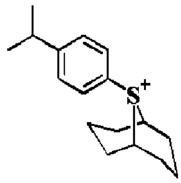
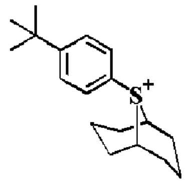
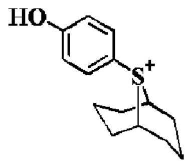

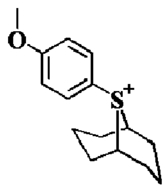
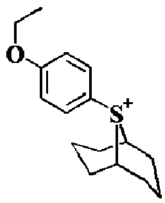
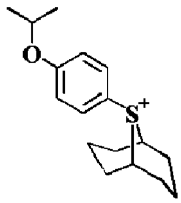
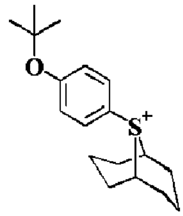
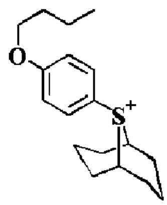

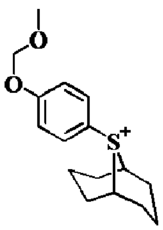
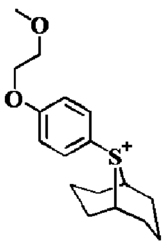
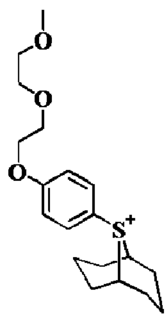
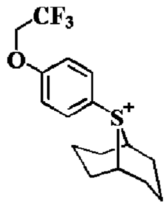
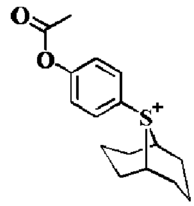

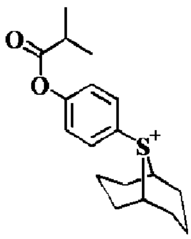
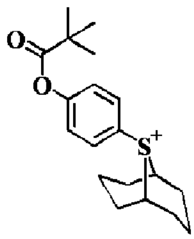
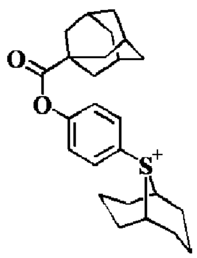
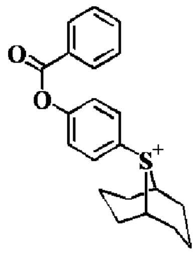
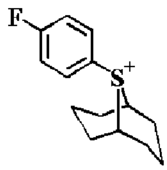

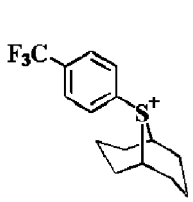
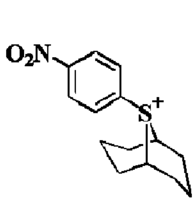
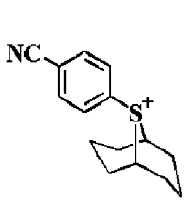
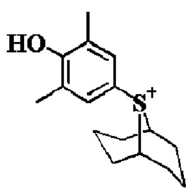
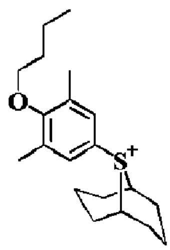

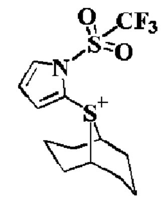
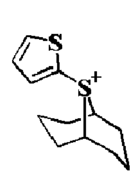
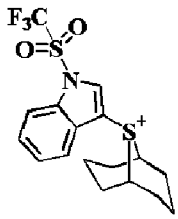
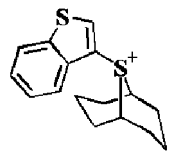
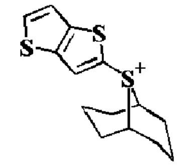

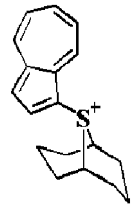
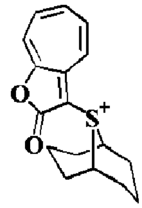
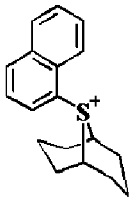
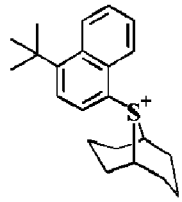
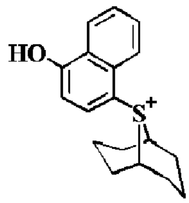

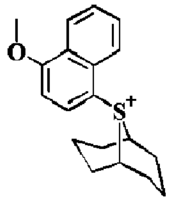
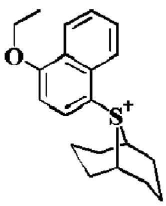
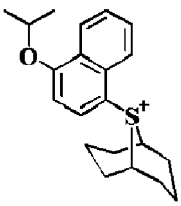
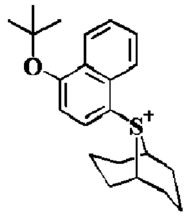
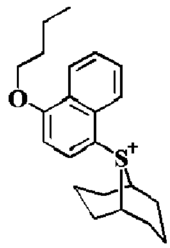

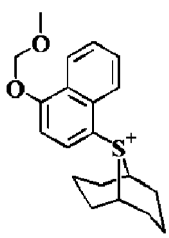
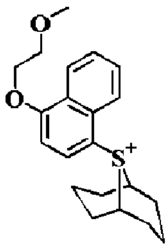
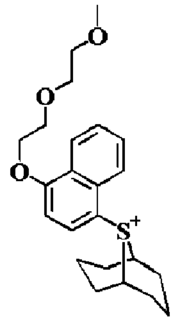
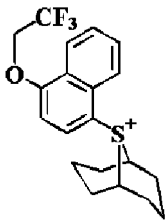
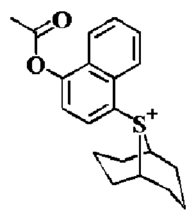

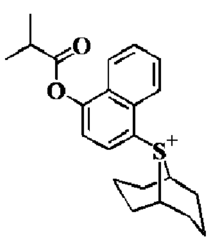
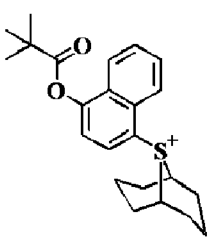
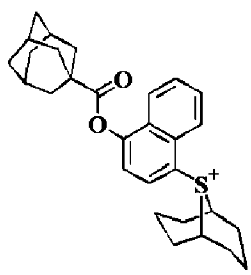
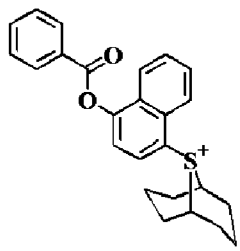

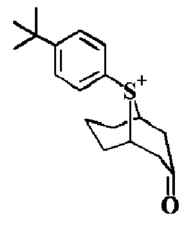
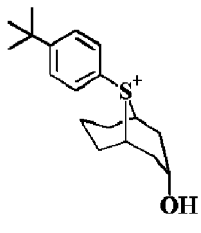
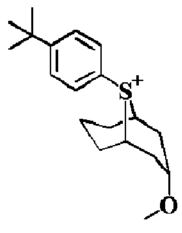
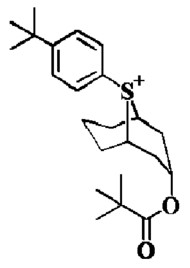
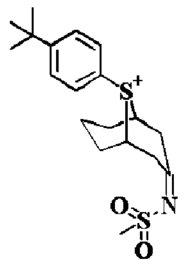

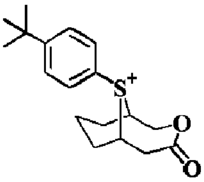
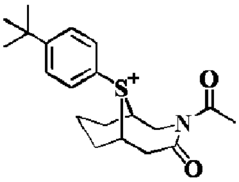
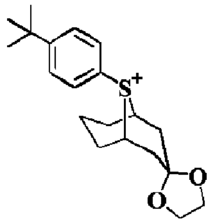
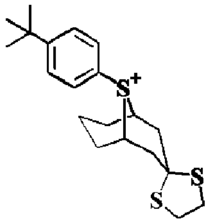

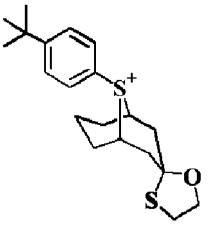
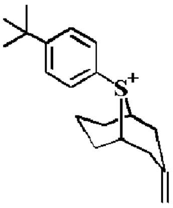
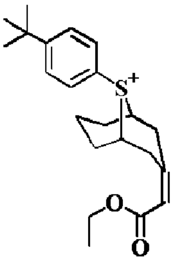
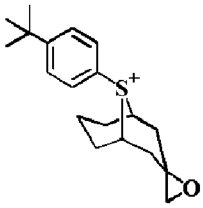
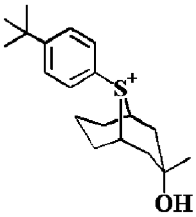

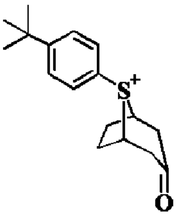
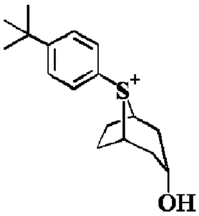
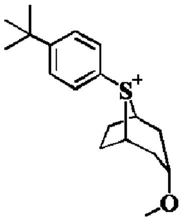
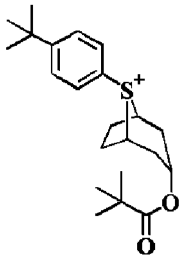
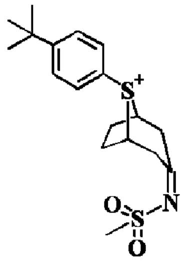

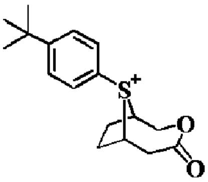
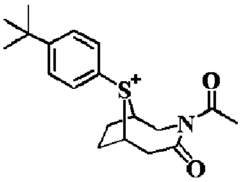
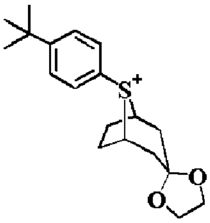
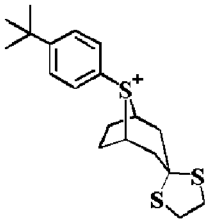

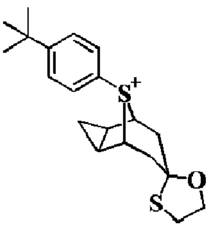
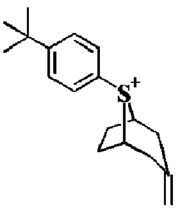
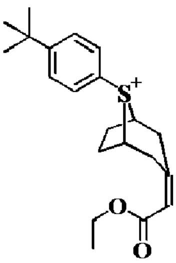
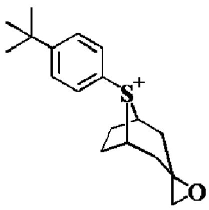
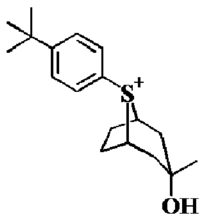

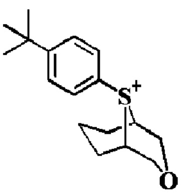
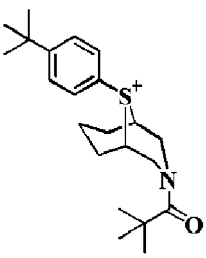
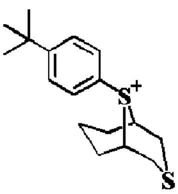
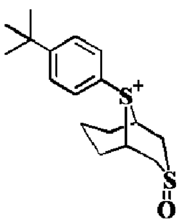
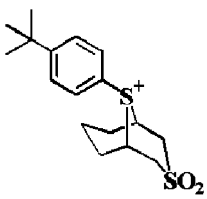

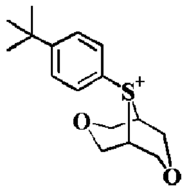
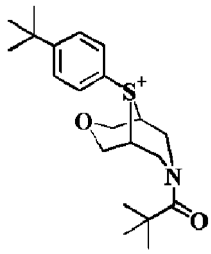
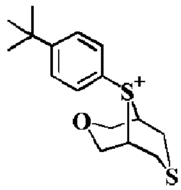
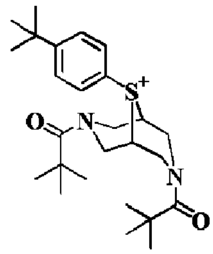
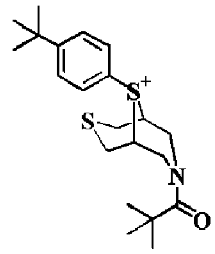

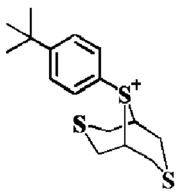
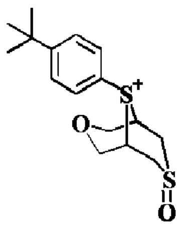
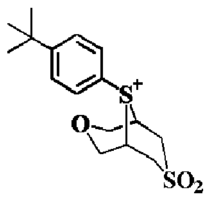
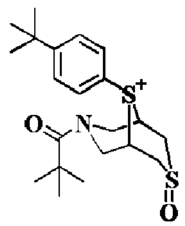
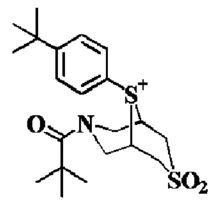

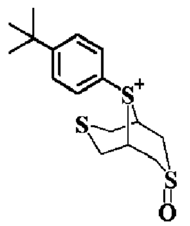
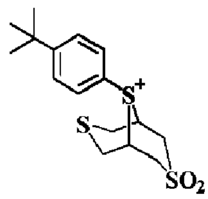

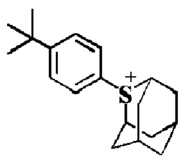
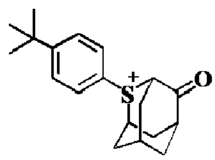
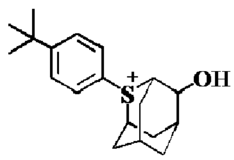
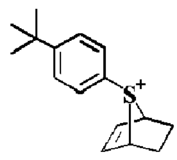
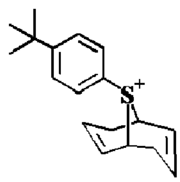

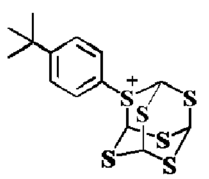
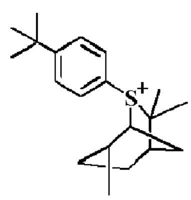
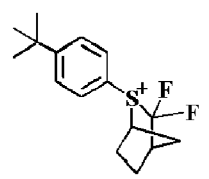

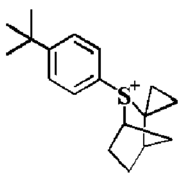
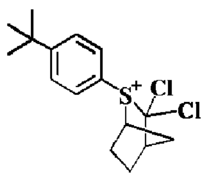
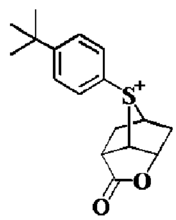
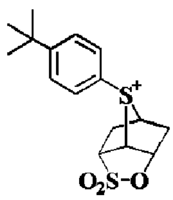

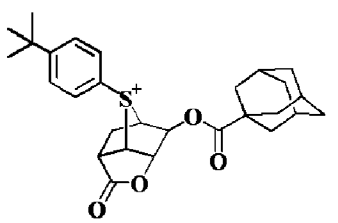
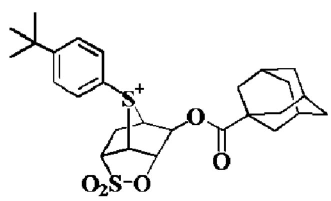

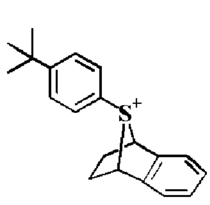
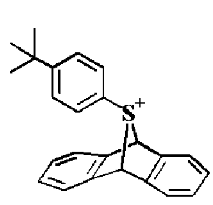
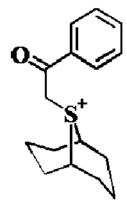
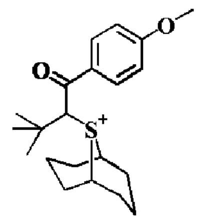

Examples of the iodonium cation represented by the formula (cation-2) include the following cations, but the iodonium cation is not limited thereto.

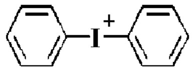
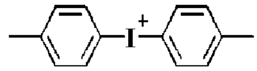
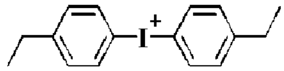
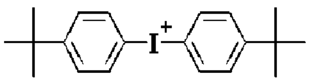
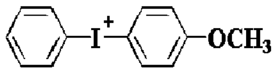
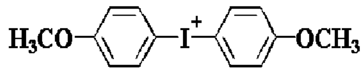
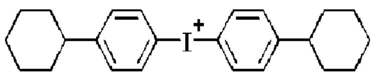
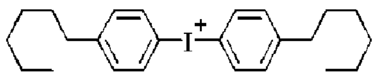
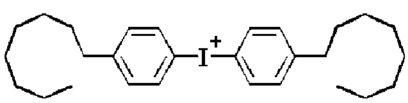
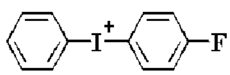
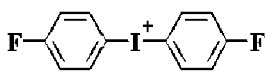
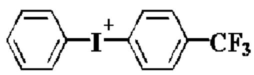
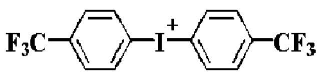
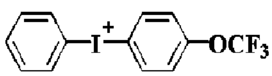
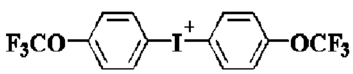
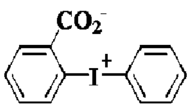

Examples of specific structures of the repeating units represented by the formulae (C1) to (C4) include any combination of the aforementioned anions and cations.

Among the repeating units c1 to c4, the repeating units c2, c3, and c4 are preferable from the viewpoint of controlling the acid diffusion, the repeating units c2 and c4 are further preferable from the viewpoint of the acid strength of the generated acid, and the repeating unit c2 is more preferable from the viewpoint of the solvent solubility.

The base polymer optionally further has a repeating unit having a structure in which a hydroxy group is protected by an acid-labile group (hereinafter, also referred to as the repeating unit d). The repeating unit d is not particularly limited as long as the unit has one or two or more of the hydroxy-group protected structures and generates the hydroxy group with decomposing the protective group by an action of an acid. The repeating unit d is preferably represented by the following formula (d1).

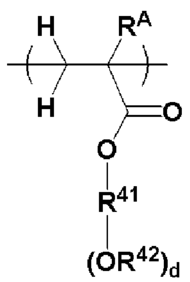
**(d1)**

In the formula (d1), $R^A$ represents the same as above. $R^{41}$ represents a (d + 1)-valent hydrocarbon group having 1 to 30 carbon atoms and optionally having a heteroatom. $R^{42}$ represents the acid-labile group. "d" represents an integer of 1 to 4.

In the formula (d1), the acid-labile group represented by $R^{42}$ is deprotected by an action of an acid to generate the hydroxy group. The structure of $R^{42}$ is not particularly limited, but an acetal structure, a ketal structure, an alkoxycarbonyl group, an alkoxymethyl group represented by the following formula (d2), etc. are preferable, and an alkoxymethyl group represented by the following formula (d2) is particularly preferable.

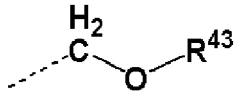

(d2)

In the formula, a broken line represents an attachment point. $R^{43}$ represents a hydrocarbyl group having 1 to 15 carbon atoms.

Specific examples of the acid-labile group represented by $R^{42}$, the alkoxymethyl group represented by the formula (d2), and the repeating unit d include groups same as those exemplified in the description of the repeating unit d described in JP 2020-111564 A.

The base polymer optionally further has a repeating unit e derived from indene, benzofuran, benzothiophene, acenaphthylene, chromone, coumarin, norbornadiene, or a derivative thereof. Examples of monomers to yield the repeating unit e include the following monomers, but the monomer is not limited thereto.

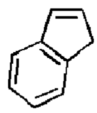 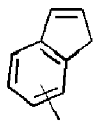 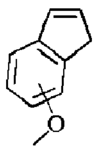 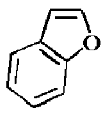 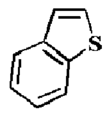 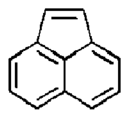 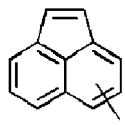 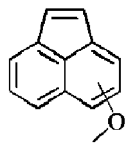

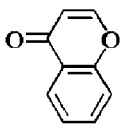 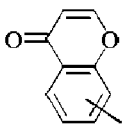 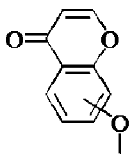 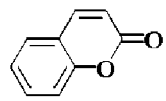 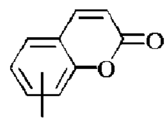 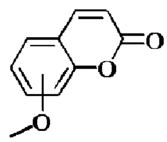

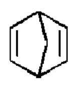 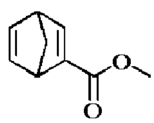

The base polymer optionally further has a repeating unit f derived from indane, vinylpyridine, or vinylcarbazole.

Content rates of the repeating units a1, a2, b1, b2, c1 to c4, d, e, and f in the inventive polymer are preferably $0 < a1 \leq 0.8$, $0 \leq a2 \leq 0.8$, $0 \leq b1 \leq 0.6$, $0 \leq b2 \leq 0.6$, $0 \leq c1 \leq 0.4$, $0 \leq c2 \leq 0.4$, $0 \leq c3 \leq 0.4$, $0 \leq c4 \leq 0.4$, $0 \leq d \leq 0.5$, $0 \leq e \leq 0.3$, and $0 \leq f \leq 0.3$, and more preferably $0 < a1 \leq 0.7$, $0 \leq a2 \leq 0.7$, $0 \leq b1 \leq 0.5$, $0 \leq b2 \leq 0.5$, $0 \leq c1 \leq 0.3$, $0 \leq c2 \leq 0.3$, $0 \leq c3 \leq 0.3$, $0 \leq c4 \leq 0.3$, $0 \leq d \leq 0.3$, $0 \leq e \leq 0.3$, and $0 \leq f \leq 0.3$.

The polymer preferably has a weight-average molecular weight (Mw) of 1,000 to 500,000, more preferably 3,000 to 100,000. The Mw within this range yields sufficient etching resistance, and has no risk of deterioration in resolution caused by failure to achieve a difference in a dissolution rate before and after the exposure. The Mw in the present invention is a value in terms of polystyrene by gel permeation chromatography (GPC) using tetrahydrofuran (THF) or N,N-dimethylformamide (DMF) as a solvent.

Since a molecular weight distribution (Mw/Mn) of the polymer has a larger effect as the pattern rule becomes smaller, the Mw/Mn is preferably 1.0 to 2.0, which indicates narrow distribution, to obtain the resist composition suitably used for a fine pattern size. Within the above range, polymers having a small molecular weight and a large molecular weight are reduced, and there is no risk of a foreign matter on a pattern and deterioration in a pattern shape after the exposure.

To synthesize the polymer, for example, monomers to yield the aforementioned repeating units and a radical polymerization initiator are added into an organic solvent, and the mixture is heated to perform polymerization.

Examples of the organic solvent used in the polymerization include toluene, benzene, THF, diethyl ether, dioxane, cyclohexane, cyclopentane, methyl ethyl ketone (MEK), propylene glycol monomethyl ether acetate (PGMEA),

and γ-butyrolactone (GBL). Examples of the polymerization initiator include 2,2'-azobisisobutyronitrile (AIBN), 2,2'-azobis(2,4-dimethylvaleronitrile), dimethyl 2,2-azobis(2-methylpropionate), 1,1'-azobis(1-acetoxy-1-phenylethane), benzoyl peroxide, and lauroyl peroxide. An addition amount of these initiators is preferably 0.01 to 25 mol% relative to a total of the monomers to be polymerized. The reaction temperature is preferably 50 to 150°C, and more preferably 60 to 100°C. The reaction time is preferably 2 to 24 hours, and from the viewpoint of production efficiency, more preferably 2 to 12 hours.

The polymerization initiator may be added into a solution of the monomers and fed into a reaction vessel, or an initiator solution is prepared separately from the monomer solution and each of the solutions may be independently fed into a reaction vessel. Since a radical generated from the initiator may proceed the polymerization reaction during the waiting time to generate a polymer having an ultra-high molecular weight, the monomer solution and the initiator solution are preferably each independently prepared and added dropwise from the viewpoint of quality control. The acid-labile group may be introduced into the monomer to be used as it is, or may be protected or partially protected after the polymerization. To regulate the molecular weight, known chain transfer agents, such as dodecyl mercaptan and 2-mercaptoethanol, may be used in combination. In this case, an addition amount of these chain transfer agents is preferably 0.01 to 20 mol% relative to the total of the monomers to be polymerized.

When the monomer has a hydroxy group, the hydroxy group may be substituted with an acetal group, such as an ethoxyethyl group, easily deprotected by an acid during the polymerization, and the protected hydroxy group may be deprotected by a weak acid and water after the polymerization. Alternatively, the hydroxy group may be substituted with an acetyl group, a formyl group, a pivaloyl group, etc. to be subjected to alkaline hydrolysis after the polymerization.

When hydroxystyrene or hydroxyvinylnaphthalene is copolymerized, hydroxystyrene or hydroxyvinylnaphthalene and the other monomers may be heat-polymerized in the organic solvent with adding the radical polymerization initiator. Alternatively, acetoxystyrene or acetoxyvinylnaphthalene may be used, and the acetoxy group is deprotected with alkaline hydrolysis after the polymerization to be converted into polyhydroxystyrene or hydroxypolyvinylnaphthalene.

As a base in the alkaline hydrolysis, aqueous ammonia, triethylamine, etc. can be used. The reaction temperature is preferably -20 to 100°C, and more preferably 0 to 60°C. The reaction time is preferably 0.2 to 100 hours, and more preferably 0.5 to 20 hours.

An amount of each monomer in the monomer solution is appropriately set so as to be a preferable content rate of the above repeating units, for example.

With the polymer obtained in the manufacturing method, a reaction solution obtained by the polymerization reaction may be a final product. Alternatively, a powder obtained via a purification step, such as reprecipitation method in which the polymerization solution is added into a poor solvent to obtain a powder, may be treated as a final product. From the viewpoints of operation efficiency and quality stabilization, the powder obtained in the purification step is preferably dissolved in a solvent for forming a polymer solution to be operated as a final product.

Specific examples of the solvent used in this case include solvents described in paragraphs [0144] to [0145] of JP 2008-111103 A, and specifically include: ketones, such as cyclohexanone and methyl-2-n-pentylketone; alcohols, such as 3-methoxybutanol, 3-methyl-3-methoxybutanol, 1-methoxy-2-propanol, and 1-ethoxy-2-propanol; ethers, such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters, such as PGMEA, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones, such as GBL; alcohols, such as diacetone alcohol (DAA); alcoholic solvents having a high boiling point, such as diethylene glycol, propylene glycol, glycerin, 1,4-butanediol, and 1,3-butanediol; and a mixed solvent thereof.

In the polymer solution, a concentration of the polymer is preferably 0.01 to 30 mass%, and more preferably 0.1 to 20 mass%.

The reaction solution and the polymer solution are preferably filtered with a filter. The filtration can remove a foreign matter and gel, which may cause a defect, and is effective in terms of quality stabilization.

Examples of a material of the filter used for the filtration include a fluorocarbon, a cellulose, a nylon, a polyester, and a hydrocarbon. In the step of filtering the resist composition, the filter is preferably formed with a fluorocarbon, so-called Teflon(R), a hydrocarbon such as polyethylene and polypropylene, or nylon. A pore size of the filter can be appropriately selected according to target cleanliness, and is preferably 100 nm or smaller, and more preferably 20 nm or smaller. These filters may be used singly, or may be used in combination of a plurality of these filters. With the filtration method, the solution may be passed through the filter once, but the solution is more preferably circulated to be filtered a plurality of times. In the step for producing the polymer, the filtration step may be performed in any order and times, but the reaction solution after the polymerization reaction, the polymer solution, or both thereof are preferably filtered.

The polymer may be used singly, or may be used in combination of two or more kinds thereof having different composition ratio, Mw, and/or Mw/Mn. The base polymer (B) optionally contains, in addition to the above polymer, a hydrogenated ring-opening metathesis polymer. Polymers described in JP 2003-66612 A can be used.

(C) Organic Solvent

The organic solvent of the component (C) is not particularly limited as long as it can dissolve each component described above and each component described later. Examples of such an organic solvent include: ketones, such as cyclopentanone, cyclohexanone, and methyl-2-n-pentyl ketone; alcohols, such as 3-methoxybutanol, 3-methyl-3-methoxybutanol, 1-methoxy-2-propanol, and 1-ethoxy-2-propanol; keto alcohols, such as DAA; ethers, such as PGME, ethylene glycol monomethyl ether, propylene glycol monoethyl ether, ethylene glycol monoethyl ether, propylene glycol dimethyl ether, and diethylene glycol dimethyl ether; esters, such as PGMEA, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones, such as GBL; and a mixed solvent thereof.

Among these organic solvents, preferable are 1-ethoxy-2-propanol, PGMEA, cyclohexanone, GBL, DAA, and a mixed solvent thereof, which have particularly excellent solubility of the base polymer of the component (B).

A use amount of the organic solvent is preferably 200 to 5,000 parts by mass, and more preferably 400 to 3,500 parts by mass, relative to 80 parts by mass of the base polymer (B). The organic solvent (C) may be used singly, or may be used with mixing two or more kinds thereof.

(D) Quencher

Examples of the quencher (D) include onium salts represented by the following formula (5) or (6).

$$R^{q1}\text{-}SO_3^- \ Mq^+ \qquad (5)$$

$$R^{q2}\text{-}CO_2^- \ Mq^+ \qquad (6)$$

In the formula (5), $R^{q1}$ represents a hydrogen atom or a monovalent hydrocarbon group (hydrocarbyl group) having 1 to 40 carbon atoms and optionally having a heteroatom, except for a group in which a hydrogen atom bonded to a carbon atom at the $\alpha$-position of the sulfo group is substituted with a fluorine atom or a fluoroalkyl group. In the formula (6), $R^{q2}$ represents a hydrogen atom or a monovalent hydrocarbon group (hydrocarbyl group) having 1 to 40 carbon atoms and optionally having a heteroatom.

Specific examples of the hydrocarbyl group represented by $R^{q1}$ include: alkyl groups, such as a methyl group, an ethyl group, a propyl group, an isopropyl group, an n-butyl group, a sec-butyl group, a tert-butyl group, an n-pentyl group, a tert-pentyl group, an n-hexyl group, an n-octyl group, a 2-ethylhexyl group, an n-nonyl group, and an n-decyl group; cyclic saturated hydrocarbyl groups, such as a cyclopentyl group, a cyclohexyl group, a cyclopentylmethyl group, a cyclopentylethyl group, a cyclopentylbutyl group, a cyclohexylmethyl group, a cyclohexylethyl group, a cyclohexylbutyl group, a norbornyl group, a tricyclo[5.2.1.0$^{2,6}$]decanyl group, and an adamantyl group; and aryl groups, such as a phenyl group, a naphthyl group, and an anthracenyl group. A part or all of hydrogen atoms in these groups are optionally substituted with a group having a heteroatom, such as an oxygen atom, a sulfur atom, a nitrogen atom, and a halogen atom. A part of carbon atoms in these groups is optionally substituted with a group having a heteroatom, such as an oxygen atom, a sulfur atom, and a nitrogen atom. As a result, optionally contained are a hydroxy group, a cyano group, a carbonyl group, an ether bond, an ester bond, a sulfonate ester bond, a carbonate bond, a lactone ring, a sultone ring, a carboxylic anhydride, a haloalkyl group, etc.

Specific examples of the hydrocarbyl group represented by $R^{q2}$ include: the substituents exemplified as the specific examples of $R^{q1}$; fluorinated alkyl groups, such as a trifluoromethyl group and a trifluoroethyl group; and fluorinated aryl groups, such as a pentafluorophenyl group and a 4-trifluoromethylphenyl group.

Examples of the anion of the onium salt represented by the formula (5) include the following anions, but the anion is not limited thereto.

$CH_3SO_3^-$

$CH_3CH_2SO_3^-$

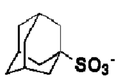
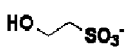
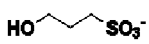

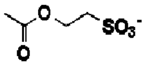 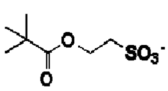 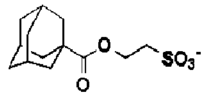 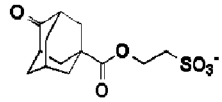

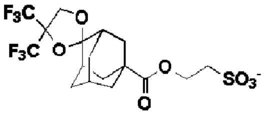 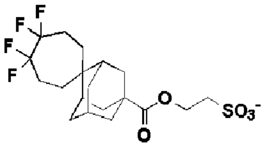 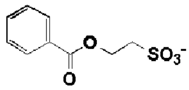 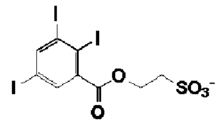

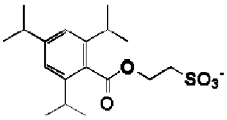 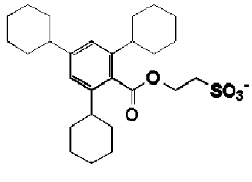 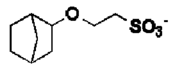 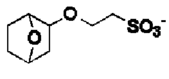

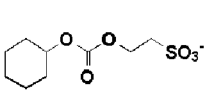 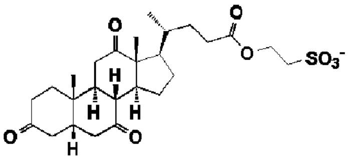 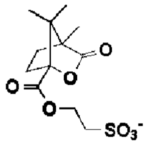 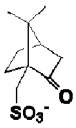 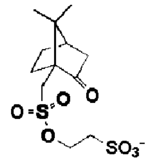

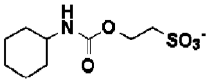 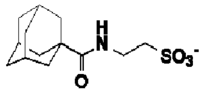 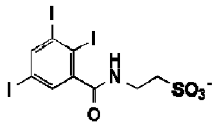 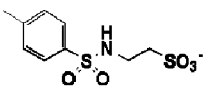

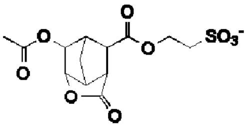 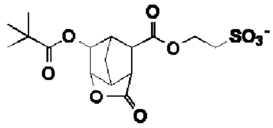 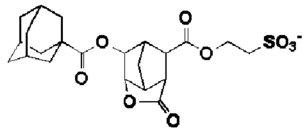

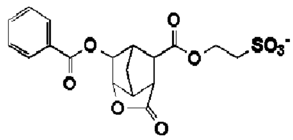 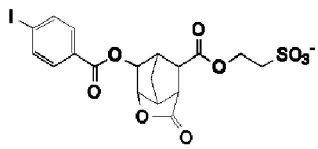 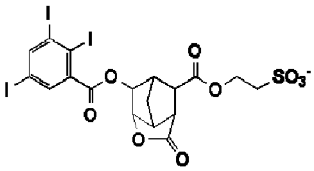

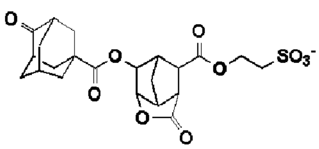 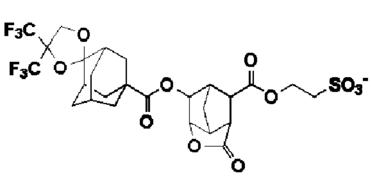 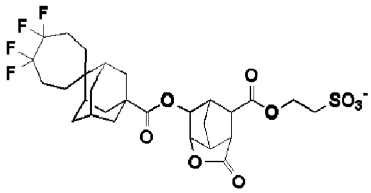

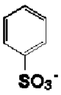 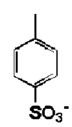 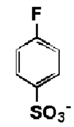 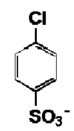 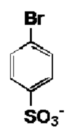 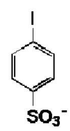 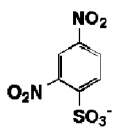 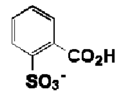 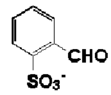

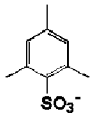 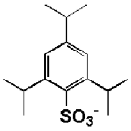 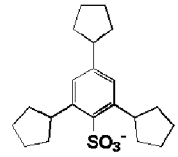 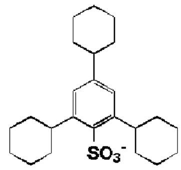 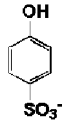 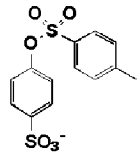 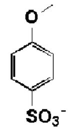

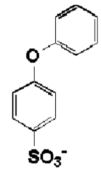 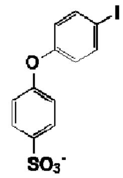 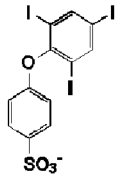 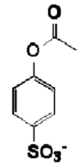 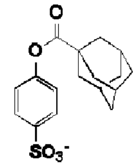

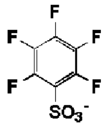 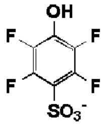 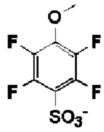 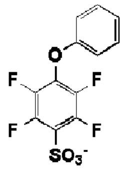 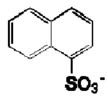 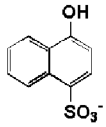 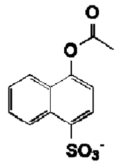

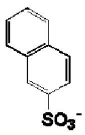 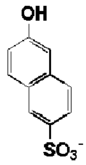 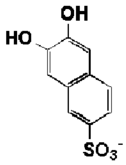 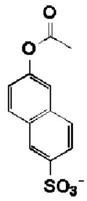 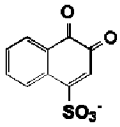 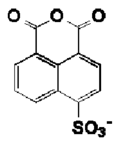 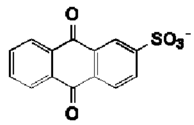

Examples of the anion of the onium salt represented by the formula (6) include the following anions, but the anion is not limited thereto.

$HCO_2^-$

$CH_3CO_2^-$

$CH_3CH_2CO_2^-$

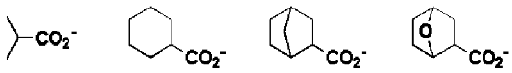

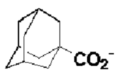 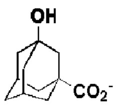 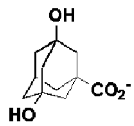 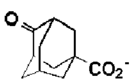 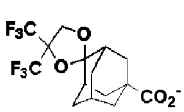 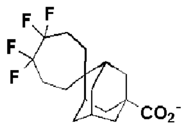

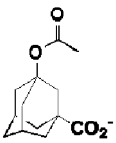 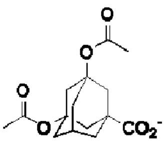 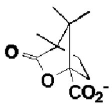 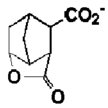 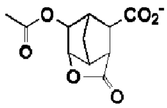 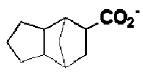

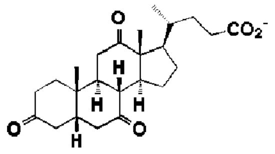 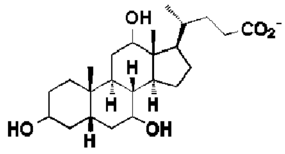 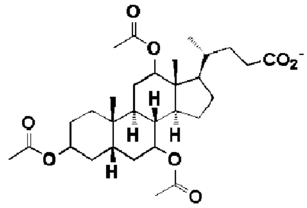

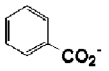 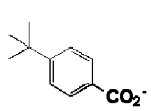 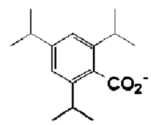 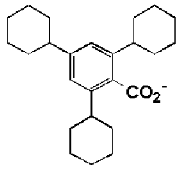 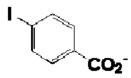 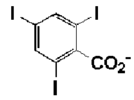

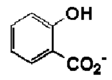 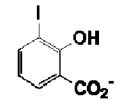 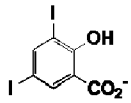 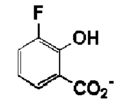 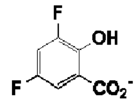 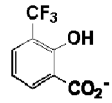 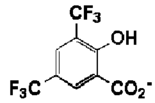

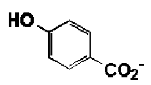 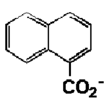 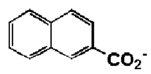 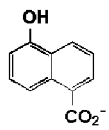 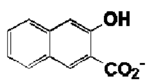 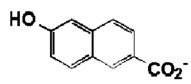

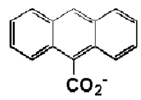 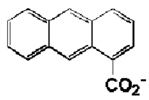 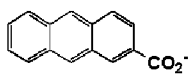 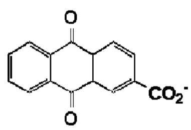 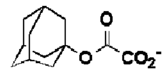

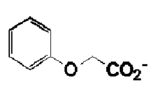 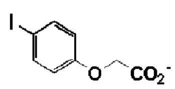 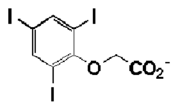

$CF_3CO_2^-$

$CF_3CH_2CO_2^-$

$CF_3CF_2CO_2^-$

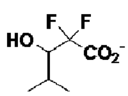 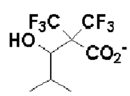 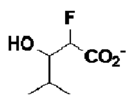 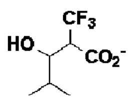

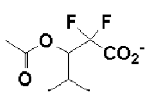 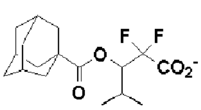 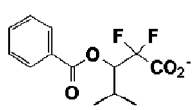 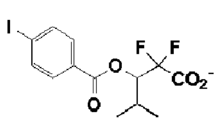 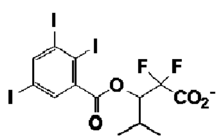

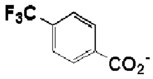 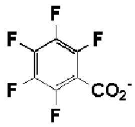 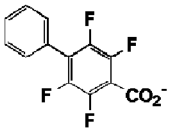 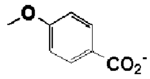

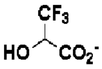 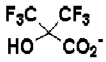 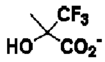 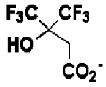

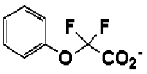 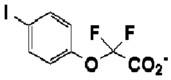 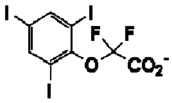 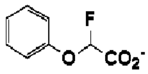 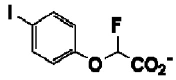 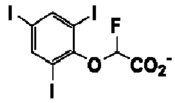

In the formulae (5) and (6), $Mq^+$ represents an onium cation. The onium cation is preferably represented by the following formula (cation-1), (cation-2), or (cation-3).

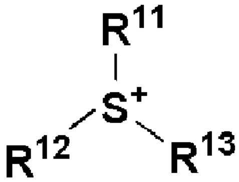

(cation-1)

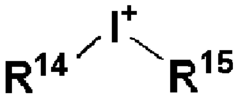

(cation-2)

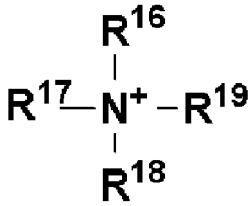

(cation-3)

Examples of the cation represented by the formulae (cation-1) and (cation-2) include cations same as $A^+$ in the formulae (C2) to (C4) . In (cation-3), $R^{16}$ to $R^{19}$ each independently represent a hydrocarbyl group having 1 to 40 carbon atoms and optionally having a heteroatom. $R^{16}$ and $R^{17}$ are optionally bonded each other to form a ring together with the nitrogen atom to which $R^{16}$ and $R^{17}$ are bonded. Examples of the hydrocarbyl group include groups same as those exemplified in the description of $R^{11}$ to $R^{15}$ in the formulae (cation-1) and (cation-2).

With the onium cation represented by $Mq^+$, examples of the ammonium cation represented by (cation-3) include the following cations, but the cation is not limited thereto.

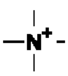 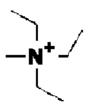 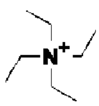 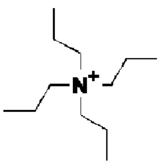 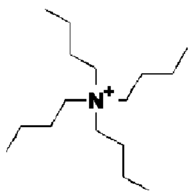 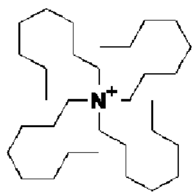

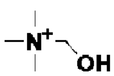 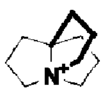 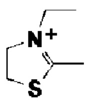 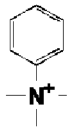 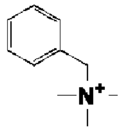 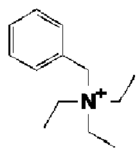 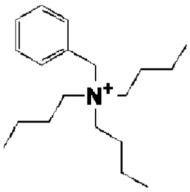

Specific examples of the onium salt represented by the formula (5) or (6) include any combination of the aforementioned anions and cations. These onium salts are easily prepared by an ion-exchange reaction using a known organic chemical method. About the ion-exchange reaction, JP 2007-145797 A can be referred, for example.

The onium salt represented by the formula (5) or (6) acts as a quencher in the inventive chemically amplified resist composition. This is because each counter anion of the onium salt is a conjugated base of a weak acid. The weak acid herein means an acid exhibiting acidity that cannot deprotect the acid-labile group in the acid-labile group-containing

unit used for the base polymer.

The onium salt represented by the formula (5) or (6) functions as a quencher when used in combination with an onium-salt type photoacid generator having a conjugated base of a strong acid, such as an $\alpha$-fluorinated sulfonic acid, as a counter anion. That is, when an onium salt to generate a strong acid, such as an $\alpha$-fluorinated sulfonic acid, and an onium salt to generate a weak acid, such as non-fluorinated sulfonic acid and a carboxylic acid, are mixed to be used, the strong acid generated from the photoacid generator by high-energy ray irradiation collides the unreacted onium salt having the weak acid anion to release the weak acid with salt exchange, resulting in generation of an onium salt having the strong acid anion. This process exchanges the strong acid into the weak acid having low catalytic ability, and the acid is apparently deactivated to enable to control the acid diffusion.

Usable for the quencher (D) are: an onium salt having a sulfonium cation and a phenoxide anion portion in the same molecule, described in JP 6848776 B; an onium salt having a sulfonium cation and a carboxylate anion portion in the same molecule, described in JP 6583136 B and JP 2020-200311 A; and an onium salt having an iodonium cation and a carboxylate anion portion in the same molecule, descried in JP 6274755 B.

When the photoacid generator to generate the strong acid is an onium salt, the strong acid generated by high-energy ray irradiation can be exchanged into the weak acid, as described above. Meanwhile, it is considered that the weak acid generated by high-energy ray irradiation hardly collides the unreacted onium salt to generate the strong acid to cause salt exchange. This is because of a phenomenon that an onium cation is more likely to form an ion pair with an anion of a stronger acid.

When the onium salt represented by the formula (5) or (6) is contained as the onium-salt type quencher (D), a content thereof is preferably 0.1 to 20 parts by mass, and more preferably 0.1 to 10 parts by mass, relative to 80 parts by mass of the base polymer (B). The onium-salt type quencher of the component (D) within the above range is preferable in terms of good resolution without considerable deterioration in the sensitivity. The onium salt represented by the formula (5) or (6) can be used singly, or used in combination of two or more kinds thereof.

The inventive chemically amplified resist composition may further comprise a nitrogen-containing quencher. In the present invention, the nitrogen-containing quencher is referred to a material that traps the acid generated from the photoacid generator in the chemically amplified resist composition for inhibiting the diffusion toward an unexposed portion to form a desired pattern.

Examples of the nitrogen-containing quencher of the component (D) include primary, secondary, or tertiary amine compounds described in paragraphs [0146] to [0164] of JP 2008-111103 A, in particular, amine compounds having a hydroxy group, an ether bond, an ester bond, a lactone ring, a cyano group, or a sulfonate ester bond. Examples thereof also include compounds in which a primary or secondary amine is protected with a carbamate group, as compounds described in JP 3790649 B.

As above, the inventive resist composition can further comprise the amine compound.

As the nitrogen-containing quencher, a sulfonium sulfonate salt having a nitrogen-containing substituent may also be used. Such a compound functions as a so-called photodegradable base. The photodegradable base functions as a quencher in an unexposed portion, and losses the quenching ability by neutralization with a generated acid of the photodegradable base itself in an exposed portion. Using the photodegradable base can further enhance the contrast between the exposed portion and the unexposed portion. As the photodegradable base, JP 2009-109595 A and JP 2012-46501 A can be referred, for example.

When the nitrogen-containing quencher of the component (D) is contained, a content thereof is preferably 0.001 to 12 parts by mass, and more preferably 0.01 to 8 parts by mass, relative to 80 parts by mass of the base polymer (B). The nitrogen-containing compound may be used singly, or may be used in combination of two or more kinds thereof.

(E) Other Photoacid Generator

The inventive chemically amplified resist composition optionally includes a photoacid generator other than the component (A) as a component (E) (hereinafter, also referred to as the other photoacid generator). The other photoacid generator is not particularly limited as long as it is a compound to generate an acid with high-energy ray irradiation. Examples of preferable other photoacid generators include photoacid generators represented by the following formula (3) or (4).

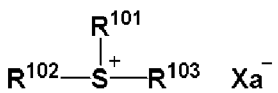

(3)

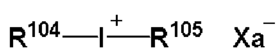

(4)

In the formulae (3) and (4), $R^{101}$ to $R^{105}$ each independently represent a hydrocarbyl group having 1 to 20 carbon atoms and optionally having a heteroatom. Any two of $R^{101}$, $R^{102}$, and $R^{103}$ are optionally bonded each other to form a ring together with the sulfur atom to which $R^{101}$, $R^{102}$, and $R^{103}$ are bonded. Examples of the hydrocarbyl group

include groups same as those exemplified in the description of $R^{11}$ to $R^{15}$ in the formulae (cation-1) and (cation-2) .

In the formula (3), examples of the sulfonium cation include cations same as those exemplified as the sulfonium cation represented by the formula (cation-1).

In the formula (4), examples of the iodonium cation include cations same as those exemplified as the iodonium cation represented by the formula (cation-2).

In the formulae (3) and (4), examples of $Xa^-$include anions same as those exemplified in the formula (1) .

As the other photoacid generator of the component (E), a photoacid generator represented by the following formula (V) is also preferable.

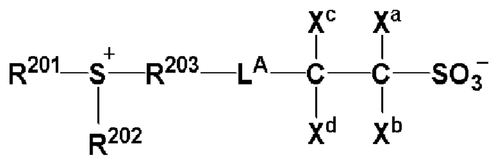

(V)

In the formula (V), $R^{201}$ and $R^{202}$ each independently represent a hydrocarbyl group having 1 to 30 carbon atoms and optionally having a heteroatom. $R^{203}$ represents a hydrocarbylene group having 1 to 30 carbon atoms and optionally having a heteroatom. Any two of $R^{201}$, $R^{202}$, and $R^{203}$ are optionally bonded each other to form a ring together with the sulfur atom to which $R^{201}$, $R^{202}$, and $R^{203}$ are bonded each other.

The hydrocarbyl group represented by $R^{201}$ and $R^{202}$ may be a saturated or unsaturated group, and may be any of linear, branched, and cyclic groups. Specific examples thereof include: alkyl groups having 1 to 30 carbon atoms, such as a methyl group, an ethyl group, a propyl group, an isopropyl group, an n-butyl group, a sec-butyl group, a tert-butyl group, a tert-pentyl group, an n-pentyl group, an n-hexyl group, an n-octyl group, a 2-ethylhexyl group, an n-nonyl group, and an n-decyl group; cyclic saturated hydrocarbyl groups having 3 to 30 carbon atoms, such as a cyclopentyl group, a cyclohexyl group, a cyclopentylmethyl group, a cyclopentylethyl group, a cyclopentylbutyl group, a cyclohexylmethyl group, a cyclohexylethyl group, a cyclohexylbutyl group, a norbornyl group, an oxanorbornyl group, a tricyclo[5.2.1.$0^{2,6}$]decanyl group, and an adamantyl group; aryl groups having 6 to 30 carbon atoms, such as a phenyl group, a methylphenyl group, an ethylphenyl group, an n-propylphenyl group, an isopropylphenyl group, an n-butylphenyl group, an isobutylphenyl group, a sec-butylphenyl group, a tert-butylphenyl group, a naphthyl group, a methylnaphthyl group, an ethylnaphthyl group, an n-propylnaphthyl group, an isopropylnaphthyl group, an n-butylnaphthyl group, an isobutylnaphthyl group, a sec-butylnaphthyl group, a tert-butylnaphthyl group, and an anthracenyl group; and groups obtained by combining these groups. A part or all of hydrogen atoms in the hydrocarbyl groups is optionally substituted with a group having a heteroatom, such as an oxygen atom, a sulfur atom, a nitrogen atom, and a halogen atom. A part of $-CH_2-$ constituting the hydrocarbyl groups is optionally substituted with a group having a heteroatom, such as an oxygen atom, a sulfur atom, and a nitrogen atom. As a result, optionally contained are a hydroxy group, a cyano group, a fluorine atom, a chlorine atom, a bromine atom, an iodine atom, a carbonyl group, an ether bond, an ester bond, a sulfonate ester bond, a carbonate group, a lactone ring, a sultone ring, a carboxylic anhydride, a haloalkyl group, etc.

The hydrocarbylene group represented by $R^{203}$ may be a saturated or unsaturated group, and may be any of linear, branched, and cyclic groups. Specific examples thereof include: alkanediyl groups having 1 to 30 carbon atoms, such as a methanediyl group, an ethane-1,1-diyl group, an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, a dodecane-1,12-diyl group, a tridecane-1,13-diyl group, a tetradecane-1,14-diyl group, a pentadecane-1,15-diyl group, a hexadecane-1,16-diyl group, and a heptadecane-1,17-diyl group; cyclic saturated hydrocarbylene groups having 3 to 30 carbon atoms, such as a cyclopentanediyl group, a cyclohexanediyl group, a norbornanediyl group, and an adamantanediyl group; and cyclic unsaturated hydrocarbylene groups, such as a phenylene group, a methylphenylene group, an ethylphenylene group, an n-propylphenylene group, an isopropylphenylene group, an n-butylphenylene group, an isobutylphenylene group, a sec-butylphenylene group, a tert-butylphenylene group, a naphthylene group, a methylnaphthylene group, an ethylnaphthylene group, an n-propylnaphthylene group, an isopropylnaphthylene group, an n-butylnaphthylene group, an isobutylnaphthylene group, a sec-butylnaphthylene group, and a tert-butylnaphthylene group. A part or all of hydrogen atoms in the hydrocarbylene groups are optionally substituted with a group having a heteroatom, such as an oxygen atom, a sulfur atom, a nitrogen atom, and a halogen atom. A part of $-CH_2-$ constituting the hydrocarbylene groups is optionally substituted with a group having a heteroatom, such as an oxygen atom, a sulfur atom, and a nitrogen atom. As a result, optionally contained are a hydroxy group, a cyano group, a fluorine atom, a chlorine atom, a bromine atom, an iodine atom, a carbonyl group, an ether bond, an ester bond, a sulfonate ester bond, a carbonate group, a lactone ring, a sultone ring, a carboxylic anhydride, a haloalkyl group, etc. The heteroatom is preferably an oxygen atom.

In the formula (V), $L^A$ represents a single bond, an ether bond, or a hydrocarbylene group having 1 to 20 carbon atoms and optionally having a heteroatom. The hydrocarbylene group may be a saturated or unsaturated group, and

may be any of linear, branched, and cyclic groups. Specific examples thereof include groups same as those exemplified as the hydrocarbylene group represented by $R^{203}$.

In the formula (V), $X^a$, $X^b$, $X^c$, and $X^d$ each independently represent a hydrogen atom, a fluorine atom, or a trifluoromethyl group. At least one of $X^a$, $X^b$, $X^c$, and $X^d$ represents a fluorine atom or a trifluoromethyl group.

The photoacid generator represented by the formula (V) is preferably a photoacid generator represented by the following formula (V').

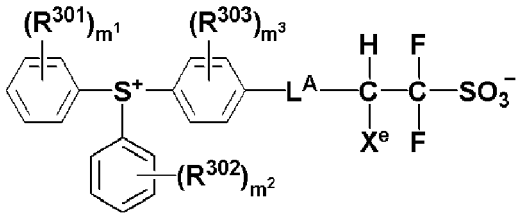

(V')

In the formula (V'), $L^A$ represents the same as above. $X^e$ represents a hydrogen atom or a trifluoromethyl group, and preferably a trifluoromethyl group. $R^{301}$, $R^{302}$, and $R^{303}$ each independently represent a hydrogen atom or a hydrocarbyl group having 1 to 20 carbon atoms and optionally having a heteroatom. The hydrocarbyl group may be a saturated or unsaturated group, and may be any of linear, branched, and cyclic groups. Specific examples thereof include groups same as $R^{12}$ in the formula (1). "$m^1$" and "$m^2$" each independently represent an integer of 0 to 5. "$m^3$" represents an integer of 0 to 4.

Examples of the photoacid generator represented by the formula (V) include photoacid generators same as those exemplified as photoacid generators represented by the formula (2) in JP 2017-026980 A.

Among the above other photoacid generators, the photoacid generators having the anion represented by the formula (1A') or (1D) are particularly preferable since having small acid diffusion and excellent solubility in the solvent. The photoacid generators represented by the formula (V') is particularly preferable since having extremely small acid diffusion.

When the photoacid generator of the component (E) is contained, a content thereof is preferably 0.1 to 40 parts by mass, and more preferably 0.5 to 20 parts by mass, relative to 80 parts by mass of the base polymer (B). The addition amount of the photoacid generator of the component (E) within the above range is preferable because of good resolution and no risk of generation of a foreign matter problem after the development or during the removal of the resist film. The photoacid generator of the component (E) may be used singly, or may be used in combination of two or more kinds thereof.

(F) Surfactant Insoluble or Hardly Soluble in Water and Soluble in Alkaline Developer; and/or Surfactant Insoluble or Hardly Soluble in Water and Alkaline Developer

The inventive chemically amplified resist composition optionally further comprises: (F) a surfactant insoluble or hardly soluble in water and soluble in an alkaline developer; and/or a surfactant insoluble or hardly soluble in water and an alkaline developer. As such a surfactant, surfactants described in JP 2010-215608 A and JP 2011-16746 A can be referred.

Among the surfactants described in the above patent publications, the surfactant insoluble or hardly soluble in water and an alkaline developer is preferably FC-4430 (manufactured by 3M Company), SURFLON(R) S-381 (manufactured by AGC Seimi Chemical Co., Ltd.), OLFIN(R) E1004 (manufactured by Nissin Chemical Industry Co., Ltd.), KH-20 and KH-30 (manufactured by AGC Seimi Chemical Co., Ltd.), and an oxetane ring-opening polymerized product represented by the following formula (surf-1).

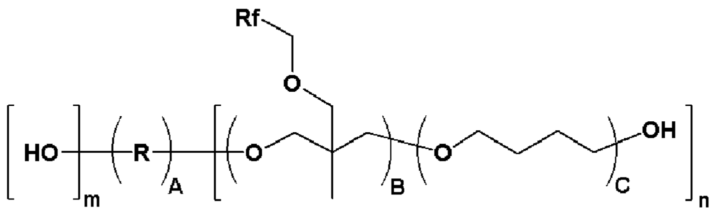

(surf-1)

Here, R, Rf, A, B, C, "m", and "n" are applied only in the formula (surf-1) regardless of the above description. R represents a divalent to tetravalent aliphatic group having 2 to 5 carbon atoms. Examples of the divalent aliphatic group include an ethylene group, a 1,4-butylene group, a 1,2-propylene group, a 2,2-dimethyl-1,3-propylene group, and a 1,5-pentylene group. Examples of the trivalent or tetravalent aliphatic group include the following groups.

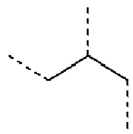 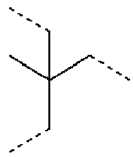 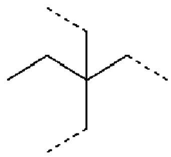 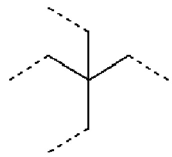

In the formula, a broken line represents an attachment point. The groups are partial structures derived from glycerol, trimethylolethane, trimethylolpropane, and pentaerythritol, respectively.

Among these, a 1,4-butylene group and a 2,2-dimethyl-1,3-propylene group are preferable.

Rf represents a trifluoromethyl group or a pentafluoroethyl group, and preferably a trifluoromethyl group. "m" represents an integer of 0 to 3. "n" represents an integer of 1 to 4. A sum of "n" and "m", which represents a valency of R, represents an integer of 2 to 4. A represents 1. B represents an integer of 2 to 25, and preferably represents an integer of 4 to 20. C represents an integer of 0 to 10, and preferably represents 0 or 1. With each constituting unit in the formula (surf-1), the order is not stipulated, and may be block-bonded or random-bonded. Manufacturing of the surfactant of the partially fluorinated oxetane ring-opening polymerized product is described in detail in US 5650483 B.

In the ArF immersion exposure without a resist protective film, the surfactant insoluble or hardly soluble in water and soluble in an alkaline developer has a function of reducing penetration of water or leaching by orientation on a surface of the resist film. Thus, such a surfactant is useful for inhibiting elution of a water-soluble component from the resist film to reduce damage of an exposure apparatus. Such a surfactant is also useful because such a surfactant becomes soluble during development with an alkaline aqueous solution after the exposure and after post exposure bake (PEB), and hardly forms a foreign matter causing a defect. Such a surfactant, which has a property of being insoluble or hardly soluble in water and soluble in an alkaline developer, is preferably a polymer surfactant, which is also referred to as a hydrophobic resin. In particular, such a surfactant preferably has high water repellency and improves water-slipping property.

Examples of such a polymer surfactant include a polymer having at least one selected from repeating units represented by any of the following formulae (8A) to (8E).

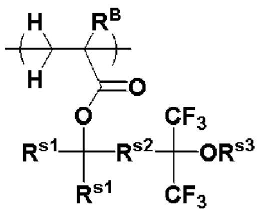
(8A)

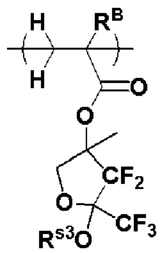
(8B)

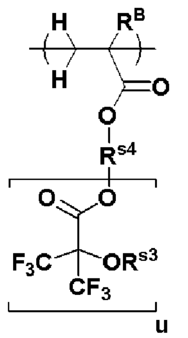
(8C)

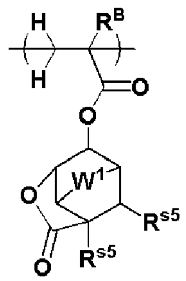
(8D)

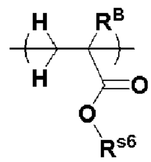
(8E)

In the formulae (8A) to (8E), $R^B$ represents a hydrogen atom, a fluorine atom, a methyl group, or a trifluoromethyl group. $W^1$ represents $-CH_2-$, $-CH_2CH_2-$, - O-, or separated two -H. $R^{s1}$ each independently represents a hydrogen atom or a hydrocarbyl group having 1 to 10 carbon atoms. $R^{s2}$ represents a single bond or a linear or branched hydrocarbylene group having 1 to 5 carbon atoms. $R^{s3}$ each independently represents a hydrogen atom, a hydrocarbyl group or fluorinated hydrocarbyl group having 1 to 15 carbon atoms, or an acid-labile group. When $R^{s3}$ represents a hydrocarbyl group or a fluorinated hydrocarbyl group, an ether bond or a carbonyl group is optionally interposed between a carbon-carbon bond. $R^{s4}$ represents a (u + 1) -valent hydrocarbon group or fluorinated hydrocarbon group having 1 to 20 carbon atoms. "u" represents an integer of 1 to 3. $R^{s5}$ each independently represents a hydrogen atom or a group represented by -C(=O)-O-$R^{s7}$. $R^{s7}$ represents a fluorinated hydrocarbyl group having 1 to 20 carbon atoms. $R^{s6}$ represents a hydrocarbyl group or fluorinated hydrocarbyl group having 1 to 15 carbon atoms, and an ether bond or a carbonyl group is optionally interposed between a carbon-carbon bond thereof. Here, $R^B$ and "u" are applied only in the formulae (8A) to (8E).

The hydrocarbyl group represented by $R^{s1}$ may be any of linear, branched, and cyclic groups. Specific examples thereof include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, a cyclopentyl group, an n-hexyl group, a cyclohexyl group, an n-heptyl, an n-octyl group, an n-nonyl group, an n-decyl group, an adamantyl group, and a norbornyl group. Among these, groups having 1 to 6 carbon atoms are preferable.

The hydrocarbylene group represented by $R^{s2}$ may be any of linear, branched, and cyclic groups. Specific

examples thereof include a methylene group, an ethylene group, a propylene group, a butylene group, and a pentylene group.

The hydrocarbyl group represented by $R^{s3}$ or $R^{s6}$ may be any of linear, branched, and cyclic groups. Specific examples thereof include alkyl groups, alkenyl groups, and alkynyl groups, and the hydrocarbyl group is preferably alkyl groups. Examples of the alkyl group include the groups exemplified as the hydrocarbyl group represented by $R^{s1}$, and in addition, an n-undecyl group, an n-dodecyl group, a tridecyl group, a tetradecyl group, and a pentadecyl group. Examples of the fluorinated hydrocarbyl group represented by $R^{s3}$ or $R^{s6}$ include groups in which a part or all of hydrogen atoms bonded to a carbon atom in the above hydrocarbyl group are substituted with a fluorine atom. As described above, an ether bond or a carbonyl group is optionally interposed between a carbon-carbon bond thereof.

Examples of the acid-labile group represented by $R^{s3}$ include: the groups represented by the aforementioned formulae (AL-1) to (AL-3); tertiary hydrocarbyl groups having 4 to 20, preferably 4 to 15, carbon atoms; trialkylsilyl groups in which each alkyl group has 1 to 6 carbon atoms; and an oxoalkyl groups having 4 to 20 carbon atoms.

The (u + 1)-valent hydrocarbon group or fluorinated hydrocarbon group represented by $R^{s4}$ may be any of linear, branched, and cyclic groups. Specific examples thereof include groups obtained by further removing u hydrogen atoms from the above hydrocarbyl group, fluorinated hydrocarbyl group, etc.

The fluorinated hydrocarbyl group represented by $R^{s7}$ may be any of linear, branched, and cyclic groups. Specific examples thereof include groups in which a part or all of hydrogen atoms in the above hydrocarbyl group are substituted with a fluorine atom. Specific examples thereof include a trifluoromethyl group, a 2,2,2-trifluoroethyl group, a 3,3,3-trifluoro-1-propyl group, a 3,3,3-trifluoro-2-propyl group, a 2,2,3,3-tetrafluoropropyl group, a 1,1,1,3,3,3-hexafluoroisopropyl group, a 2,2,3,3,4,4,4-heptafluorobutyl group, a 2,2,3,3,4,4,5,5-octafluoropentyl group, a 2, 2, 3, 3, 4, 4, 5, 5, 6, 6, 7, 7-dodecafluoroheptyl group, a 2-(perfluorobutyl)ethyl group, a 2-(perfluorohexyl)ethyl group, a 2-(perfluorooctyl)ethyl group, and a 2-(perfluorodecyl)ethyl group.

Examples of the repeating units represented by any one of the formulae (8A) to (8E) include the following repeating units, but the repeating unit is not limited thereto. In the following formulae, $R^B$ represents the same as above.

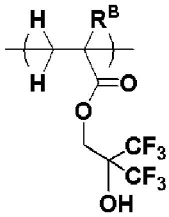
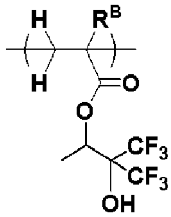
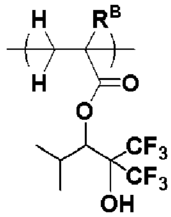
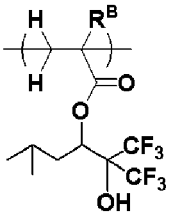
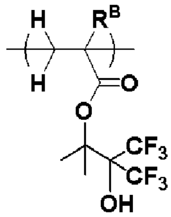
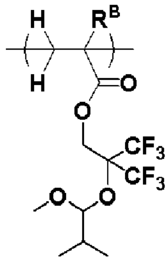

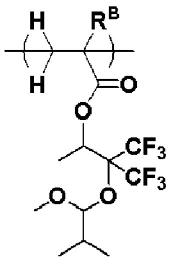
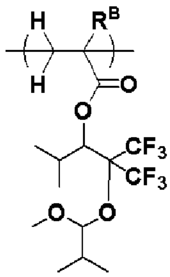
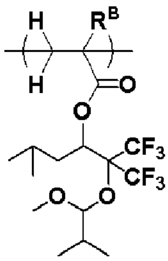
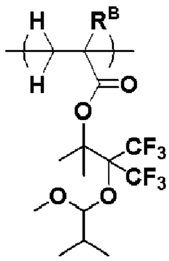
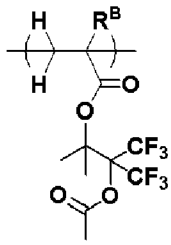
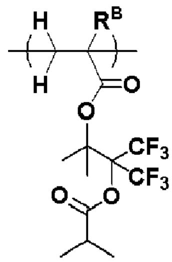

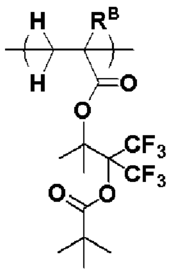
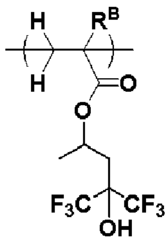
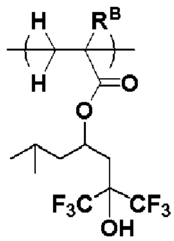
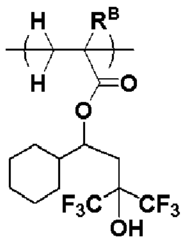
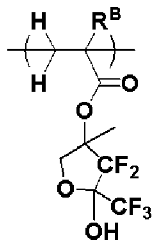
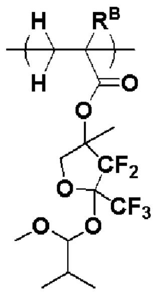

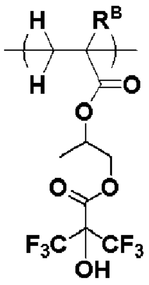 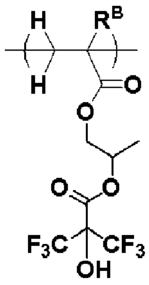 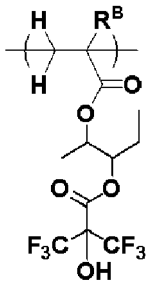 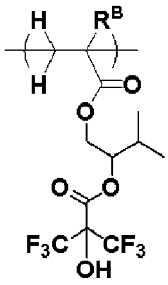 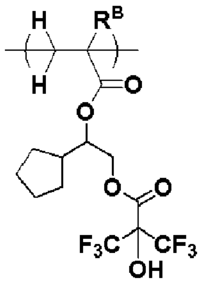 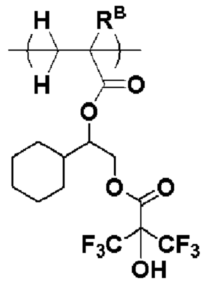

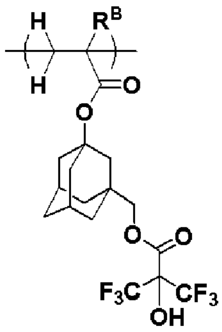 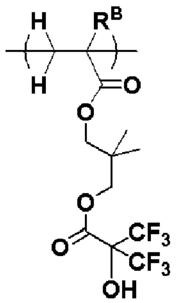 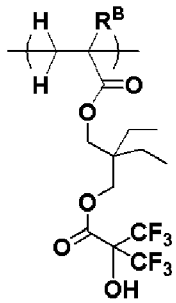 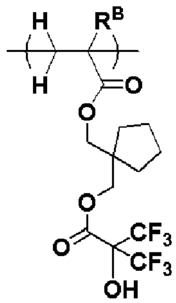

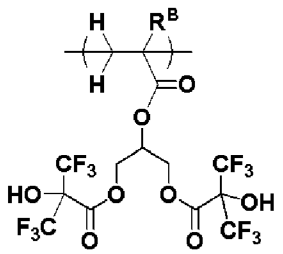 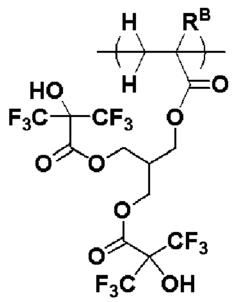 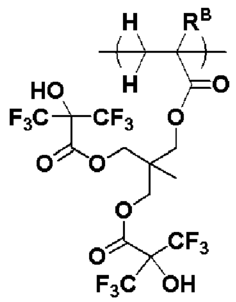 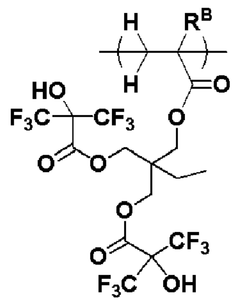

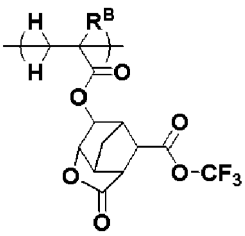 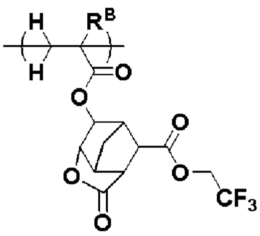 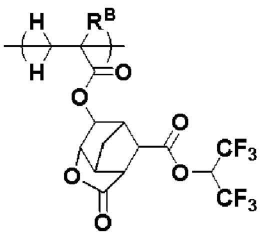 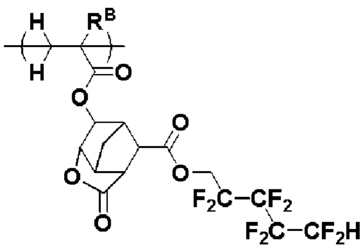

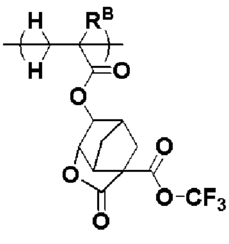 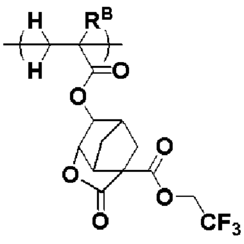 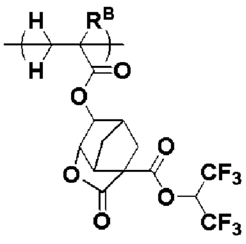 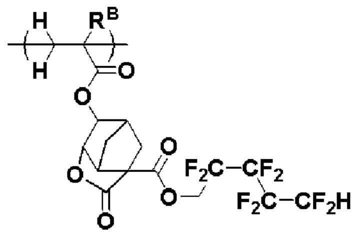

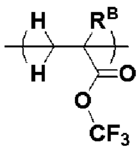 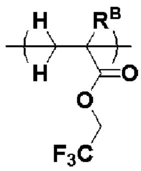 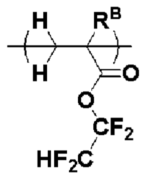 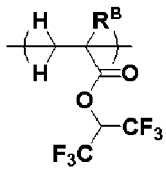 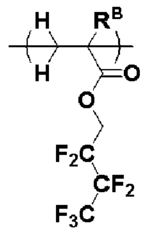 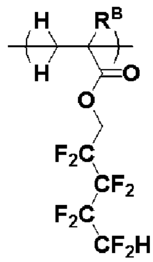 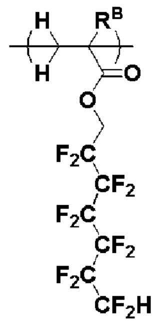

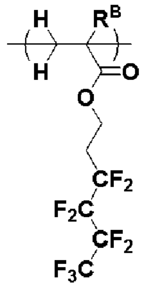 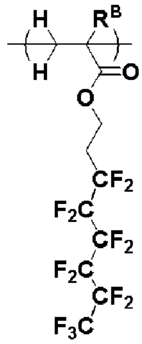 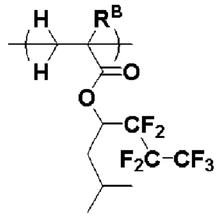

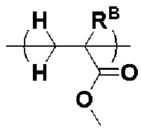 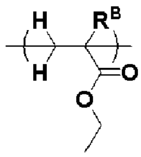 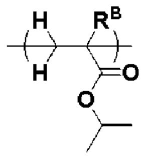 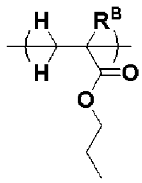 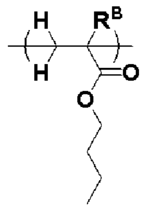 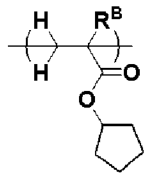 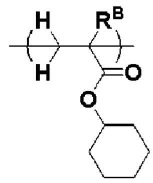

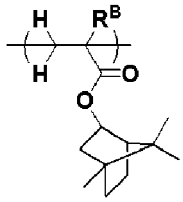 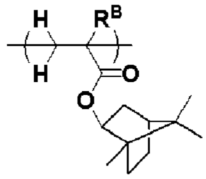 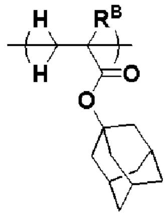 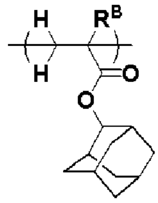

The polymer surfactant optionally further has a repeating unit other than the repeating units represented by the formulae (8A) to (8E). Examples of the other repeating unit include repeating units obtained from methacrylic acid, an $\alpha$-trifluoromethylacrylic acid derivative, etc. In the polymer surfactant, a content of the repeating units represented by the formulae (8A) to (8E) is preferably 20 mol% or more, more preferably 60 mol% or more, and further preferably 100 mol% in all the repeating units.

Mw of the polymer surfactant is preferably 1,000 to 500,000, and more preferably 3,000 to 100,000. Mw/Mn is preferably 1.0 to 2.0, and more preferably 1.0 to 1.6.

Examples of a method for synthesizing the polymer surfactant include the following method. Into monomers having an unsaturated bond to yield the repeating unit represented by the formulae (8A) to (8E) and, as necessary, the other repeating unit in an organic solvent, and a radical initiator is added and heated to be polymerized. Examples of the organic solvent used in the polymerization include toluene, benzene, THF, diethyl ether, and dioxane. Examples of the polymerization initiator include AIBN, 2,2'-azobis(2,4-dimethylvaleronitrile), dimethyl 2,2-azobis(2-methylpropionate), benzoyl peroxide, and lauroyl peroxide. The reaction temperature is preferably 50 to 100°C. The reaction time is preferably 4 to 24 hours. The acid-labile group may be introduced into the monomer to be used as it is, or may be protected or partially protected after the polymerization.

When the polymer surfactant is synthesized, known chain transfer agents, such as dodecyl mercaptan and 2-mercaptoethanol, may be used to regulate the molecular weight. In this case, an addition amount of these chain transfer

agents is preferably 0.01 to 10 mol% relative to the total number of moles of the monomers to be polymerized.

When the surfactant of the component (F) is contained, a content thereof is preferably 0.1 to 50 parts by mass, and more preferably 0.5 to 10 parts by mass, relative to 80 parts by mass of the base polymer (B). When the addition amount is 0.1 part by mass or more, a sweepback contact angle between the resist film surface and water is sufficiently improved. When the addition amount is 50 parts by mass or less, the resist film surface has a low dissolution rate in the developer to sufficiently maintain the height of the formed fine pattern.

(G) Other Components

The inventive chemically amplified resist composition optionally includes: compounds to be decomposed by an acid to generate an acid (acid amplifying compounds); organic acid derivatives; fluorine-substituted alcohols; compounds to change in its solubility in a developer by an action of an acid, the compound having Mw of 3,000 or less (dissolution inhibitors); etc. as another component (G). As the acid amplifying compound, compounds described in JP 2009-269953 A or JP 2010-215608 A can be referred. When the acid amplifying compound is contained, a content thereof is preferably 0 to 5 parts by mass, and more preferably 0 to 3 parts by mass, relative to 80 parts by mass of the base polymer (B). If the content is too high, it is difficult to control the acid diffusion, and deterioration in resolution and deterioration in the pattern shape may occur. As the organic acid derivative, the fluorine-substituted alcohol, and the dissolution inhibitor, compounds described in JP 2009-269953 A or JP 2010-215608 A can be referred.

Patterning Process

The inventive patterning process comprises steps of: forming a resist film on a substrate using the aforementioned chemically amplified resist composition; exposing the resist film to high energy ray, such as KrF excimer laser light, ArF excimer laser light, electron beam (EB), or extreme ultraviolet ray (EUV) having a wavelength of 3 to 15 nm; and developing the exposed resist film using a developer.

As the substrate, a substrate for manufacturing an integrated circuit (such as Si, $SiO_2$, SiN, SiON, TiN, WSi, BPSG, SOG, and an organic anti-reflective film) or a substrate for manufacturing a mask circuit (such as Cr, CrO, CrON, $MoSi_2$, and $SiO_2$) can be used, for example.

The resist film can be used by, for example: applying the above chemically amplified resist composition by a method such as spin-coating so that the film thickness is 0.05 to 2 μm; and prebaking the coating film on a hot plate at preferably 60 to 150°C for 1 to 10 minutes, more preferably 80 to 140°C for 1 to 5 minutes.

In the patterning process, the high energy ray is preferably KrF excimer laser light, ArF excimer laser light, electron beam, or extreme ultraviolet ray having a wavelength of 3 to 15 nm.

When the KrF excimer laser light, the ArF excimer laser light, or the EUV is used, the exposure of the resist film can be performed by using a mask for forming a target pattern, and irradiating such ray at an exposure dose of preferably 1 to 200 $mJ/cm^2$, more preferably 10 to 100 $mJ/cm^2$. When the EB is used, the exposure can be performed by using a mask for forming a target pattern or directly, and irradiating the EB at an exposure dose of preferably 1 to 300 $\mu C/cm^2$, more preferably 10 to 200 $\mu C/cm^2$.

The exposure can be performed by a common exposure method, or can be performed by using an immersion method in which a liquid having a refractive index of 1.0 or more is interposed between the resist film and a projection lens. In this case, a protective film insoluble in water can be used.

The protective film insoluble in water, which is used for preventing an eluted material from the resist film and for improving water-slipping property on the film surface, roughly includes two types. One is an organic-solvent removal type, and the other is alkaline aqueous solution-soluble type. With the former type, the protective film is required to be removed by an organic solvent not dissolving the resist film before development with an alkaline aqueous solution. With the latter type, the protective film is soluble in an alkaline developer and removed together with a soluble portion in the resist film. The latter protective film is particularly preferably a material containing a base polymer having a 1,1,1,3,3,3-hexafluoro-2-propanol residue group, which is insoluble in water and soluble in the alkaline developer, and dissolved in a solvent. Examples of such a solvent include an alcoholic solvent having 4 or more carbon atoms, an ether solvent having 8 to 12 carbon atoms, and a mixed solvent thereof. The aforementioned surfactant insoluble in water and soluble in an alkaline developer can be dissolved in the alcoholic solvent having 4 or more carbon atoms, the ether solvent having 8 to 12 carbon atoms, or the mixed solvent thereof to form the above material.

PEB may be performed after the exposure. The PEB can be performed by heating, for example, on a hot plate preferably at 60 to 150°C for 1 to 5 minutes, more preferably 80 to 140°C for 1 to 3 minutes.

A developer of an alkaline aqueous solution, such as tetramethylammonium hydroxide (TMAH) at preferably 0.1 to 5 mass%, more preferably 2 to 3 mass%, can be used, for example. The development can be performed for preferably 0.1 to 3 minutes, more preferably 0.5 to 2 minutes, by a common method, such as a dip method, a puddle method, and a spray method, to form a target pattern on the substrate with dissolving the exposed portion.

As a means for patterning process, after the resist film formation, the acid generator, etc. may be extracted from the film surface by performing a rinse with pure water (post soak), particles may be washed away, and rinsing (post soak) may be performed to remove water remained on the film after the exposure.

Furthermore, a pattern may be formed by a double-patterning method. Examples of the double-patterning method include: a trench method in which a first exposure and etching process a foundation of a 1:3 trench pattern, and a position-shifted second exposure forms the 1:3 trench pattern to form a 1:1 pattern; and a line method in which a first exposure and etching process a first foundation of a 1:3 isolated left pattern, and a position-shifted second exposure forms a second foundation having the formed 1:3 isolated left pattern under the first foundation to form 1:1 pattern with a half pitch.

In the inventive patterning process, a negative-tone development method can be used. This method uses an organic solvent as the developer instead of the developer of the alkaline aqueous solution, and the unexposed portion is dissolved to achieve the development.

As the developer for this organic solvent development, usable are 2-octanone, 2-nonanone, 2-heptanone, 3-heptanone, 4-heptanone, 2-hexanone, 3-hexanone, diisobutyl ketone, methylcyclohexanone, acetophenone, methylacetophenone, propyl acetate, butyl acetate, isobutyl acetate, pentyl acetate, butenyl acetate, isopentyl acetate, propyl formate, butyl formate, isobutyl formate, pentyl formate, isopentyl formate, methyl valerate, methyl pentenoate, methyl crotonate, ethyl crotonate, methyl propionate, ethyl propionate, ethyl 3-ethoxypropionate, methyl lactate, ethyl lactate, propyl lactate, butyl lactate, isobutyl lactate, pentyl lactate, isopentyl lactate, methyl 2-hydroxyisobutyrate, ethyl 2-hydroxyisobutyrate, methyl benzoate, ethyl benzoate, phenyl acetate, benzyl acetate, methyl phenylacetate, benzyl formate, phenylethyl formate, methyl 3-phenylpropionate, benzyl propionate, ethyl phenylacetate, 2-phenylethyl acetate, etc. These organic solvents can be used singly, or two or more kinds thereof can be mixed to use.

EXAMPLE

Hereinafter, the present invention will be specifically described with showing Synthesis Examples, Examples, and Comparative Examples, but the present invention is not limited to the following Examples. Used apparatuses were as follows.

- IR: NICOLET 6700, manufactured by Thermo Fisher Scientific K.K.
- $^1$H-NMR: ECA-500, manufactured by JEOL Ltd.
- MALDI TOF-MS: S3000, manufactured by JEOL Ltd.

[1] Synthesis of Onium Salt

Example I-1: Synthesis of PAG-1

(1) Synthesis of Intermediate In-1

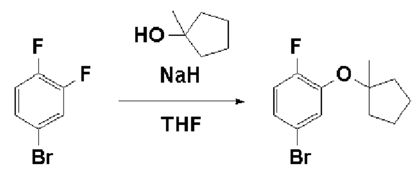


M-1 In-1

Under a nitrogen atmosphere, sodium hydride (purity: 55 mass%, 43.6 g) was dispersed in THF (240 ml), and a solution composed of 1-methylcyclopentanol (114.2 g) and THF (120 ml) was added dropwise thereinto. After the dropwise addition, the mixture was heated under reflux for 4 hours to prepare a metal alkoxide. Thereafter, a raw material M-1 (193.0 g) was added dropwise thereinto, and the mixture was heated under reflux for 26 hours for aging. The reaction liquid was cooled with an ice bath, and the reaction was terminated with water (400 ml). The target product was extracted twice with a solvent composed of toluene (200 ml) and ethyl acetate (200 ml), a common aqueous work-up was performed, the solvent was evaporated, and purification was performed with distillation to obtain 210.3 g of an intermediate In-1 as a colorless oil (77% yield).

(2) Synthesis of Intermediate In-2

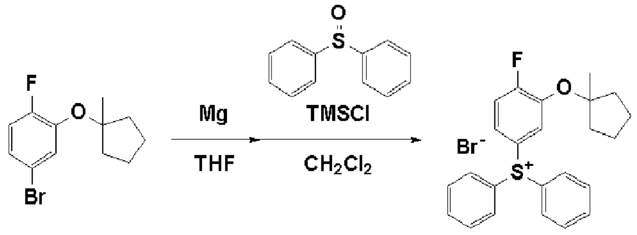


In-1 In-2

Under a nitrogen atmosphere, a Grignard reagent was prepared from magnesium (3.7 g), THF (120 g), and the intermediate In-1 (41.0 g). The reaction system was cooled to 10°C or lower, and a solution composed of diphenyl sulfoxide (10.1 g) and methylene chloride (50 g) was added. After the addition, chlorotrimethylsilane (22.6 g) was added dropwise with maintaining the internal temperature of 20°C or lower. After the dropwise addition, the mixture was aged at an internal temperature of 20°C or lower for 2 hours. After the aging, the reaction system was cooled, and an aqueous solution composed of ammonium chloride (15 g), 20 mass% hydrochloric acid (15 g), and water (100 g) was added dropwise to terminate the reaction. Thereafter, methanol (50 g) and diisopropyl ether (200 g) were added, and the aqueous layer was separated. Then, the separated aqueous layer was washed twice with hexane (200 g). Into the washed aqueous layer, methylene chloride (100 g) was added to extract the target product. Thereafter, a common aqueous-work up was performed, and the solvent was evaporated to obtain 19.5 g of an intermediate In-2 as a colorless oil (85% yield).

(3) Synthesis of PAG-1

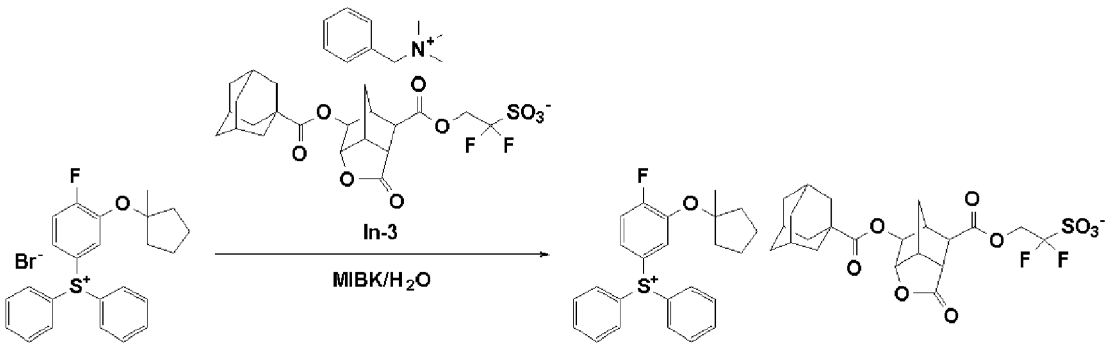


In-2 PAG-1

Under a nitrogen atmosphere, the intermediate In-2 (5.1 g), an intermediate In-3 (6.5 g), methyl isobutyl ketone (60 g), methylene chloride (15 g), and water (30 g) were added, and the mixture was stirred at a room temperature for 30 minutes. The organic layer was separated, washed with water, and then condensed under a reduced pressure. The residue was recrystallized with diisopropyl ether to obtain 8.75 g of PAG-1 as a white crystal (97% yield).

IR spectrum data and a TOF-MS result of PAG-1 are shown below. FIG. 1 shows a result of a nuclear magnetic resonance spectrum ($^1$H-NMR in DMSO-$d_6$).

IR (D-ATR): ν = 3489, 3064, 2969, 2908, 2853, 1786, 1731, 1601, 1500, 1477, 1448, 1404, 1380, 1324, 1225, 1238, 1181, 1104, 1076, 1035, 1011, 939, 795, 751, 719, 685, 642, 585, 551, 524, 503, 460 $cm^{-1}$
MALDI TOF-MS: POSITIVE $M^+$ 379 (corresponding to $C_{24}H_{24}FOS^+$)
NEGATIVE $M^-$ 503 (corresponding to $C_{22}H_{25}F_2O_9S^-$)

Example I-2: Synthesis of PAG-2

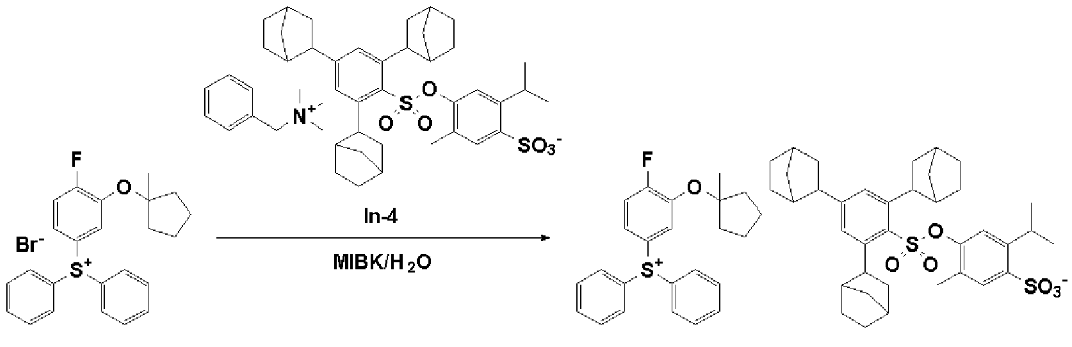


In-2 PAG-2

PAG-2 was synthesized in the same manner as in Example I-1 (3) except that the intermediate In-3 was changed to an intermediate In-4 (yielded amount: 14.0 g, 91% yield).

IR spectrum data and a TOF-MS result of PAG-2 are shown below. FIG. 2 shows a result of a nuclear magnetic resonance spectrum ($^1$H-NMR in DMSO-$d_6$).

IR (D-ATR): ν = 3444, 3061, 2952, 2870, 1597, 1558, 1496, 1478, 1447, 1403, 1383, 1361, 1311, 1296, 1269, 1183, 1125, 1082, 1036, 999, 969, 926, 906, 883, 816, 758, 716, 684, 641, 594, 561, 545, 504 $cm^{-1}$
MALDI TOF-MS: POSITIVE $M^+$ 379 (corresponding to $C_{24}H_{24}FOS^+$)
NEGATIVE $M^-$ 651 (corresponding to $C_{37}H_{47}O_6S_2^-$)

Example I-3: Synthesis of PAG-3

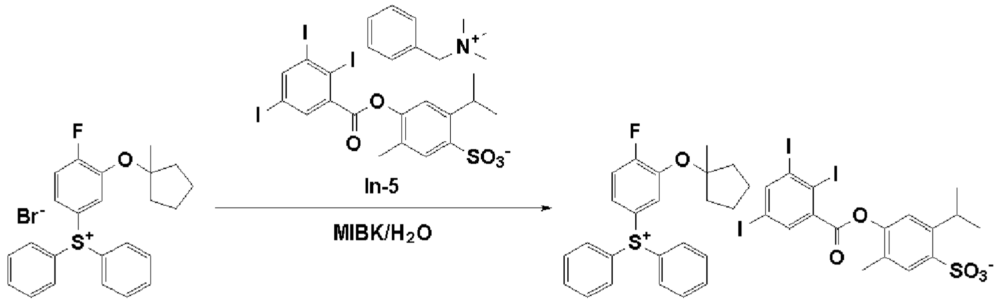


In-2 PAG-3

PAG-3 was synthesized in the same manner as in Example I-1 (3) except that the intermediate In-3 was changed to an intermediate In-5 (yielded amount: 10.6 g, 97% yield).

IR spectrum data and a TOF-MS result of PAG-3 are shown below. FIG. 3 shows a result of a nuclear magnetic resonance spectrum ($^1$H-NMR in DMSO-$d_6$).

IR (D-ATR): ν = 3451, 3060, 2969, 2870, 1749, 1600, 1570, 1497, 1446, 1398, 1379, 1365, 1326, 1268, 1252, 1215, 1199, 1177, 1143, 1122, 1093, 1038, 1001, 931, 871, 812, 750, 717, 685, 656, 640, 617, 591, 561, 527, 504 $cm^{-1}$
MALDI TOF-MS: POSITIVE $M^+$ 379 (corresponding to $C_{24}H_{24}FOS^+$)
NEGATIVE $M^-$ 711 (corresponding to $C_{17}H_{14}I_2O_5S^-$)

Example I-4: Synthesis of PAG-4

(1) Synthesis of Intermediate In-6

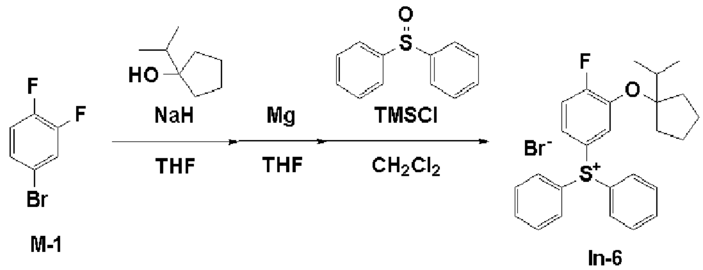


M-1

In-6

An intermediate In-6 was synthesized in the same manners as in Example I-1 (1) and Example I-1 (2) except that 1-methylcyclopentanol was changed to 1-isopropylcyclopentanol (yielded amount: 24.8 g, 72% yield in two steps).

(2) Synthesis of PAG-4

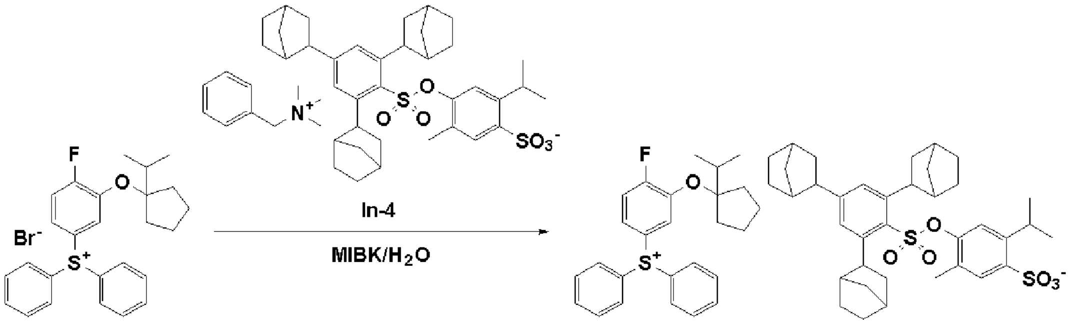


In-6

PAG-4

PAG-4 was synthesized in the same manner as in Example I-2 except that the intermediate In-2 was changed to the intermediate In-6 (yielded amount: 10.3 g, 97% yield).

IR spectrum data and a TOF-MS result of PAG-4 are shown below. FIG. 4 shows a result of a nuclear magnetic resonance spectrum ($^1$H-NMR in DMSO-$d_6$).

IR (D-ATR): ν = 3060, 2954, 2870, 1599, 1559, 1504, 1476, 1446, 1406, 1386, 1359, 1311, 1288, 1266, 1237, 1214, 1202, 1183, 1162, 1130, 1082, 1036, 998, 970, 958, 913, 882, 815, 790, 745, 716, 682, 641, 601, 590, 562, 545, 511, 494, 465 $cm^{-1}$
MALDI TOF-MS: POSITIVE $M^+$ 407 (corresponding to $C_{26}H_{28}FOS^+$)
NEGATIVE $M^-$ 651 (corresponding to $C_{37}H_{47}O_6S_2^-$)

Example I-5: Synthesis of PAG-5

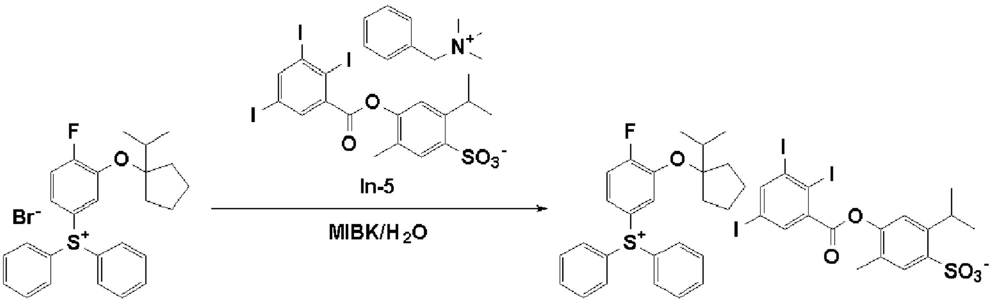


In-6

PAG-5

PAG-5 was synthesized in the same manner as in Example I-3 except that the intermediate In-2 was changed to the intermediate In-6 (yielded amount: 10.3 g, 92% yield).

IR spectrum data and a TOF-MS result of PAG-5 are shown below. FIG. 5 shows a result of a nuclear magnetic resonance spectrum ($^1$H-NMR in DMSO-$d_6$).

IR (D-ATR): ν = 3452, 3060, 2988, 2871, 1749, 1599, 1569, 1498, 1477, 1446, 1397, 1365, 1325, 1267, 1252, 1214, 1175, 1143, 1123, 1092, 1038, 1000, 968, 953, 912, 870, 808, 749, 717, 685, 656, 640, 617, 591, 560, 527, 504 $cm^{-1}$.
MALDI TOF-MS: POSITIVE $M^+$ 407 (corresponding to $C_{26}H_{28}FOS^+$)
NEGATIVE $M^-$ 711 (corresponding to $C_{17}H_{14}I_3O_5S^-$)

Examples I-6 to I-17: Synthesis of PAG-6 to PAG-17

Each onium salt was synthesized with a corresponding raw material and each organic synthesis reaction. Structures of onium salts used for chemically amplified resist compositions are shown below.

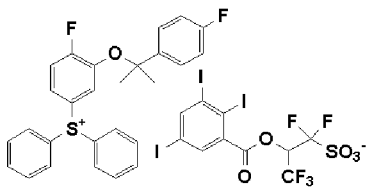

PAG-6

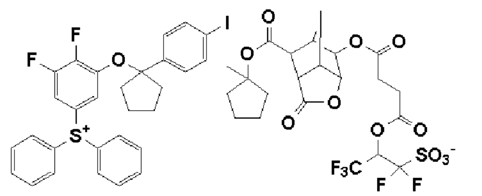

PAG-7

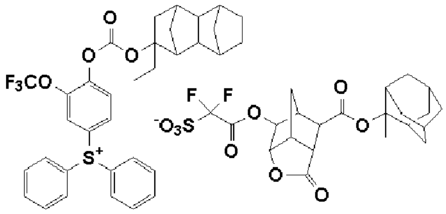

PAG-8

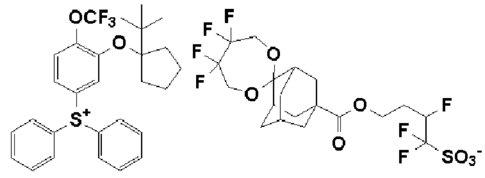

PAG-9

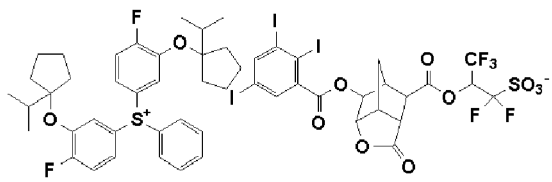

PAG-10

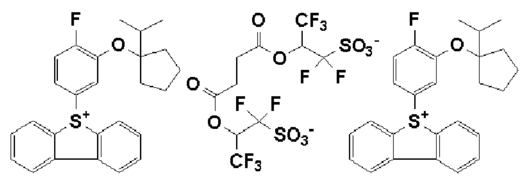

PAG-11

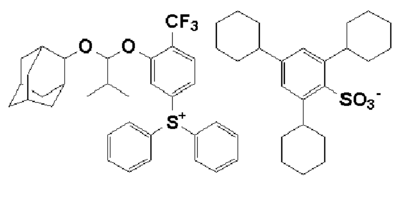

PAG-12

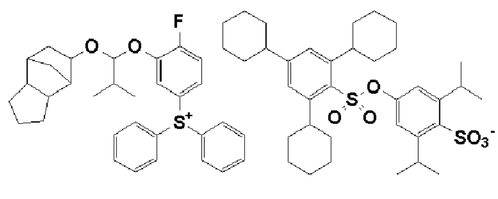

PAG-13

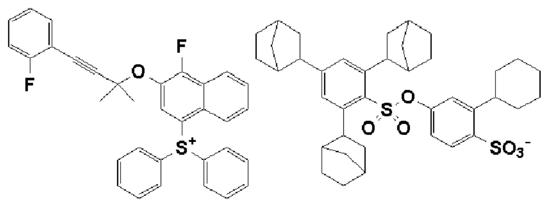

PAG-14

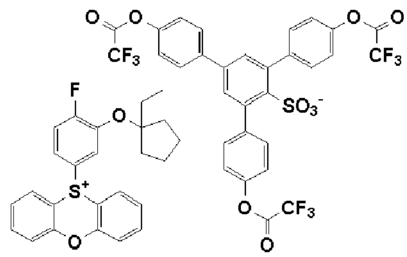

PAG-15

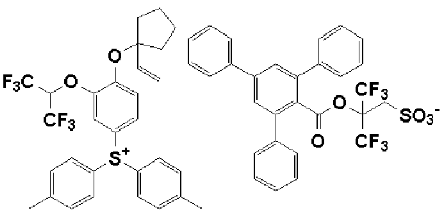

PAG-16

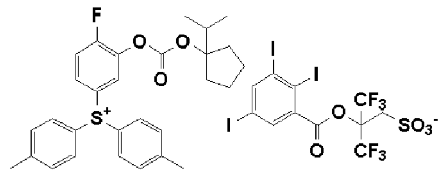

PAG-17

[2] Synthesis of Base Polymer

A base polymer used for a chemically amplified resist composition was synthesized by a method described below. Mw and Mn of the obtained polymer were measured in terms of polystyrene by GPC using THF as a solvent.

Synthesis Example: Synthesis of Base Polymers (Polymers P-1 to P-6)

Each monomer was combined and subjected to a copolymerization reaction in THF being a solvent. The reaction solution was poured into methanol, the precipitated solid was washed with hexane, and then isolated and dried to obtain base polymers (polymers P-1 to P-6) having compositions described below. The composition of the obtained base polymer was determined by $^1$H-NMR, and Mw and Mw/Mn was determined by GPC (solvent: THF, standard: polystyrene).

Polymer P-1
Mw=5,200
Mw/Mn=1.45

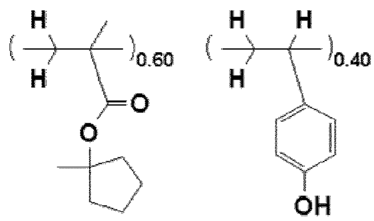

Polymer P-2
Mw=7,000
Mw/Mn=1.60

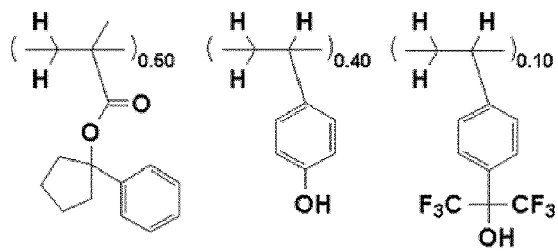

Polymer P-3
Mw=9,200
Mw/Mn=1.82

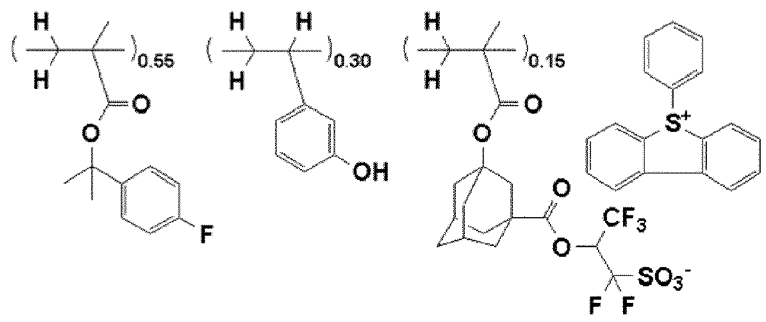

Polymer P-4
Mw=5,000
Mw/Mn=1.58

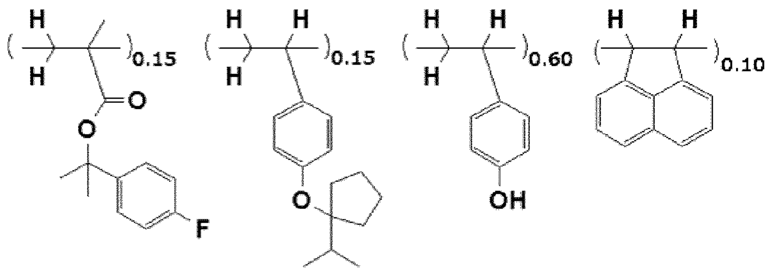

Polymer P-5
Mw=5,400
Mw/Mn=1.61

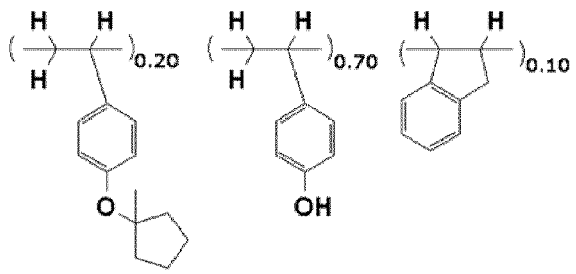

Polymer P-6
Mw=6,400
Mw/Mn=1.51

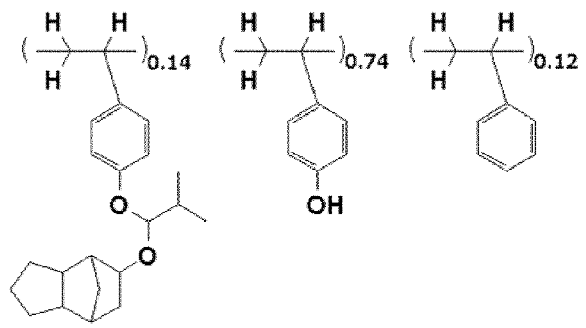

[3] Preparation of Chemically Amplified Resist Composition

Examples 1-1 to 1-32 and Comparative Examples 1-1 to 1-24

The inventive sulfonium salts (photoacid generators: PAG-1 to PAG-17), comparative photoacid generators (PAG-A to PAG-L), the base polymers (P-1 to P-6), quenchers (Q-1 to Q-6), and a hydrophobic resin F-1 were dissolved into solvents containing 100 ppm of FC-4430, manufactured by 3M Company, as a surfactant at compositions shown in the following Table 1 and Table 2 to prepare solutions. The solutions were filtered with a Teflon(R) filter with 0.2 μm to prepare chemically amplified resist compositions (R-1 to R-32 and CR-1 to CR-24).

[Table 1]

| | Resist composition | Base resin (parts by mass) | Photoacid generator (parts by mass) | Quencher (parts by mass) | Hydrophobic resin (parts by mass) | Solvent 1 (parts by mass) | Solvent 2 (parts by mass) | Solvent 3 (parts by mass) |
|---|---|---|---|---|---|---|---|---|
| Example 1-1 | R-1 | P-1 (80) | PAG-1 (22) | Q-1 (6.0) | - | PGMEA (2,200) | DAA (900) | |
| Example 1-2 | R-2 | P-1 (80) | PAG-6 (24) | Q-1 (6.0) | - | PGMEA (2,200) | DAA (900) | |
| Example 1-3 | R-3 | P-1 (80) | PAG-7 (23) | Q-1 (6.0) | - | PGMEA (2,200) | DAA (900) | |
| Example 1-4 | R-4 | P-1 (80) | PAG-8 (20) | Q-1 (6.0) | - | PGMEA (2,200) | DAA (900) | |
| Example 1-5 | R-5 | P-1 (80) | PAG-9 (24) | Q-1 (6.0) | - | PGMEA (2,200) | DAA (900) | |
| Example 1-6 | R-6 | P-1 (80) | PAG-10 (20) | Q-1 (6.0) | - | PGMEA (2,200) | DAA (900) | |
| Example 1-7 | R-7 | P-1 (80) | PAG-11 (11) | Q-1 (6.0) | - | PGMEA (2,200) | DAA (900) | |
| Example 1-8 | R-8 | P-1 (80) | PAG-16 (25) | Q-2 (6.0) | - | PGMEA (2,200) | DAA (900) | |
| Example 1-9 | R-9 | P-2 (80) | PAG-1 (22) | Q-1 (3.4) Q-6 (3.4) | - | PGMEA (2,200) | DAA (900) | |
| Example 1-10 | R-10 | P-2 (80) | PAG-6 (24) | Q-2 (6.2) | - | PGMEA (2,200) | DAA (900) | - |
| Example 1-11 | R-11 | P-2 (80) | PAG-8 (21) | Q-3 (5.8) | - | PGMEA (2,200) | DAA (900) | - |
| Example 1-12 | R-12 | P-2 (80) | PAG-16 (25) | Q-2 (6.2) | - | PGMEA (2,200) | DAA (900) | - |
| Example 1-13 | R-13 | P-3 (80) | PAG-1 (12) | Q-1 (7.8) | - | PGMEA (2,200) | DAA (900) | - |
| Example 1-14 | R-14 | P-3 (80) | PAG-6 (10) | Q-2 (3.4) Q-6 (3.4) | - | PGMEA (2,200) | DAA (900) | - |
| Example 1-15 | R-15 | P-3 (80) | PAG-10 (10) | Q-3 (5.8) | - | PGMEA (2,200) | DAA (900) | - |
| Example 1-16 | R-16 | P-4 (80) | PAG-2 (6) | Q-4 (2.5) | F-1 (1.5) | EL (1,700) | PGME (360) | PGMEA (340) |
| Example 1-17 | R-17 | P-4 (80) | PAG-3 (6) | Q-4 (2.5) | F-1 (1.5) | EL (1,700) | PGME (360) | PGMEA (340) |
| Example 1-18 | R-18 | P-4 (80) | PAG-4 (6) | Q-4 (2.5) | F-1 (1.5) | EL (1,700) | PGME (360) | PGMEA (340) |
| Example 1-19 | R-19 | P-4 (80) | PAG-5 (6) | Q-4 (2.5) | F-1 (1.5) | EL (1,700) | PGME (360) | PGMEA (340) |
| Example 1-20 | R-20 | P-4 (80) | PAG-12 (6) | Q-4 (2.5) | F-1 (1.5) | EL (1,700) | PGME (360) | PGMEA (340) |

(continued)

| | Resist composition | Base resin (parts by mass) | Photoacid generator (parts by mass) | Quencher (parts by mass) | Hydrophobic resin (parts by mass) | Solvent 1 (parts by mass) | Solvent 2 (parts by mass) | Solvent 3 (parts by mass) |
|---|---|---|---|---|---|---|---|---|
| Example 1-21 | R-21 | P-4 (80) | PAG-13 (6) | Q-4 (2.5) | F-1 (1.5) | EL (1,700) | PGME (360) | PGMEA (340) |
| Example 1-22 | R-22 | P-4 (80) | PAG-14 (6) | Q-4 (2.5) | F-1 (1.5) | EL (1,700) | PGME (360) | PGMEA (340) |
| Example 1-23 | R-23 | P-4 (80) | PAG-15 (6) | Q-4 (2.5) | F-1 (1.5) | EL (1,700) | PGME (360) | PGMEA (340) |
| Example 1-24 | R-24 | P-4 (80) | PAG-16 (4) | Q-4 (2.5) | F-1 (1.5) | EL (1,700) | PGME (360) | PGMEA (340) |
| Example 1-25 | R-25 | P-5 (80) | PAG-4 (8) | Q-4 (2.5) | F-1 (1.5) | EL (1,700) | PGME (360) | PGMEA (340) |
| Example 1-26 | R-26 | P-5 (80) | PAG-5 (8) | Q-4 (2.5) | F-1 (1.5) | EL (1,700) | PGME (360) | PGMEA (340) |
| Example 1-27 | R-27 | P-5 (80) | PAG-13 (6) | Q-4 (2.5) | F-1 (1.5) | EL (1,700) | PGME (360) | PGMEA (340) |
| Example 1-28 | R-28 | P-5 (80) | PAG-15 (8) | Q-4 (2.5) | F-1 (1.5) | EL (1,700) | PGME (360) | PGMEA (340) |
| Example 1-29 | R-29 | P-5 (80) | PAG-16 (4) | Q-5 (3.0) | F-1 (1.5) | EL (1,700) | PGME (360) | PGMEA (340) |
| Example 1-30 | R-30 | P-6 (80) | PAG-4 (6) | Q-4 (7.8) | F-1 (1.5) | EL (1,700) | PGME (360) | PGMEA (340) |
| Example 1-31 | R-31 | P-6 (80) | PAG-16 (5) | Q-5 (2.8) | F-1 (1.5) | EL (1,700) | PGME (360) | PGMEA (340) |
| Example 1-32 | R-32 | P-6 (80) | PAG-17 (5) | Q-5 (2.8) | F-1 (1.5) | EL (1,700) | PGME (360) | PGMEA (340) |

[Table 2]

| | Resist composition | Base resin (parts by mass) | Photoacid generator (parts by mass) | Quencher (parts by mass) | Hydrophobic resin (parts by mass) | Solvent 1 (parts by mass) | Solvent 2 (parts by mass) | Solvent 3 (parts by mass) |
|---|---|---|---|---|---|---|---|---|
| Comparative Example 1-1 | CR-1 | P-1 (80) | PAG-A (22) | Q-1 (6.0) | - | PGMEA (2,200) | DAA (900) | - |
| Comparative Example 1-2 | CR-2 | P-1 (80) | PAG-B (21) | Q-1 (6.2) | - | PGMEA (2,200) | DAA (900) | - |
| Comparative Example 1-3 | CR-3 | P-1 (80) | PAG-C (23) | Q-1 (6.1) | - | PGMEA (2,200) | DAA (900) | - |
| Comparative Example 1-4 | CR-4 | P-1 (80) | PAG-D (22) | Q-1 (5.8) | - | PGMEA (2,200) | DAA (900) | - |
| Comparative Example 1-5 | CR-5 | P-1 (80) | PAG-E (20) | Q-1 (6.0) | - | PGMEA (2,200) | DAA (900) | - |
| Comparative Example 1-6 | CR-6 | P-1 (80) | PAG-F (22) | Q-1 (6.2) | - | PGMEA (2,200) | DAA (900) | - |
| Comparative Example 1-7 | CR-7 | P-2 (80) | PAG-B (20) | Q-2 (6.2) | - | PGMEA (2,200) | DAA (900) | - |
| Comparative Example 1-8 | CR-8 | P-2 (80) | PAG-C (22) | Q-3 (5.8) | - | PGMEA (2,200) | DAA (900) | - |
| Comparative Example 1-9 | CR-9 | P-2 (80) | PAG-F (20) | Q-3 (5.8) | - | PGMEA (2,200) | DAA (900) | - |
| Comparative Example 1-10 | CR-10 | P-3 (80) | PAG-C (12) | Q-1 (7.8) | - | PGMEA (1,400) | DAA (900) | - |
| Comparative Example 1-11 | CR-11 | P-3 (80) | PAG-E (10) | Q-3 (5.6) | - | PGMEA (1,400) | DAA (900) | - |
| Comparative Example 1-12 | CR-12 | P-3 (80) | PAG-D (10) | Q-2 (3.4)<br>Q-6 (3.4) | - | PGMEA (1,400) | DAA (900) | - |
| Comparative Example 1-13 | CR-13 | P-4 (80) | PAG-G (6) | Q-4 (2.5) | F-1 (1.5) | EL (1,700) | PGME (360) | PGMEA (340) |
| Comparative Example 1-14 | CR-14 | P-4 (80) | PAG-H (6) | Q-4 (2.5) | F-1 (1.5) | EL (1,700) | PGME (360) | PGMEA (340) |

(continued)

| | Resist composition | Base resin (parts by mass) | Photoacid generator (parts by mass) | Quencher (parts by mass) | Hydrophobic resin (parts by mass) | Solvent 1 (parts by mass) | Solvent 2 (parts by mass) | Solvent 3 (parts by mass) |
|---|---|---|---|---|---|---|---|---|
| Comparative Example 1-15 | CR-15 | P-4 (80) | PAG-1 (6) | Q-4 (2.5) | F-1 (1.5) | EL (1,700) | PGME (360) | PGMEA (340) |
| Comparative Example 1-16 | CR-16 | P-4 (80) | PAG-J (6) | Q-4 (2.5) | F-1 (1.5) | EL (1,700) | PGME (360) | PGMEA (340) |
| Comparative Example 1-17 | CR-17 | P-4 (80) | PAG-K (6) | Q-4 (2.5) | F-1 (1.5) | EL (1,700) | PGME (360) | PGMEA (340) |
| Comparative Example 1-18 | CR-18 | P-4 (80) | PAG-L (4) | Q-4 (2.5) | F-1 (1.5) | EL (1,700) | PGME (360) | PGMEA (340) |
| Comparative Example 1-19 | CR-19 | P-5 (80) | PAG-H (6) | Q-4 (2.5) | F-1 (1.5) | EL (1,700) | PGME (360) | PGMEA (340) |
| Comparative Example 1-20 | CR-20 | P-5 (80) | PAG-1 (6) | Q-4 (2.5) | F-1 (1.5) | EL (1,700) | PGME (360) | PGMEA (340) |
| Comparative Example 1-21 | CR-21 | P-5 (80) | PAG-J (6) | Q-4 (2.5) | F-1 (1.5) | EL (1,700) | PGME (360) | PGMEA (340) |
| Comparative Example 1-22 | CR-22 | P-5 (80) | PAG-L (4) | Q-5 (2.6) | F-1 (1.5) | EL (1,700) | PGME (360) | PGMEA (340) |
| Comparative Example 1-23 | CR-23 | P-6 (80) | PAG-J (6) | Q-4 (2.5) | F-1 (1.5) | EL (1,700) | PGME (360) | PGMEA (340) |
| Comparative Example 1-24 | CR-24 | P-6 (80) | PAG-L (4) | Q-5 (2.6) | F-1 (1.5) | EL (1,700) | PGME (360) | PGMEA (340) |

In Tables 1 and 2, the solvents, the hydrophobic resin F-1, the comparative photoacid generators PAG-A to PAG-L, and the quenchers Q-1 to Q-6 were as follows.
-Solvent: PGMEA (Propylene glycol monomethyl ether acetate)

DAA (Diacetone alcohol)

EL (Ethyl lactate)

PGME (Propylene glycol monomethyl ether)

-Hydrophobic resin: F-1

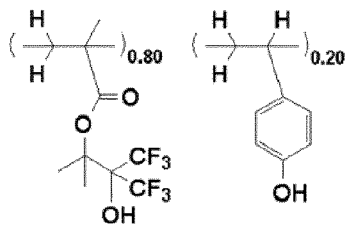

Hydrophobic resin F-1
Mw=6,000
Mw/Mn=1.53

-Comparative photoacid generator: PAG-A to PAG-L

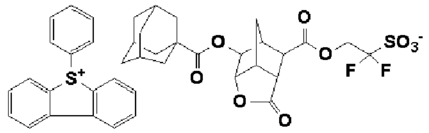

PAG-A

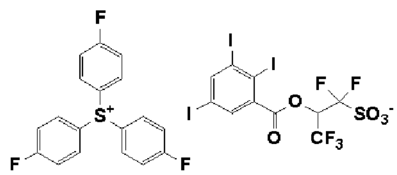

PAG-B

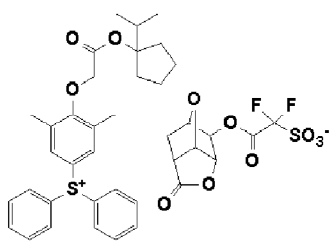

PAG-C

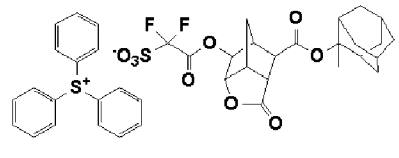

PAG-D

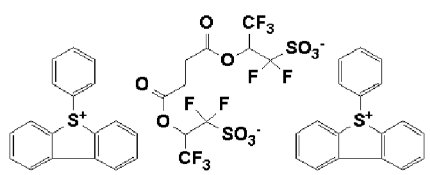

PAG-E

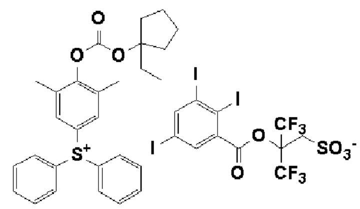

PAG-F

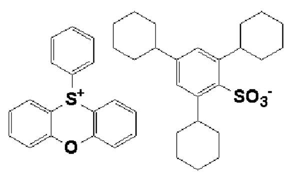

PAG-G

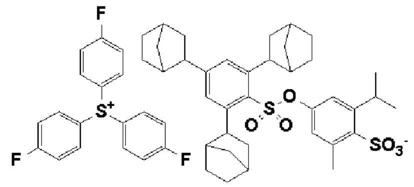

PAG-H

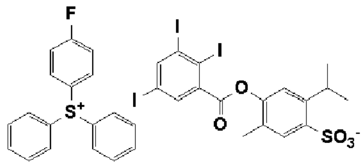

PAG-I

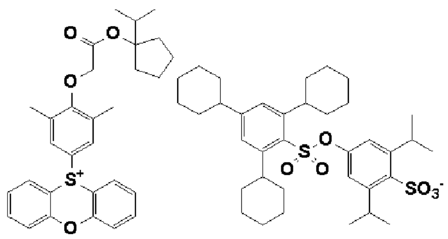

PAG-J

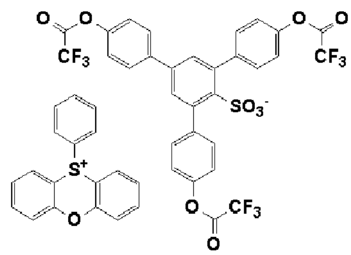

PAG-K

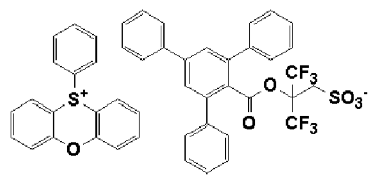

PAG-L

-Quencher: Q-1 to Q-6

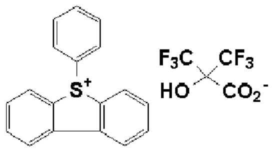

Q-1

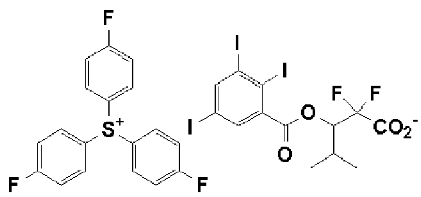

Q-2

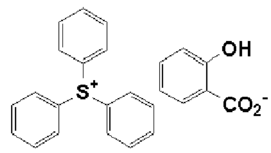

Q-3

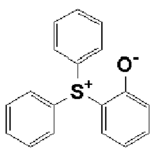

Q-4

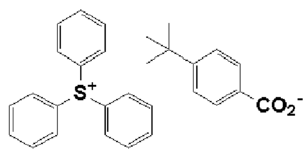

Q-5

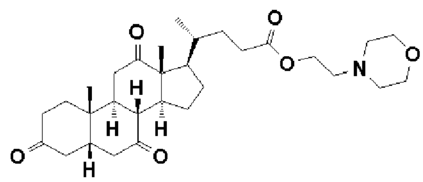

Q-6

[4] EUV Lithography Evaluation (1)

Examples 2-1 to 2-15 and Comparative Examples 2-1 to 2-12

Each of the chemically amplified resist compositions (R-1 to R-15 and CR-1 to CR-12) shown in Table 1 and Table 2 was applied by spin-coating on a Si substrate on which a silicon-containing spin-on-hard mask SHB-A940, manufactured by Shin-Etsu Chemical Co., Ltd. (silicon content of 43 mass%), was formed with 20 nm in film thickness. Then, the substrate was prebaked at 100°C for 60 seconds using a hot plate to produce a resist film with 50 nm in film

thickness. This resist film was exposed by using an EUV scanner NXE3300 (NA 0.33, σ 0.9/0.6, dipole illumination), manufactured by ASML Holding N.V. The exposure was performed with a LS pattern with 18 nm on wafer size and 36 nm in pitch, and with changing an exposure dose and focus (exposure dose pitch: 1 mJ/cm$^2$, focus pitch: 0.020 μm). After the exposure, PEB was performed at a temperature shown in Table 3 and Table 4 for 60 seconds. Thereafter, puddle development with a 2.38 mass% aqueous TMAH solution for 30 seconds, rinse with a surfactant-containing rinsing material, and spin-drying were performed to obtain a positive-type pattern.

The obtained LS pattern was observed with a length-measurement SEM (CG6300), manufactured by Hitachi High-Technologies Corporation, to evaluate sensitivity, exposure latitude (EL), LWR, depth of focus (DOF), and collapse limit in accordance with the following methods. Table 3 and Table 4 show the results.

Sensitivity Evaluation

An optimum exposure dose $E_{op}$ (mJ/cm$^2$) to yield the LS pattern with 18 nm in line width and 36 nm in pitch was determined to specify this value as a sensitivity. A smaller sensitivity value indicates higher sensitivity.

EL Evaluation

From exposure doses to form the LS pattern within a range of ± 10% of 18 nm space width (16.2 to 19.8 nm), EL (unit: %) was determined by the following equation. The larger the EL value, the better the performance.

$$EL\ (\%) = (|E_1 - E_2| / E_{op}) \times 100$$

$E_1$: An optimum exposure dose to yield a LS pattern with 16.2 nm in line width and 36 nm in pitch.
$E_2$: An optimum exposure dose to yield a LS pattern with 19.8 nm in line width and 36 nm in pitch.
$E_{op}$: An optimum exposure dose to yield the LS pattern with 18 nm in line width and 36 nm in pitch.

LWR Evaluation

In the LS pattern obtained by irradiation at $E_{op}$, sizes at 10 positions in the longitudinal direction of the line were measured. From the results, a tripled value (3σ) of a standard variation (σ) was determined as LWR. A smaller LWR value can yield a pattern with smaller roughness and uniform line width.

DOF Evaluation

As evaluation of depth of focus, determined was a focus range that formed within a range of the 18 nm size ± 10% (16.2 to 19.8 nm) of the LS pattern. A larger DOF value indicates wider depth of focus.

Evaluation of Collapse Limit of Line Pattern

Line sizes of the LS patterns at each exposure dose with the optimum focus were measured at 10 positions in the longitudinal direction. A narrowest line size obtained without collapse was specified as a collapse limit size. A smaller limit size value indicates excellent collapse limit.

[Table 3]

| | Resist composition | PEB temperature (°C) | Optimum exposure dose (mJ/cm$^2$) | EL (%) | LWR (nm) | DOF (nm) | Collapse limit (nm) |
|---|---|---|---|---|---|---|---|
| Example 2-1 | R-1 | 95 | 43 | 18 | 2.8 | 120 | 10.6 |
| Example 2-2 | R-2 | 95 | 42 | 18 | 2.7 | 110 | 11 |
| Example 2-3 | R-3 | 90 | 41 | 17 | 2.9 | 120 | 10.3 |
| Example 2-4 | R-4 | 95 | 42 | 19 | 2.8 | 120 | 10.5 |

(continued)

| | Resist composition | PEB temperature (°C) | Optimum exposure dose (mJ/cm$^2$) | EL (%) | LWR (nm) | DOF (nm) | Collapse limit (nm) |
|---|---|---|---|---|---|---|---|
| Example 2-5 | R-5 | 95 | 41 | 18 | 2.9 | 110 | 11.1 |
| Example 2-6 | R-6 | 95 | 40 | 17 | 2.7 | 100 | 10.2 |
| Example 2-7 | R-7 | 90 | 41 | 19 | 2.8 | 120 | 10.4 |
| Example 2-8 | R-8 | 95 | 43 | 19 | 2.7 | 120 | 11.2 |
| Example 2-9 | R-9 | 95 | 42 | 18 | 2.9 | 100 | 11.1 |
| Example 2-10 | R-10 | 90 | 41 | 17 | 3 | 120 | 10.6 |
| Example 2-11 | R-11 | 95 | 42 | 17 | 2.8 | 110 | 10.8 |
| Example 2-12 | R-12 | 95 | 44 | 18 | 2.9 | 100 | 10.5 |
| Example 2-13 | R-13 | 90 | 38 | 19 | 2.8 | 120 | 10.6 |
| Example 2-14 | R-14 | 95 | 39 | 18 | 2.8 | 110 | 11 |
| Example 2-15 | R-15 | 95 | 39 | 17 | 2.9 | 120 | 10.8 |

[Table 4]

| | Resist composition | PEB temperature (°C) | Optimum exposure dose (mJ/cm$^2$) | EL (%) | LWR (nm) | DOF (nm) | Collapse limit (nm) |
|---|---|---|---|---|---|---|---|
| Comparative Example 2-1 | CR-1 | 95 | 50 | 17 | 3.8 | 80 | 15.3 |
| Comparative Example 2-2 | CR-2 | 95 | 49 | 18 | 3.7 | 90 | 14.9 |
| Comparative Example 2-3 | CR-3 | 90 | 43 | 15 | 4 | 70 | 14.5 |
| Comparative Example 2-4 | CR-4 | 90 | 44 | 17 | 3.8 | 80 | 13.9 |
| Comparative Example 2-5 | CR-5 | 90 | 42 | 18 | 3.8 | 80 | 15.1 |
| Comparative Example 2-6 | CR-6 | 90 | 43 | 17 | 3.9 | 90 | 14.6 |
| Comparative Example 2-7 | CR-7 | 95 | 48 | 17 | 3.8 | 90 | 14.8 |
| Comparative Example 2-8 | CR-8 | 90 | 43 | 18 | 4.1 | 70 | 13.8 |

(continued)

| | Resist composition | PEB temperature (°C) | Optimum exposure dose ($mJ/cm^2$) | EL (%) | LWR (nm) | DOF (nm) | Collapse limit (nm) |
|---|---|---|---|---|---|---|---|
| Comparative Example 2-9 | CR-9 | 100 | 43 | 19 | 3.8 | 80 | 14.3 |
| Comparative Example 2-10 | CR-10 | 95 | 43 | 18 | 3.9 | 90 | 15.2 |
| Comparative Example 2-11 | CR-11 | 95 | 42 | 16 | 3.7 | 80 | 14.8 |
| Comparative Example 2-12 | CR-12 | 100 | 43 | 17 | 4.2 | 70 | 14.3 |

From the results shown in Table 3 and Table 4, the chemically amplified resist composition containing the inventive photoacid generator has been found to have excellent EL, LWR, and DOF with good sensitivity. The composition has a small collapse limit value, and also has been confirmed to hardly cause pattern collapse even in fine pattern formation. Therefore, the inventive chemically amplified resist composition has been demonstrated to be suitable for a material for EUV lithography.

[5] EUV Lithography Evaluation (2)

Examples 3-1 to 3-15 and Comparative Examples 3-1 to 3-12

Each of the chemically amplified resist compositions (R-1 to R-15 and CR-1 to CR-12) shown in Table 1 and Table 2 was applied by spin-coating on a Si substrate on which a silicon-containing spin-on-hard mask SHB-A940, manufactured by Shin-Etsu Chemical Co., Ltd. (silicon content of 43 mass%), was formed with 20 nm in film thickness. Then, the substrate was prebaked at 105°C for 60 seconds using a hot plate to produce a resist film with 50 nm in film thickness. This resist film was exposed by using an EUV scanner NXE3400 (NA 0.33, $\sigma$ 0.9/0.6, quadrupole illumination, 46 nm in pitch on wafer size, hole pattern mask with +20% bias), manufactured by ASML Holding N.V. Then, PEB was performed at a temperature shown in Table 5 and Table 6 for 60 seconds by using a hot plate, and development was performed with a 2.38 mass% aqueous TMAH solution for 30 seconds to form a hole pattern with 23 nm in size.

Using a length-measurement SEM (CG6300), manufactured by Hitachi High-Technologies Corporation, an exposure dose when the hole size was formed with 23 nm was measured to specify this exposure dose as a sensitivity. Sizes of 50 holes were measured in this time, and a tripled value ($3\sigma$) of a standard variation ($\sigma$) calculated from the results was determined as size variation (CDU). Table 5 and Table 6 show the results.

[Table 5]

| | Resist composition | PEB Temperature (°C) | Optimum exposure dose ($mJ/cm^2$) | CDU (nm) |
|---|---|---|---|---|
| Example 3-1 | R-1 | 90 | 26 | 2.2 |
| Example 3-2 | R-2 | 95 | 24 | 2.4 |
| Example 3-3 | R-3 | 90 | 24 | 2.3 |
| Example 3-4 | R-4 | 90 | 25 | 2.5 |
| Example 3-5 | R-5 | 90 | 27 | 2.3 |
| Example 3-6 | R-6 | 90 | 25 | 2.6 |
| Example 3-7 | R-7 | 85 | 24 | 2.4 |
| Example 3-8 | R-8 | 90 | 26 | 2.3 |
| Example 3-9 | R-9 | 95 | 24 | 2.3 |
| Example 3-10 | R-10 | 90 | 25 | 2.4 |
| Example 3-11 | R-11 | 90 | 26 | 2.5 |

(continued)

| | Resist composition | PEB Temperature (°C) | Optimum exposure dose (mJ/cm$^2$) | CDU (nm) |
|---|---|---|---|---|
| Example 3-12 | R-12 | 85 | 27 | 2.3 |
| Example 3-13 | R-13 | 90 | 24 | 2.2 |
| Example 3-14 | R-14 | 90 | 25 | 2.4 |
| Example 3-15 | R-15 | 90 | 24 | 2.6 |

[Table 6]

| | Resist composition | PEB Temperature (°C) | Optimum exposure dose (mJ/cm$^2$) | CDU (nm) |
|---|---|---|---|---|
| Comparative Example 3-1 | CR-1 | 90 | 35 | 3.5 |
| Comparative Example 3-2 | CR-2 | 95 | 32 | 3.3 |
| Comparative Example 3-3 | CR-3 | 90 | 25 | 3.1 |
| Comparative Example 3-4 | CR-4 | 85 | 26 | 2.9 |
| Comparative Example 3-5 | CR-5 | 90 | 24 | 3 |
| Comparative Example 3-6 | CR-6 | 85 | 27 | 3.2 |
| Comparative Example 3-7 | CR-7 | 90 | 26 | 2.8 |
| Comparative Example 3-8 | CR-8 | 90 | 25 | 3.2 |
| Comparative Example 3-9 | CR-9 | 90 | 26 | 2.9 |
| Comparative Example 3-10 | CR-10 | 85 | 25 | 2.8 |
| Comparative Example 3-11 | CR-11 | 95 | 26 | 2.9 |
| Comparative Example 3-12 | CR-12 | 90 | 25 | 3.1 |

From the results shown in Table 5 and Table 6, the inventive chemically amplified resist composition has been confirmed to have good sensitivity and excellent CDU.

[6] EB Lithography Evaluation

Examples 4-1 to 4-17 and Comparative Examples 4-1 to 4-12

Each of the chemically amplified resist compositions (R-16 to R-32 and CR-13 to CR-24) was applied by spin-coating on a photomask blanks with 152-mm square having a chromium outermost surface by using ACT-M (manufactured by Tokyo Electron Ltd.). The composition was prebaked at 110°C for 600 seconds using a hot plate to produce a resist film with 80 nm in film thickness.

Furthermore, the composition was exposed by using an electron beam exposure apparatus (EBM-5000plus,

manufactured by NuFlare Technology, Inc., acceleration voltage of 50 kV), PEB was performed at 110°C for 600 seconds, and development was performed with a 2.38 mass% aqueous TMAH solution to obtain a positive-type pattern.

The obtained resist pattern was evaluated as follows. The mask blanks with the produced pattern was observed with the above SEM (scanning electron microscope) to measure LER of a 200-nm line-and-space (LS). An exposure dose at which the 200-nm 1:1 LS was resolved with 1:1 was specified as an optimum exposure dose ($\mu C/cm^2$), and a minimum size at the exposure dose at which the 200-nm LS was resolved with 1:1 was specified as a resolution (critical resolution). Development loading (Variation of development loading) was evaluated as follows. Each disposed on a substrate were: a 200-nm LS pattern formed with the exposure dose ($\mu C/cm^2$) at which the designed 200-nm 1:1 LS was resolved with a ratio of 1:1; and dummy patterns with a density of 15%, 25%, 33%, 45%, 50%, 55%, 66%, 75%, 85%, or 95%, the dummy patterns disposed on around the 200-nm LS pattern. The size of the space portion of the 200-nm LS pattern was measured and differences in the sizes of the sparse or dense patterns were compared. Whether the pattern had a rectangular shape or not was determined by visual observation.

A dissolution rate in an excessively exposed portion was calculated by: applying the resist solution on a 8-inch silicon wafer by spin-coating; baking the resist solution at 110°C for 60 seconds to form a resist film with 90 nm in film thickness; then exposing the resist film to KrF excimer laser light at an exposure dose ($mJ/cm^2$) at which a 200-nm 1:1 line-and-space (LS) was resolved with 1:1; baking the exposed resist film at 110°C for 60 seconds; and developing the baked resist film at 23°C with a 2.38 mass% TMAH aqueous solution using a resist development analyzer (RDA-800, manufactured by Litho Tech Japan Corporation). Table 7 and Table 8 show the results.

[Table 7]

| | Resist composition | Optimum exposure dose ($\mu C/cm^2$) | Critical resolution (nm) | LER (nm) | Variation of development loading ($\Delta$nm) | Pattern shape | Dissolution rate of excessively exposed portion (nm/s) |
|---|---|---|---|---|---|---|---|
| Example 4-1 | R-16 | 260 | 30 | 4.5 | 1.6 | Rectangular | 770 |
| Example 4-2 | R-17 | 270 | 30 | 4.4 | 1.7 | Rectangular | 750 |
| Example 4-3 | R-18 | 260 | 30 | 4 . 7 | 1.6 | Rectangular | 680 |
| Example 4-4 | R-19 | 260 | 30 | 4.4 | 1.5 | Rectangular | 700 |
| Example 4-5 | R-20 | 270 | 30 | 4.5 | 1.6 | Rectangular | 710 |
| Example 4-6 | R-21 | 260 | 35 | 4.7 | 1.7 | Rectangular | 690 |
| Example 4-7 | R-22 | 260 | 30 | 4.3 | 1.5 | Rectangular | 720 |
| Example 4-8 | R-23 | 270 | 30 | 4.4 | 1.6 | Rectangular | 730 |
| Example 4-9 | R-24 | 260 | 35 | 4.5 | 1.6 | Rectangular | 750 |
| Example 4-10 | R-25 | 265 | 30 | 4.6 | 1.7 | Rectangular | 760 |
| Example 4-11 | R-26 | 260 | 30 | 4.4 | 1.5 | Rectangular | 740 |
| Example 4-12 | R-27 | 265 | 30 | 4.5 | 1.7 | Rectangular | 720 |
| Example 4-13 | R-28 | 260 | 35 | 4.6 | 1.6 | Rectangular | 730 |

(continued)

| | Resist composition | Optimum exposure dose ($\mu C/cm^2$) | Critical resolution (nm) | LER (nm) | Variation of development loading ($\Delta nm$) | Pattern shape | Dissolution rate of excessively exposed portion (nm/s) |
|---|---|---|---|---|---|---|---|
| Example 4-14 | R-29 | 270 | 30 | 4.5 | 1.6 | Rectangular | 750 |
| Example 4-15 | R-30 | 260 | 35 | 4.7 | 1.7 | Rectangular | 760 |
| Example 4-16 | R-31 | 255 | 30 | 4.5 | 1.5 | Rectangular | 710 |
| Example 4-17 | R-32 | 260 | 30 | 4.4 | 1.6 | Rectangular | 740 |

[Table 8]

| | Resist composition | Optimum exposure dose ($\mu C/cm^2$) | Critical resolution (nm) | LER (nm) | Variation of development loading ($\Delta nm$) | Pattern shape | Dissolution rate of excessively exposed portion (nm/s) |
|---|---|---|---|---|---|---|---|
| Comparative Example 4-1 | CR-13 | 280 | 45 | 5.5 | 2.1 | Rounded head | 400 |
| Comparative Example 4-2 | CR-14 | 290 | 40 | 5.4 | 2.3 | Rounded head | 450 |
| Comparative Example 4-3 | CR-15 | 280 | 40 | 5.6 | 2.2 | Rounded head | 340 |
| Comparative Example 4-4 | CR-16 | 270 | 45 | 5.1 | 2.2 | Rounded head | 410 |
| Comparative Example 4-5 | CR-17 | 265 | 50 | 5.4 | 2.4 | Rounded head | 430 |
| Comparative Example 4-6 | CR-18 | 270 | 45 | 5.5 | 2.3 | Rounded head | 510 |
| Comparative Example 4-7 | CR-19 | 280 | 45 | 5.5 | 2.1 | Rounded head | 400 |
| Comparative Example 4-8 | CR-20 | 270 | 50 | 5.6 | 2.3 | Rounded head | 430 |
| Comparative Example 4-9 | CR-21 | 285 | 60 | 5.7 | 2.2 | Rounded head | 470 |
| Comparative Example 4-10 | CR-22 | 270 | 45 | 5 | 2 . 1 | Rounded head | 430 |
| Comparative Example 4-11 | CR-23 | 280 | 45 | 5.6 | 2.2 | Rounded head | 460 |
| Comparative Example 4-12 | CR-24 | 290 | 50 | 5.3 | 2.1 | Rounded head | 510 |

From the results shown in Table 7 and Table 8, all of the chemically amplified resist compositions containing the inventive photoacid generator exhibited good resolution, LER, and pattern rectangularity, and exhibited a reduced value of the development loading. Therefore, the inventive chemically amplified resist composition has been demonstrated to be suitable also for a material for EB lithography.

The present specification includes the following aspects.

[1]: A sulfonium salt represented by the following formula (1),

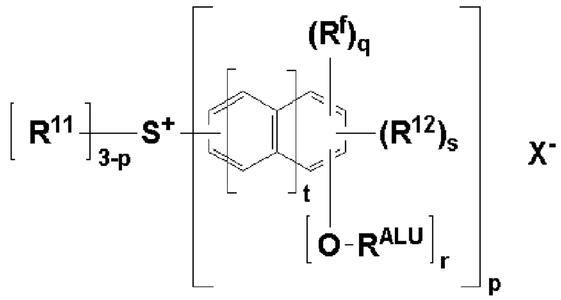

(1)

wherein "p" represents an integer of 1 to 3; $R^{11}$ represents a hydrocarbyl group having 1 to 20 carbon atoms and optionally having a heteroatom; two of the three substituents bonded to the sulfonium cation are optionally bonded each other to form a ring together with the sulfur atom to which the two substituents are bonded; $R^f$ represents a fluorine atom, a fluorine-atom-containing alkyl group, a fluorine-atom-containing alkoxy group, or a fluorine-atom-containing sulfide group, each group having 1 to 6 carbon atoms; "q" represents an integer of 1 to 4, and when $q \geq 2$, $R^f$ may be same as or different from each other; $R^{ALU}$ represents an acid-labile group formed together with the adjacent oxygen atom; "r" represents an integer of 1 to 4; $R^{12}$ represents a hydrocarbyl group having 1 to 20 carbon atoms and optionally having a heteroatom; "s" represents an integer of 0 to 4; "t" represents an integer of 0 to 2; $q + r + s \leq 5$ when t = 0, $q + r + s \leq 7$ when t = 1, and $q + r + s \leq 9$ when t = 2; $R^f$ and -O-$R^{ALU}$ are bonded to adjacent carbon atoms; and $X^-$ represents a non-nucleophilic counterion having no polymerizable group.

[2]: The sulfonium salt according to [1], wherein in the formula (1), $R^{ALU}$ is represented by the following formula (ALU-1) or (ALU-2),

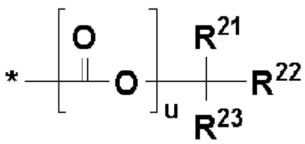

(ALU-1)

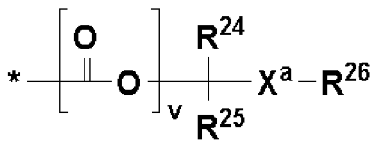

(ALU-2)

wherein in the formula (ALU-1), $R^{21}$, $R^{22}$, and $R^{23}$ each independently represent a hydrocarbyl group having 1 to 10 carbon atoms and optionally having a substituent; any two of $R^{21}$, $R^{22}$, and $R^{23}$ are optionally bonded each other to form a ring; "u" represents an integer of 0 or 1; in the formula (ALU-2), $R^{24}$ and $R^{25}$ each independently represent a hydrogen atom or a hydrocarbyl group having 1 to 10 carbon atoms and optionally having a substituent; $R^{26}$ represents a hydrocarbyl group having 1 to 20 carbon atoms, or $R^{26}$ is optionally bonded to $R^{24}$ or $R^{25}$ each other to form a heterocyclic group having 3 to 20 carbon atoms together with $X^a$ and the carbon atom to which $R^{24}$ and $R^{25}$ are bonded; $-CH_2-$ contained in the hydrocarbyl group and the heterocyclic group is optionally substituted with -O- or -S-; $X^a$ represents an oxygen atom or a sulfur atom; "v" represents an integer of 0 or 1; and "*" represents a bond to the adjacent oxygen atom.

[3]: The sulfonium salt according to [1] or [2], wherein in the formula (1), $X^-$ being the non-nucleophilic counterion having no polymerizable group represents a sulfonate anion, an imide anion, or a methide anion.

[4]: A photoacid generator, comprising the sulfonium salt according to any one of [1] to [3].

[5]: A resist composition, comprising the photoacid generator according to [4].

[6]: The resist composition according to [5], further comprising a base resin having a repeating unit represented by the following formula (a1) or (a2),

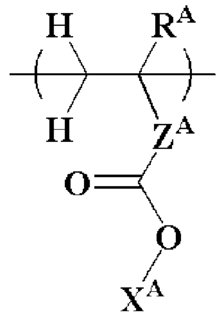
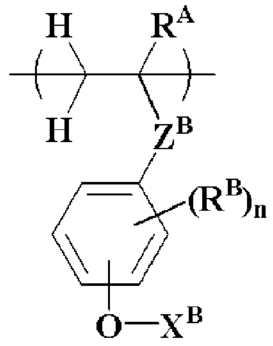

(a1) (a2)

wherein $R^A$ each independently represents a hydrogen atom, a fluorine atom, a methyl group, or a trifluoromethyl group; $Z^A$ represents a single bond, a phenylene group, a naphthylene group, or (main chain)-C(=O)-O-$Z^{A1}$-; $Z^{A1}$ represents a linear, branched, or cyclic alkanediyl group having 1 to 10 carbon atoms, a phenylene group or a naphthylene group, the alkanediyl group optionally having a hydroxy group, an ether bond, an ester bond, or a lactone ring; $Z^B$ represents a single bond or (main chain)-C(=O)-O-; $X^A$ and $X^B$ each independently represent an acid-labile group; $R^B$ represents a linear, branched, or cyclic monovalent hydrocarbon group having 1 to 20 carbon atoms and optionally having a heteroatom; and "n" represents an integer of 0 to 4.

[7]: The resist composition according to [6], wherein the base resin further has a repeating unit represented by the following formula (b1) or (b2),

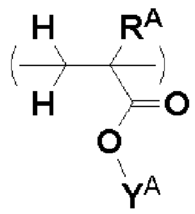
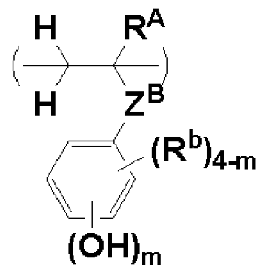

(b1) (b2)

wherein $R^A$ and $Z^B$ represent the same as above; $Y^A$ represents a hydrogen atom or a polar group having one or more structures selected from a hydroxy group other than a phenolic hydroxy group, a cyano group, a carbonyl group, a carboxy group, an ether bond, an ester bond, a sulfonate ester bond, a sulfonamide bond, a carbonate bond, a lactone ring, a sultone ring, a sulfur atom, and a carboxylic anhydride; $R^b$ represents a linear, branched, or cyclic monovalent hydrocarbon group having 1 to 20 carbon atoms and optionally having a heteroatom; and "m" represents an integer of 1 to 4.

[8]: The resist composition according to [6] or [7], wherein the base resin further has at least one repeating unit selected from repeating units represented by the following formulae (C1) to (C4),

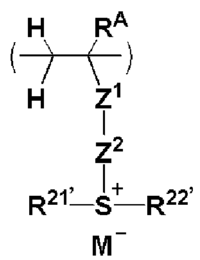
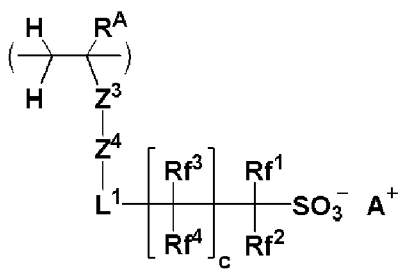
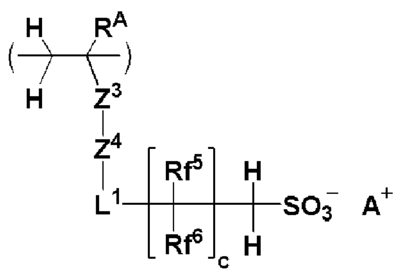

(C1) (C2) (C3)

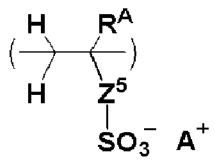

(C4)

wherein $R^A$ represents the same as above; $Z^1$ represents a single bond or a phenylene group; $Z^2$ represents a single bond, *-C(=O)-O-$Z^{21}$-, *-C (=O)-NH-$Z^{21}$-, or *-O-$Z^{21}$-; $Z^{21}$ represents an aliphatic hydrocarbylene group having 1 to 6 carbon atoms, a phenylene group, or a divalent group obtained by combining these groups, $Z^{21}$ optionally having a carbonyl group, an ester bond, an ether bond, or a hydroxy group; $Z^3$ represents a single bond, a phenylene group, a naphthylene group, or *-C(=O)-O-$Z^{31}$-; $Z^{31}$ represents an aliphatic hydrocarbylene group having 1 to 10 carbon atoms, a phenylene group or a naphthylene group, the aliphatic hydrocarbylene group optionally having a hydroxy group, an ether bond, an ester bond, or a lactone ring; $Z^4$ represents a single bond, a methylene group, or *-$Z^{41}$-C(=O)-O-; $Z^{41}$ represents a hydrocarbylene group having 1 to 20 carbon atoms and optionally having a heteroatom, an ether bond, or an ester bond; $Z^5$ represents a single bond, a methylene group, an ethylene group, a phenylene group, a fluorinated phenylene group, a phenylene group substituted with a trifluoromethyl group, *-C(=O)-O-$Z^{51}$-, *-C (=O)-N(H)-$Z^{51}$-, or *-O-$Z^{51}$-; $Z^{51}$ represents an aliphatic hydrocarbylene group having 1 to 6 carbon atoms, a phenylene group, a fluorinated phenylene group, or a phenylene group substituted with a trifluoromethyl group, and $Z^{51}$ optionally has a carbonyl group, an ester bond, an ether bond, or a hydroxy group; "*" represents an attachment point to a carbon atom in the main chain or to a group bonding to the main chain; $R^{21'}$ and $R^{22'}$ are optionally bonded each other to form a ring together with the sulfur atom to which $R^{21'}$ and $R^{22'}$ are bonded; $L^1$ represents a single bond, an ether bond, an ester bond, a carbonyl group, a sulfonate ester bond, a carbonate bond, or a carbamate bond; $Rf^1$ and $Rf^2$ each independently represent a fluorine atom or a fluorinated alkyl group having 1 to 6 carbon atoms; $Rf^3$ and $Rf^4$ each independently represent a hydrogen atom, a fluorine atom, or a fluorinated alkyl group having 1 to 6 carbon atoms; $Rf^5$ and $Rf^6$ each independently represent a hydrogen atom, a fluorine atom, or a fluorinated alkyl group having 1 to 6 carbon atoms; not all $Rf^5$ and $Rf^6$ simultaneously represent hydrogen atoms; $M^-$ represents a non-nucleophilic counterion; $A^+$ represents an onium cation; and "c" represents an integer of 0 to 3.

[9]: The resist composition according to any one of [6] to [8], further comprising an organic solvent.

[10]: The resist composition according to any one of [6] to [9], further comprising a compound represented by the following formula (5) or (6),

$$R^{q1}\text{-}SO_3^-\ Mq^+ \qquad (5)$$

$$R^{q2}\text{-}CO_2^-\ Mq^+ \qquad (6)$$

wherein $R^{q1}$ represents a hydrogen atom or a monovalent hydrocarbon group having 1 to 40 carbon atoms and optionally having a heteroatom, $R^{q1}$ excluding a group in which a hydrogen atom bonded to a carbon atom at an $\alpha$-position of the sulfo group is substituted with a fluorine atom or a fluoroalkyl group; $R^{q2}$ represents a hydrogen atom or a monovalent hydrocarbon group having 1 to 40 carbon atoms and optionally having a heteroatom; and $Mq^+$ represents an onium cation.

[11]: The resist composition according to any one of [6] to [10], further comprising a photoacid generator other than the photoacid generator according to [4].

[12]: The resist composition according to any one of [6] to [11], further comprising an amine compound.

[13]: The resist composition according to any one of [6] to [12], further comprising: a surfactant insoluble or hardly soluble in water and soluble in an alkaline developer; and/or a surfactant insoluble or hardly soluble in water and an alkaline developer.

[14]: A patterning process, comprising steps of: forming a resist film on a substrate using the resist composition according to any one of [6] to [13]; exposing the resist film to high energy ray; and developing the exposed resist film using a developer.

[15]: The patterning process according to [14], wherein the high energy ray is KrF excimer laser light, ArF excimer laser light, electron beam, or extreme ultraviolet ray having a wavelength of 3 to 15 nm.

It should be noted that the present invention is not limited to the above-described embodiments. The embodiments are just examples, and any examples that substantially have the same feature and demonstrate the same functions

and effects as those in the technical concept disclosed in claims of the present invention are included in the technical scope of the present invention.

## Claims

1. A sulfonium salt represented by the following formula (1),

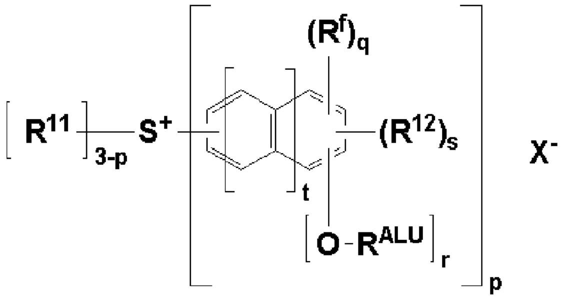

(1)

wherein "p" represents an integer of 1 to 3; $R^{11}$ represents a hydrocarbyl group having 1 to 20 carbon atoms and optionally having a heteroatom; two of the three substituents bonded to the sulfonium cation are optionally bonded each other to form a ring together with the sulfur atom to which the two substituents are bonded; $R^f$ represents a fluorine atom, a fluorine-atom-containing alkyl group, a fluorine-atom-containing alkoxy group, or a fluorine-atom-containing sulfide group, each group having 1 to 6 carbon atoms; "q" represents an integer of 1 to 4, and when $q \geq 2$, $R^f$ may be same as or different from each other; $R^{ALU}$ represents an acid-labile group formed together with the adjacent oxygen atom; "r" represents an integer of 1 to 4; $R^{12}$ represents a hydrocarbyl group having 1 to 20 carbon atoms and optionally having a heteroatom; "s" represents an integer of 0 to 4; "t" represents an integer of 0 to 2; $q + r + s \leq 5$ when t = 0, $q + r + s \leq 7$ when t = 1, and $q + r + s < 9$ when t = 2; $R^f$ and -O-$R^{ALU}$ are bonded to adjacent carbon atoms; and $X^-$ represents a non-nucleophilic counterion having no polymerizable group.

2. The sulfonium salt according to claim 1, wherein in the formula (1), $R^{ALU}$ is represented by the following formula (ALU-1) or (ALU-2),

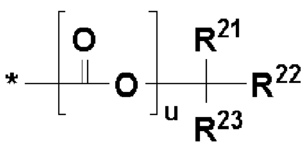 (ALU-1) 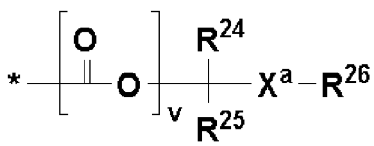 (ALU-2)

wherein in the formula (ALU-1), $R^{21}$, $R^{22}$, and $R^{23}$ each independently represent a hydrocarbyl group having 1 to 10 carbon atoms and optionally having a substituent; any two of $R^{21}$, $R^{22}$, and $R^{23}$ are optionally bonded each other to form a ring; "u" represents an integer of 0 or 1; in the formula (ALU-2), $R^{24}$ and $R^{25}$ each independently represent a hydrogen atom or a hydrocarbyl group having 1 to 10 carbon atoms and optionally having a substituent; $R^{26}$ represents a hydrocarbyl group having 1 to 20 carbon atoms, or $R^{26}$ is optionally bonded to $R^{24}$ or $R^{25}$ each other to form a heterocyclic group having 3 to 20 carbon atoms together with $X^a$ and the carbon atom to which $R^{24}$ and $R^{25}$ are bonded; $-CH_2-$ contained in the hydrocarbyl group and the heterocyclic group is optionally substituted with -O- or -S-; $X^a$ represents an oxygen atom or a sulfur atom; "v" represents an integer of 0 or 1; and "*" represents a bond to the adjacent oxygen atom.

3. The sulfonium salt according to claim 1 or claim 2, wherein in the formula (1), $X^-$ being the non-nucleophilic counterion having no polymerizable group represents a sulfonate anion, an imide anion, or a methide anion.

4. A photoacid generator, comprising the sulfonium salt according to any one of claims 1 to 3.

5. A resist composition, comprising the photoacid generator according to claim 4.

6. The resist composition according to claim 5, further comprising a base resin having a repeating unit represented by the following formula (a1) or (a2),

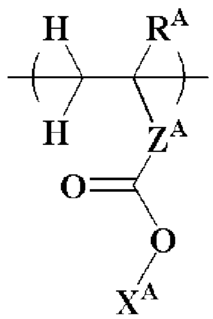

(a1)

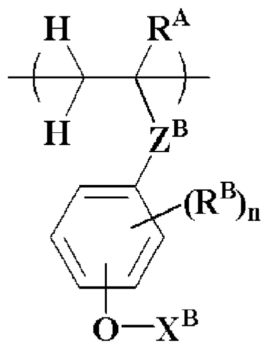

(a2)

wherein $R^A$ each independently represents a hydrogen atom, a fluorine atom, a methyl group, or a trifluoromethyl group; $Z^A$ represents a single bond, a phenylene group, a naphthylene group, or (main chain)-C(=O)-O-$Z^{A1}$-; $Z^{A1}$ represents a linear, branched, or cyclic alkanediyl group having 1 to 10 carbon atoms, a phenylene group or a naphthylene group, the alkanediyl group optionally having a hydroxy group, an ether bond, an ester bond, or a lactone ring; $Z^B$ represents a single bond or (main chain)-C(=O)-O-; $X^A$ and $X^B$ each independently represent an acid-labile group; $R^B$ represents a linear, branched, or cyclic monovalent hydrocarbon group having 1 to 20 carbon atoms and optionally having a heteroatom; and "n" represents an integer of 0 to 4.

**7.** The resist composition according to claim 6, wherein the base resin further has a repeating unit represented by the following formula (b1) or (b2),

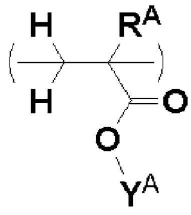

(b1)

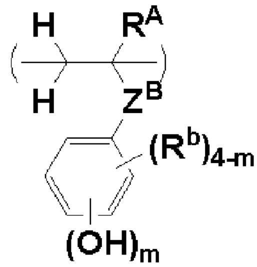

(b2)

wherein $R^A$ and $Z^B$ represent the same as above; $Y^A$ represents a hydrogen atom or a polar group having one or more structures selected from a hydroxy group other than a phenolic hydroxy group, a cyano group, a carbonyl group, a carboxy group, an ether bond, an ester bond, a sulfonate ester bond, a sulfonamide bond, a carbonate bond, a lactone ring, a sultone ring, a sulfur atom, and a carboxylic anhydride; $R^b$ represents a linear, branched, or cyclic monovalent hydrocarbon group having 1 to 20 carbon atoms and optionally having a heteroatom; and "m" represents an integer of 1 to 4.

**8.** The resist composition according to claim 6 or claim 7, wherein the base resin further has at least one repeating unit selected from repeating units represented by the following formulae (C1) to (C4),

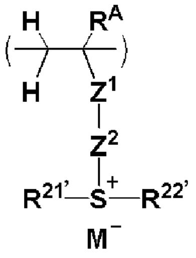

(C1)

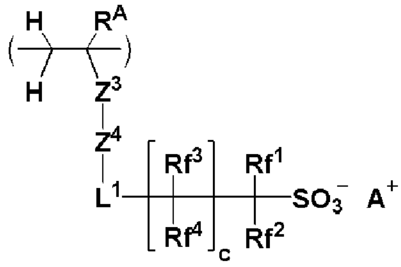

(C2)

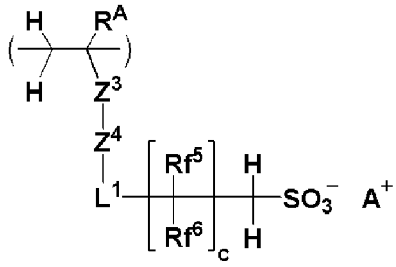

(C3)

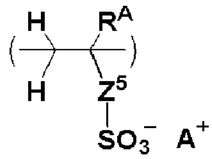

(C4)

wherein $R^A$ represents the same as above; $Z^1$ represents a single bond or a phenylene group; $Z^2$ represents a single bond, *-C(=O)-O-$Z^{21}$-, *-C (=O)-NH-$Z^{21}$-, or *-O-$Z^{21}$-; $Z^{21}$ represents an aliphatic hydrocarbylene group having 1 to 6 carbon atoms, a phenylene group, or a divalent group obtained by combining these groups, $Z^{21}$ optionally having a carbonyl group, an ester bond, an ether bond, or a hydroxy group; $Z^3$ represents a single bond, a phenylene group, a naphthylene group, or *-C(=O)-O-$Z^{31}$-; $Z^{31}$ represents an aliphatic hydrocarbylene group having 1 to 10 carbon atoms, a phenylene group or a naphthylene group, the aliphatic hydrocarbylene group optionally having a hydroxy group, an ether bond, an ester bond, or a lactone ring; $Z^4$ represents a single bond, a methylene group, or *-$Z^{41}$-C(=O)-O-; $Z^{41}$ represents a hydrocarbylene group having 1 to 20 carbon atoms and optionally having a heteroatom, an ether bond, or an ester bond; $Z^5$ represents a single bond, a methylene group, an ethylene group, a phenylene group, a fluorinated phenylene group, a phenylene group substituted with a trifluoromethyl group, *-C(=O)-O-$Z^{51}$-, *-C (=O)-N(H)-$Z^{51}$-, or *-O-$Z^{51}$-; $Z^{51}$ represents an aliphatic hydrocarbylene group having 1 to 6 carbon atoms, a phenylene group, a fluorinated phenylene group, or a phenylene group substituted with a trifluoromethyl group, and $Z^{51}$ optionally has a carbonyl group, an ester bond, an ether bond, or a hydroxy group; "*" represents an attachment point to a carbon atom in the main chain or to a group bonding to the main chain; $R^{21'}$ and $R^{22'}$ are optionally bonded each other to form a ring together with the sulfur atom to which $R^{21'}$ and $R^{22'}$ are bonded; $L^1$ represents a single bond, an ether bond, an ester bond, a carbonyl group, a sulfonate ester bond, a carbonate bond, or a carbamate bond; $Rf^1$ and $Rf^2$ each independently represent a fluorine atom or a fluorinated alkyl group having 1 to 6 carbon atoms; $Rf^3$ and $Rf^4$ each independently represent a hydrogen atom, a fluorine atom, or a fluorinated alkyl group having 1 to 6 carbon atoms; $Rf^5$ and $Rf^6$ each independently represent a hydrogen atom, a fluorine atom, or a fluorinated alkyl group having 1 to 6 carbon atoms; not all $Rf^5$ and $Rf^6$ simultaneously represent hydrogen atoms; $M^-$ represents a non-nucleophilic counterion; $A^+$ represents an onium cation; and "c" represents an integer of 0 to 3.

**9.** The resist composition according to any one of claims 6 to 8, further comprising an organic solvent.

**10.** The resist composition according to any one of claims 6 to 9, further comprising a compound represented by the following formula (5) or (6),

$$R^{q1}\text{-}SO_3^-\ Mq^+ \qquad (5)$$

$$R^{q2}\text{-}CO_2^-\ Mq^+ \qquad (6)$$

wherein $R^{q1}$ represents a hydrogen atom or a monovalent hydrocarbon group having 1 to 40 carbon atoms and optionally having a heteroatom, $R^{q1}$ excluding a group in which a hydrogen atom bonded to a carbon atom at an α-position of the sulfo group is substituted with a fluorine atom or a fluoroalkyl group; $R^{q2}$ represents a hydrogen atom or a monovalent hydrocarbon group having 1 to 40 carbon atoms and optionally having a heteroatom; and $Mq^+$ represents an onium cation.

**11.** The resist composition according to any one of claims 6 to 10, further comprising a photoacid generator other than the photoacid generator according to claim 5.

**12.** The resist composition according to any one of claims 6 to 11, further comprising an amine compound.

**13.** The resist composition according to any one of claims 6 to 12, further comprising:

a surfactant insoluble or hardly soluble in water and soluble in an alkaline developer; and/or
a surfactant insoluble or hardly soluble in water and an alkaline developer.

**14.** A patterning process, comprising steps of:

forming a resist film on a substrate using the resist composition according to any one of claims 6 to 13;
exposing the resist film to high energy ray; and
developing the exposed resist film using a developer.

**15.** The patterning process according to claim 14, wherein the high energy ray is KrF excimer laser light, ArF excimer laser light, electron beam, or extreme ultraviolet ray having a wavelength of 3 to 15 nm.

[FIG. 1]

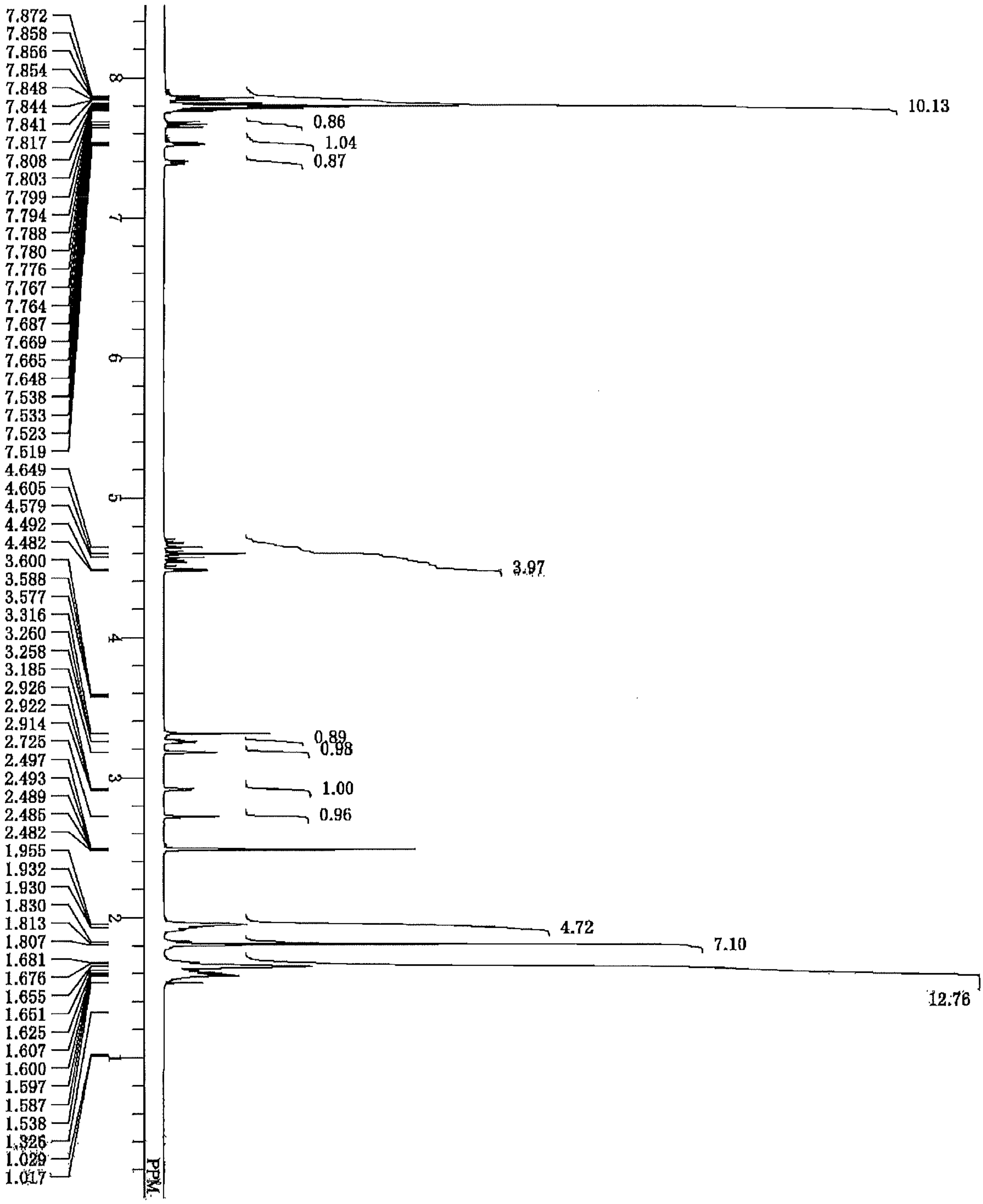

[FIG. 2]

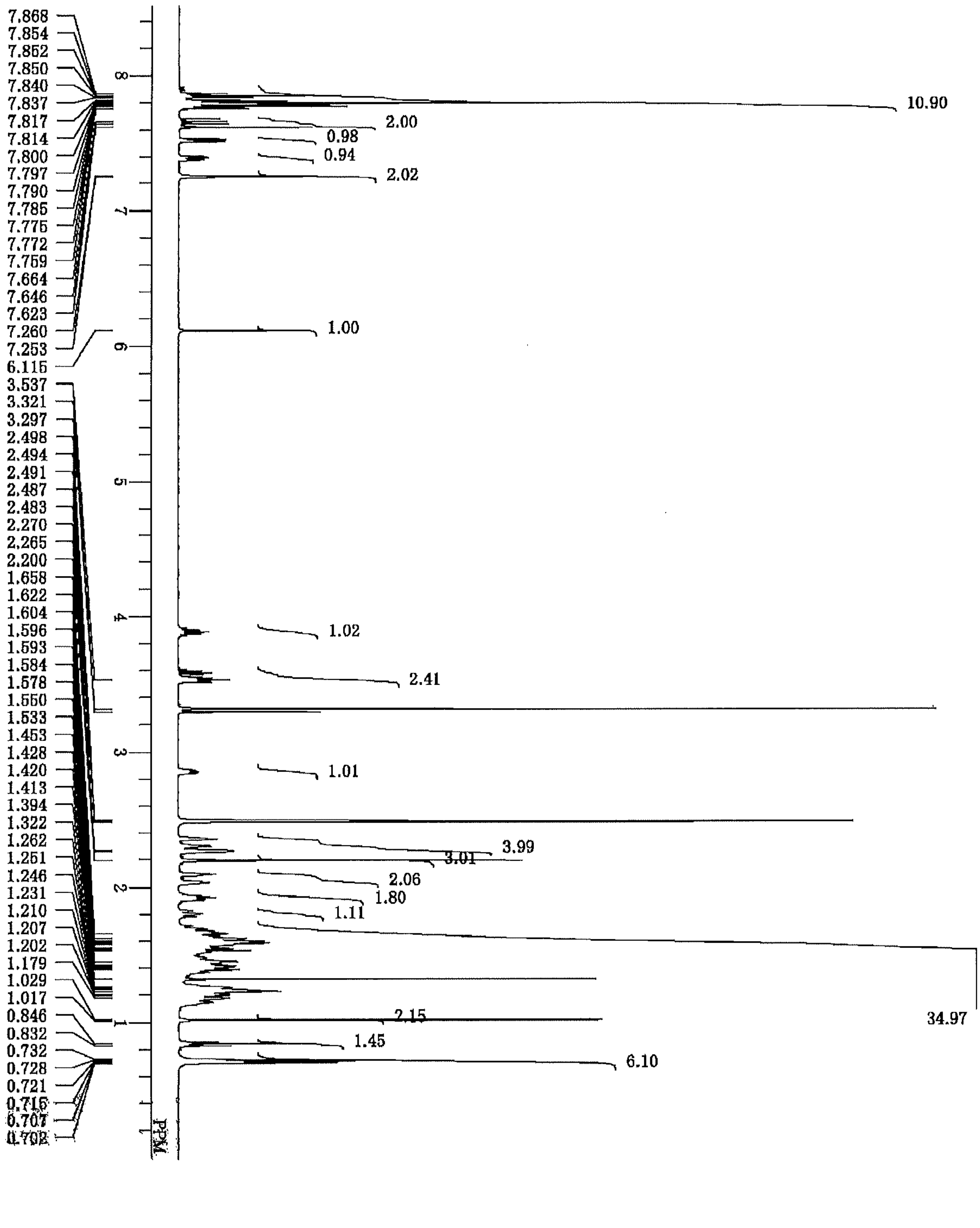
7.868
7.854
7.852
7.850
7.840
7.837
7.817
7.814
7.800
7.797
7.790
7.785
7.775
7.772
7.759
7.664
7.646
7.623
7.260
7.253
6.115
3.537
3.321
3.297
2.498
2.494
2.491
2.487
2.483
2.270
2.265
2.200
1.658
1.622
1.604
1.596
1.593
1.584
1.578
1.550
1.533
1.453
1.428
1.420
1.413
1.394
1.322
1.262
1.261
1.246
1.231
1.210
1.207
1.202
1.179
1.029
1.017
0.846
0.832
0.732
0.728
0.721
0.716
0.707
0.702
10.90
2.00
0.98
0.94
2.02
1.00
1.02
2.41
1.01
3.99
3.01
2.06
1.80
1.11
34.97
2.15
1.45
6.10
PPM

[FIG. 3]

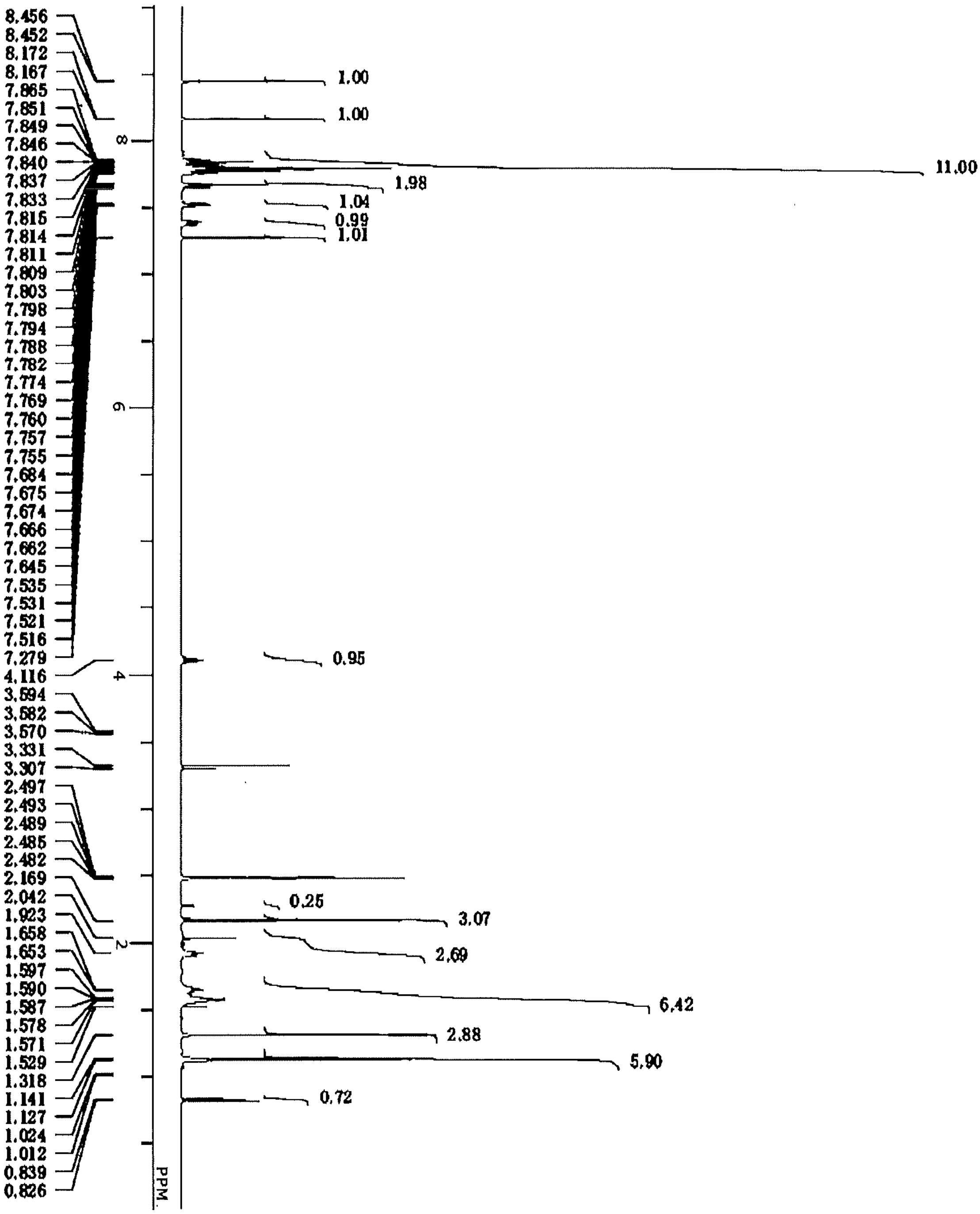
8.456
8.452
8.172
8.167
7.865
7.851
7.849
7.846
7.840
7.837
7.833
7.815
7.814
7.811
7.809
7.803
7.798
7.794
7.788
7.782
7.774
7.769
7.760
7.757
7.755
7.684
7.675
7.674
7.666
7.662
7.645
7.535
7.531
7.521
7.516
7.279
4.116
3.594
3.582
3.570
3.331
3.307
2.497
2.493
2.489
2.485
2.482
2.169
2.042
1.923
1.658
1.653
1.597
1.590
1.587
1.578
1.571
1.529
1.318
1.141
1.127
1.024
1.012
0.839
0.826
8
6
4
2
PPM.
1.00
1.00
11.00
1.98
1.04
0.99
1.01
0.95
0.25
3.07
2.69
6.42
2.88
5.90
0.72

[FIG. 4]

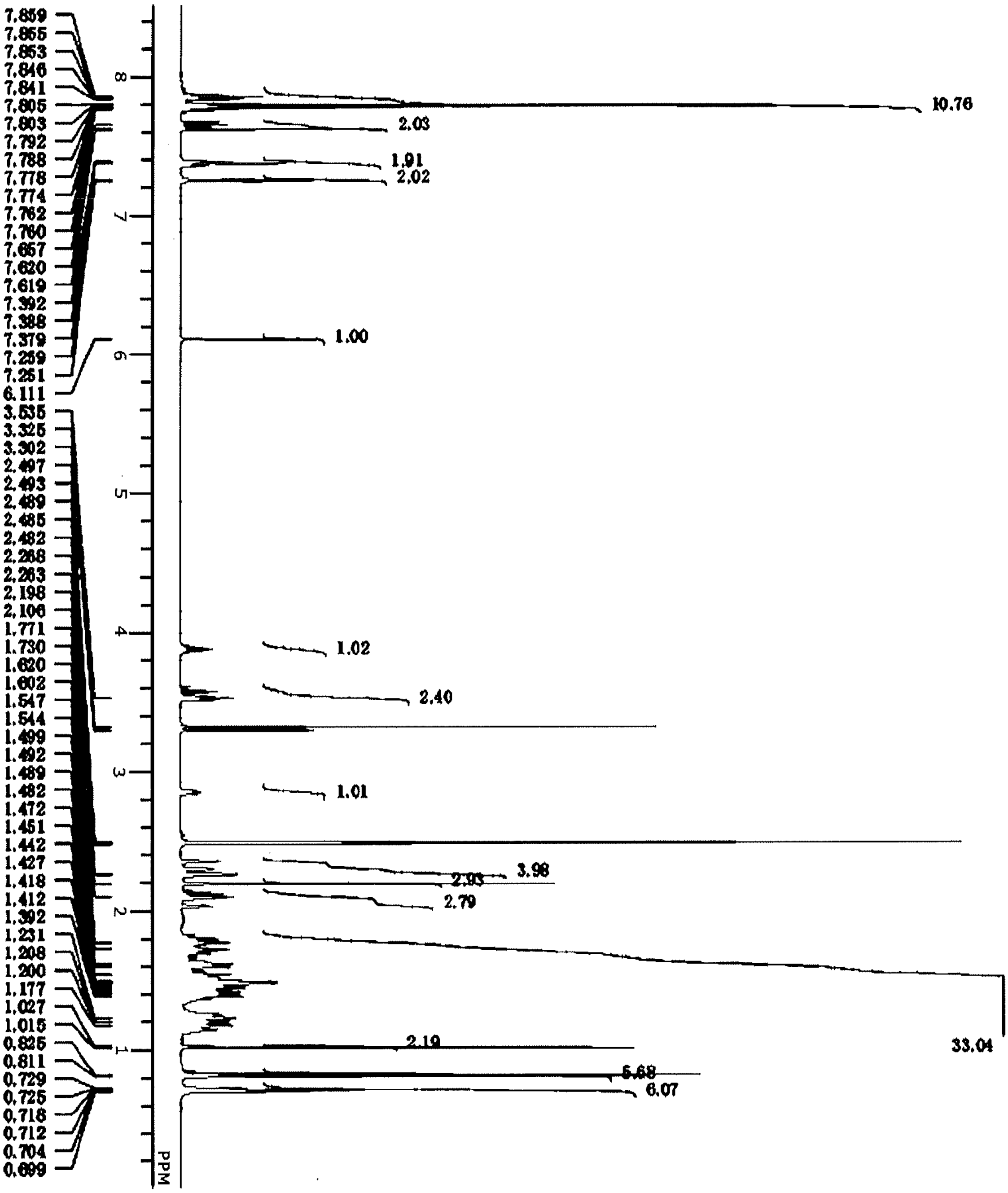
7.859
7.855
7.853
7.846
7.841
7.805
7.803
7.792
7.788
7.778
7.774
7.762
7.760
7.667
7.620
7.619
7.392
7.388
7.379
7.259
7.251
6.111
3.535
3.325
3.302
2.497
2.493
2.489
2.485
2.482
2.268
2.263
2.198
2.106
1.771
1.730
1.620
1.602
1.547
1.544
1.499
1.492
1.489
1.482
1.472
1.451
1.442
1.427
1.418
1.412
1.392
1.231
1.208
1.200
1.177
1.027
1.015
0.825
0.811
0.729
0.725
0.718
0.712
0.704
0.699
10.76
2.03
1.91
2.02
1.00
1.02
2.40
1.01
3.98
2.93
2.79
33.04
2.19
5.68
6.07
PPM

[FIG. 5]

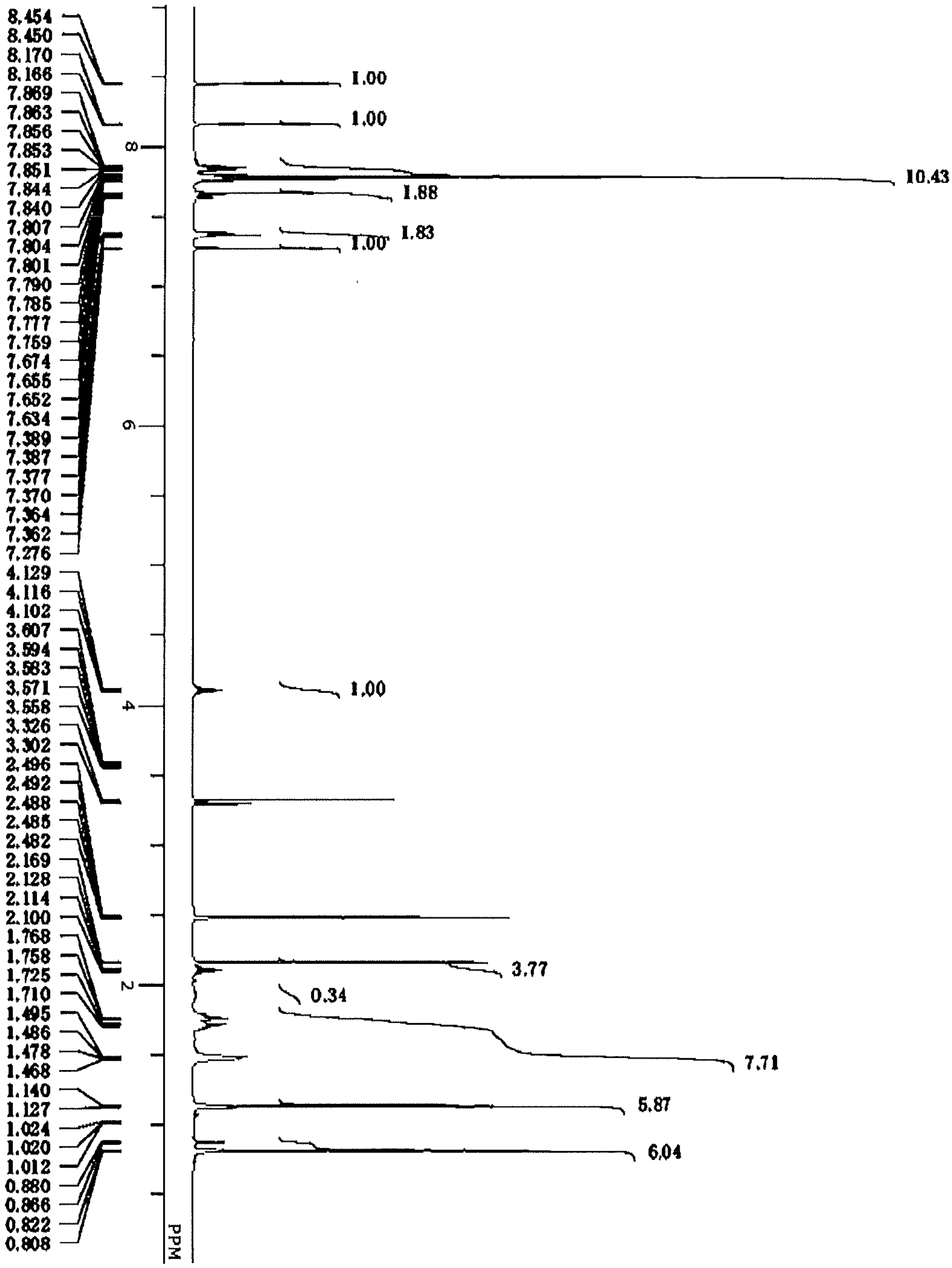
8.454
8.450
8.170
8.166
7.869
7.863
7.856
7.853
7.851
7.844
7.840
7.807
7.804
7.801
7.790
7.785
7.777
7.759
7.674
7.655
7.652
7.634
7.389
7.387
7.377
7.370
7.364
7.362
7.276
4.129
4.116
4.102
3.607
3.594
3.583
3.571
3.558
3.326
3.302
2.496
2.492
2.488
2.485
2.482
2.169
2.128
2.114
2.100
1.768
1.758
1.725
1.710
1.495
1.486
1.478
1.468
1.140
1.127
1.024
1.020
1.012
0.880
0.866
0.822
0.808
8
6
4
2
PPM
1.00
1.00
10.43
1.88
1.83
1.00
1.00
3.77
0.34
7.71
5.87
6.04

Europäisches Patentamt
European Patent Office
Office européen des brevets

# EUROPEAN SEARCH REPORT

Application Number
EP 23 17 1817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2021/055652 A1 (HATAKEYAMA JUN [JP] ET AL) 25 February 2021 (2021-02-25)<br>* para. [0002], [0124], [0143], [0144], [0170] *<br>* pages 100-102 *<br>* Table 1 *<br>----- | 1,3-11, 14,15<br>2,12,13 | INV.<br>G03F7/004<br>G03F7/039 |
| X<br>A | US 2021/188770 A1 (FUJIWARA TAKAYUKI [JP] ET AL) 24 June 2021 (2021-06-24)<br>* para. [0002], [0159]-[0161], [0388] *<br>* example 1-5 *<br>* Table 3 *<br>* pages 84 and 97 *<br>----- | 1,2,4-7, 9,11, 13-15<br>3,8,10, 12 | |
| X<br>A | WO 2021/199841 A1 (FUJIFILM CORP [JP]) 7 October 2021 (2021-10-07)<br>* page 155 *<br>* Tables 3 & 4 *<br>* para. [0001], [0508], [0520], [0530]-[0545] *<br>----- | 1-7,9, 11-15<br>8,10 | |
| | | | TECHNICAL FIELDS SEARCHED (IPC) |
| X | US 2019/064665 A1 (FUJIWARA TAKAYUKI [JP] ET AL) 28 February 2019 (2019-02-28)<br>* page 65 *<br>----- | 1-4 | G03F |
| A | US 2018/180992 A1 (KOTAKE MASAAKI [JP] ET AL) 28 June 2018 (2018-06-28)<br>* the whole document *<br>----- | 1-15 | |
| A | US 2020/159118 A1 (FUJII TATSUYA [JP] ET AL) 21 May 2020 (2020-05-21)<br>* the whole document *<br>----- | 1-15 | |

The present search report has been drawn up for all claims

1

| Place of search | Date of completion of the search | Examiner |
|---|---|---|
| Munich | 6 October 2023 | Borowczak, Baptiste |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 17 1817

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2023

| Patent document cited in search report | Publication date | Patent family member(s) | Publication date |
|---|---|---|---|
| US 2021055652 A1 | 25-02-2021 | KR 20210023759 A | 04-03-2021 |
| | | TW 202115493 A | 16-04-2021 |
| | | US 2021055652 A1 | 25-02-2021 |
| US 2021188770 A1 | 24-06-2021 | CN 113045465 A | 29-06-2021 |
| | | JP 2021091666 A | 17-06-2021 |
| | | KR 20210075020 A | 22-06-2021 |
| | | TW 202128602 A | 01-08-2021 |
| | | US 2021188770 A1 | 24-06-2021 |
| WO 2021199841 A1 | 07-10-2021 | CN 115244464 A | 25-10-2022 |
| | | EP 4129975 A1 | 08-02-2023 |
| | | IL 296766 A | 01-11-2022 |
| | | JP WO2021199841 A1 | 07-10-2021 |
| | | KR 20220137693 A | 12-10-2022 |
| | | TW 202136211 A | 01-10-2021 |
| | | US 2023043143 A1 | 09-02-2023 |
| | | WO 2021199841 A1 | 07-10-2021 |
| US 2019064665 A1 | 28-02-2019 | CN 109422672 A | 05-03-2019 |
| | | JP 6950357 B2 | 13-10-2021 |
| | | JP 2019038764 A | 14-03-2019 |
| | | KR 20190022403 A | 06-03-2019 |
| | | TW 201920092 A | 01-06-2019 |
| | | US 2019064665 A1 | 28-02-2019 |
| US 2018180992 A1 | 28-06-2018 | CN 108255019 A | 06-07-2018 |
| | | EP 3343291 A1 | 04-07-2018 |
| | | JP 7009978 B2 | 26-01-2022 |
| | | JP 2018109764 A | 12-07-2018 |
| | | KR 20180077082 A | 06-07-2018 |
| | | TW 201830139 A | 16-08-2018 |
| | | US 2018180992 A1 | 28-06-2018 |
| US 2020159118 A1 | 21-05-2020 | JP 7158251 B2 | 21-10-2022 |
| | | JP 2020085917 A | 04-06-2020 |
| | | KR 20200056929 A | 25-05-2020 |
| | | TW 202030551 A | 16-08-2020 |
| | | US 2020159118 A1 | 21-05-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008281974 A
- JP 2008281975 A
- JP 4554665 B
- JP 2007145797 A
- JP 5061484 B
- JP 2016147879 A
- JP 2015063472 A
- JP 5573098 B
- JP 6461919 B
- JP 5544078 B
- JP 5609569 B
- JP 6648726 B
- WO 2021200056 A
- JP 2021070692 A
- JP 2018180525 A
- JP 2021035935 A
- JP 2018092159 A
- JP 2006276759 A
- JP 2015117200 A
- JP 2016065016 A
- JP 2019202974 A
- JP 6645464 B
- JP 2015206932 A
- WO 2020158366 A
- JP 2015024989 A
- JP 2013080033 A
- JP 2013083821 A
- JP 2020111564 A
- JP 2008111103 A
- JP 2003066612 A
- JP 6848776 B
- JP 6583136 B
- JP 2020200311 A
- JP 6274755 B
- JP 3790649 B
- JP 2009109595 A
- JP 2012046501 A
- JP 2017026980 A
- JP 2010215608 A
- JP 2011016746 A
- US 5650483 B
- JP 2009269953 A


### Non-patent literature cited in the description


- *Journal of Photopolymer Science and Technology,* 2004, vol. 17 (4), 587-601